# EUROPEAN PATENT APPLICATION

(11) **EP 2 108 689 A2**
(43) Date of publication of application: **14.10.2009**
(21) Application number: 09250820.9
(22) Date of filing: 24.03.2009
(51) Int. Cl.: C09K 11/06, H05B 33/14, C07C 15/60, H01L 51/50, H01L 51/00

(54) **Novel organic electroluminescent compounds and organic electroluminescent device using the same**

(30) Priority: 02.04.2008 KR 20080030645
(71) Applicant: Gracel Display Inc., Seoul 133-833 (KR)
(72) Inventor: Lee, Mi Ae, Seoul, 121-190 (KR); Kim, Jin Ho, Seoul (KR); Cho, Young Jun, Seoul 136-060 (KR); Kwon, Hyuck Joo, Seoul 130-100 (KR); Kim, Bong Ok, Seoul 135-090 (KR); Kim, Sung Min, Seoul-city 157-886 (KR); Yoon, Seung Soo, Seoul 135-884 (KR); Kim, Chi Sik, Seoul 156-825 (KR)
(74) Representative: Kent, Venetia Katherine

(57) **Abstract**

The present invention relates to novel organic electroluminescent compounds, and organic electroluminescent devices employing the same as electroluminescent material. Specifically, the organic electroluminescent compounds according to the invention are **characterized in that** they are represented by Chemical Formula (1).

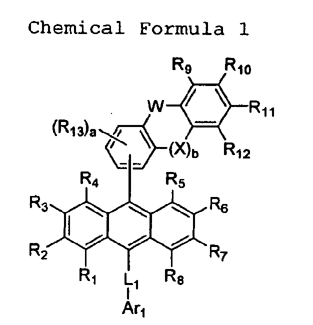

Since the organic electroluminescent compounds according to the invention have good luminous efficiency and excellent life property of material, OLED's having very good operation life can be manufactured therefrom.

## Description

### FIELD OF THE INVENTION

The present invention relates to novel organic electroluminescent compounds, and organic electroluminescent devices employing the same as electroluminescent material. Specifically, the organic electroluminescent compounds according to the present invention are represented by Chemical Formula (1): 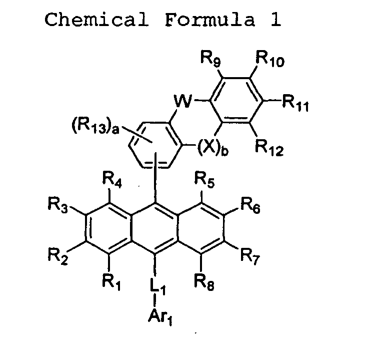
wherein, R₁ through R₈ independently represent hydrogen, halogen, (C1-C60)alkyl, (C6-C60)aryl, (C3-C60)heteroaryl, 5-or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or each of R₁ through R₈ may be linked to an adjacent substituent via (C3-C60)alkylene or (C3-C60)alkenylene to form an alicyclic ring, or a monocyclic or polycyclic aromatic ring; provided that R₁ through R₈ cannot be hydrogen all at the same time;
R₉ through R₁₂ independently represent hydrogen, halogen, (C1-C60)alkyl, (C6-C60)aryl, (C3-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or each of R₉ through R₁₂ may be linked to an adjacent substituent via (C3-C60)alkylene or (C3-C60)alkenylene to form an alicyclic ring, or a monocyclic or polycyclic aromatic ring;
R₁₃ represents halogen, (C1-C60)alkyl, (C6-C60)aryl, (C3-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl;
W and X independently represent a chemical bond, - C(R₁₄)(R₁₅)-, -N(R₁₆)-, -S-, -O-, -Si(R₁₇)(R₁₈)-, -P(R₁₉)-, - C(=O)-, -B(R₂₀)-, -In(R₂₁)-, -Se-, -Ge(R₂₂)(R₂₃)-, -Sn(R₂₄)(R₂₅)-or -Ga(R₂₆)-;
   wherein R₁₄ through R₂₆ independently represent hydrogen, halogen, (C1-C60)alkyl, (C6-C60)aryl, (C3-C60)heteroaryl, 5-or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or R₁₄ and R₁₅, R₁₇ and R₁₈, R₂₂ and R₂₃, or R₂₄ and R₂₅ may be linked via (C3-C60)alkylene or (C3-C60)alkenylene with or without a fused ring to form an alicyclic ring, or a monocyclic or polycyclic aromatic ring;
L₁ represents a chemical bond, (C6-C60)arylene or (C3-C60)heteroarylene, and the arylene or heteroarylene of L₁ may be further substituted by one or more substituent(s) selected from (C1-C60)alkyl, halogen, cyano, (C1-C60)alkoxy, (C3-C60)cycloalkyl, (C6-C60)aryl, (C3-C60)heteroaryl, adamantyl, (C7-C60)bicycloalkyl, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro, hydroxyl, tri(C1-C30)alkylsilyl, di(C1-C30)alkyl(C6-C30)arylsilyl and tri(C6-C30)arylsilyl;
Ar₁ represents (C1-C60)alkyl, (C6-C60)aryl, (C3-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or a substituent selected from the following structures: 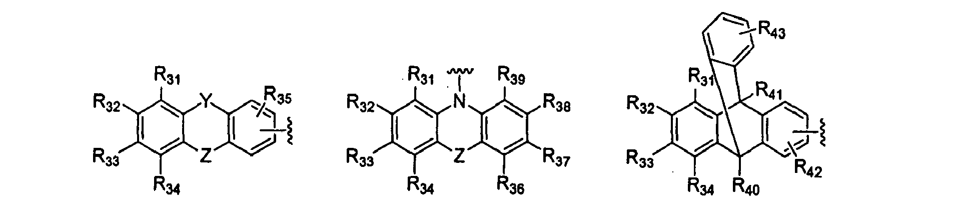 wherein, R₃₁ through R₄₃ independently represent hydrogen, halogen, (C1-C60)alkyl, (C6-C60)aryl, (C3-C60)heteroaryl, 5-or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or each of R₃₁ through R₄₃ may be linked to an adjacent substituent via (C3-C60)alkylene or (C3-C60)alkenylene with or without a fused ring to form an alicyclic ring, or a monocyclic or polycyclic aromatic ring;
Y and Z independently represent a chemical bond, - (CR₅₁R₅₂)_{c}-, -N(R₅₃)-, -S-, -O-, -Si(R₅₄)(R₅₅)-, -P(R₅₆)-, -C(=O)-, -B(R₅₇)-, -In(R₅₈)-, -Se-, -Ge(R₅₉)(R₆₀)-, -Sn(R₆₁₎(R₆₂)-, - Ga(R₆₃)- or - (R₆₄)C=C(R₆₅)-;
   wherein R₅₁ through R₆₅ independently represent hydrogen, halogen, (C1-C60)alkyl, (C6-C60)aryl, (C3-C60)heteroaryl, 5-or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or R₅₁ and R₅₂, R₅₄ and R₅₅, R₅₉ and R₆₀, R₆₁ and R₆₂ or R₆₄ and R₆₅ may be linked via (C3-C60)alkylene r (C3-C60)alkenylene with or without a fused ring to form an alicyclic ring, or a monocyclic or polycyclic aromatic ring;
   the alkyl, aryl, heteroaryl, heterocycloalkyl, cycloalkyl, trialkylsilyl, dialkylarylsilyl, triarylsilyl, adamantyl, bicycloalkyl, alkenyl, alkynyl, alkylamino or arylamino of Ar₁, R₁ through R₂₆, R₃₁ through R₄₃ and R₅₁ through R₆₃ may be further substituted by halogen, (C1-C60)alkyl, (C6-C60)aryl, (C3-C60)heteroaryl, 5- or 6-membered heterocyloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl;
a is an integer from 0 to 3; and
b and c independently represent an integer from 1 to 4.

### BACKGROUND OF THE INVENTION

Three electroluminescent materials (for red, green and blue) are employed to realize a full-colored OLED display. The important issue is to develop red, green and blue electroluminescent materials with high efficiency and long life, in order to enhance the overall feature of the organic electroluminescent (EL) devices. From the aspect of function, the EL materials are classified into host materials and dopant materials. It is generally known that a device structure having the most excellent EL properties can be fabricated with an EL layer prepared by doping a dopant to a host. Recently, development of organic EL devices with high efficiency and long life comes to the fore as an urgent subject, and particularly urgent is development of a material with far better EL properties as compared to conventional EL materials as considering EL properties required for a medium to large sized OLED panel. From this point of view, development of host material is one of the most important issues to be settled. The desired properties for the host material (serving as a solvent and energy conveyer in solid state) are high purity and appropriate molecular weight to enable vapor-deposition in vacuo. In addition, glass transition temperature and thermal decomposition temperature should be high enough to ensure thermal stability. Further, the host material should have high electrochemical stability for providing long life. It is to be easy to form an amorphous thin film, with high adhesiveness to other adjacent materials but without interlayer migration.

In the meanwhile, for conventional blue materials, a number of materials have been developed and commercialized since the development of diphenylvinyl-biphenyl (DPVBi) (Compound a) by Idemitsu-Kosan. In addition to the blue material system from Idemitsu-Kosan, dinaphthylanthracene (DNA) (Compound b), tetra(t-butyl)perylene (Compound c) system or the like have been known. However, extensive research and development should be performed with respect to these materials. The distryl compound system of Idemitsu-Kosan, which is known to have highest efficiency up to now, has 6 lm/W of power efficiency and beneficial device lifetime of more than 30,000 hr. However, when it is applied to a full-colored display, the lifetime is merely several thousand hours, owing to decrease of color purity over operation time. In case of blue electroluminescence, it becomes advantageous from the aspect of the luminous efficiency, if the electroluminescent wavelength is shifted a little toward longer wavelength. However, it is not easy to apply the material to a display of high quality because of unsatisfactory color purity in blue. Furthermore, the research and development of such materials are urgent because of the problems in color purity, efficiency and thermal stability.

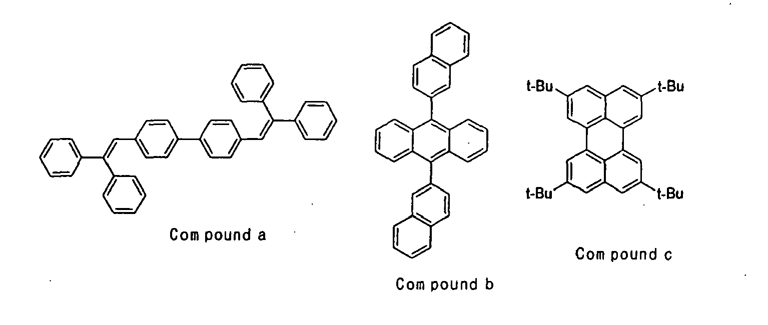

In order to develop a host material with high efficiency and long life, compounds based on different backbones have been disclosed, such as dispiro-fluorene-anthracene (TBSA), ter-spirofluorene (TSF) and bitriphenylene (BTP). These compounds, however, did not result in color purity and luminous efficiency at a sufficient level.

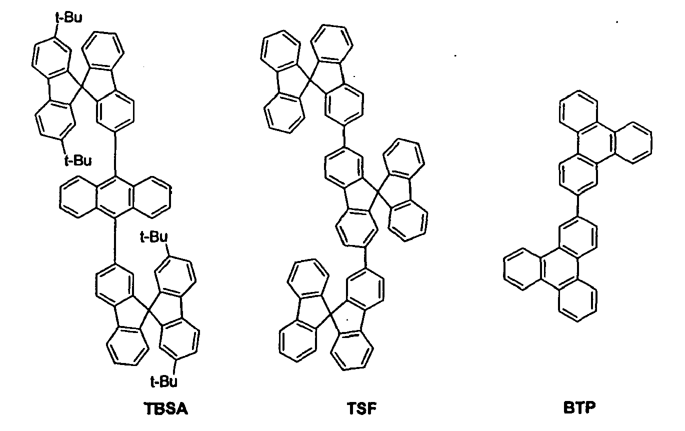

The compound TBSA as reported by Gyeongsang National University and Samsung SDI (Kwon, S. K. et al., Advanced Materials, 2001, 13, 1690; Japanese Patent Laid-Open JP 2002121547), showed luminous efficiency of 3 cd/A at 7.7 V, and relatively good color coordinate of (0.15, 0.11), but it was applied as a material for single layer, being inappropriate for practical use. The compound TSF reported by Taiwan National University (Wu, C. -C. et al., Advanced Materials, 2004, 16, 61; US Patent Publication US 2005040392) showed relatively good external quantum efficiency of 5.3%, but it was still inappropriate for practical use. The compound BTP reported by Chingwha National University of Taiwan (Cheng, C. -H. et al., Advanced Materials, 2002, 14, 1409; US Patent Publication US 2004076852) showed luminous efficiency of 2.76 cd/A and relatively good color coordinate of (0.16, 0.14), but this was still insufficient for practical use.

As described above, conventional materials are constituted of a single layer, not forming a host-dopant thin layer, and is difficult to be used practically from the aspect of color purity and efficiency. There are not enough data reliable, with respect to its long life.

In the meanwhile, according to a patent application of Mitsui Chemicals (Japan) (US Patent Publication US 7,166,240), the compounds shown below have the absorption spectra at 390 to 430 nm, with luminous efficiency of 4.6 cd/A. However, on the basis of these data, the compounds with above absorption wavelength range, electroluminescence of greenish blue color is anticipated, and the Patent Publication indicates the color as bluish green color.

Particularly, embodiment of pure blue color is impossible with the symmetrical structure of the Patent Publication, and the material, which cannot provide pure blue luminescence, is inadequate to be practically applied to a full-colored display.

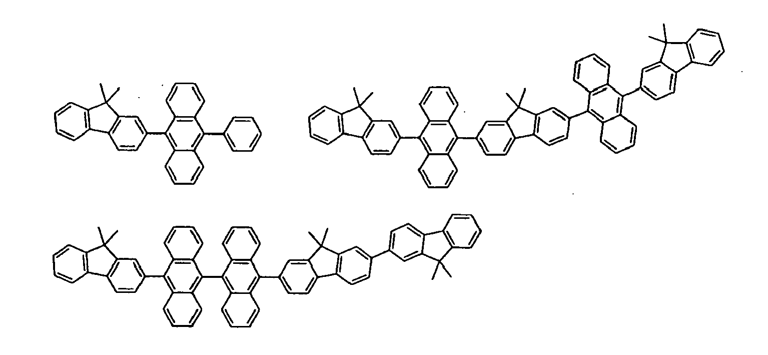

### SUMMARY OF THE INVENTION

With intensive efforts to overcome the problems of conventional techniques as described above, the present inventors have invented novel electroluminescent compounds to realize an organic electroluminescent device having excellent luminous efficiency and surprisingly improved lifetime.

The object of the present invention is to organic electroluminescent compounds having the backbone to give more excellent electroluminescent properties, longer device life and appropriate color coordinate, as compared to those of conventional host materials, with overcoming disadvantages of them.

Another object of the invention is to provide organic electroluminescent devices of high efficiency and long life, which employ said organic electroluminescent compounds as electroluminescent material.

Thus, the present invention relates to organic electroluminescent compounds represented by Chemical Formula (1), and organic electroluminescent devices comprising the same. Since the organic electroluminescent compounds according to the invention have good luminous efficiency and excellent color purity and life property of material, OLED's having very good operation life can be manufactured therefrom: 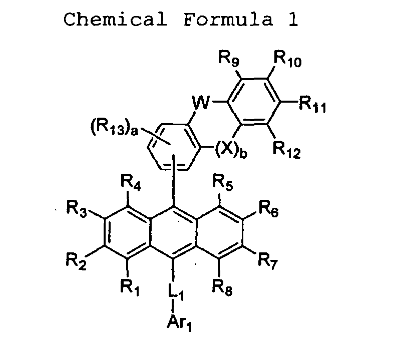
wherein, R₁ through R₈ independently represent hydrogen, halogen, (C1-C60)alkyl, (C6-C60)aryl, (C3-C60)heteroaryl, 5-or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or each of R₁ through R₈ may be linked to an adjacent substituent via (C3-C60)alkylene or (C3-C60)alkenylene to form an alicyclic ring, or a monocyclic or polycyclic aromatic ring; provided that R₁ through R₈ cannot be hydrogen all at the same time;
R₉ through R₁₂ independently represent hydrogen, halogen, (C1-C60)alkyl, (C6-C60)aryl, (C3-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or each of R₉ through R₁₂ may be linked to an adjacent substituent via (C3-C60)alkylene or (C3-C60)alkenylene to form an alicyclic ring, or a monocyclic or polycyclic aromatic ring;
R₁₃ represents halogen, (C1-C60)alkyl, (C6-C60)aryl, (C3-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl;
W and X independently represent a chemical bond, - C(R₁₄) (R₁₅)-, -N(R₁₆)-, -S-, -O-, -Si(R₁₇)(R₁₈)-, -P(R₁₉)-, - C(=O)-, -B(R₂₀)-, -In(R₂₁)-, -Se-, -Ge(R₂₂)(R₂₃)-, -Sn(R₂₄)(R₂₅)-or -Ga(R₂₆)-;
   wherein R₁₄ through R₂₆ independently represent hydrogen, halogen, (C1-C60)alkyl, (C6-C60)aryl, (C3-C60)heteroaryl, 5-or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or R₁₄ and R₁₅, R₁₇ and R₁₈, R₂₂ and R₂₃, or R₂₄ and R₂₅ may be linked via (C3-C60)alkylene or (C3-C60)alkenylene with or without a fused ring to form an alicyclic ring, or a monocyclic or polycyclic aromatic ring;
L₁ represents a chemical bond, (C6-C60)arylene or (C3-C60)heteroarylene, and the arylene or heteroarylene of L₁ may be further substituted by one or more substituent(s) selected from (C1-C60)alkyl, halogen, cyano, (C1-C60)alkoxy, (C3-C60)cycloalkyl, (C6-C60)aryl, (C3-C60)heteroaryl, adamantyl, (C7-C60)bicycloalkyl, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro, hydroxyl, tri(C1-C30)alkylsilyl, di(C1-C30)alkyl(C6-C30)arylsilyl and tri(C6-C30)arylsilyl;
Ar₁ represents (C1-C60)alkyl, (C6-C60)aryl, (C3-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or a substituent selected from the following structures: 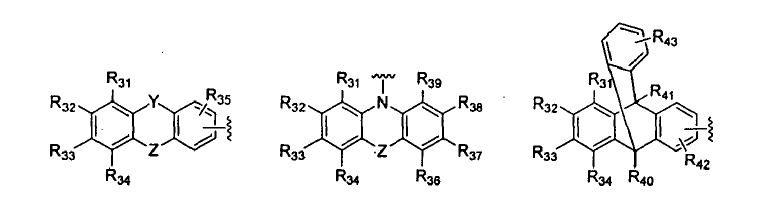 wherein, R₃₁ through R₄₃ independently represent hydrogen, halogen, (C1-C60)alkyl, (C6-C60)aryl, (C3-C60)heteroaryl, 5-or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)axyloxy, (C6-C60)arylthio, (C1-C60)alkoxycarbonyl, carboxyl, nitro or hydroxyl, or each of R₃₁ through R₄₃ may be linked to an adjacent substituent via (C3-C60)alkylene or (C3-C60)alkenylene with or without a fused ring to form an alicyclic ring, or a monocyclic or polycyclic aromatic ring;
Y and Z independently represent a chemical bond, - (CR₅₁R₅₂)_{c}-, -N(R₅₃)-, -S-, -O-, -Si(R₅₄)(R₅₅)-, -P(R₅₆)-, -C(=O) -B(R₅₇)-, -In(R₅₈)-, -Se-, -Ge(R₅₉)(R₆₀)-, -Sn(R₆₁)(R₆₂)-, - Ga(R₆₃)- or - (R₆₄)C=C(R₆₅)-;
   wherein R₅₁ through R₆₅ independently represent hydrogen, halogen, (C1-C60)alkyl, (C6-C60)aryl, (C3-C60)heteroaryl, 5-or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or R₅₁ and R₅₂, R₅₄ and R₅₅, R₅₉ and R₆₀, R₆₁ and R₆₂ or R₆₄ and R₆₅ may be linked via (C3-C60)alkylene r (C3-C60)alkenylene with or without a fused ring to form an alicyclic ring, or a monocyclic or polycyclic aromatic ring;
   the alkyl, aryl, heteroaryl, heterocycloalkyl, cycloalkyl, trialkylsilyl, dialkylarylsilyl, triarylsilyl, adamantyl, bicycloalkyl, alkenyl, alkynyl, alkylamino or arylamino of Ar₁, R₁ through R₂₆, R₃₁ through R₄₃ and R₅₁ through R₆₃ may be further substituted by halogen, (C1-C60)alkyl, (C6-C60)aryl, (C3-C60)heteroaryl, 5- or 6-membered heterocyloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl;
a is an integer from 0 to 3; and
b and c independently represent an integer from 1 to 4.

### BRIEF DESCRIPTION OF THE DRAWINGS

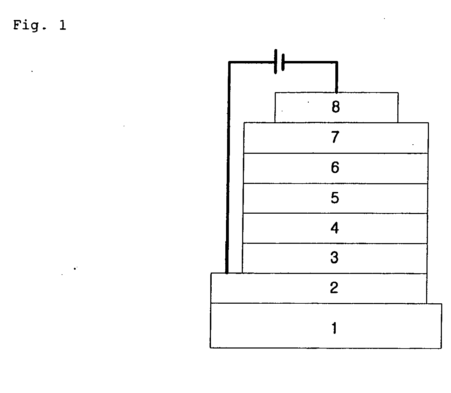

Fig. 1 is a cross-sectional view of an OLED.

### DETAILED DESCRIPTION OF THE INVENTION

Referring now to the Drawings, Fig. 1 illustrates a cross-sectional view of an OLED of the present invention comprising a Glass 1, Transparent electrode 2, Hole injecting layer 3, Hole transport layer 4, Electroluminescent layer 5, Electron transport layer 6, Electron injecting layer 7 and Al cathode 8.

The "alkyl", "alkoxy" or other substituents containing "alkyl" moiety described in the present invention include both linear and branched species.

The term "aryl" described herein means an organic radical derived from aromatic hydrocarbon via elimination of one hydrogen atom. Each ring suitably comprises a monocyclic or fused ring system containing from 4 to 7, preferably from 5 to 6 cyclic atoms. Specific examples include phenyl, naphthyl, biphenyl, anthryl, tetrahydronaphthyl, indanyl, fluorenyl, phenanthryl, triphenylenyl, pyrenyl, perylenyl, chrysenyl, naphthacenyl and fluoranthenyl, as well as aryl groups wherein aryls are linked by a chemical bond each other, but they are not restricted thereto.

The term "heteroaryl" described herein means an aryl group containing from 1 to 4 heteroatom(s) selected from N, O and S for the aromatic cyclic backbone atoms, and carbon atom(s) for remaining aromatic cyclic backbone atoms. The heteroaryl may be a 5- or 6-membered monocyclic heteroaryl or a polycyclic heteroaryl which is fused with one or more benzene ring(s), and may be partially saturated. The heteroaryl groups may include divalent aryl groups of which the heteroatoms are oxidized or quarternized to form N-oxides, quaternary salts, or the like. Specific examples include monocyclic heteroaryl groups such as furyl, thiophenyl, pyrrolyl, pyranyl, imidazolyl, pyrazolyl, thiazolyl, thiadiazolyl, isothiazolyl, isoxazolyl, oxazolyl, oxadiazolyl, triazinyl, tetrazinyl, triazolyl, tetrazolyl, furazanyl, pyridyl, pyrazinyl, pyrimidinyl, pyridazinyl; polycyclic heteroaryl groups such as benzofuranyl, benzothiophenyl, isobenzofuranyl, benzimidazolyl, benzothiazolyl, benzisothiazolyl, benzisoxazolyl, benzoxazolyl, isoindolyl, indolyl, indazolyl, benzothiadiazolyl, quinolyl, isoquinolyl, cinnolinyl, quinazolinyl, quinolizinyl, quinoxalinyl, carbazolyl, phenanthridinyl and benzodioxolyl; and corresponding N-oxides (for example, pyridyl N-oxide, quinolyl N-oxide) and quaternary salts thereof; but they are not restricted thereto.

The organic electroluminescent compound according to the invention can be selected from the compounds represented by one of Chemical Formulas (2) to (5): 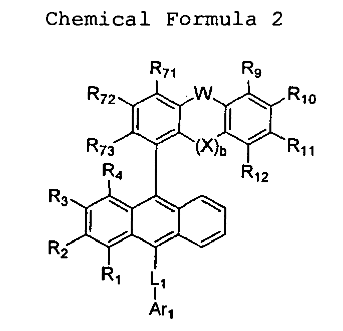 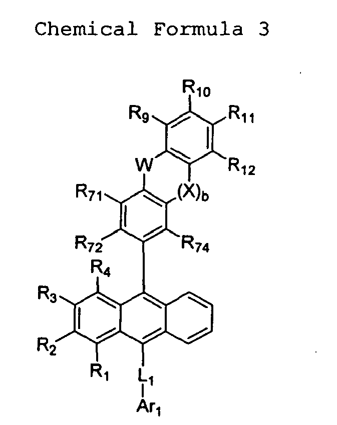 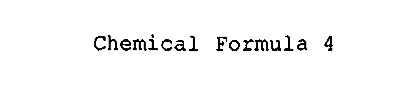 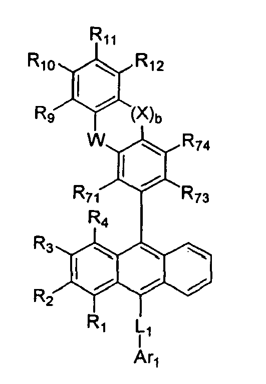 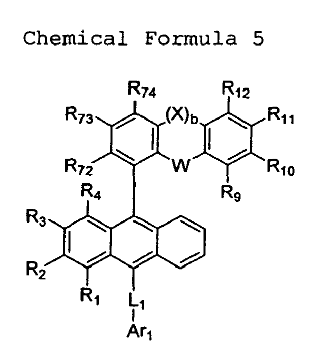
wherein, L₁, Ar₁, R₉ through R₁₂, X, W and b are defined as in Chemical Formula (1);
R₁ through R₄ independently represent hydrogen, halogen, (C1-C60)alkyl, (C6-C60)aryl, (C3-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, provided that R₁ through R₄ cannot be hydrogen all at the same time;
R₇₁ through R₇₄ independently represent hydrogen, halogen, (C1-C60)alkyl, (C6-C60)aryl, (C3-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl; and
   the alkyl, aryl, heteroaryl, heterocycloalkyl, cycloalkyl, trialkylsilyl, dialkylarylsilyl, triarylsilyl, adamantyl, bicycloalkyl, alkenyl, alkynyl, alkylamino or arylamino of R₁ through R₄, and R₇₁ through R₇₄ may be further substituted by halogen, (C1-C60)alkyl, (C6-C60)aryl, (C3-C60)heteroaryl, 5-or 6-membered heterocyloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl.

In the chemical formulas, R₁ through R₄ independently represent hydrogen, chloro, fluoro, methyl, ethyl, n-propyl, i-propyl, n-butyl, i-butyl, t-butyl, n-pentyl, i-pentyl, n-hexyl, n-heptyl, n-octyl, 2-ethylhexyl, n-nonyl, decyl, dodecyl, hexadecyl, benzyl, trifluoromethyl, perfluorethyl, trifluorethyl, perfluoropropyl, perfluorobutyl, methoxy, ethoxy, n-propoxy, i-propoxy, n-butoxy, i-butoxy, t-butoxy, n-pentoxy, i-pentoxy, n-hexyloxy, n-heptoxy, cyclopropyl, cyclobutyl, cyclopentyl, cyclohexyl, cycloheptyl, cyclooctyl, cyclononyl, cyclodecyl, morpholino, thiomorpholino, phenyl, naphthyl, biphenyl, fluorenyl, phenanthryl, anthryl, fluoranthenyl, triphenylenyl, pyrenyl, chrysenyl, naphthacenyl, perylenyl, spirobifluorenyl, pyridyl, pyrrolyl, furanyl, thiophenyl, imidazolyl, benzimidazolyl, pyrazinyl, pyrimidinyl, pyridazinyl, quinolyl, triazinyl, benzofuranyl, benzothiophenyl, pyrazolyl, indolyl, carbazolyl, thiazolyl, oxazolyl, benzothiazolyl, benzoxazolyl, phenanthrolinyl, trimethylsilyl, triethylsilyl, tripropylsilyl, tri(t-butyl)silyl, t-butyldimethylsilyl, dimethylphenylsilyl, triphenylsilyl, adamantyl, bicyclo[2.2.1]heptyl, bicyclo[2.2.2]octyl, bicyclo[3.2.1]octyl, bicyclo[5.2.0]nonyl, bicyclo[4.2.2]decyl, bicyclo[2.2.2]octyl, 4-pentylbicyclo [2.2.2]octyl, ethenyl, phenylethenyl, ethynyl, phenylethynyl, cyano, dimethylamino, diphenylamino, monomethylamino, monophenylamino, phenyloxy, phenylthio, methoxycarbonyl, ethoxycarbonyl, t-butoxycarbonyl, carboxyl, nitro or hydroxyl; provided that R₁ through R₄ cannot be hydrogen all at the same time.

In the formulas, 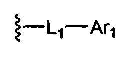 is selected from the following structures, but they are not restricted thereto: 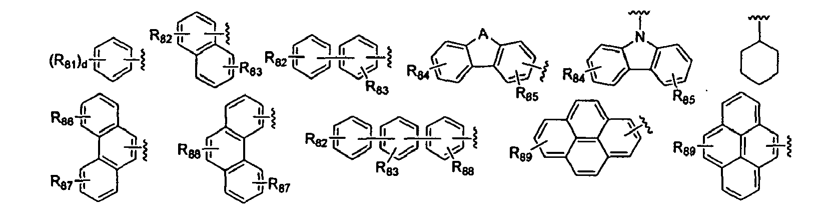 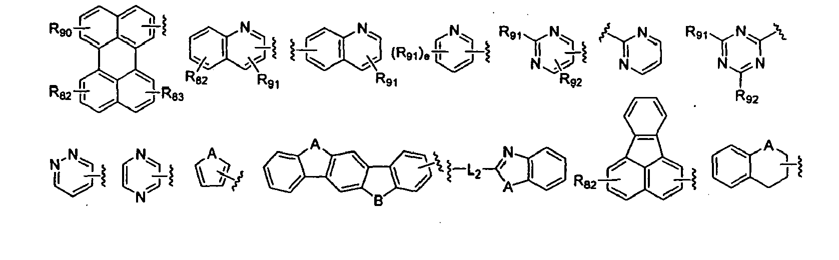 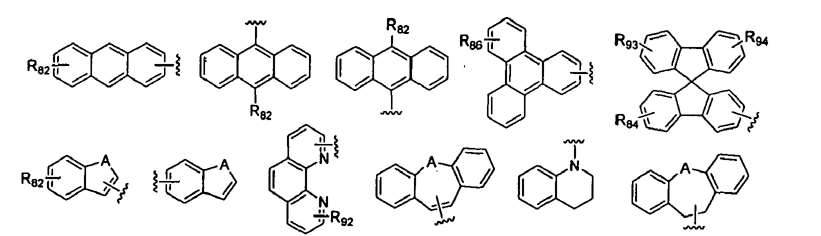 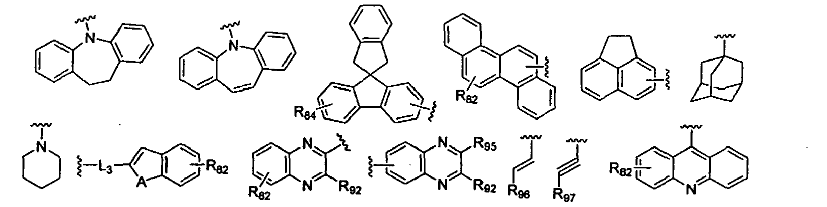 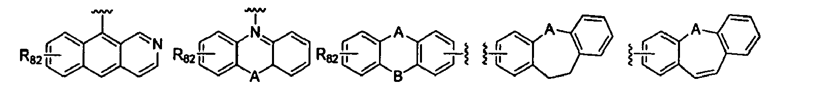
wherein, R₈₁ through R₉₇ independently represent hydrogen, halogen, (C1-C60)alkyl, (C6-C60) aryl, (C3-C60)heteroaryl, 5-or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl; the alkyl, aryl, heteroaryl, heterocycloalkyl, cycloalkyl, trialkylsilyl, dialkylarylsilyl, triarylsilyl, adamantyl, bicycloalkyl, alkenyl, alkynyl, alkylamino or arylamino of R₈₁ through R₉₇ may be further substituted by halogen, (C1-C60)alkyl, (C6-C60)aryl, (C3-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(Cl-C60)alkyl, (C6-C60)aryloxy, (C6-C60)arylthio, (C1-C60)alkoxycarbonyl, carboxyl, nitro or hydroxyl;
L₂ and L₃ independently represent a chemical bond, (C6-C60)arylene or (C3-C60)heteroarylene; the arylene or heteroarylene of L₂ and L₃ may be further substituted by one or more substituent(s) selected from (C1-C60)alkyl, halogen, cyano, (C1-C60)alkoxy, (C3-C60)cycloalkyl, (C6-C60)aryl, (C3-C60)heteroaryl, adamantyl, (C7-C60)bicycloalkyl, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro, hydroxyl, tri(C1-C30)alkylsilyl, di(C1-C30)alkyl(C6-C30)arylsilyl and tri(C6-C30)arylsilyl;
A and B independently represent a chemical bond, - C(R₁₀₁)(R₁₀₂)-, -N(R₁₀₃)-, -S-, -O-, -Si(R₁₀₄)(R₁₀₅)-, -P(R₁₀₆)-, - C(=O)-, -B(R₁₀₇)-, -In(R₁₀₈)-, -Se-, -Ge(R₁₀₉)(R₁₁₀)-, - Sn(R₁₁₁)(R₁₁₂)- or -Ga(R₁₁₃)-;
   wherein R₁₀₁ through R₁₁₃ independently represent hydrogen, halogen, (C1-C60)alkyl, (C6-C60)aryl, (C3-C60)heteroaryl, 5-or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or R₁₀₁ and R₁₀₂, R₁₀₄ and R₁₀₅, R₁₀₉ and R₁₁₀, or R₁₁₁ and R₁₁₂ may be linked via (C3-C60)alkylene or (C3-C60)alkenylene with or without a fused ring to form an alicyclic ring, or a monocyclic or polycyclic aromatic ring;
d is an integer from 1 to 5; and
e is an integer from 1 to 4.

Specifically, group 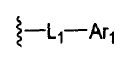 is selected from the following structures, but not restricted thereto:

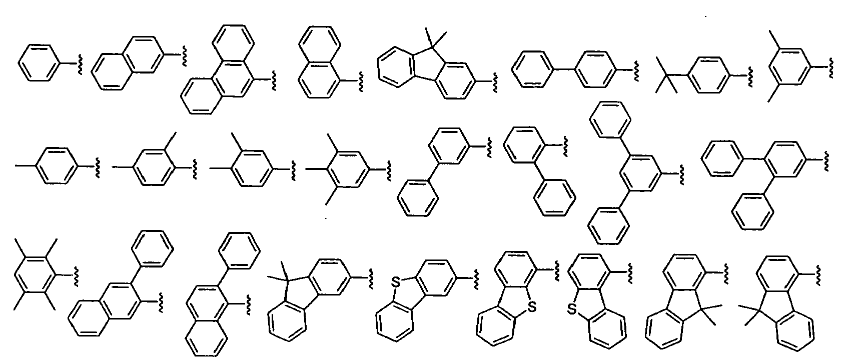

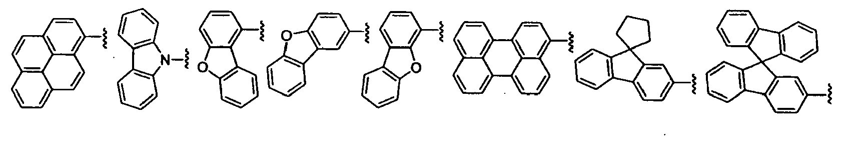

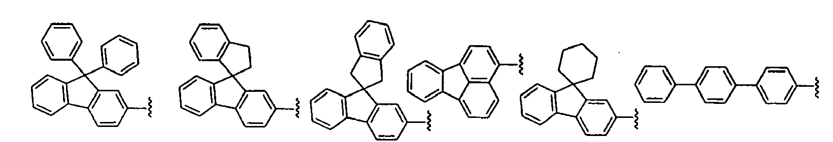

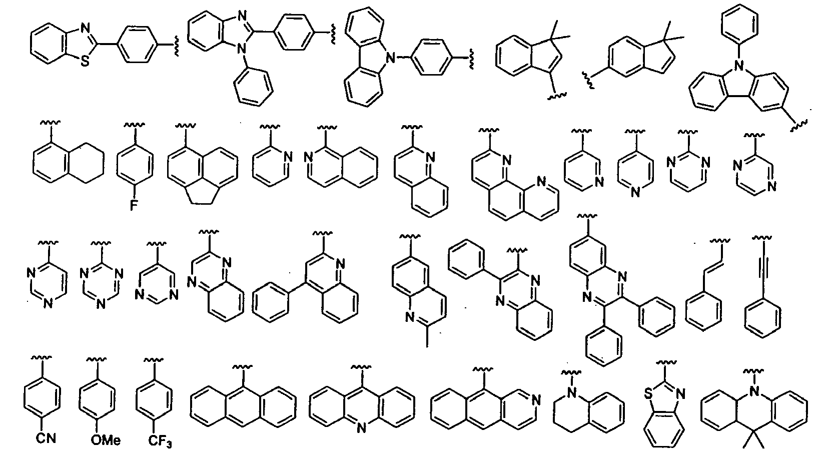

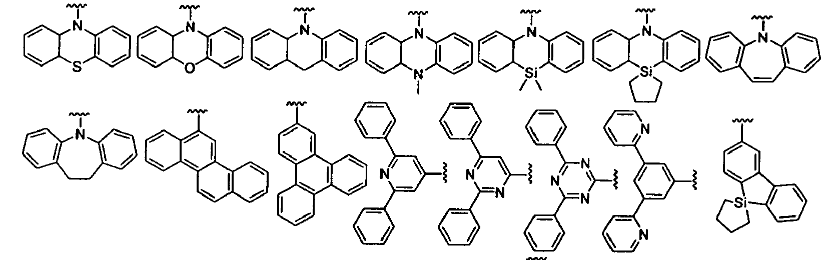

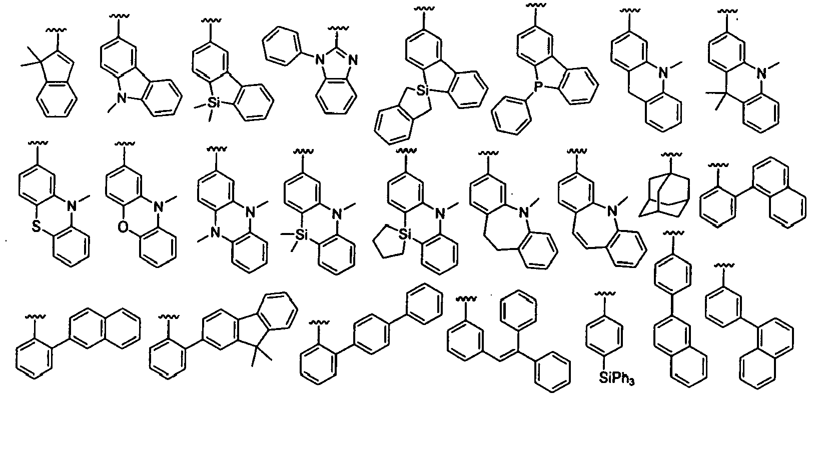

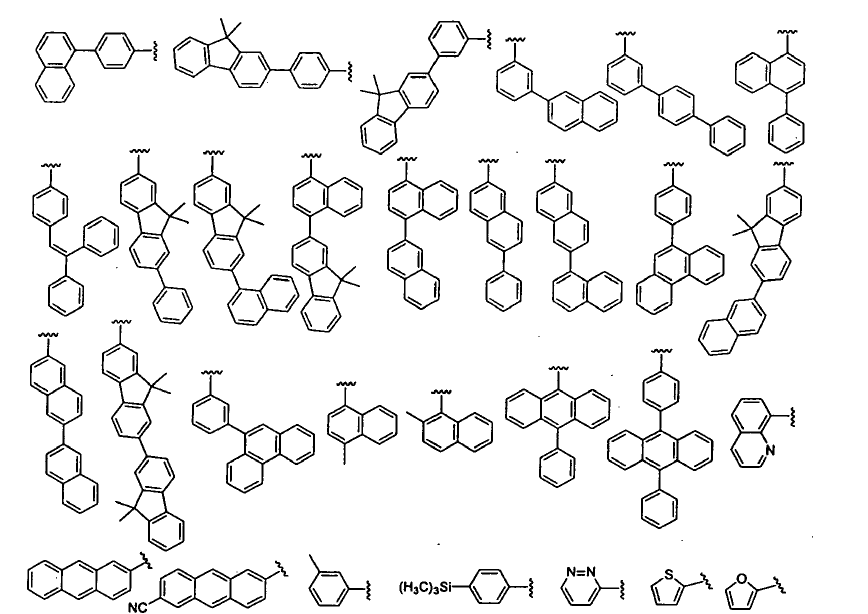

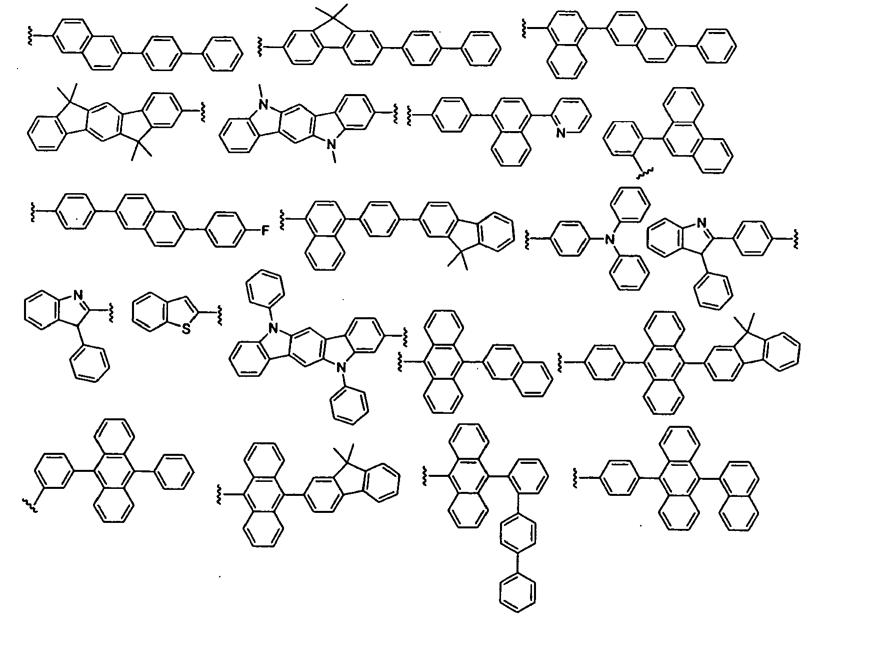

More specifically, the organic electroluminescent compounds according to the present invention can be specifically exemplified by the following compounds, but they are not restricted thereto:

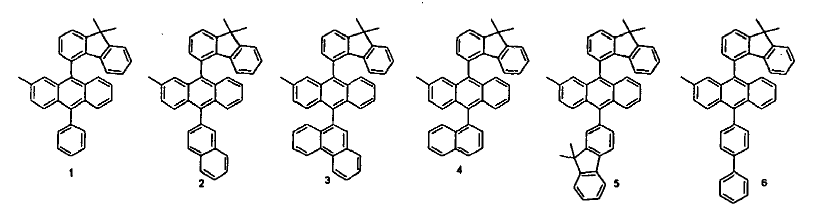

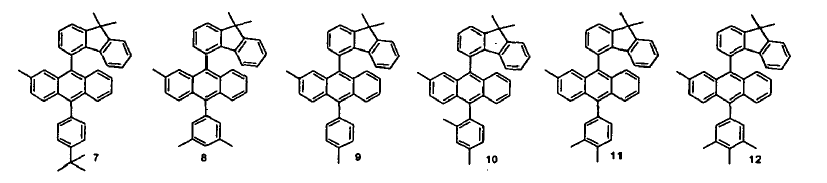

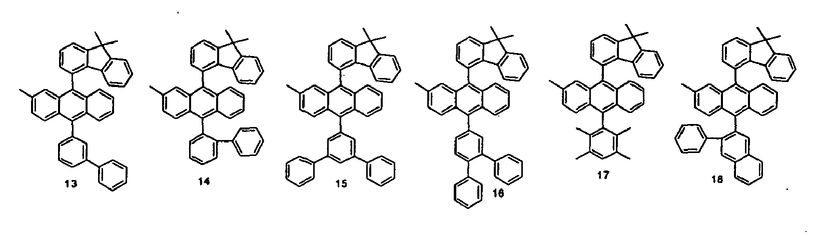

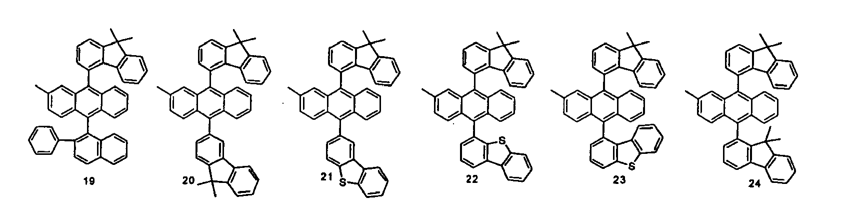

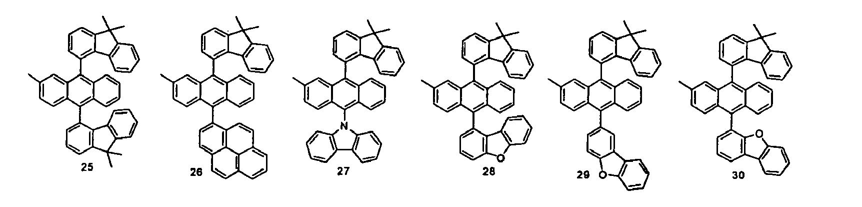

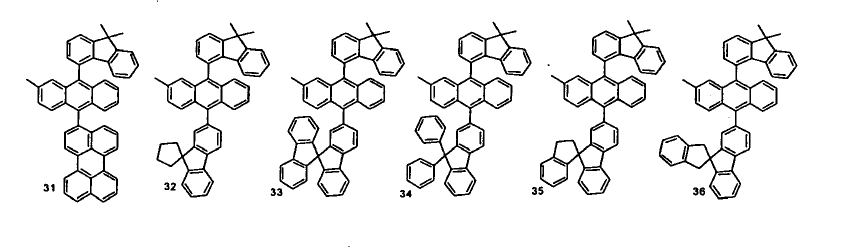

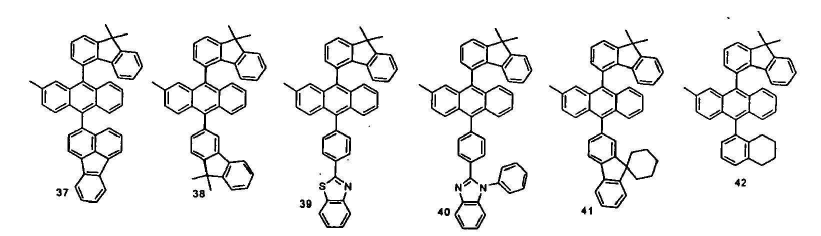

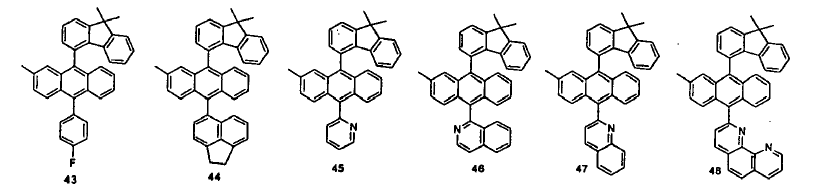

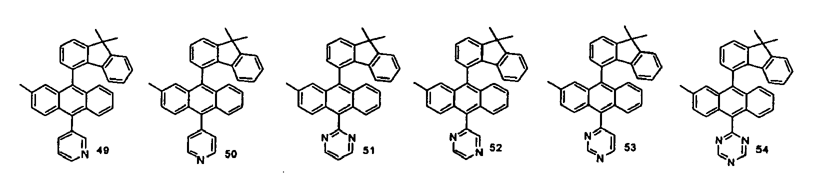

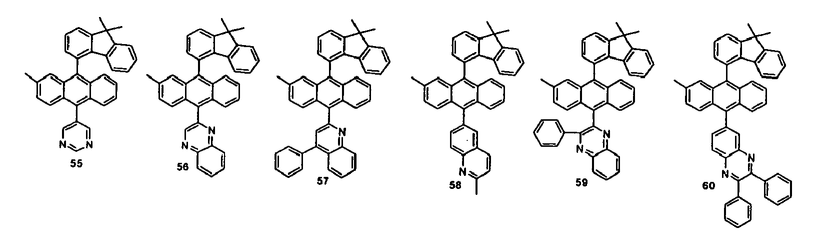

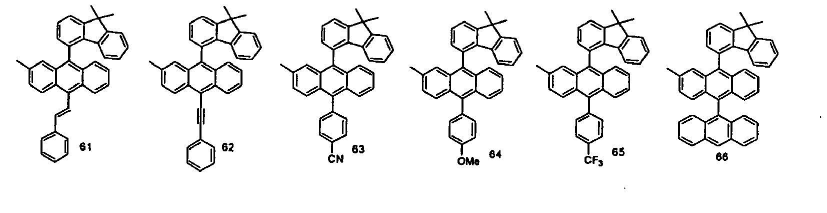

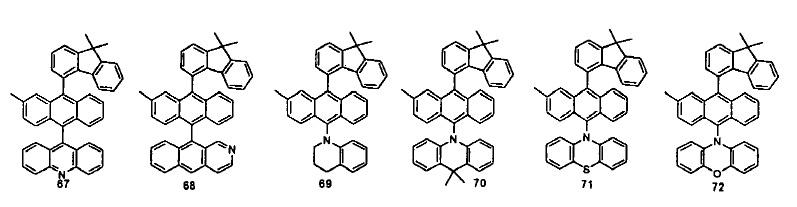

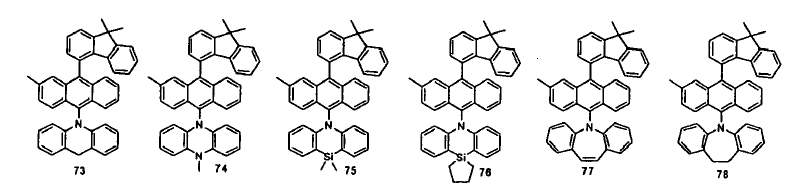

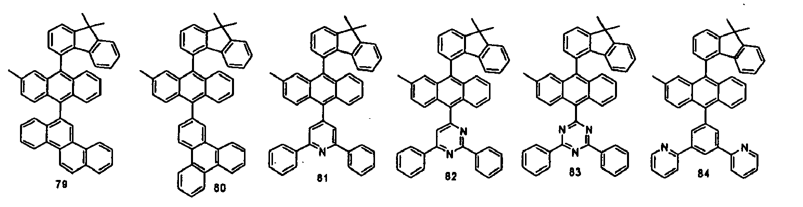

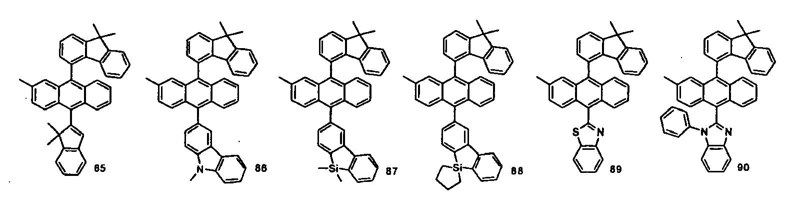

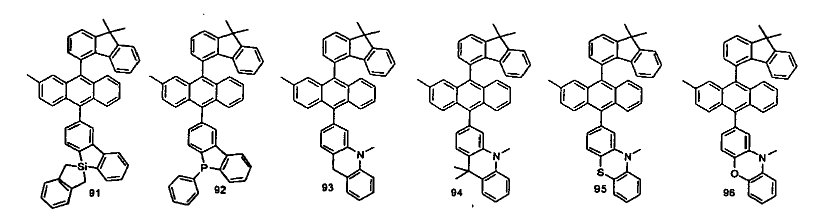

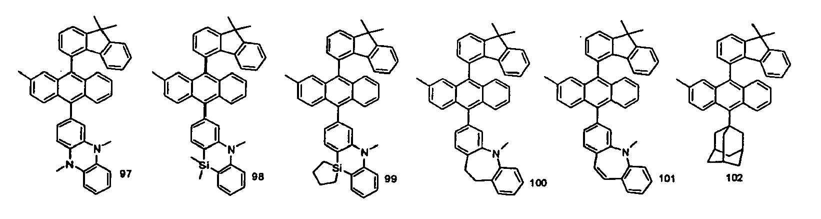

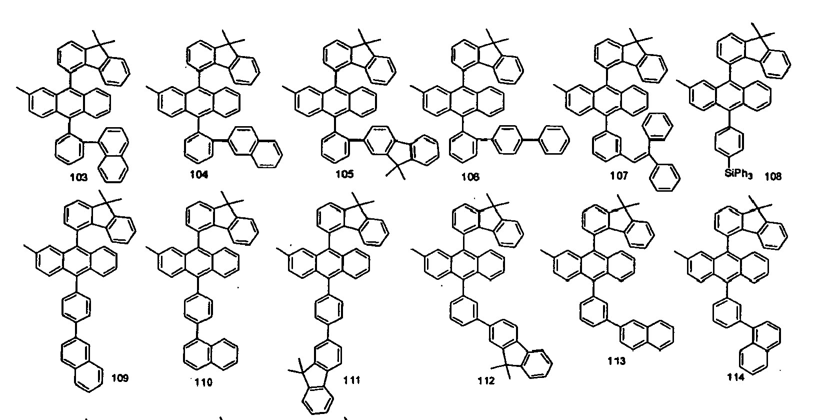

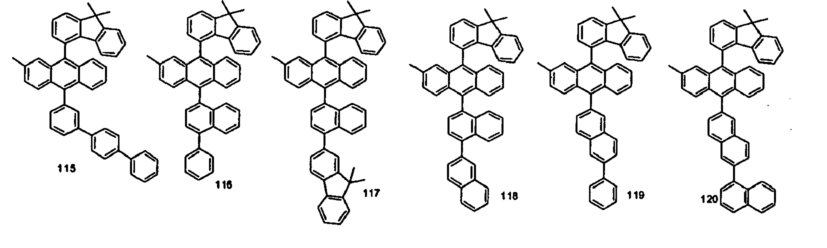

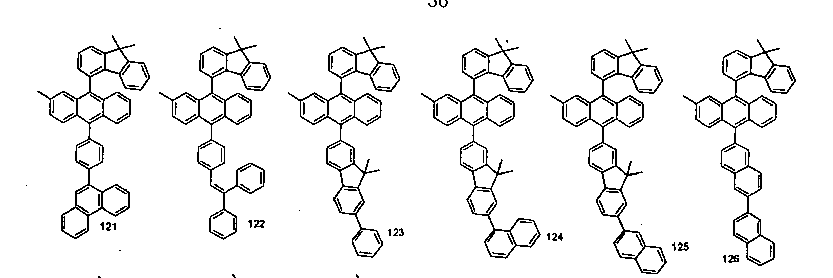

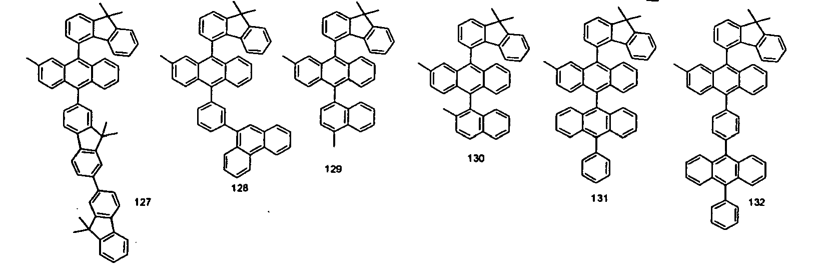

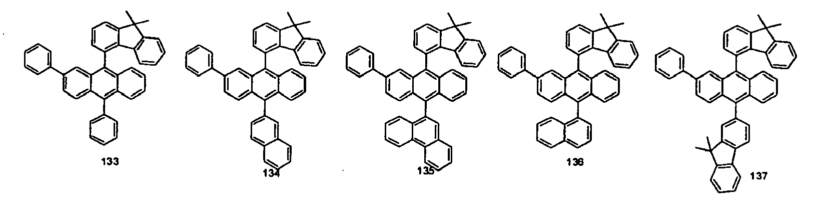

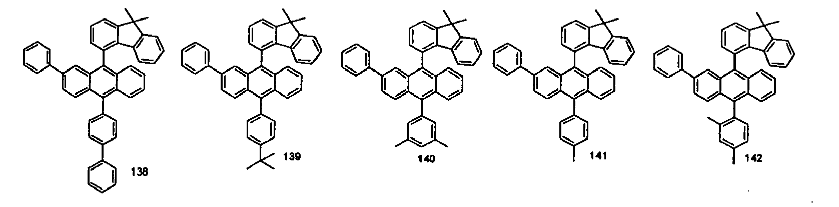

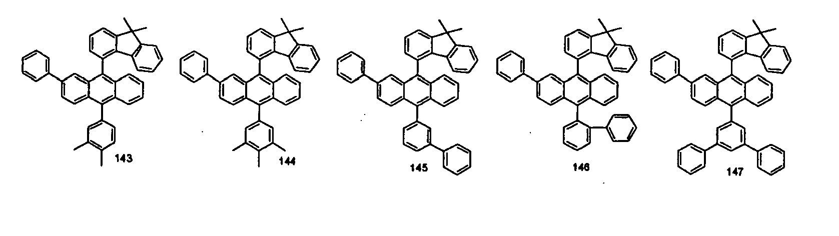

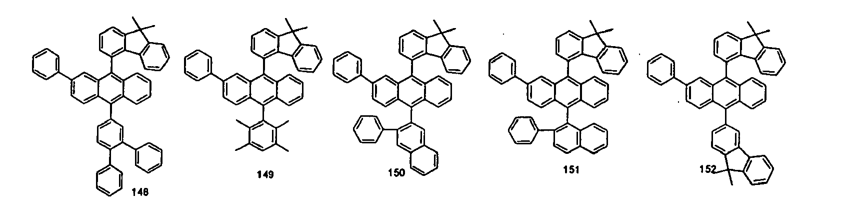

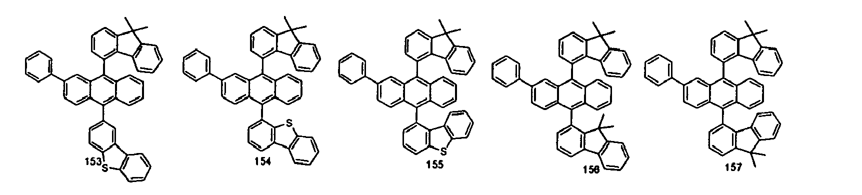

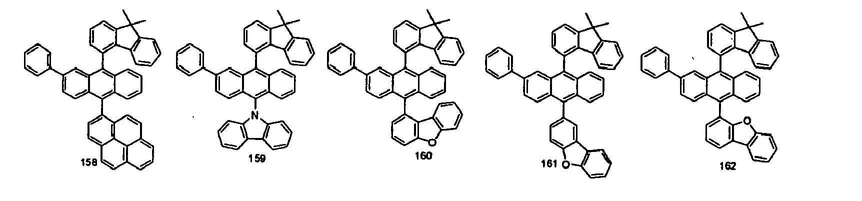

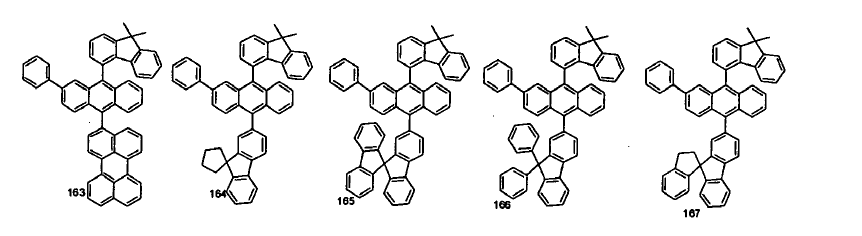

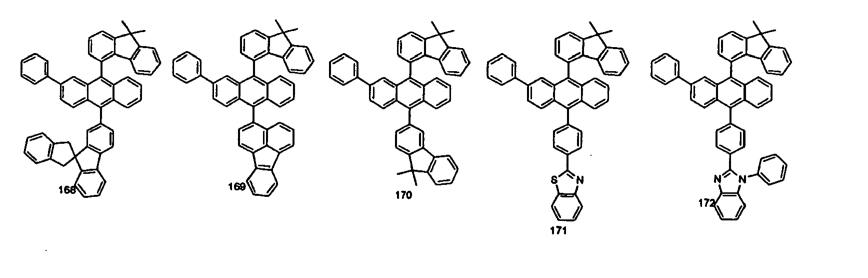

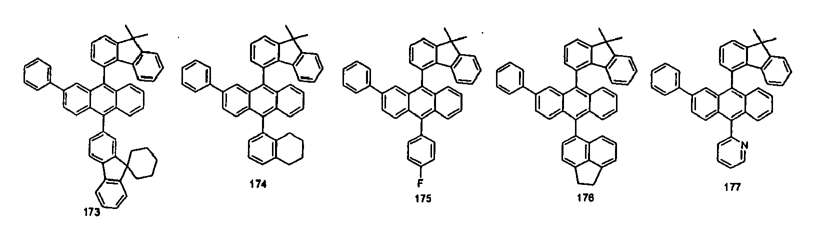

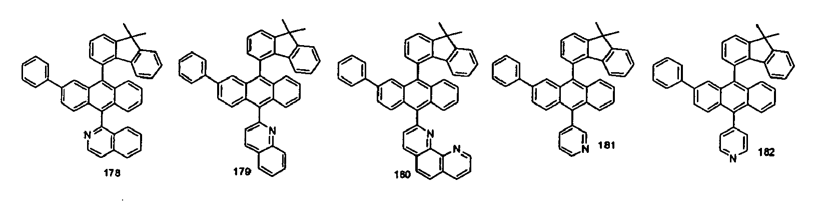

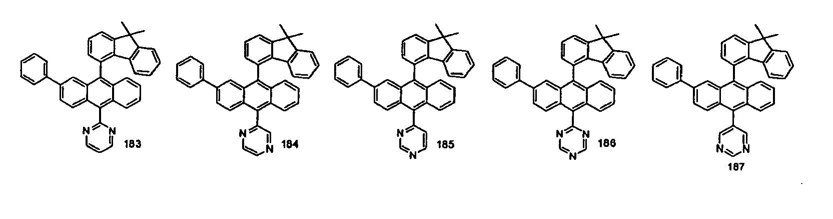

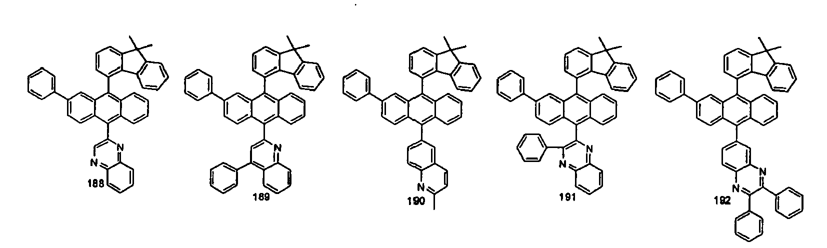

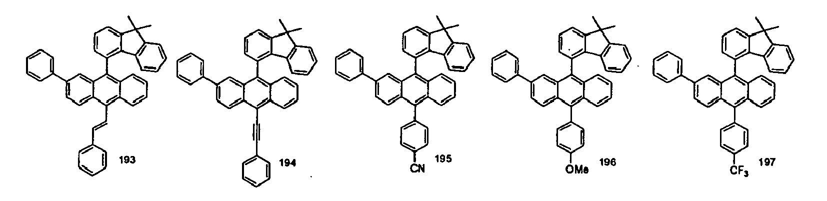

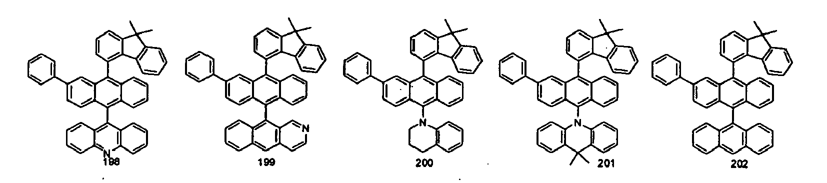

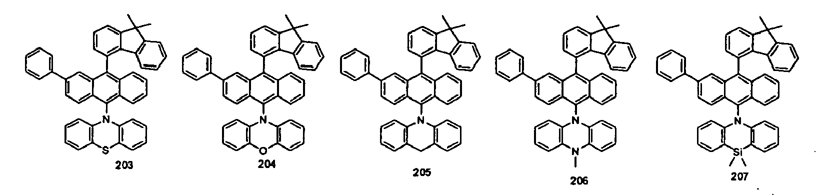

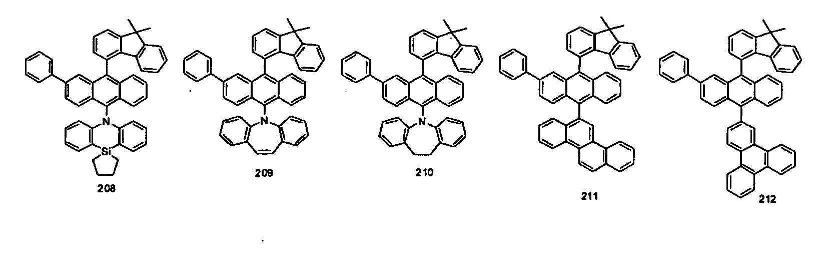

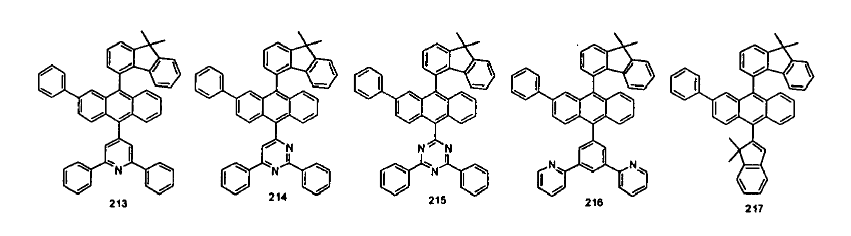

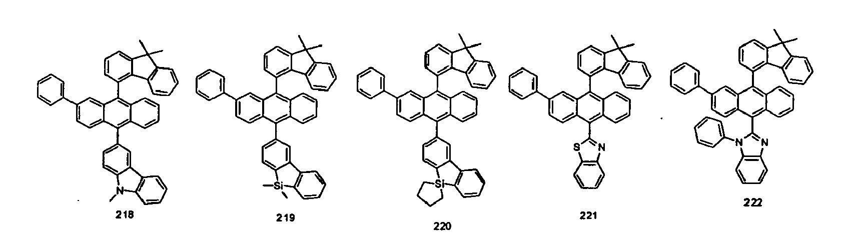

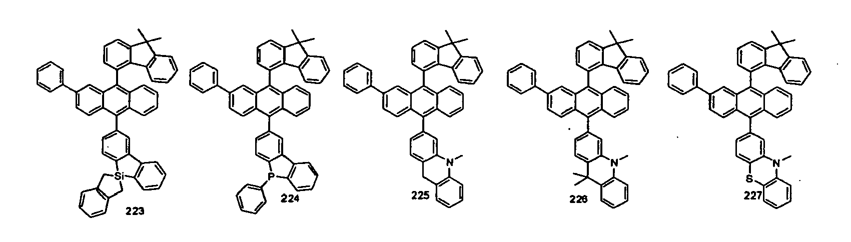

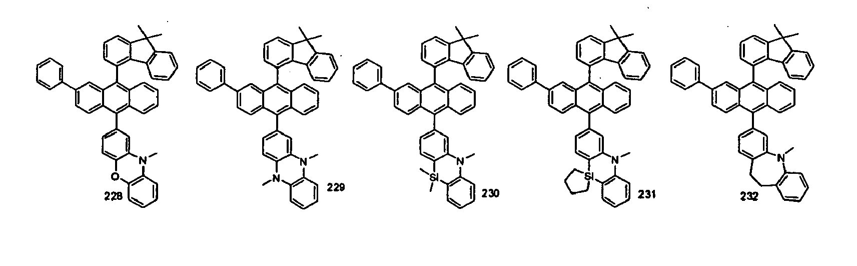

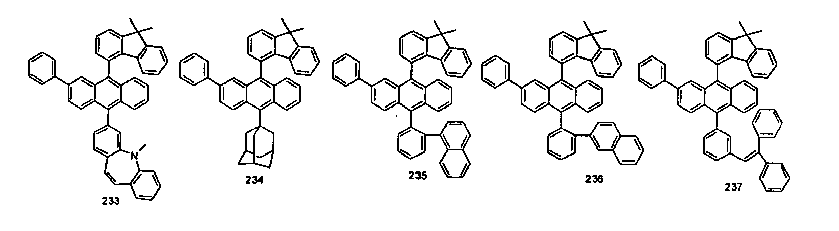

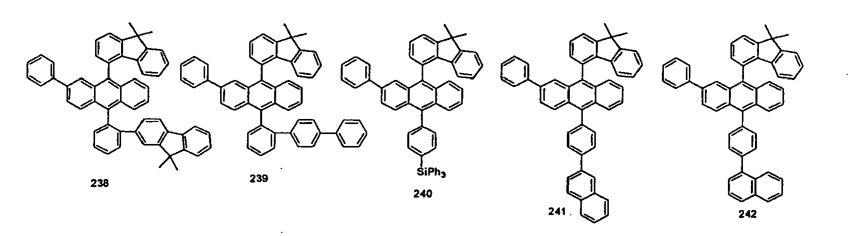

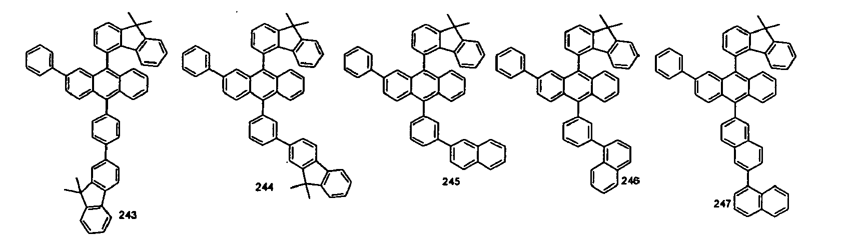

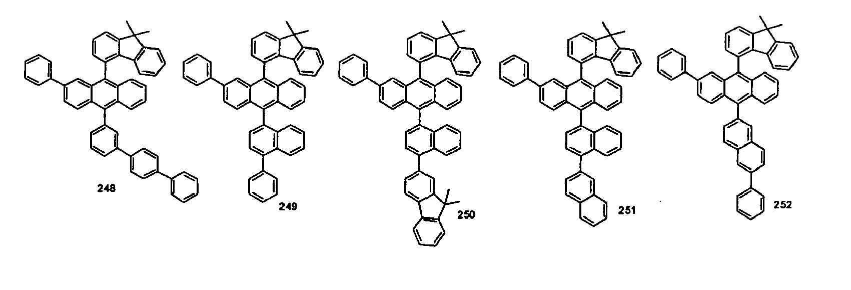

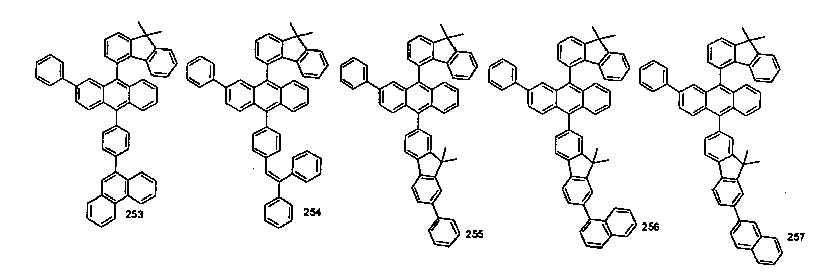

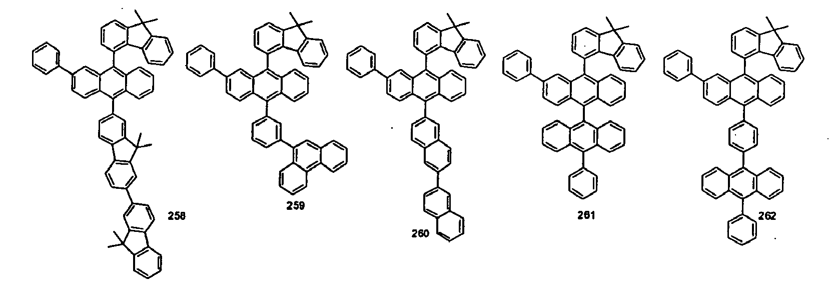

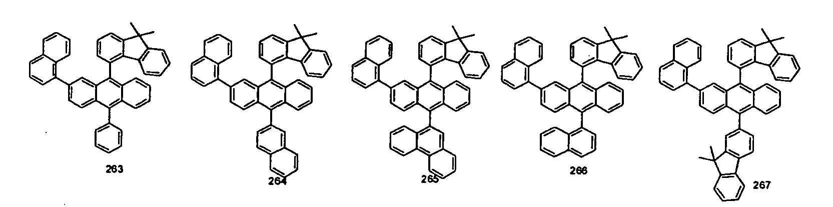

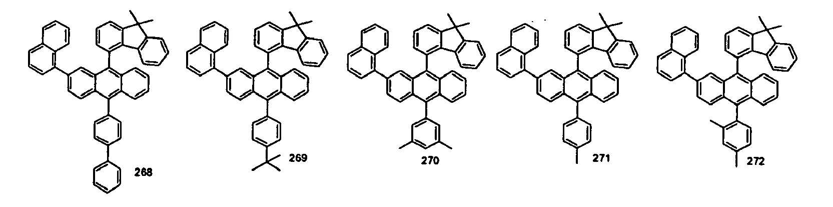

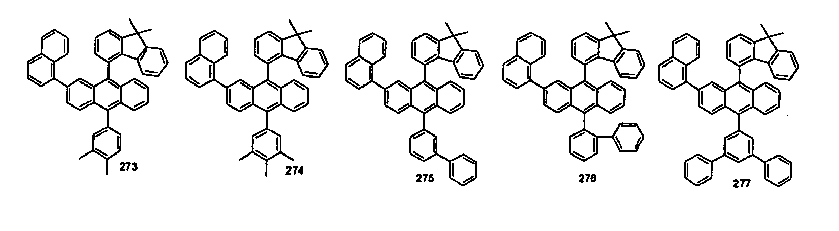

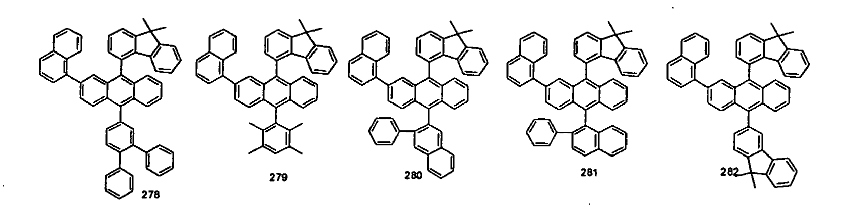

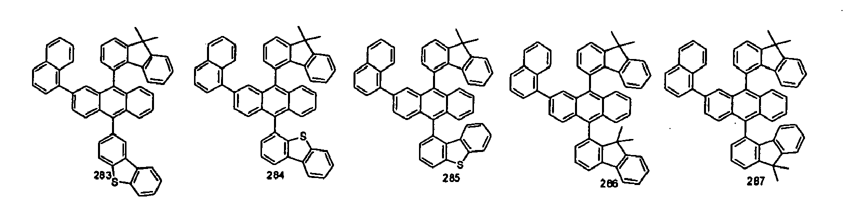

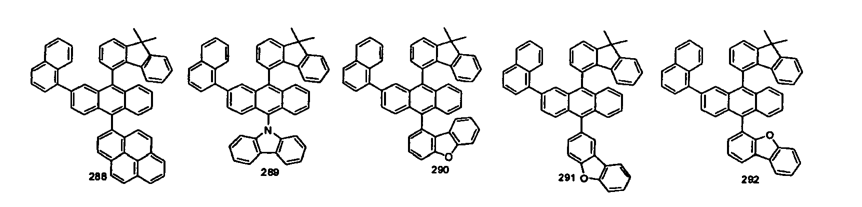

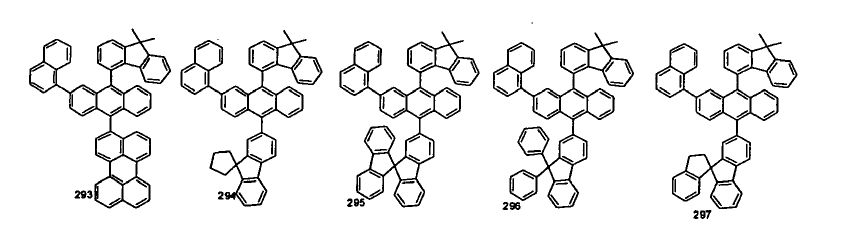

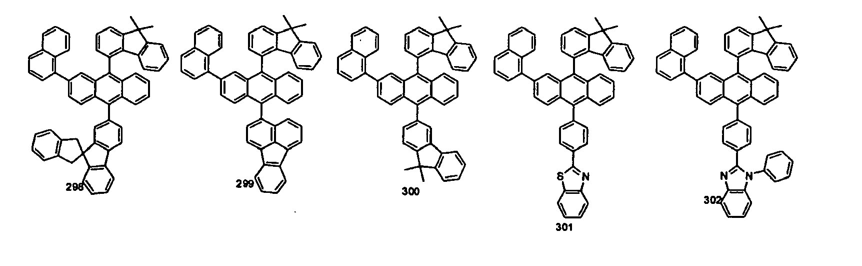

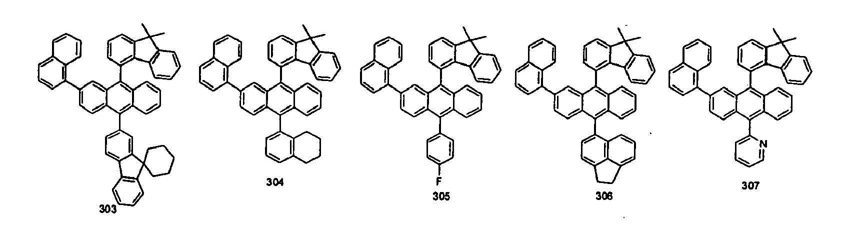

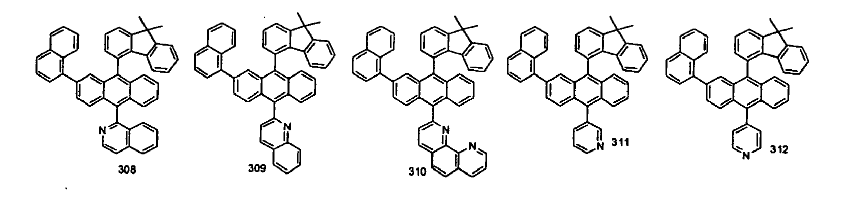

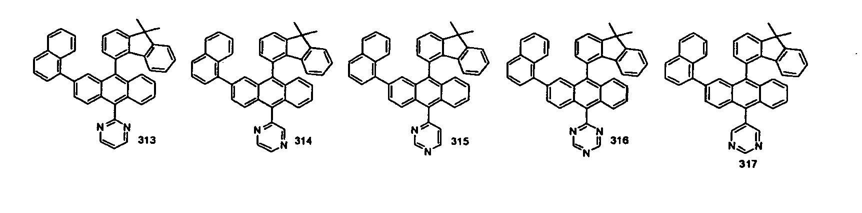

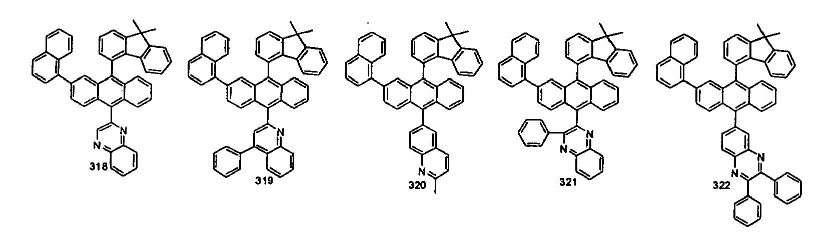

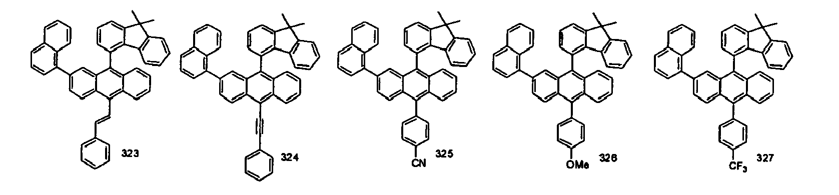

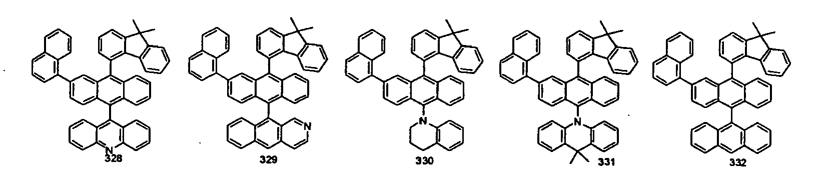

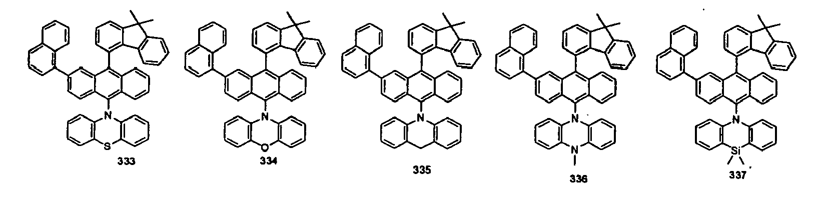

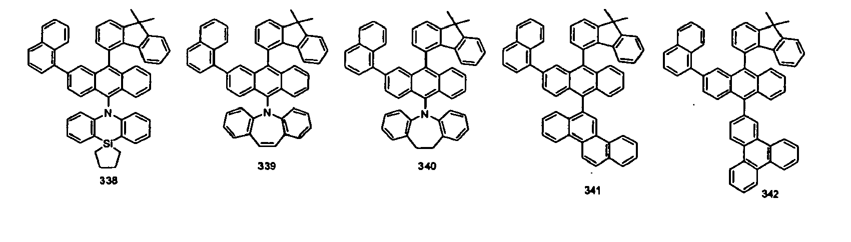

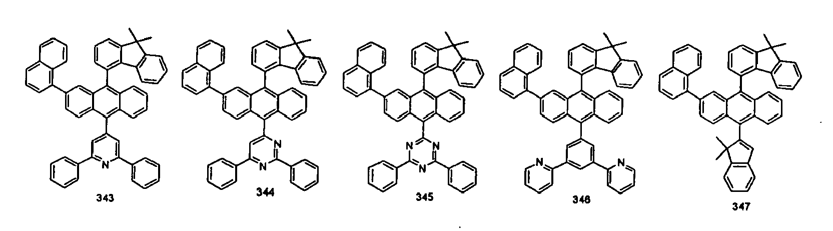

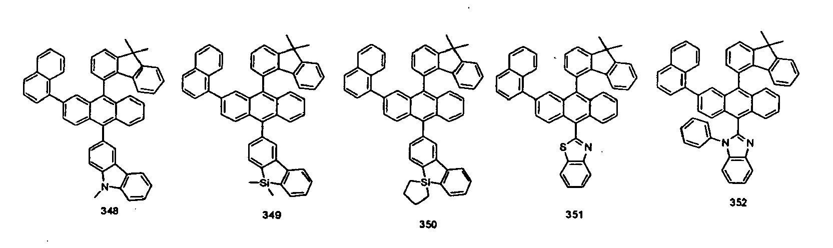

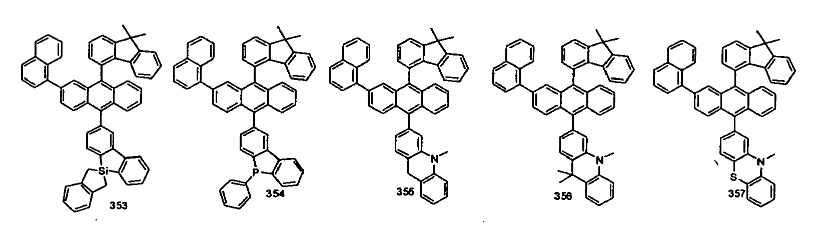

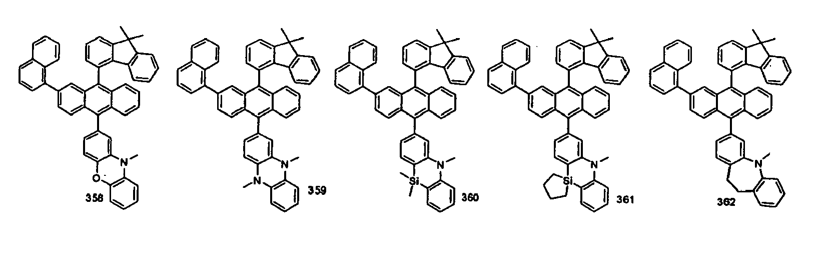

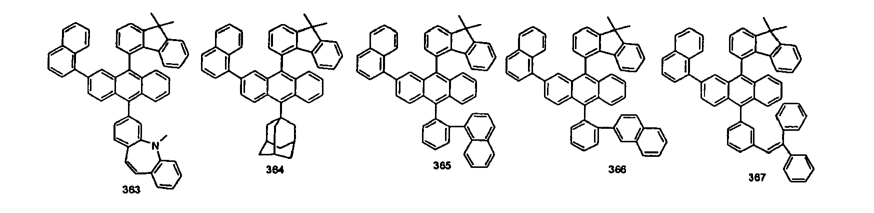

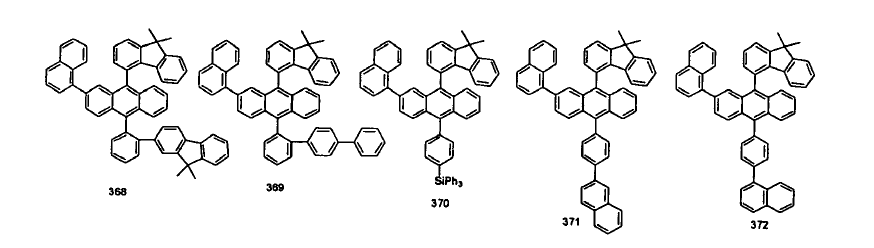

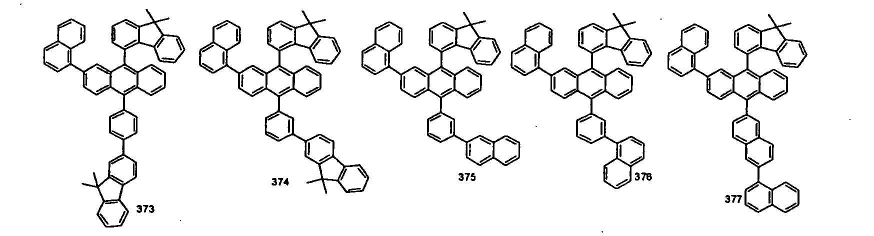

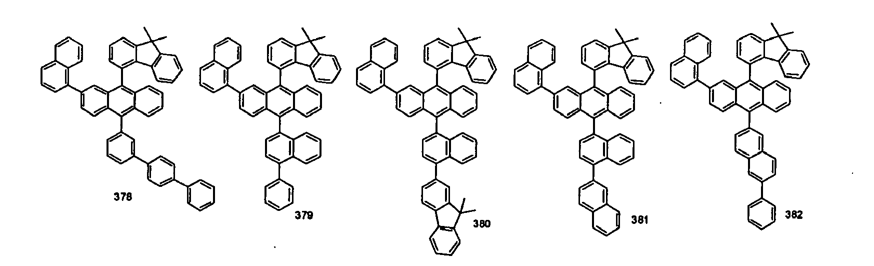

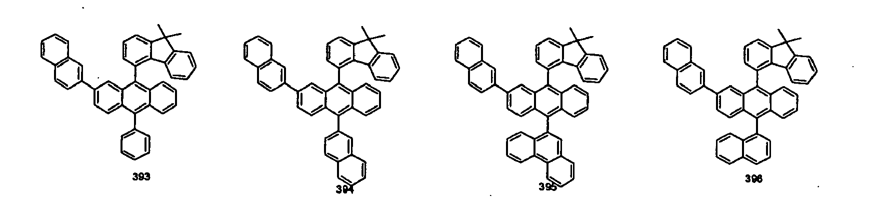

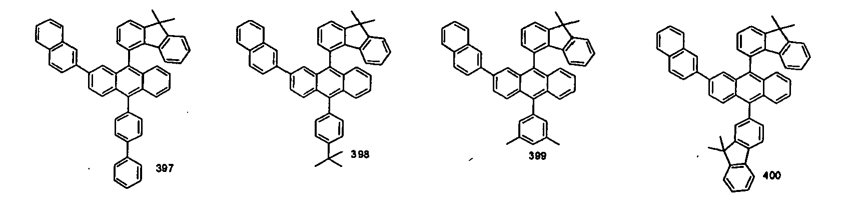

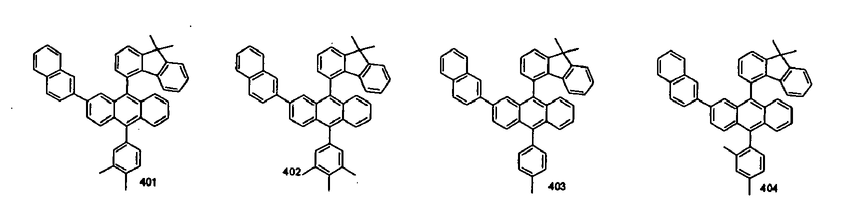

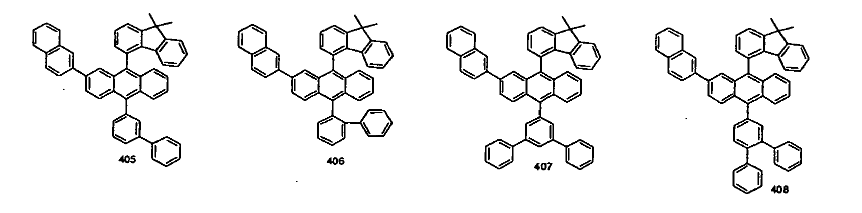

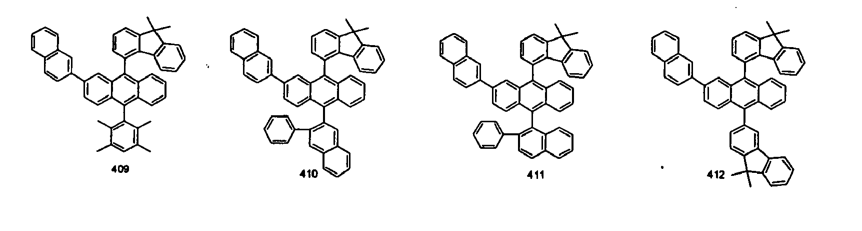

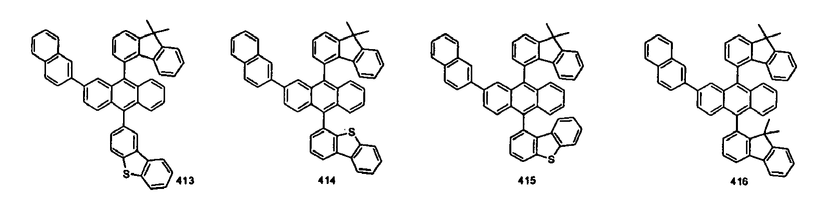

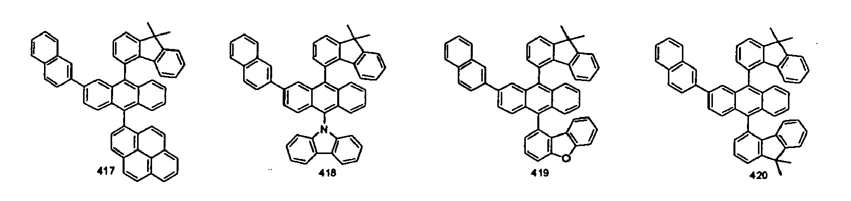

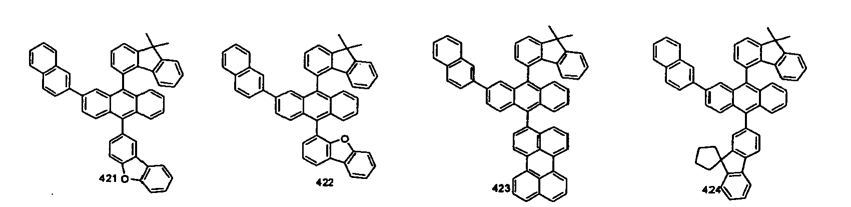

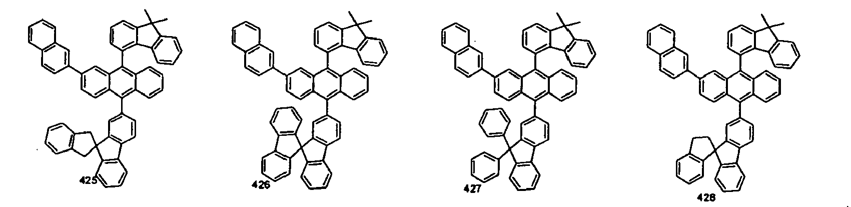

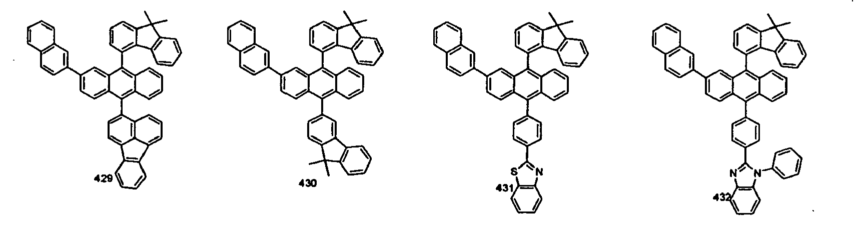

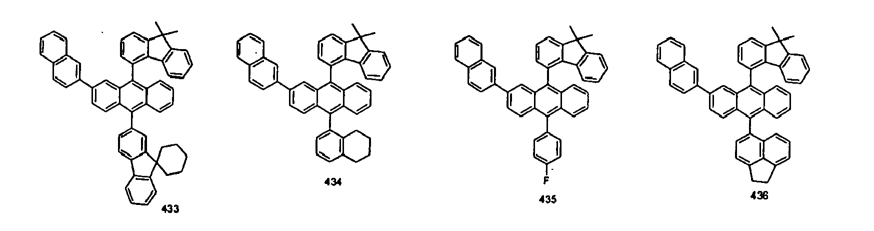

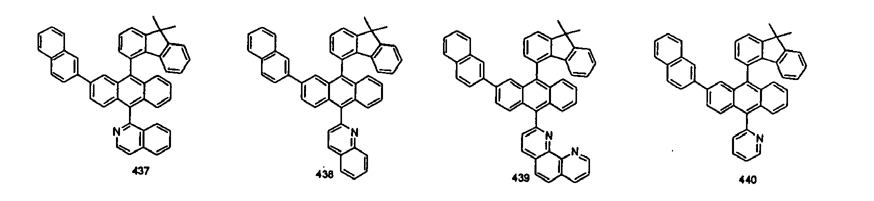

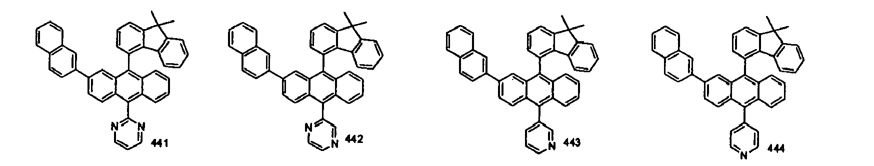

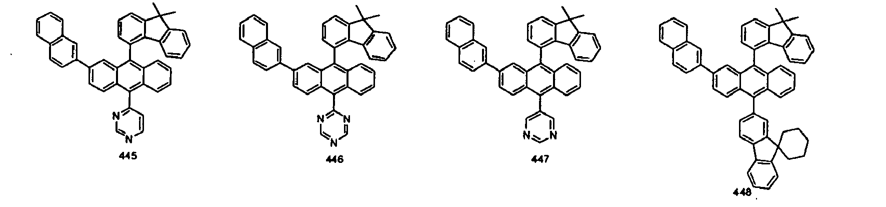

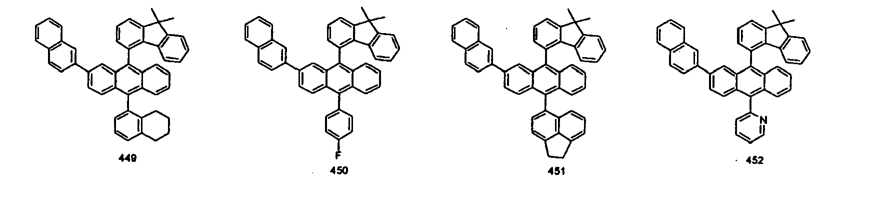

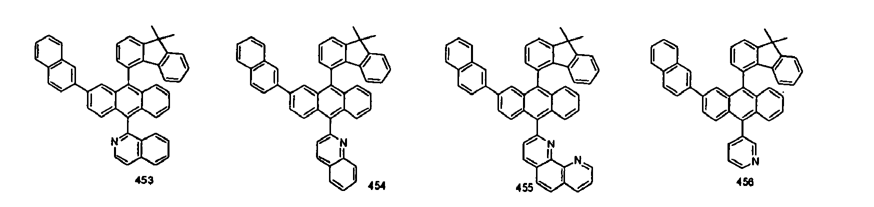

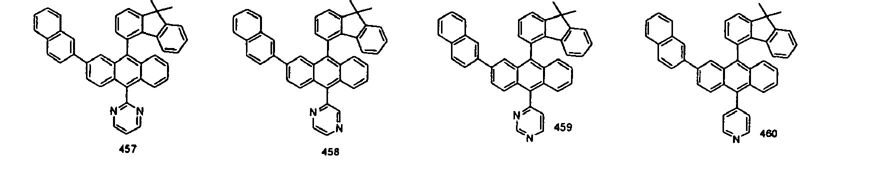

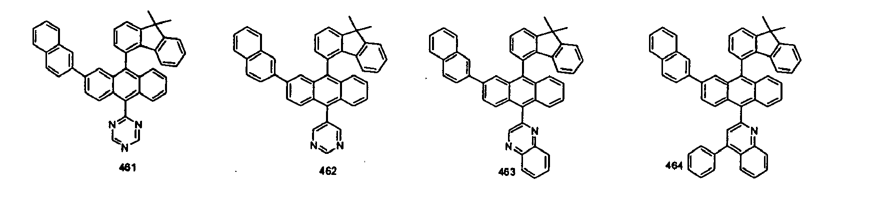

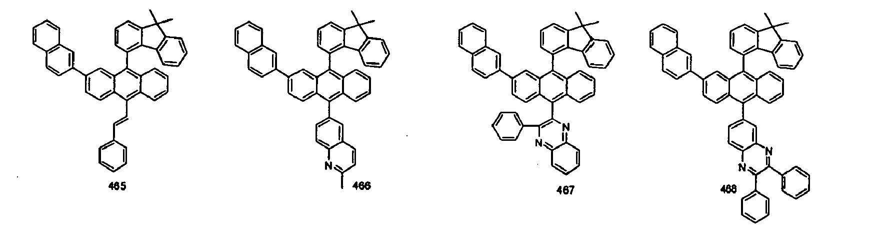

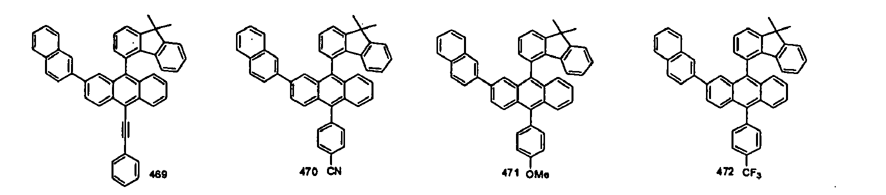

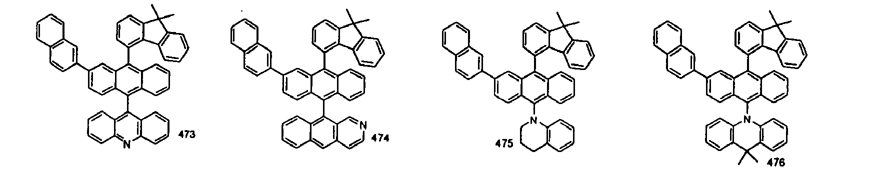

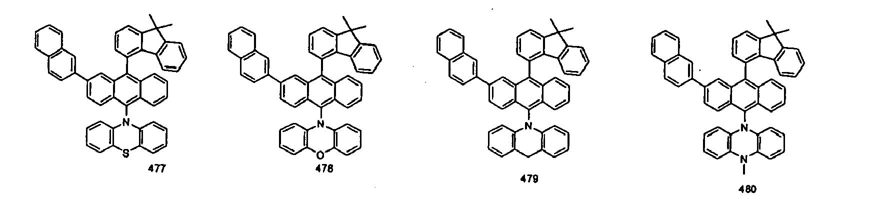

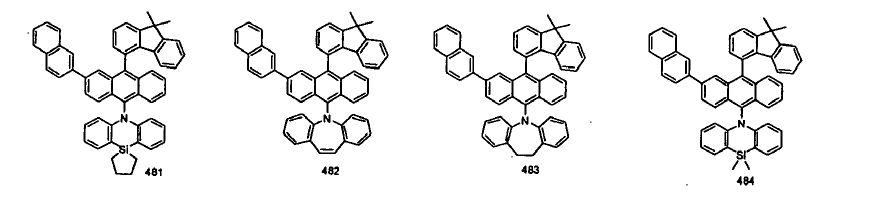

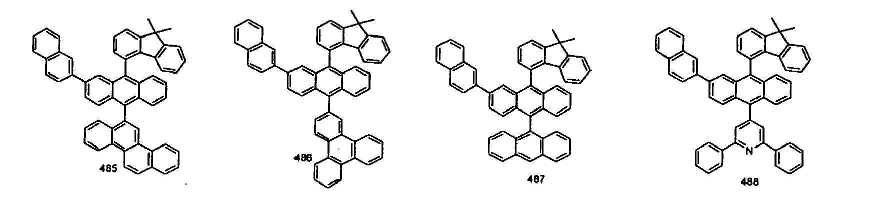

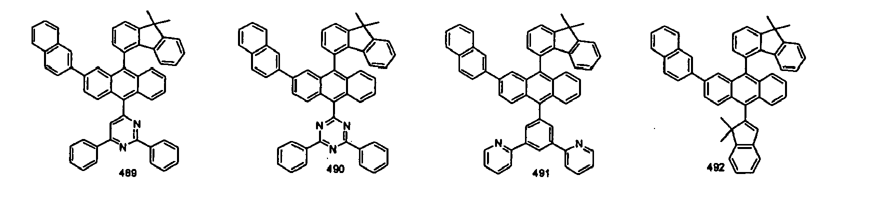

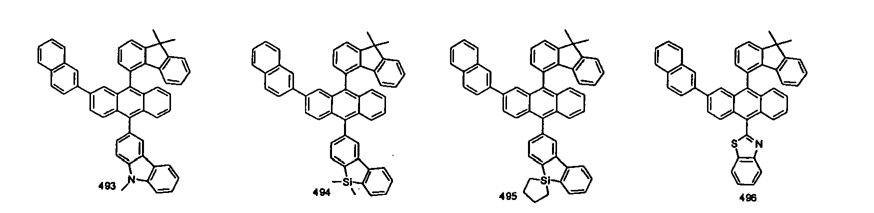

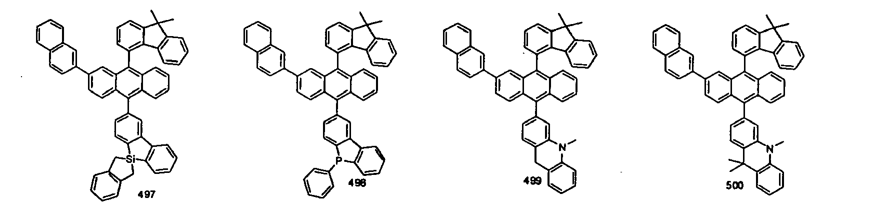

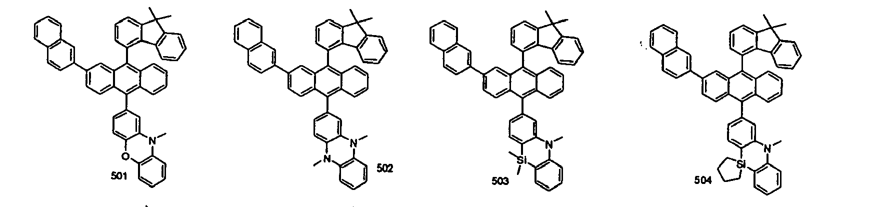

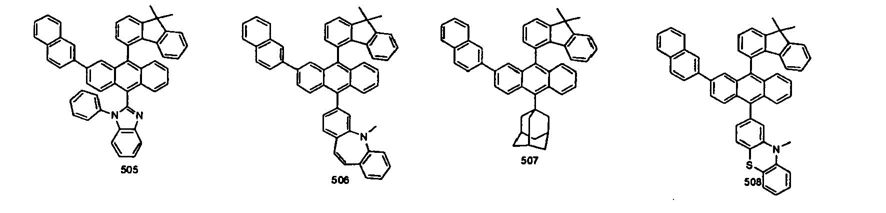

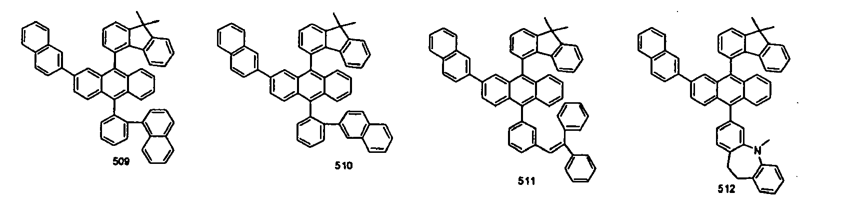

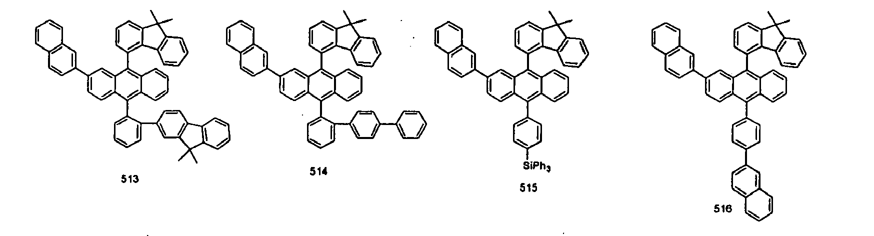

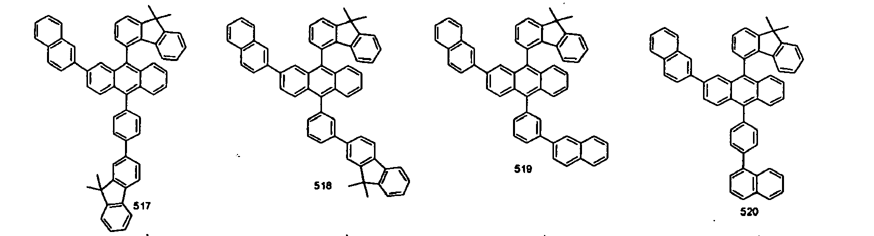

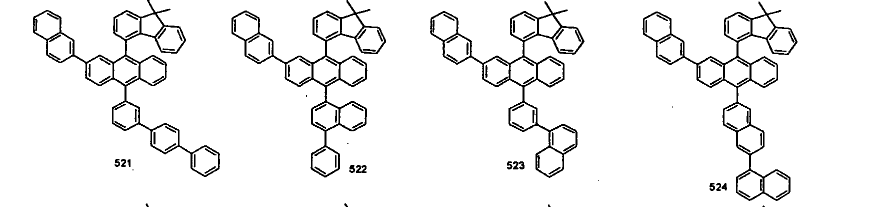

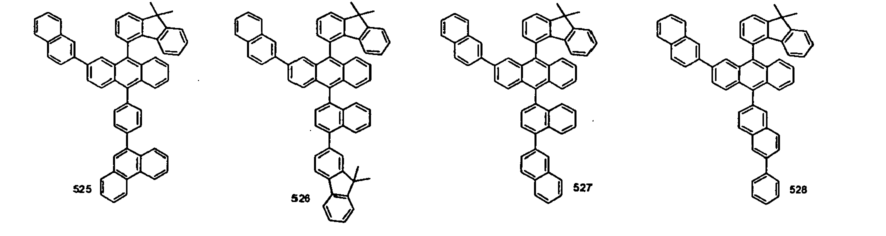

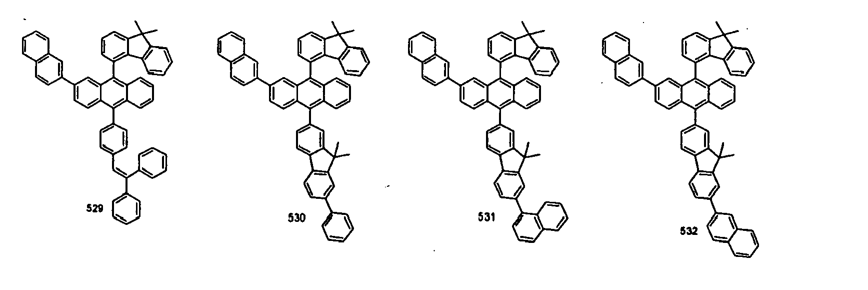

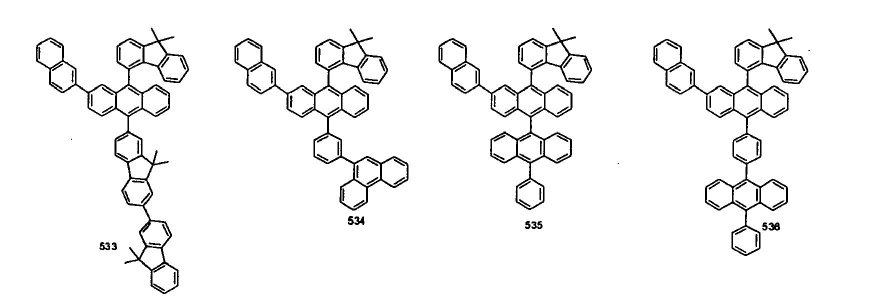

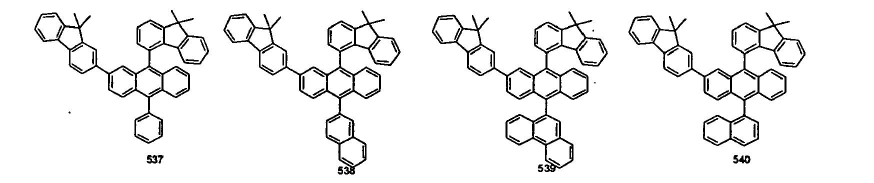

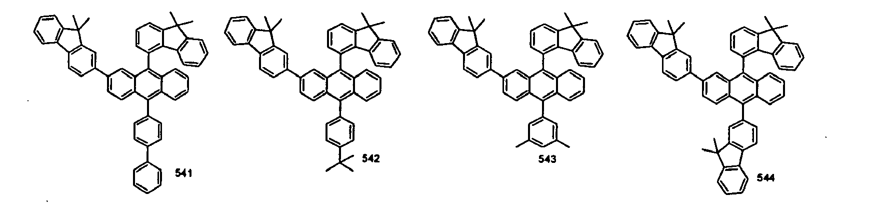

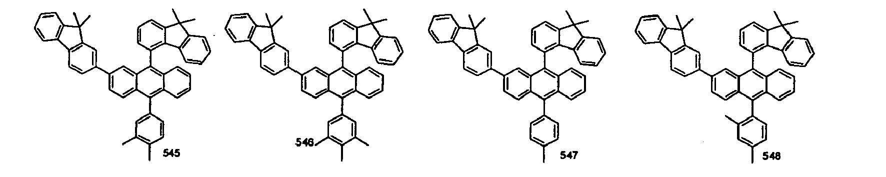

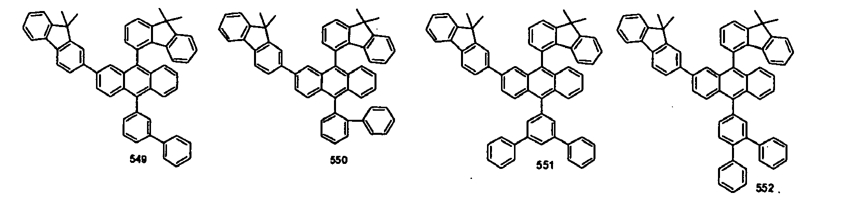

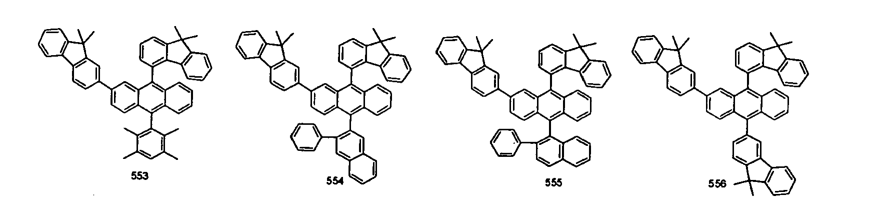

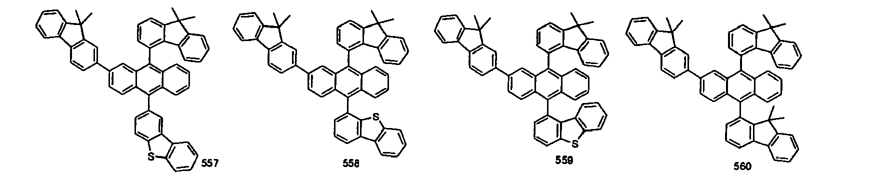

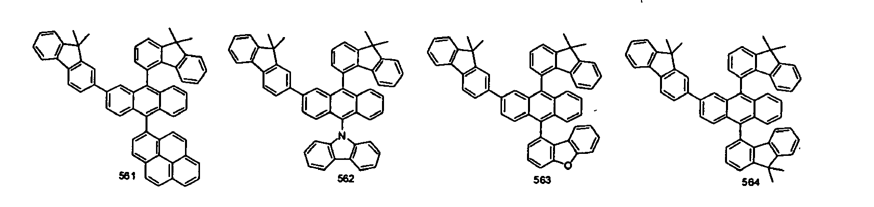

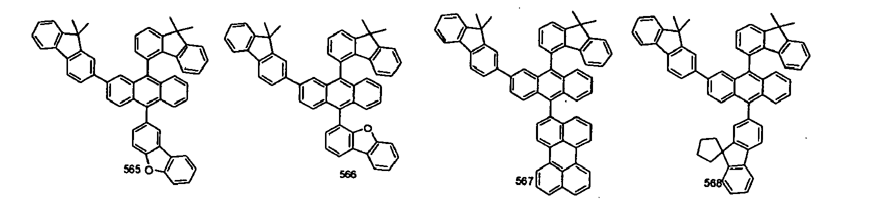

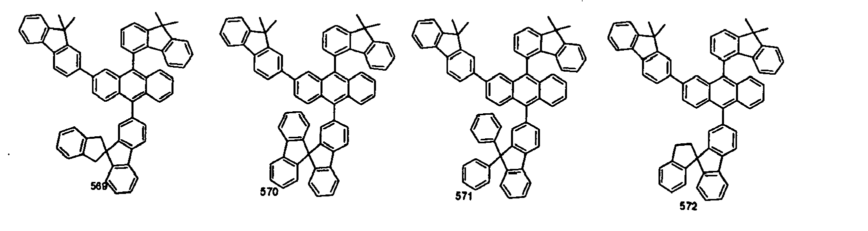

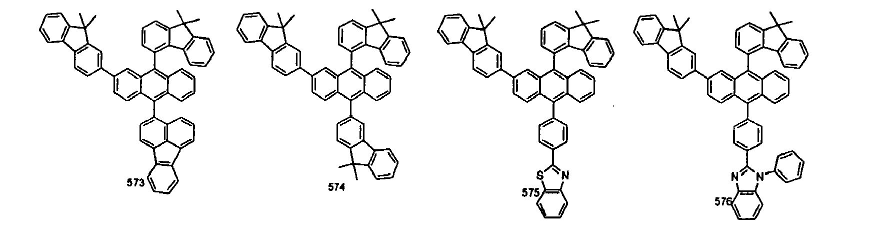

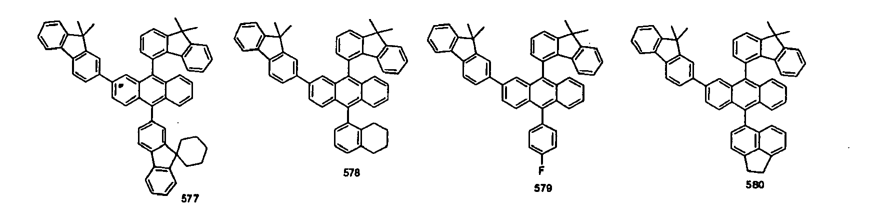

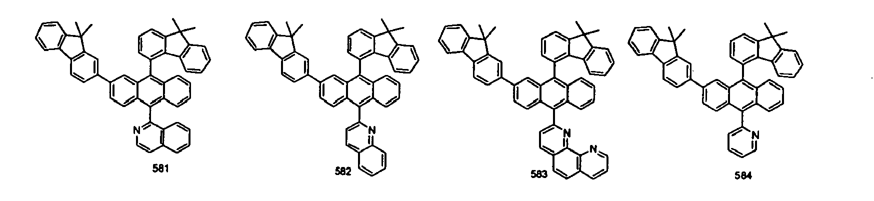

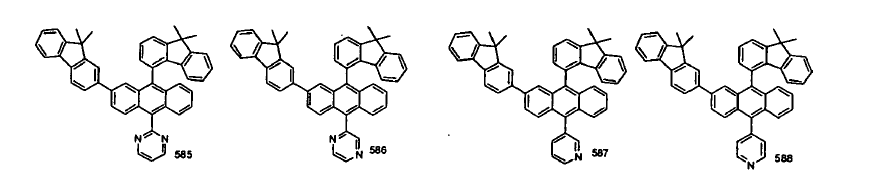

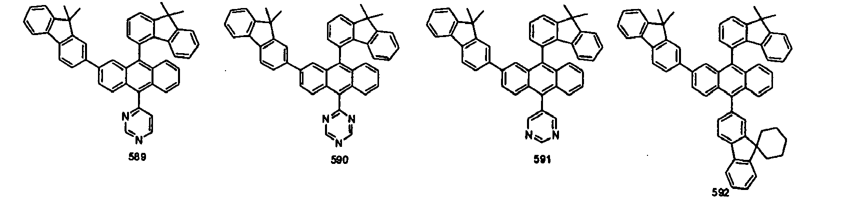

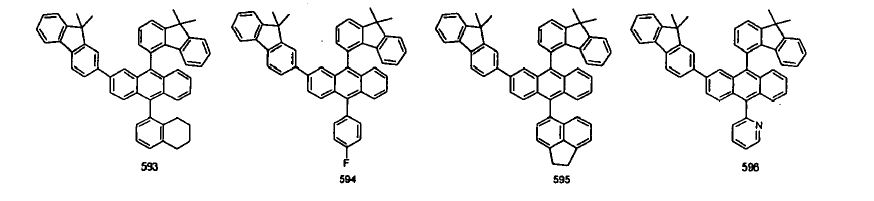

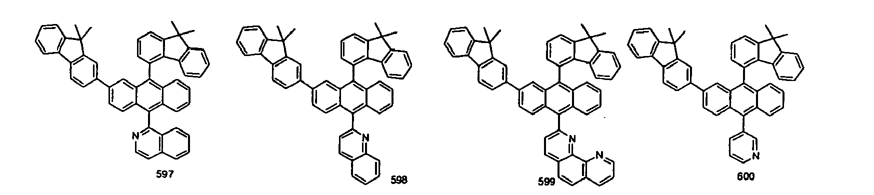

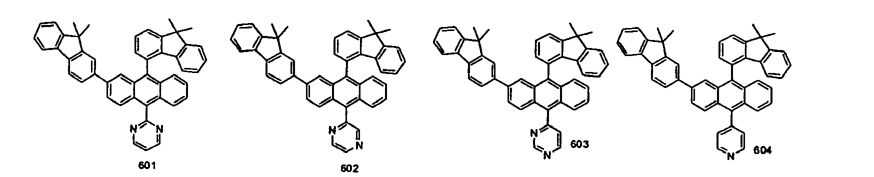

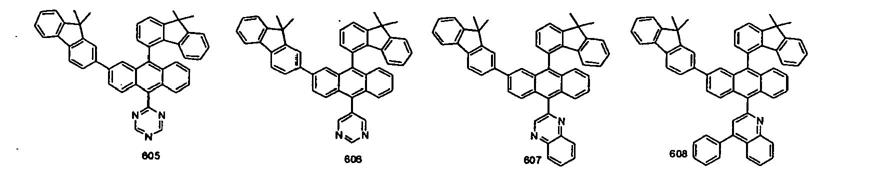

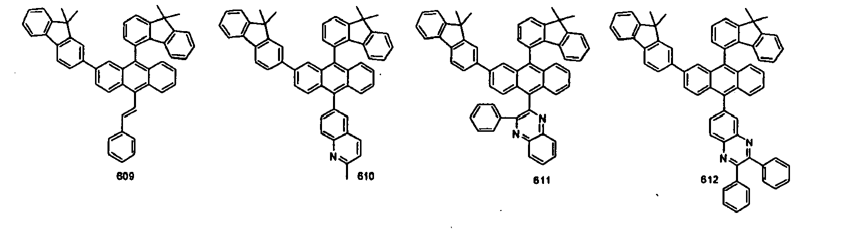

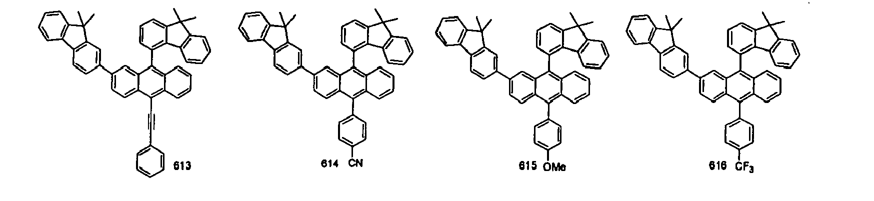

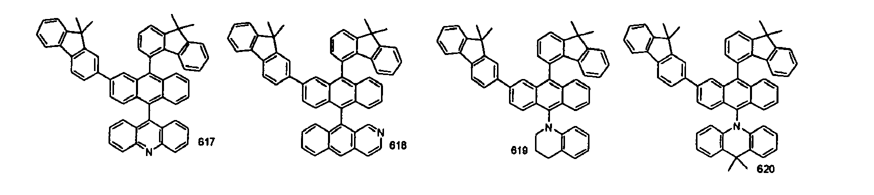

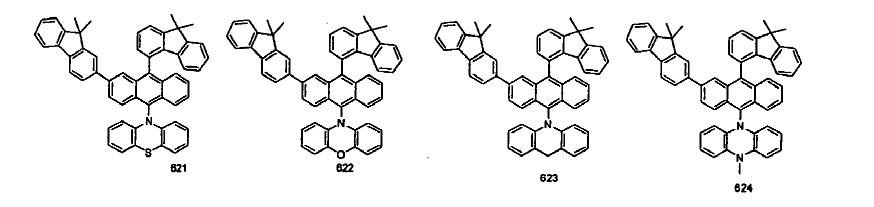

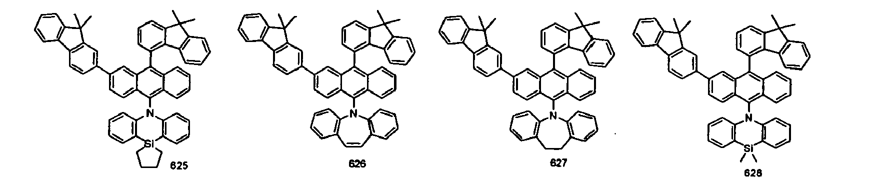

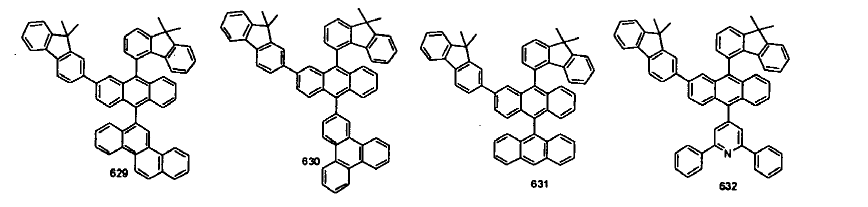

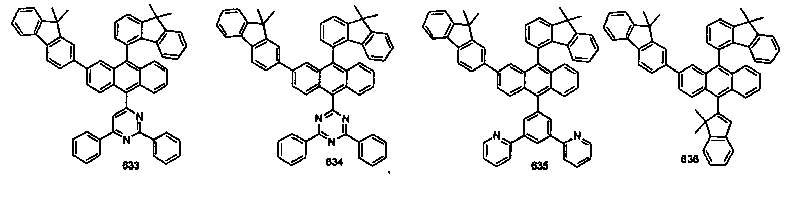

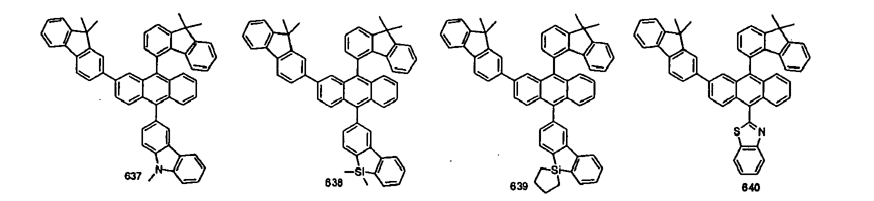

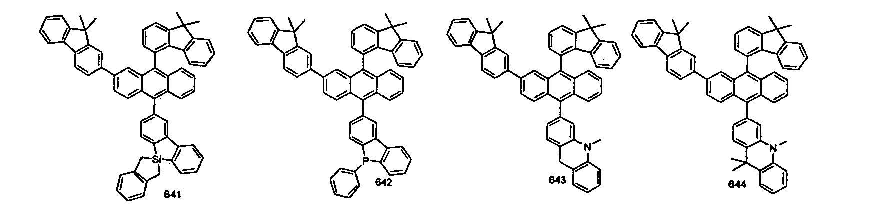

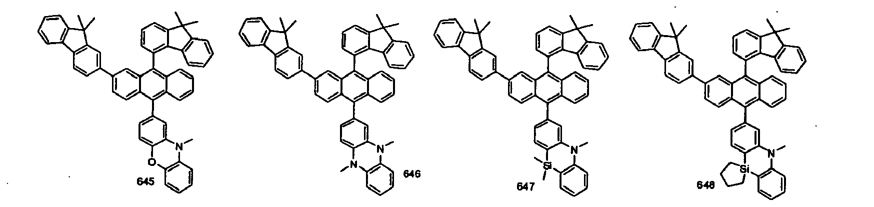

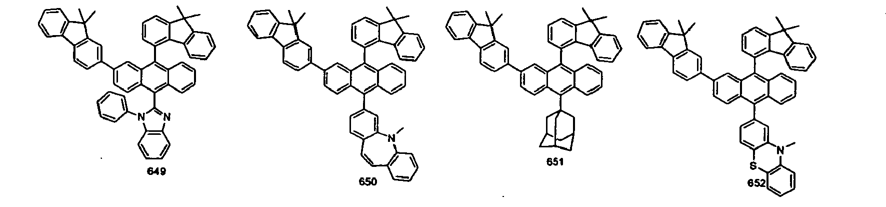

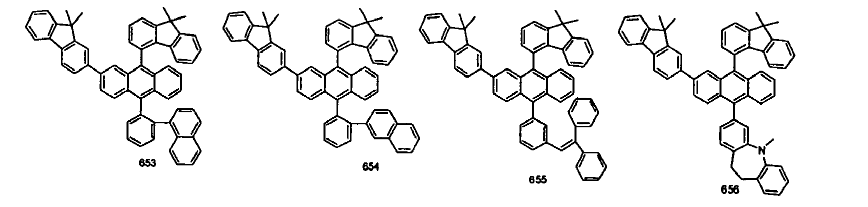

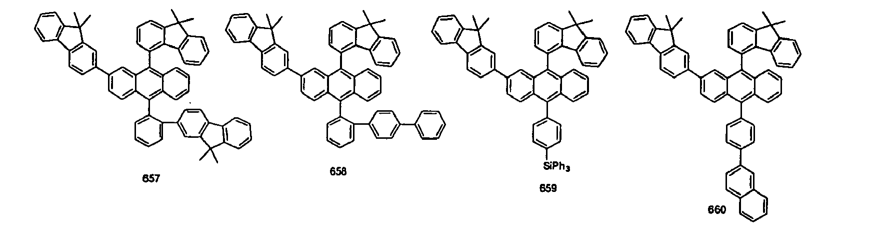

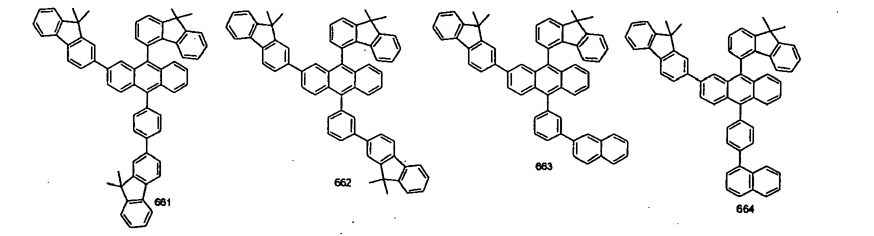

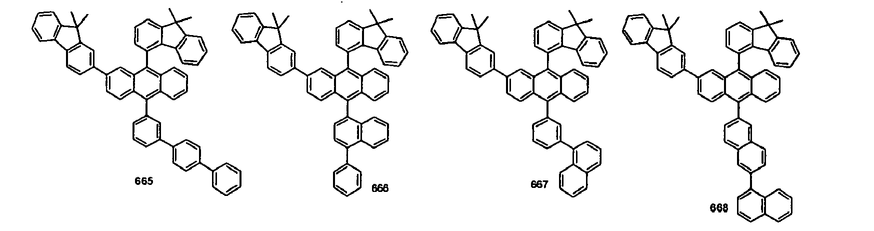

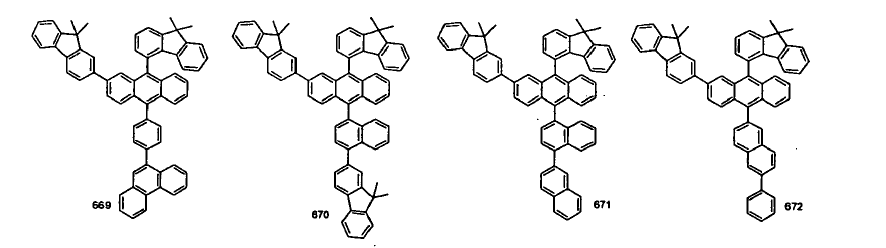

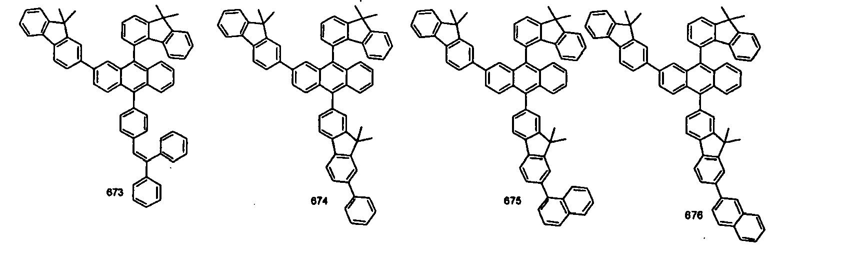

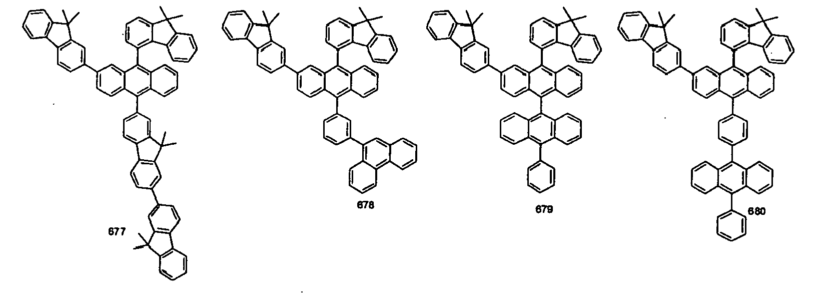

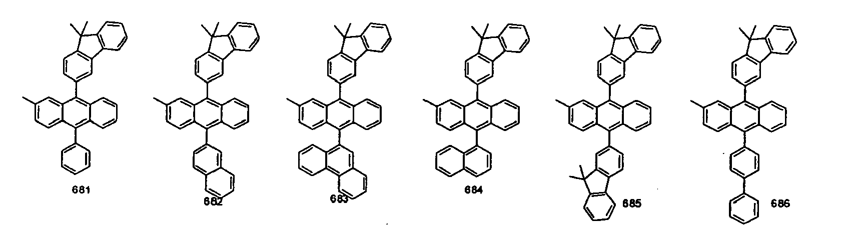

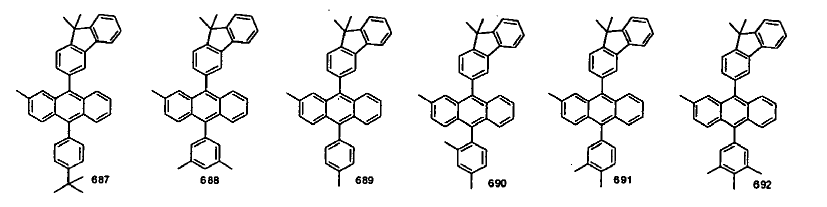

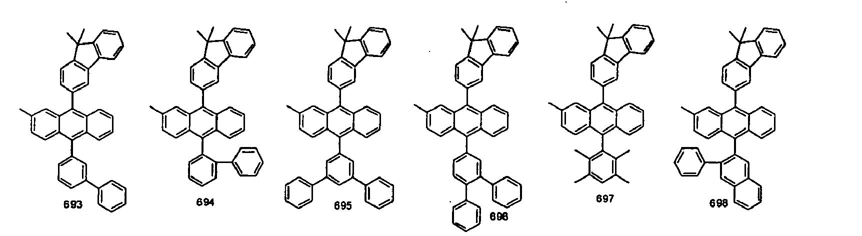

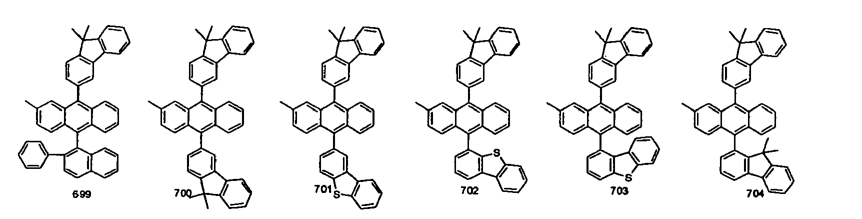

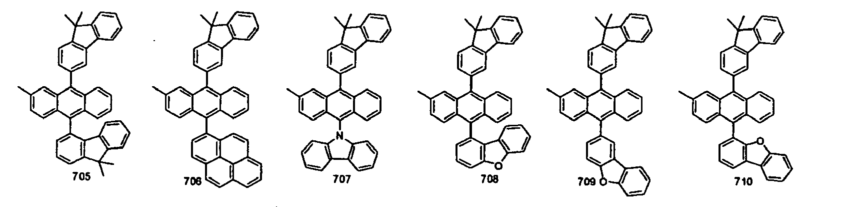

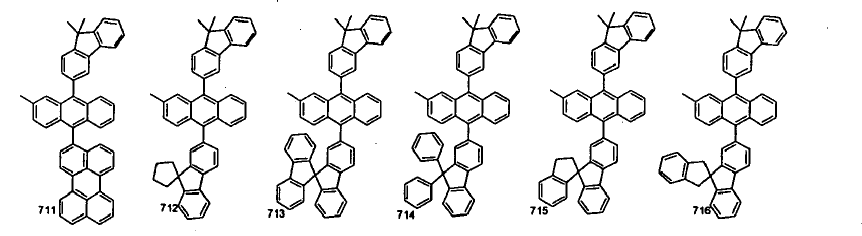

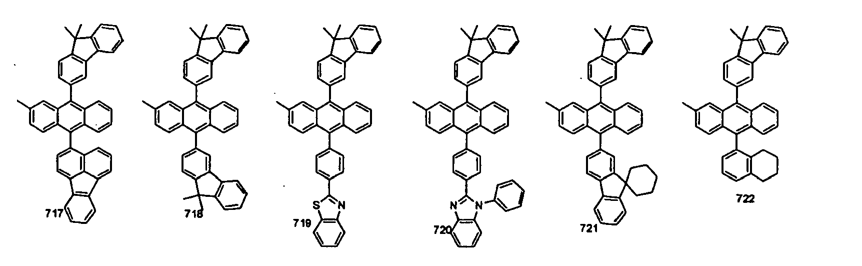

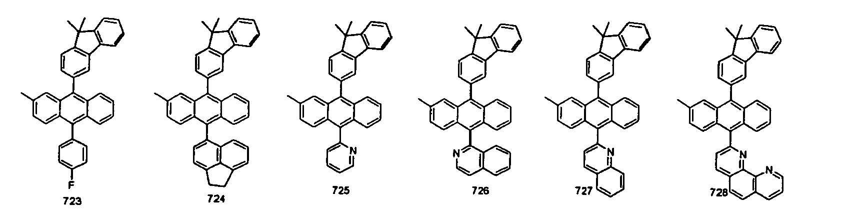

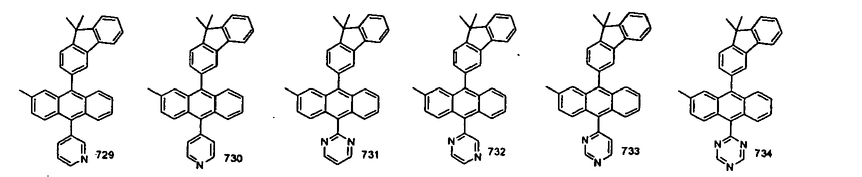

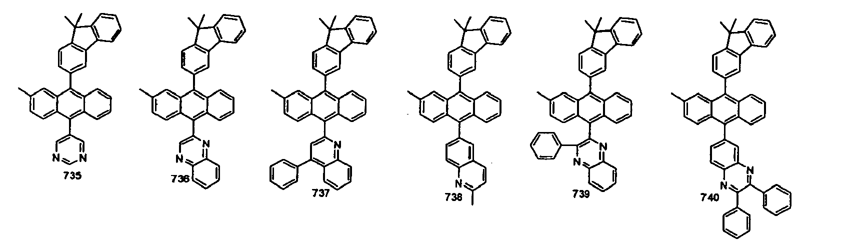

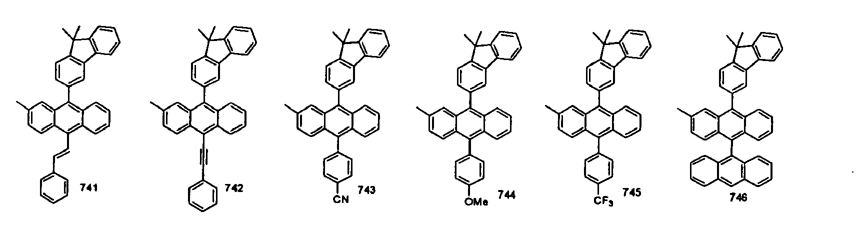

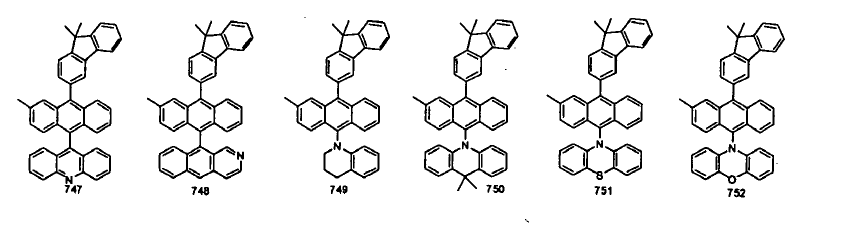

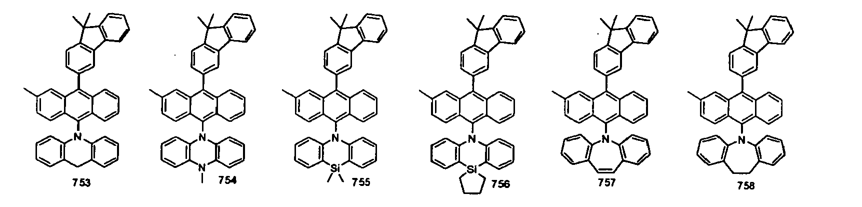

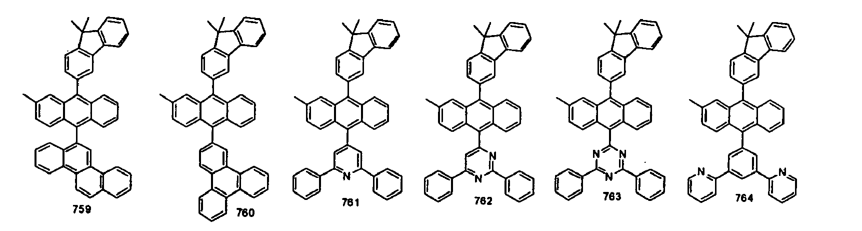

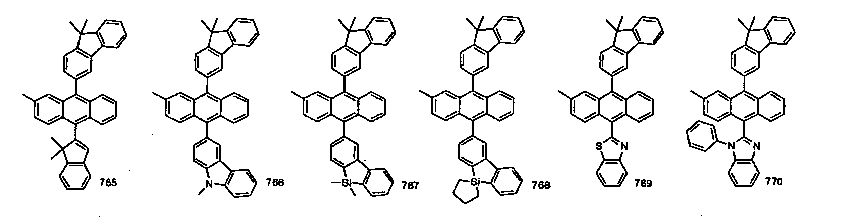

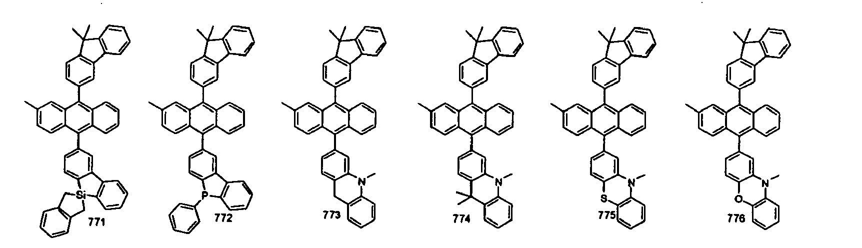

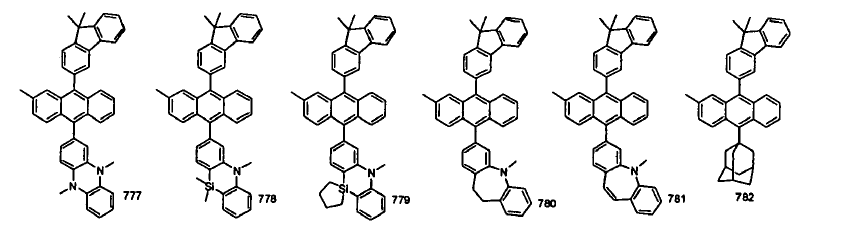

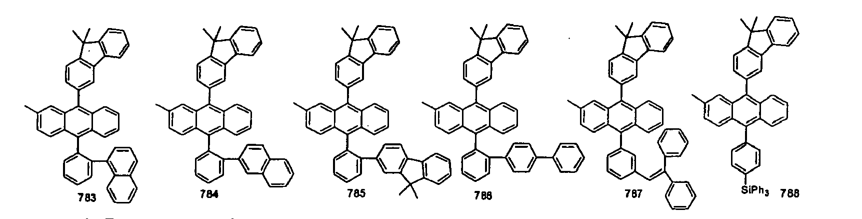

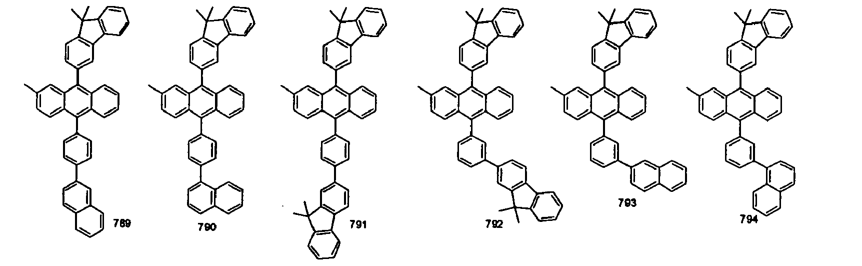

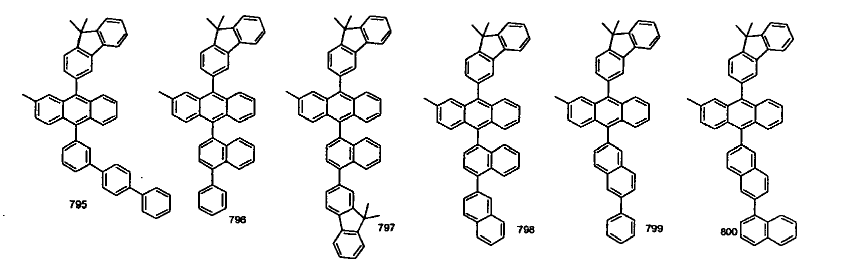

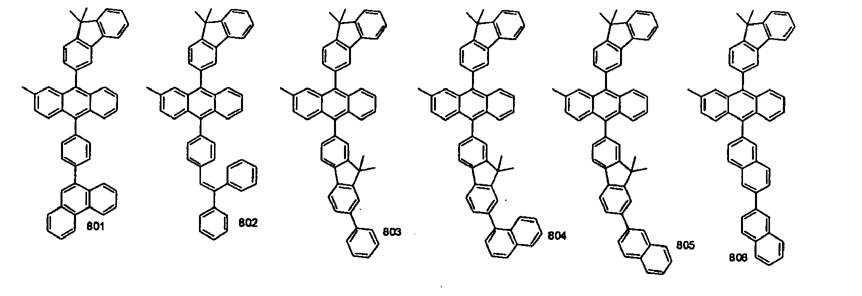

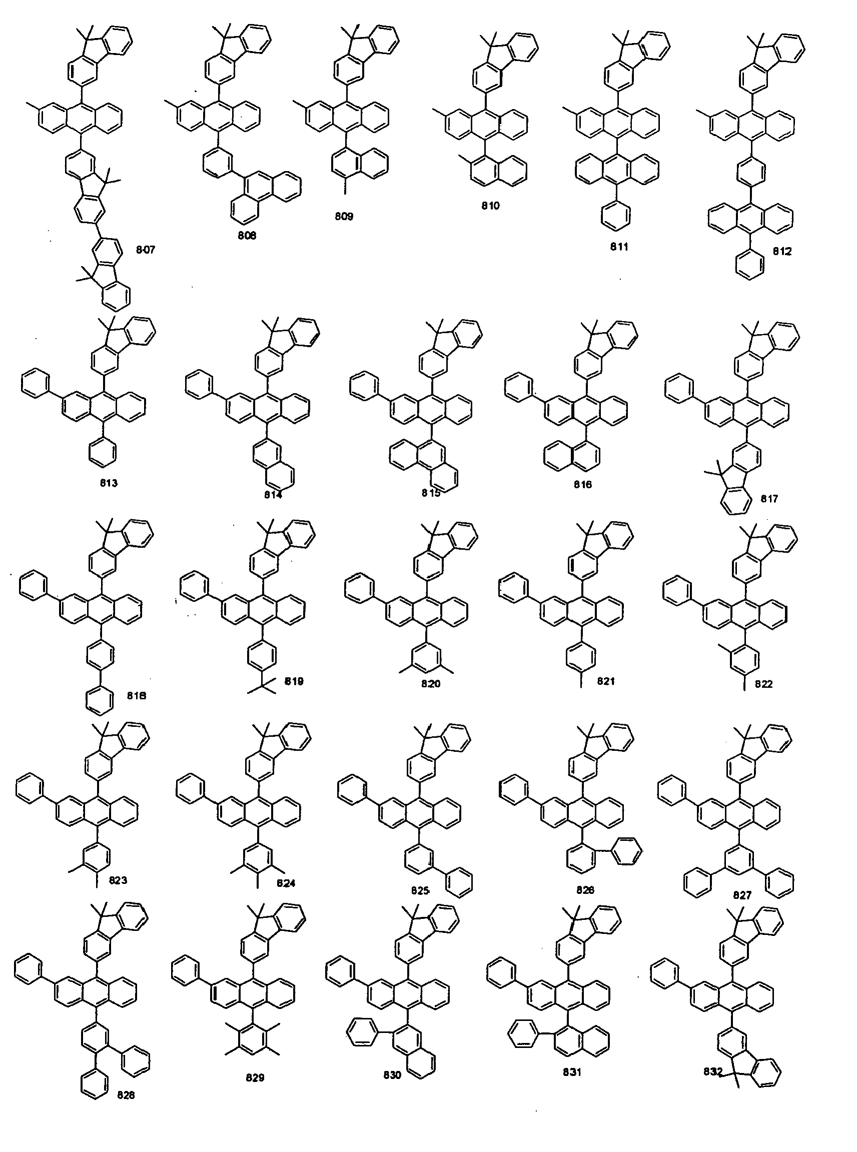

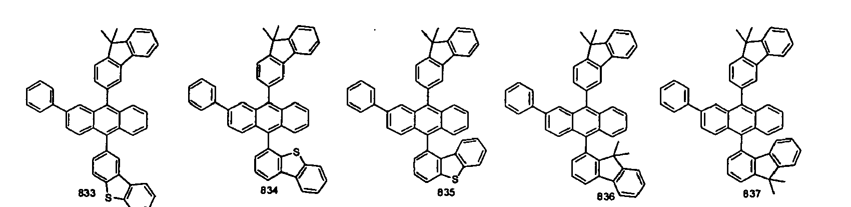

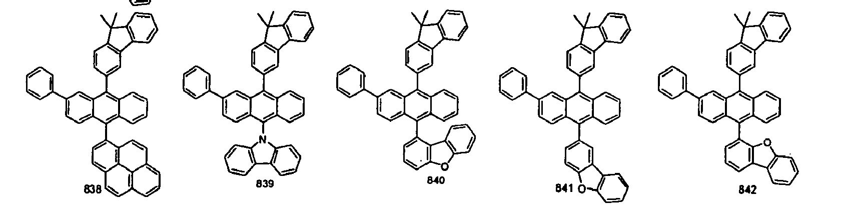

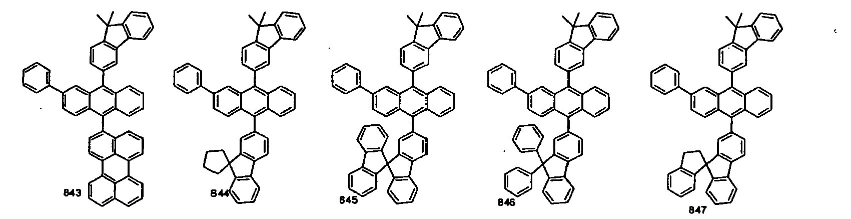

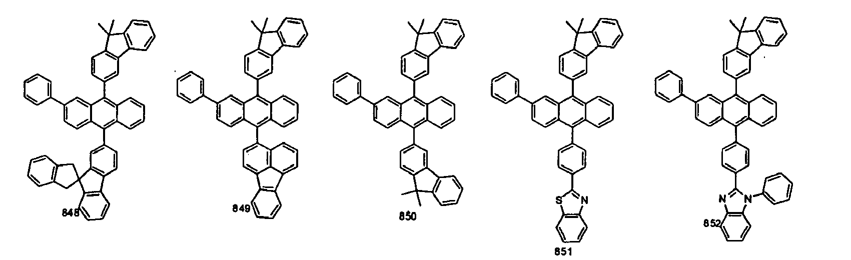

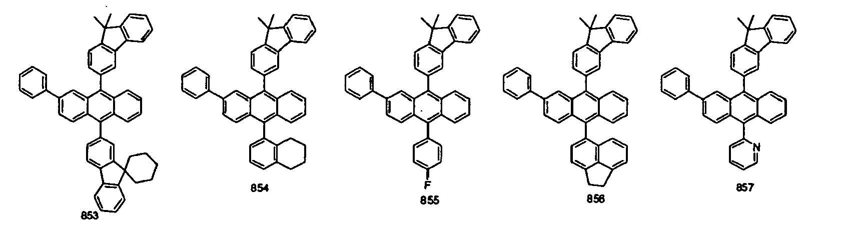

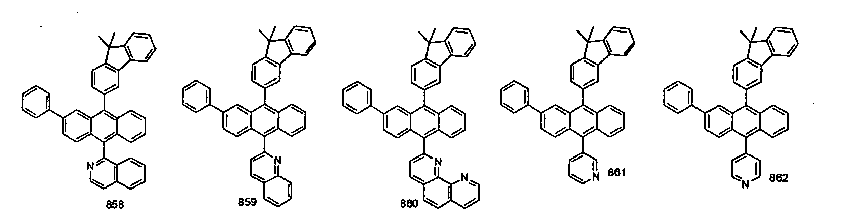

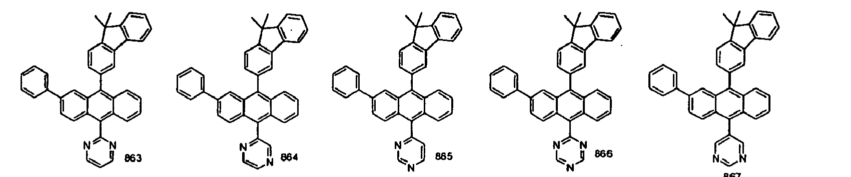

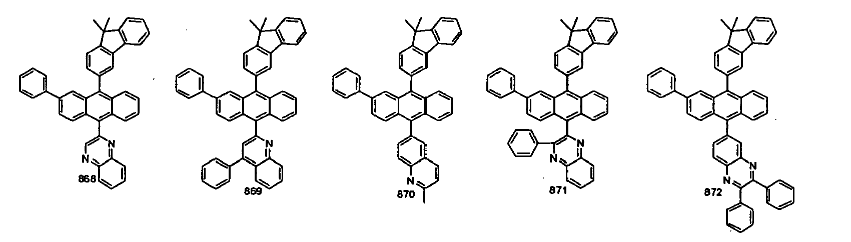

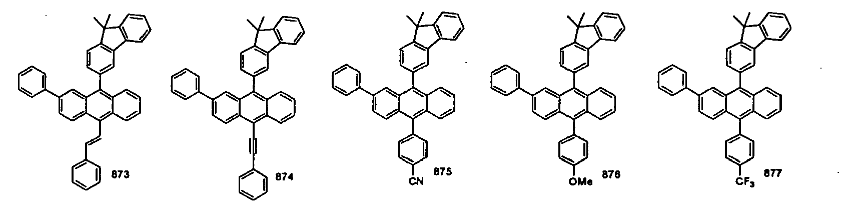

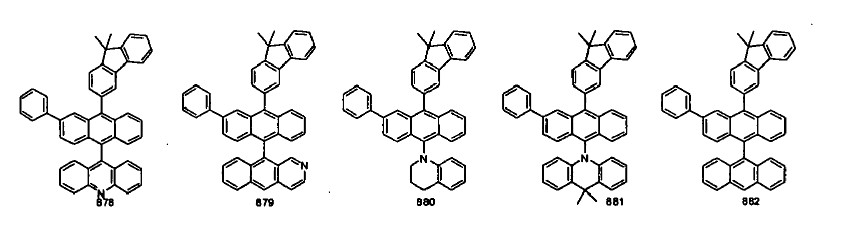

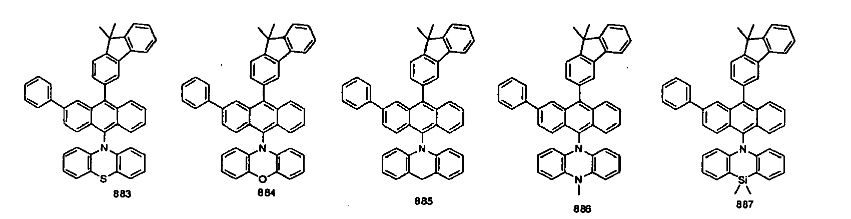

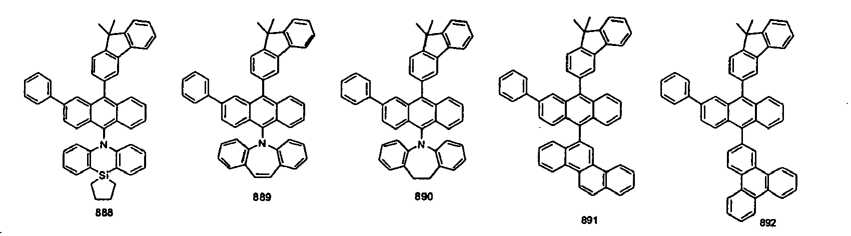

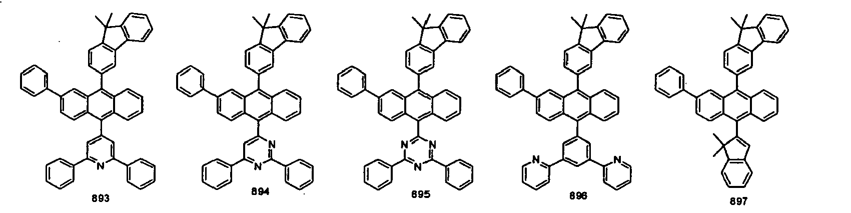

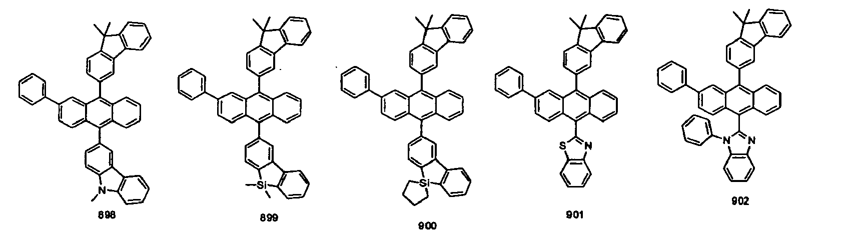

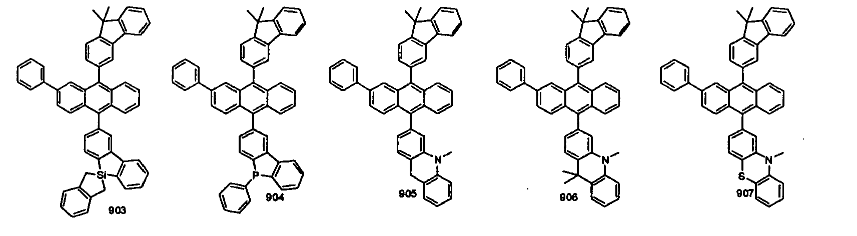

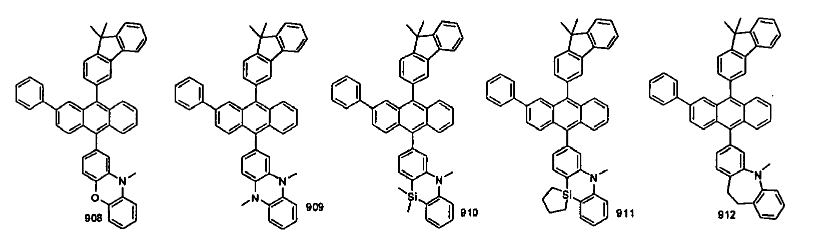

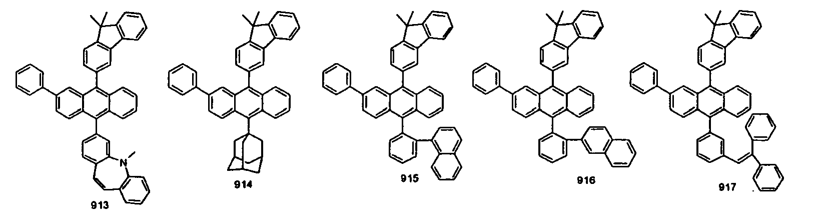

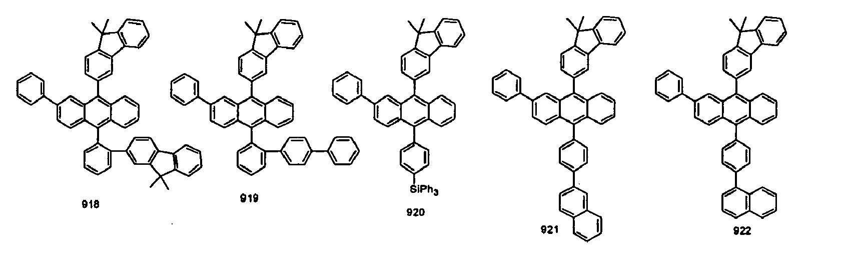

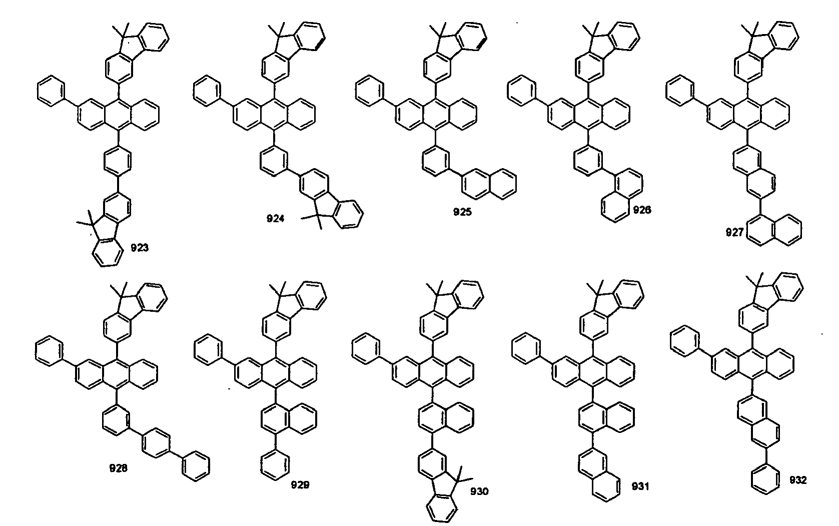

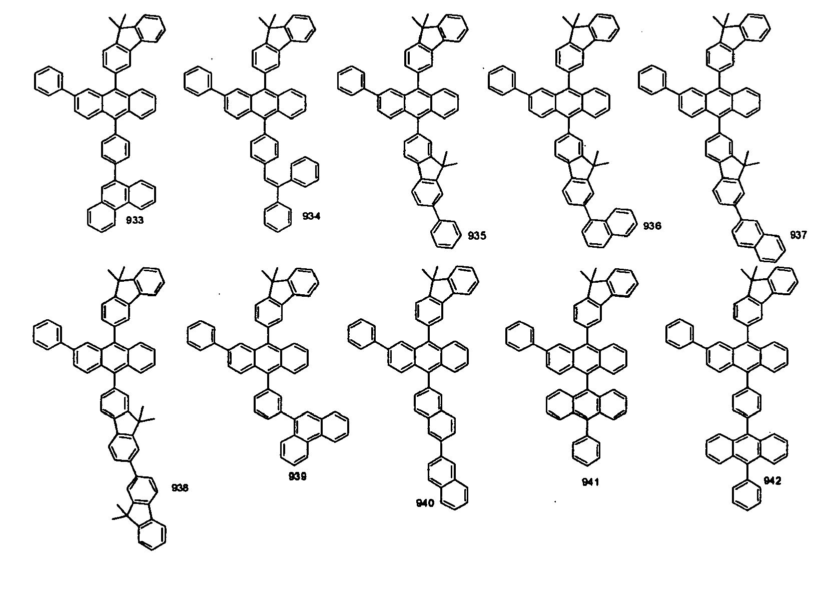

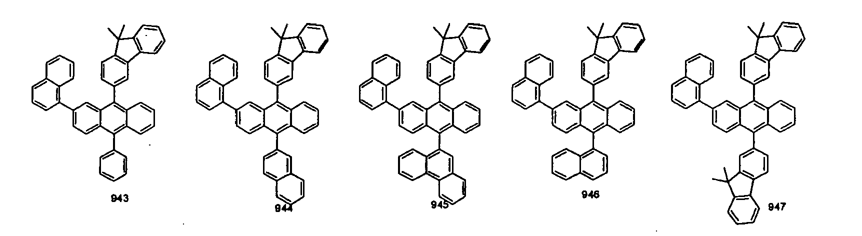

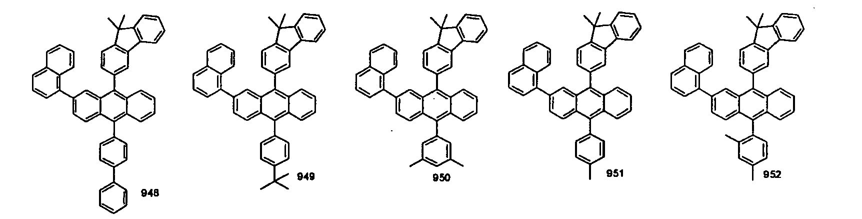

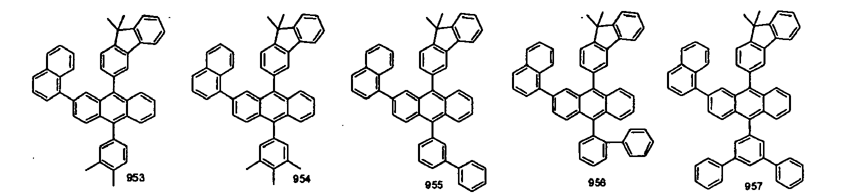

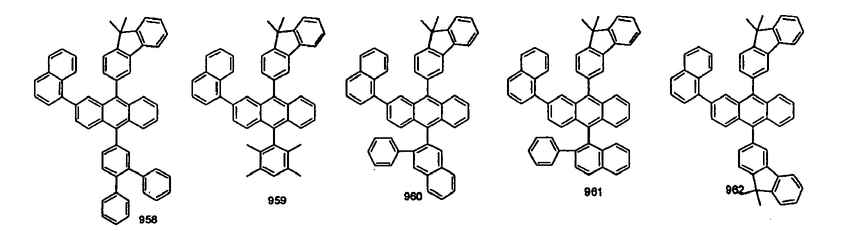

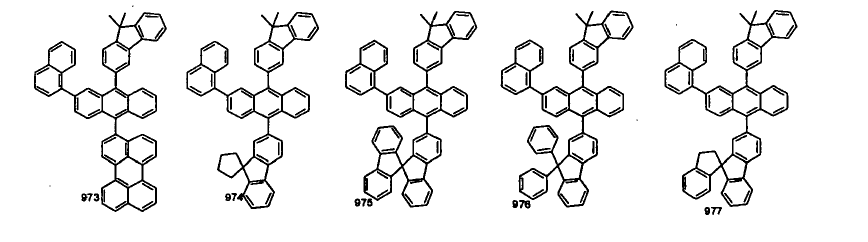

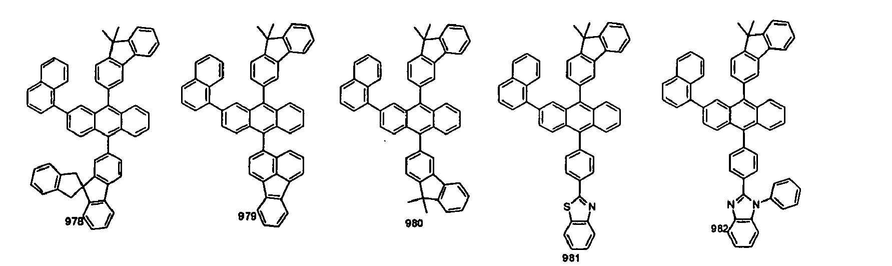

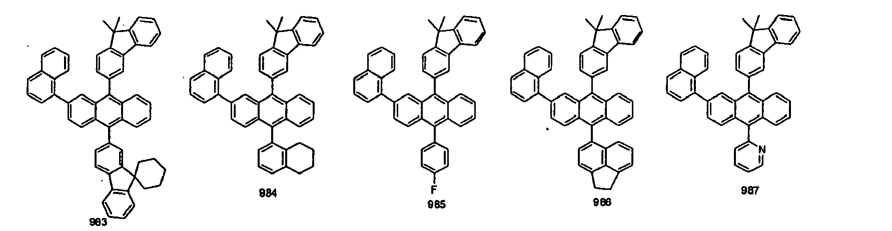

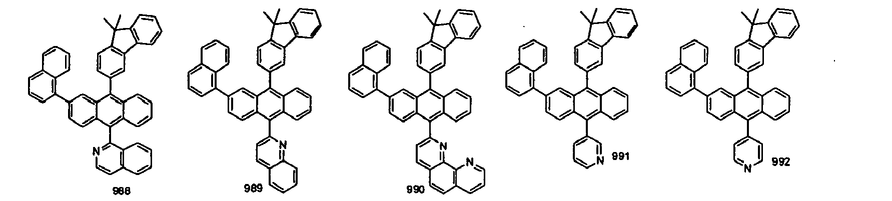

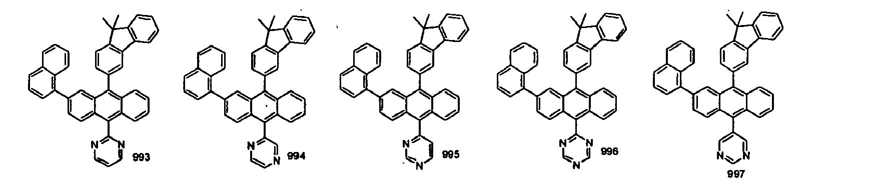

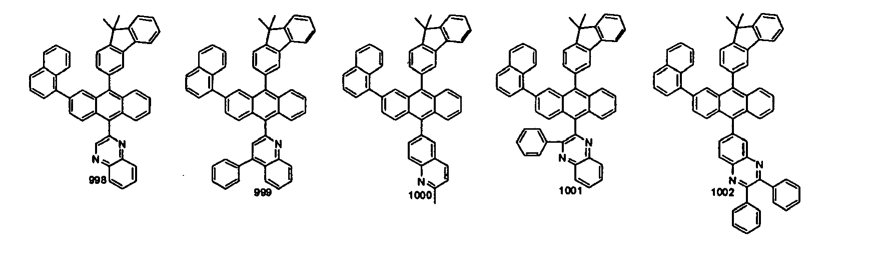

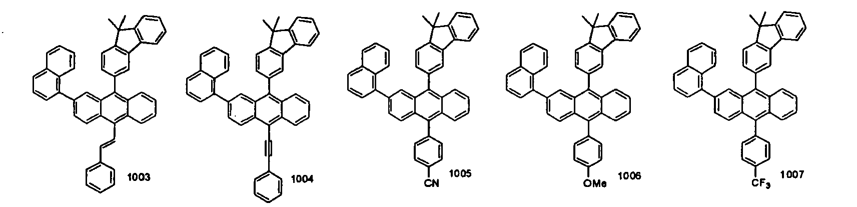

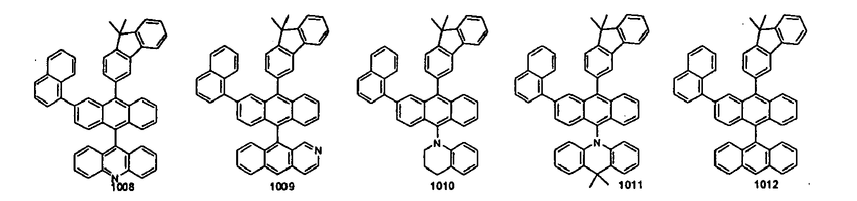

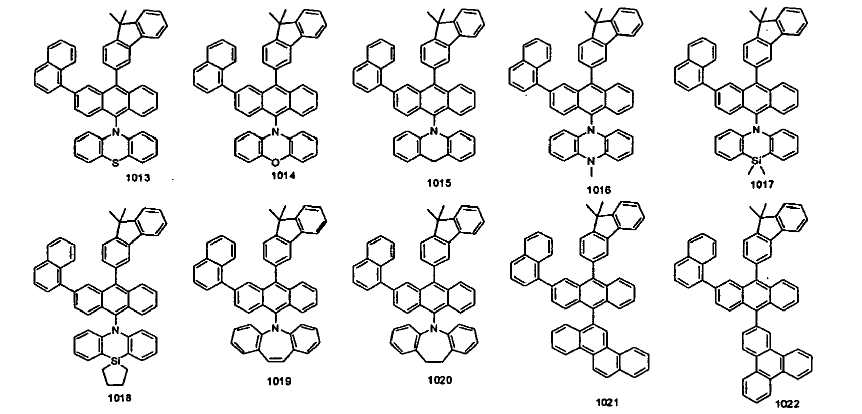

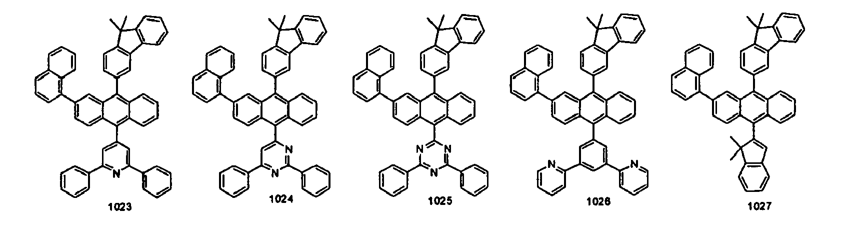

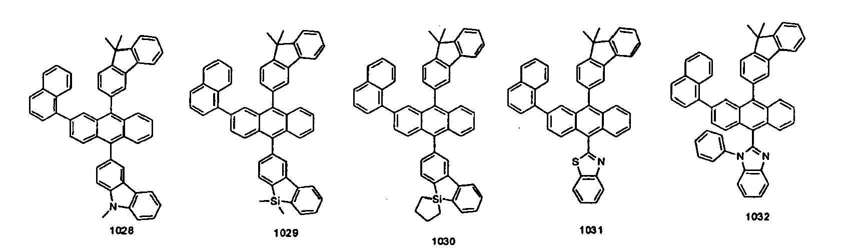

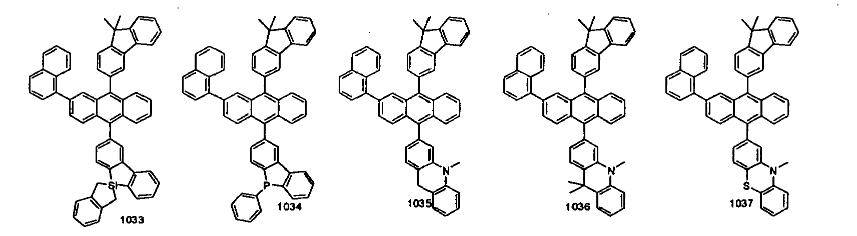

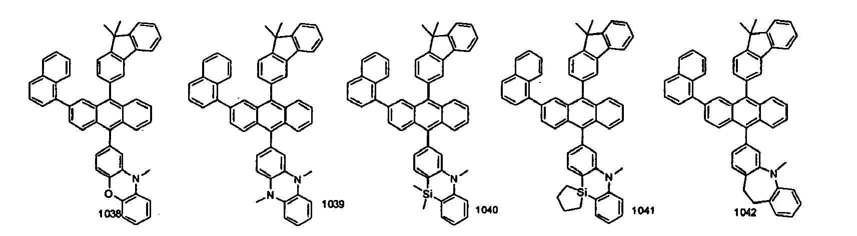

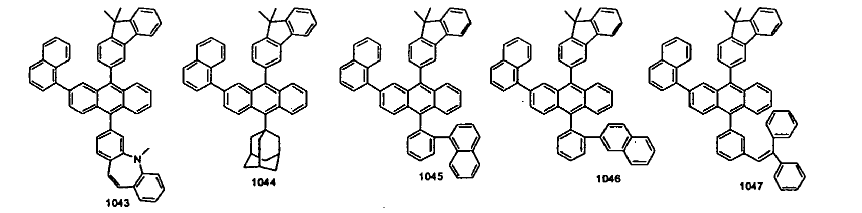

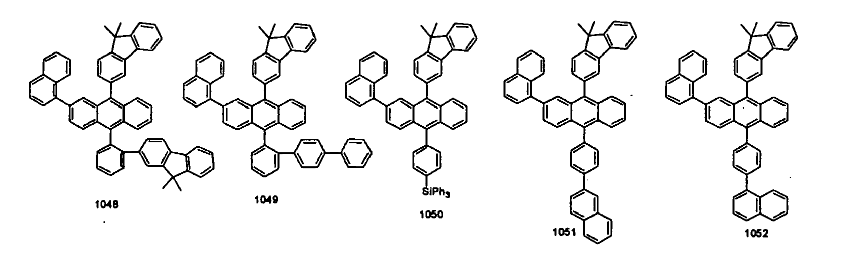

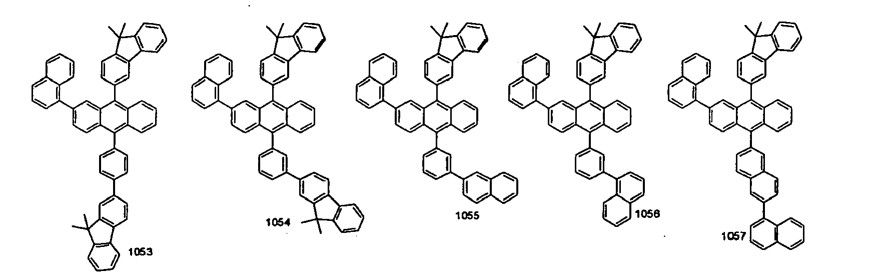

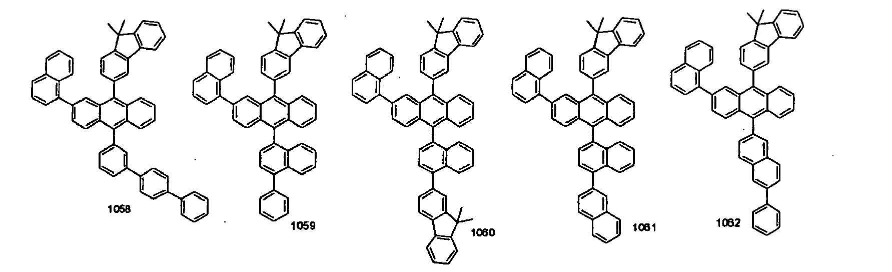

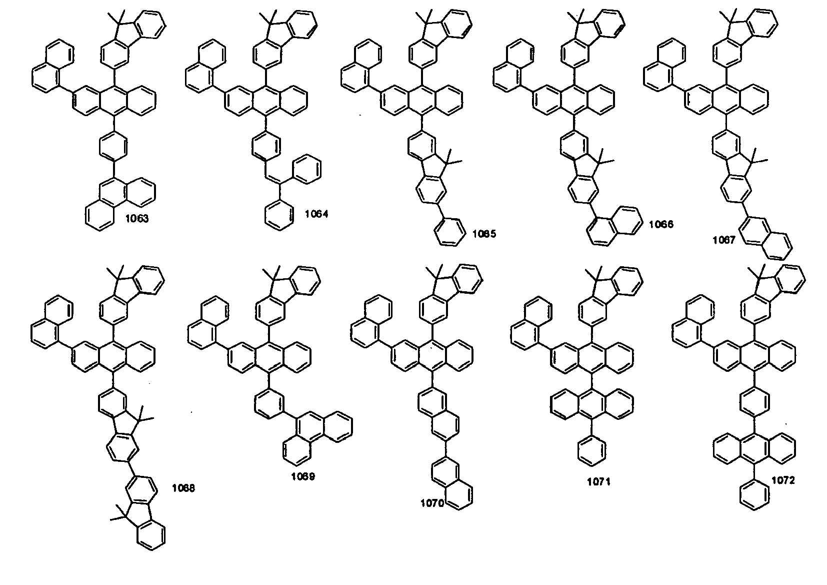

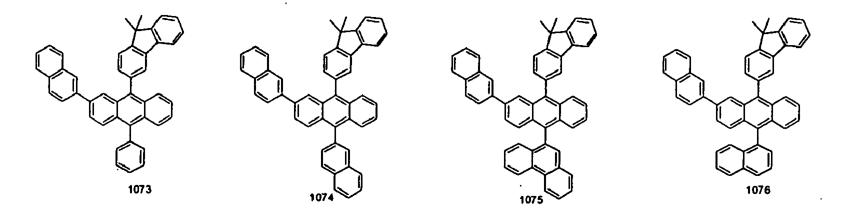

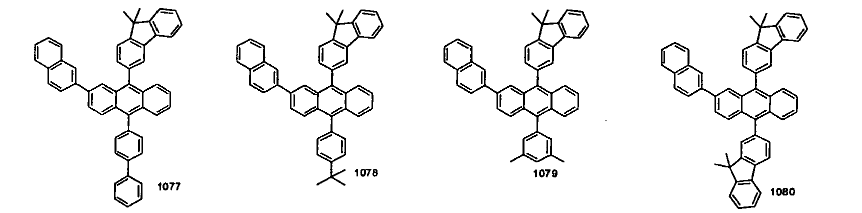

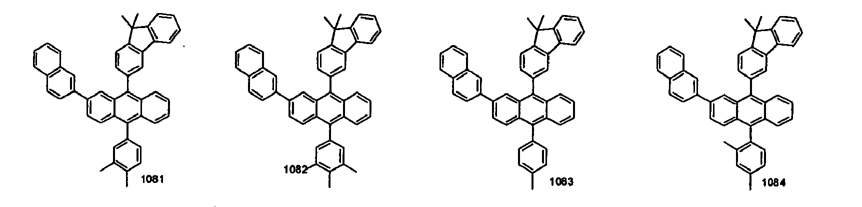

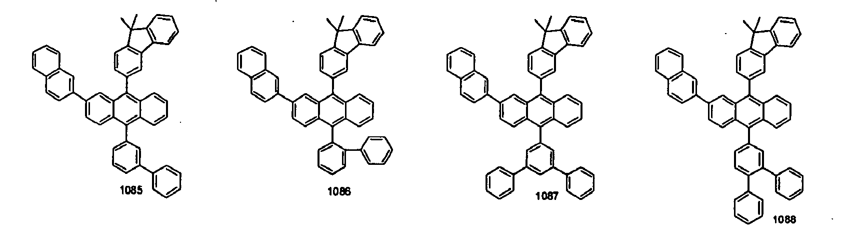

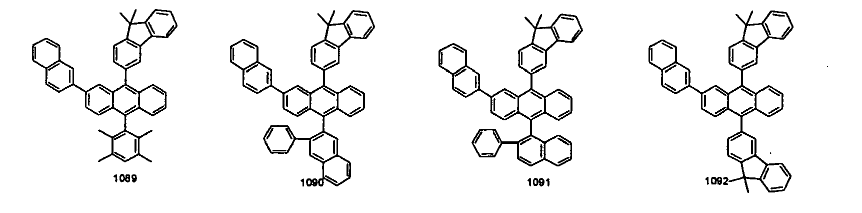

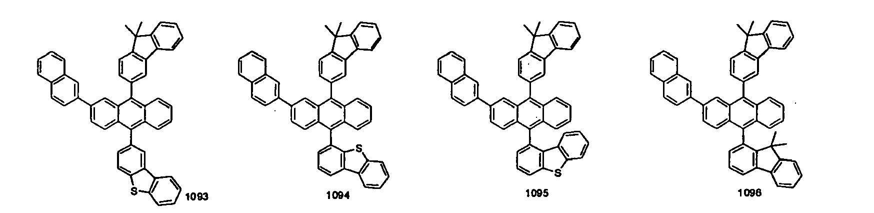

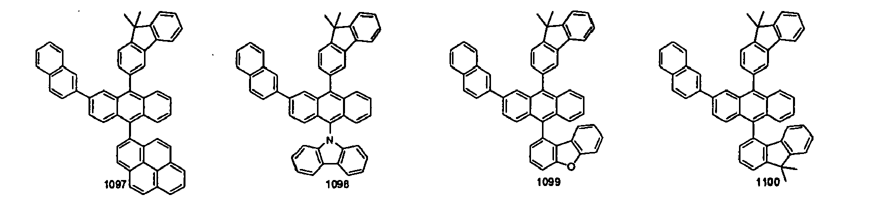

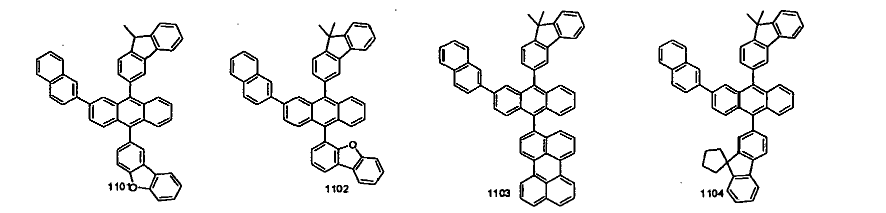

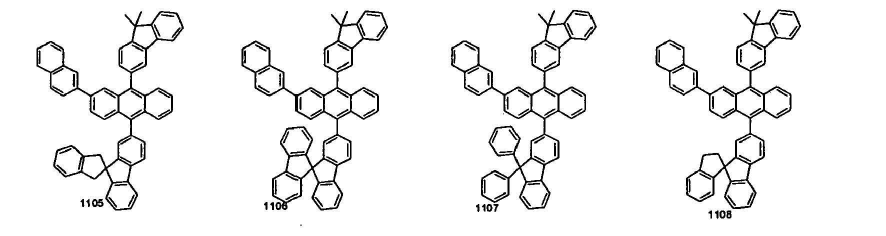

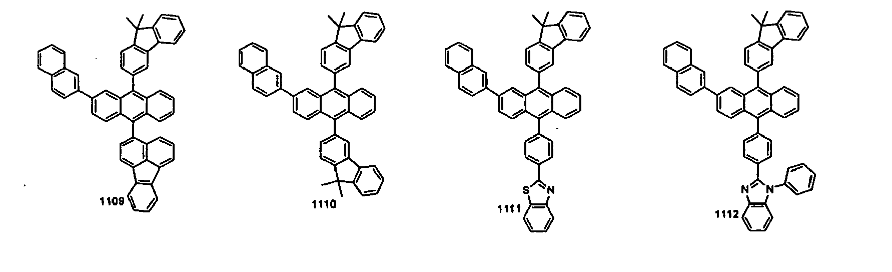

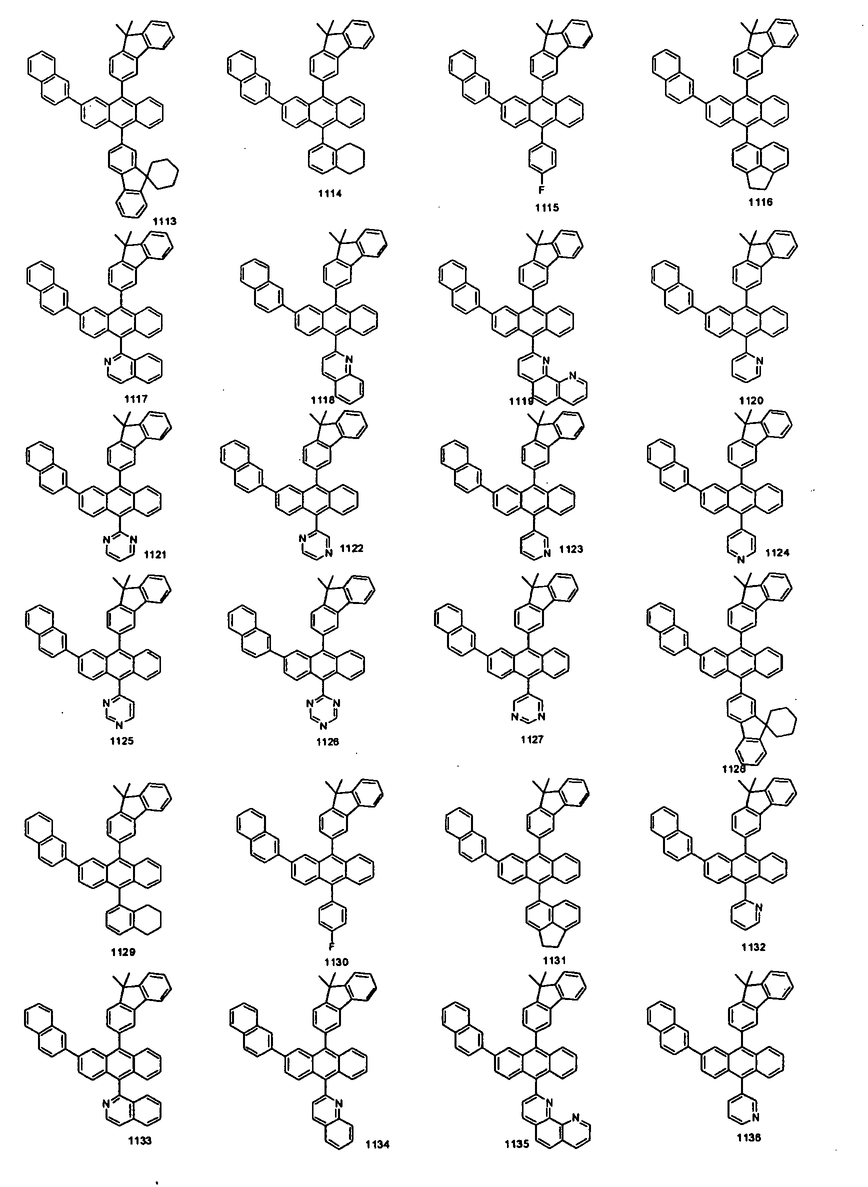

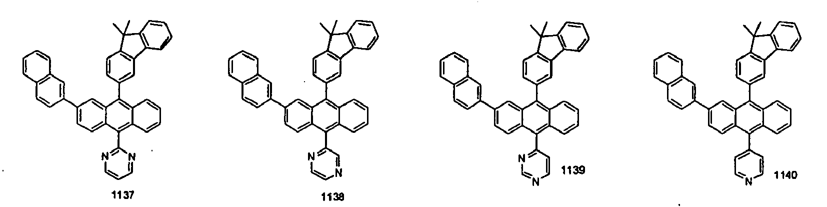

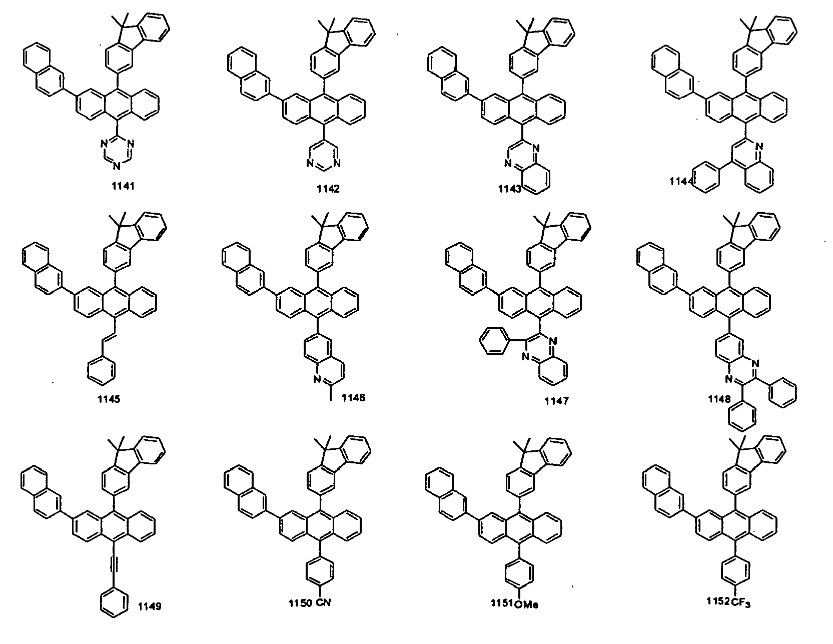

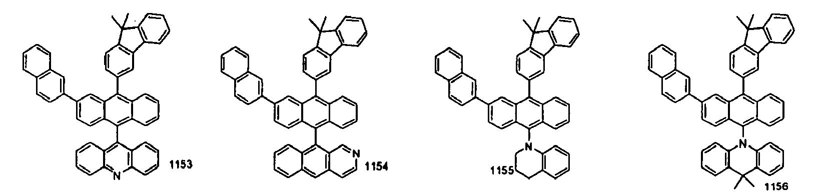

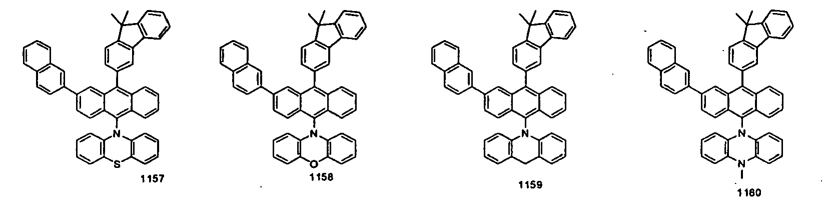

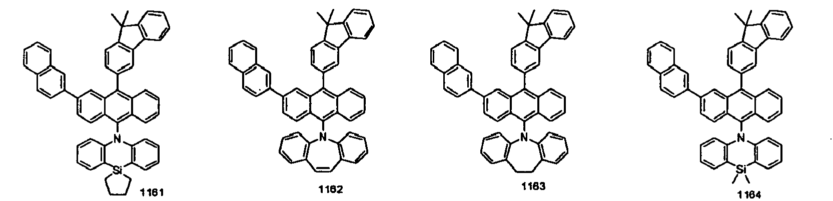

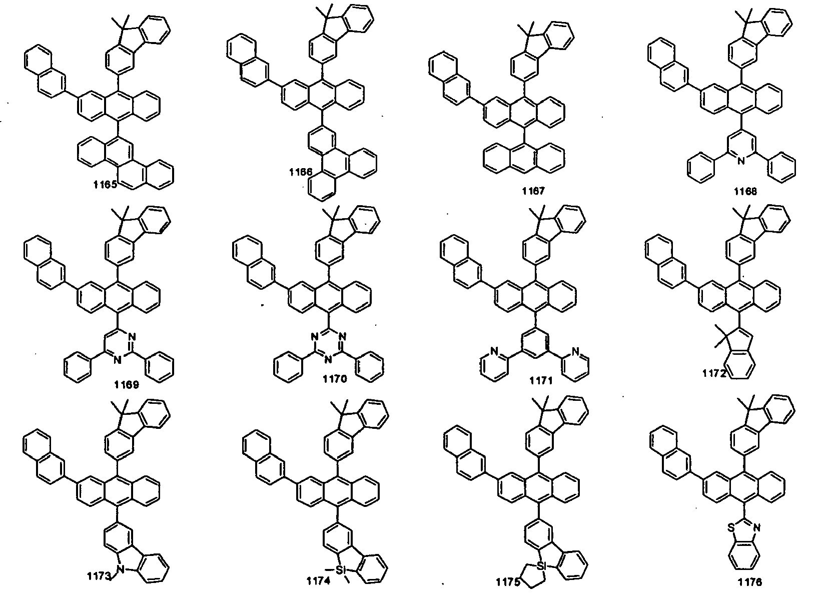

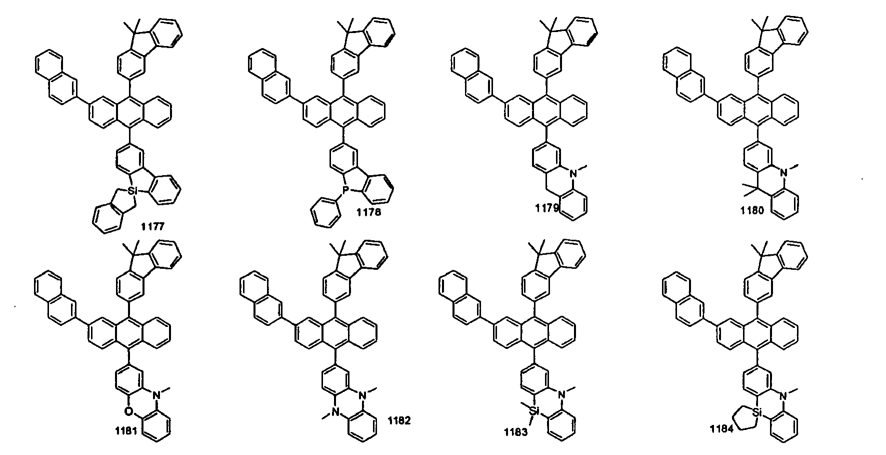

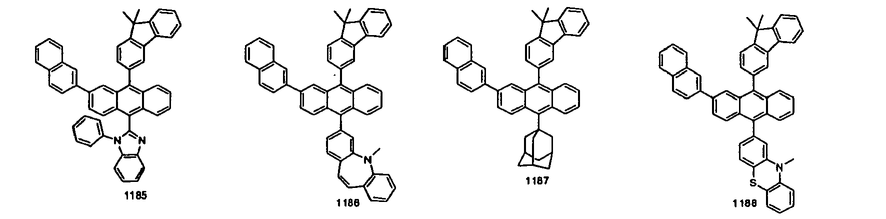

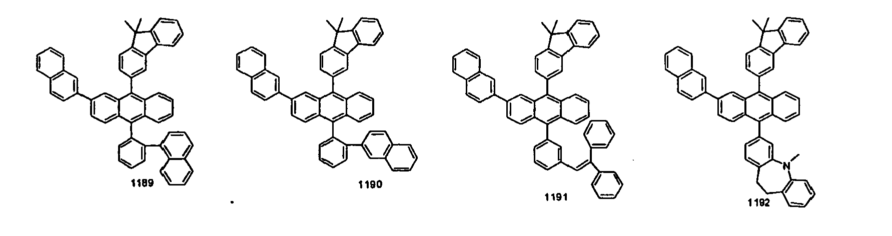

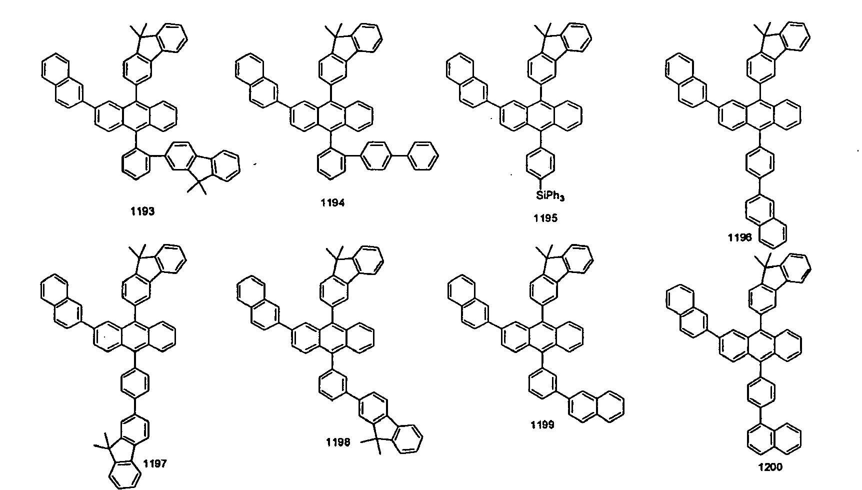

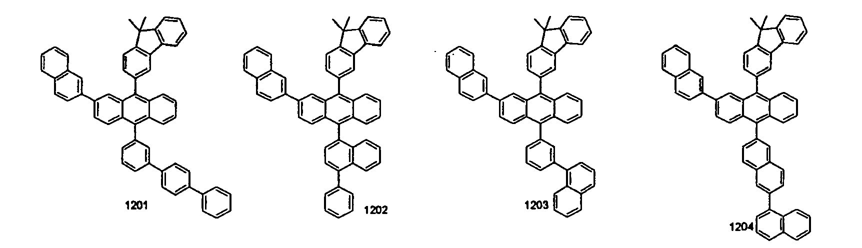

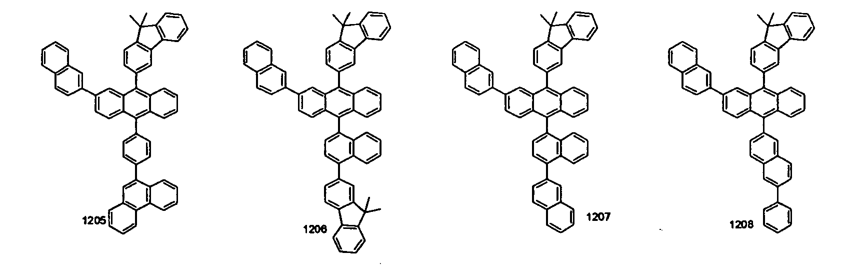

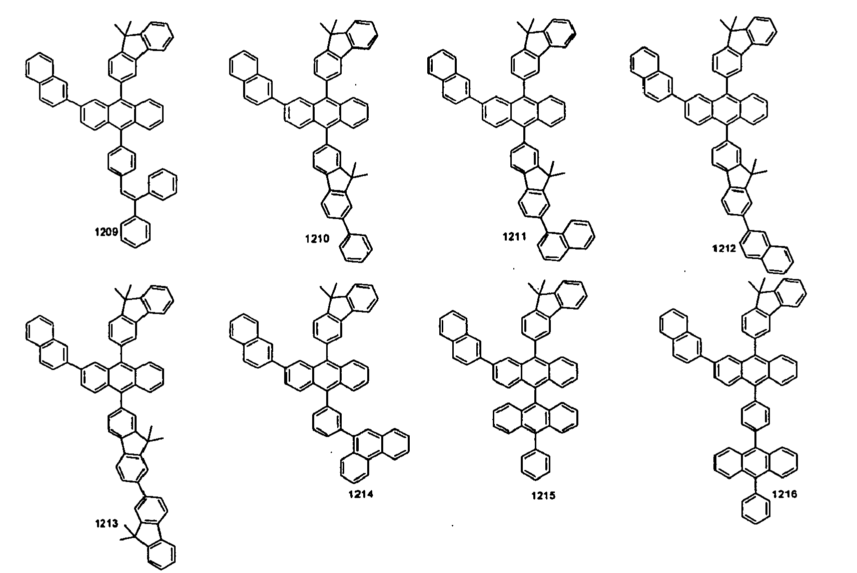

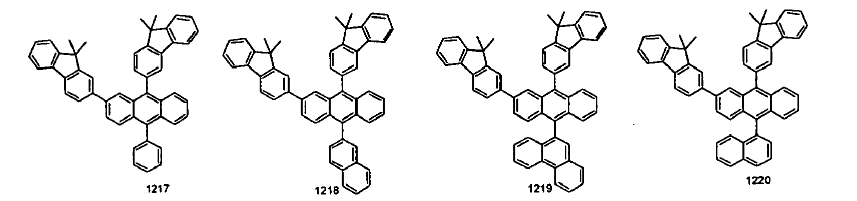

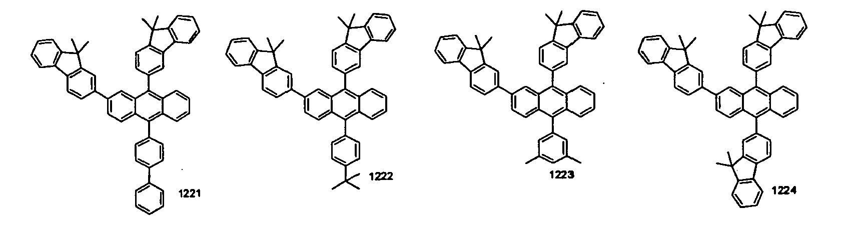

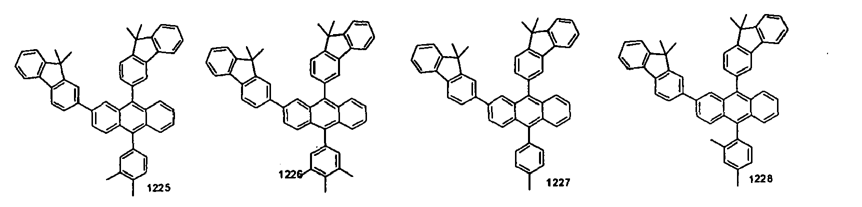

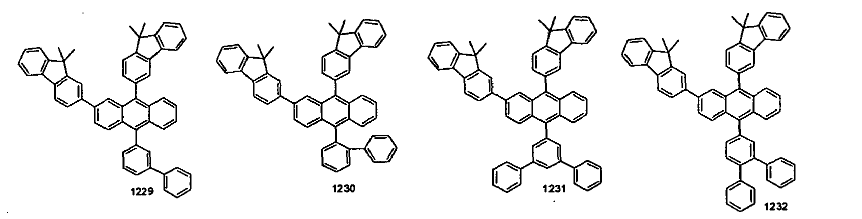

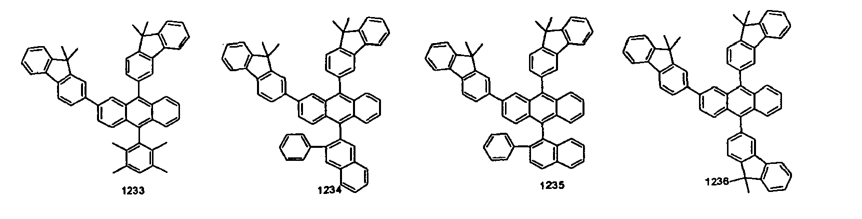

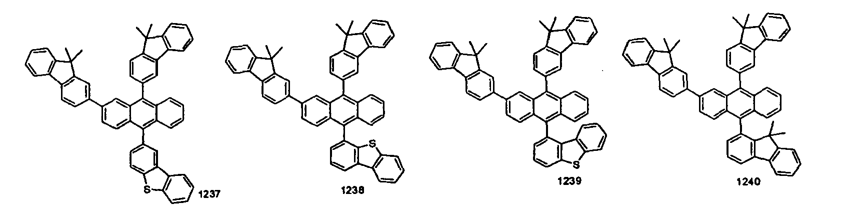

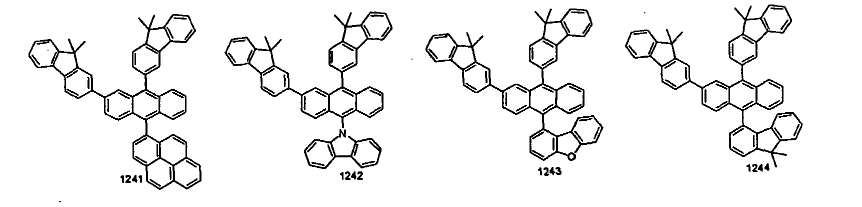

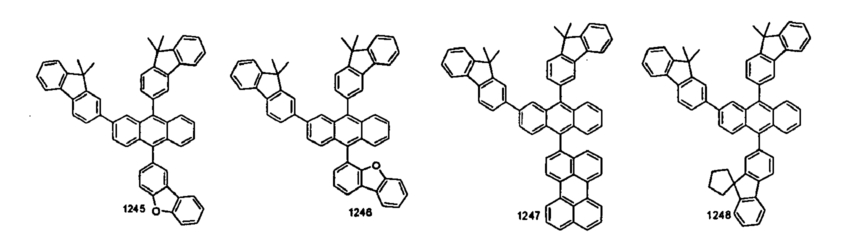

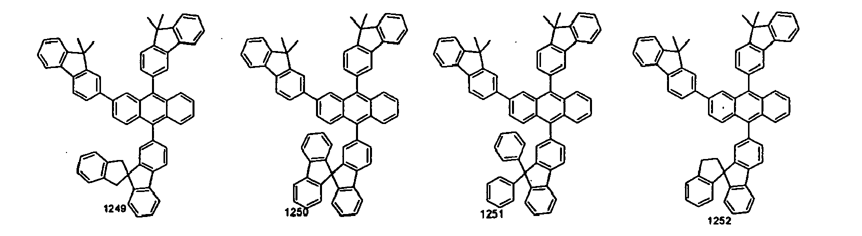

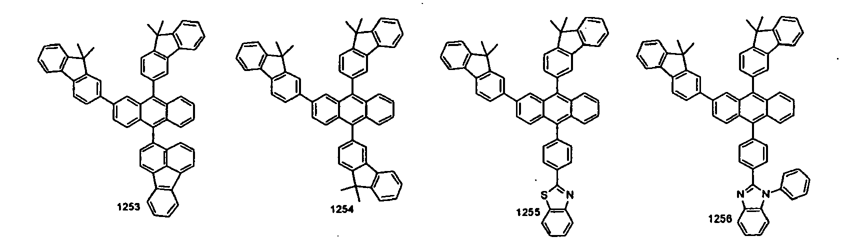

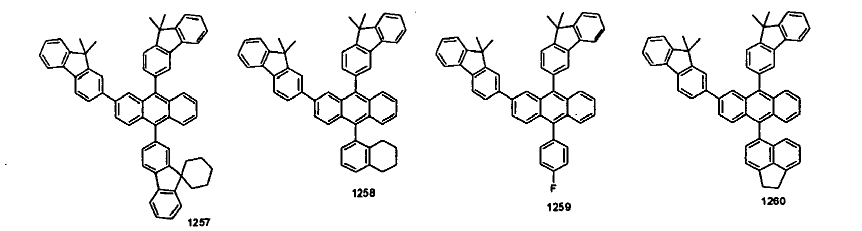

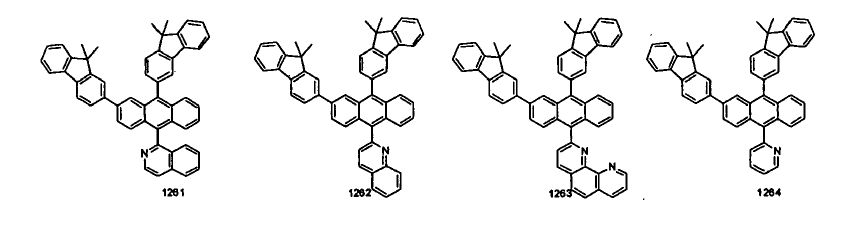

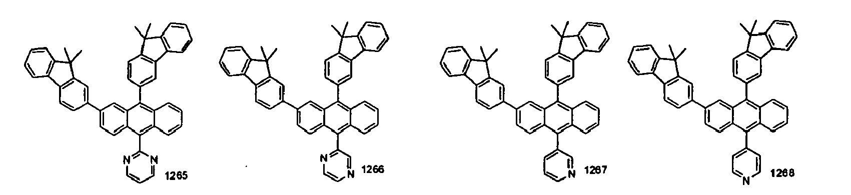

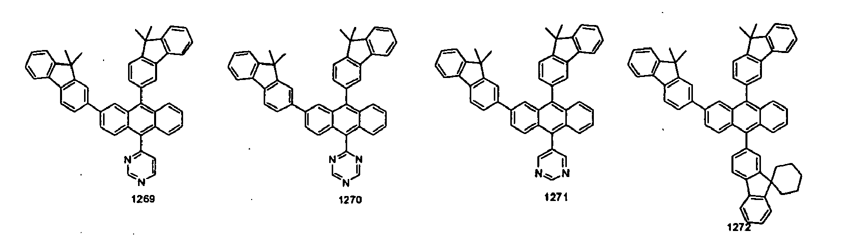

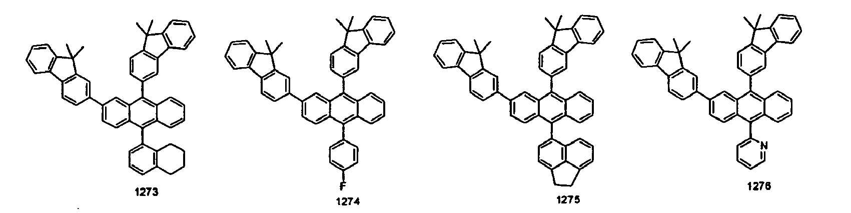

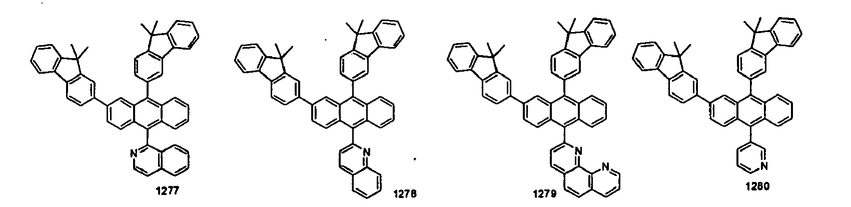

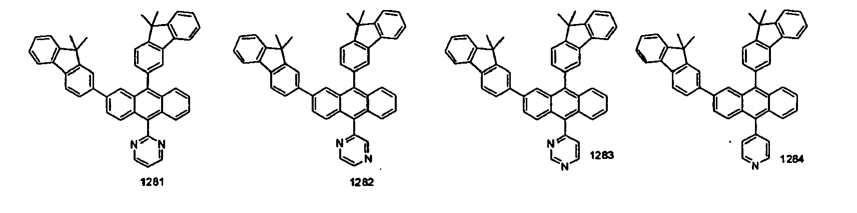

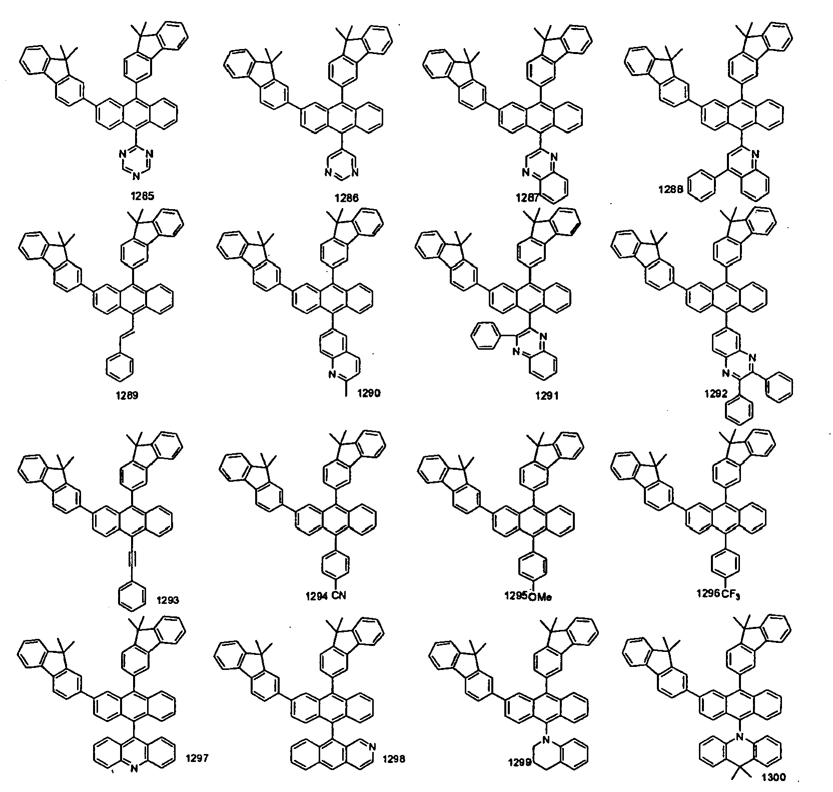

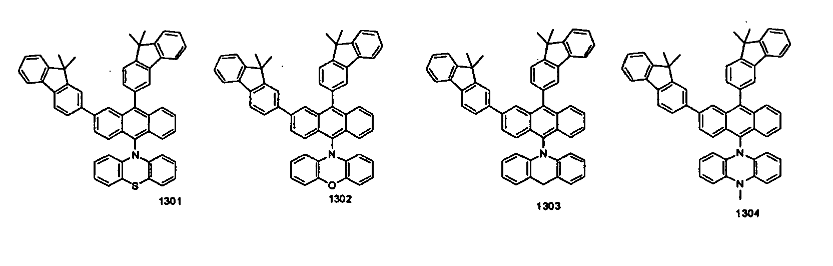

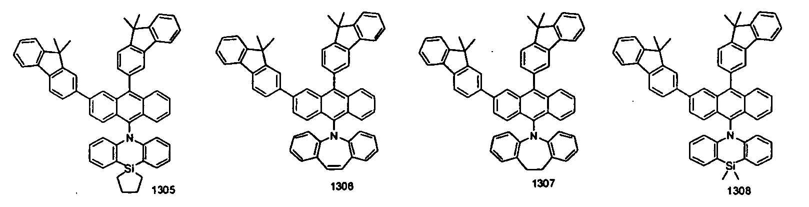

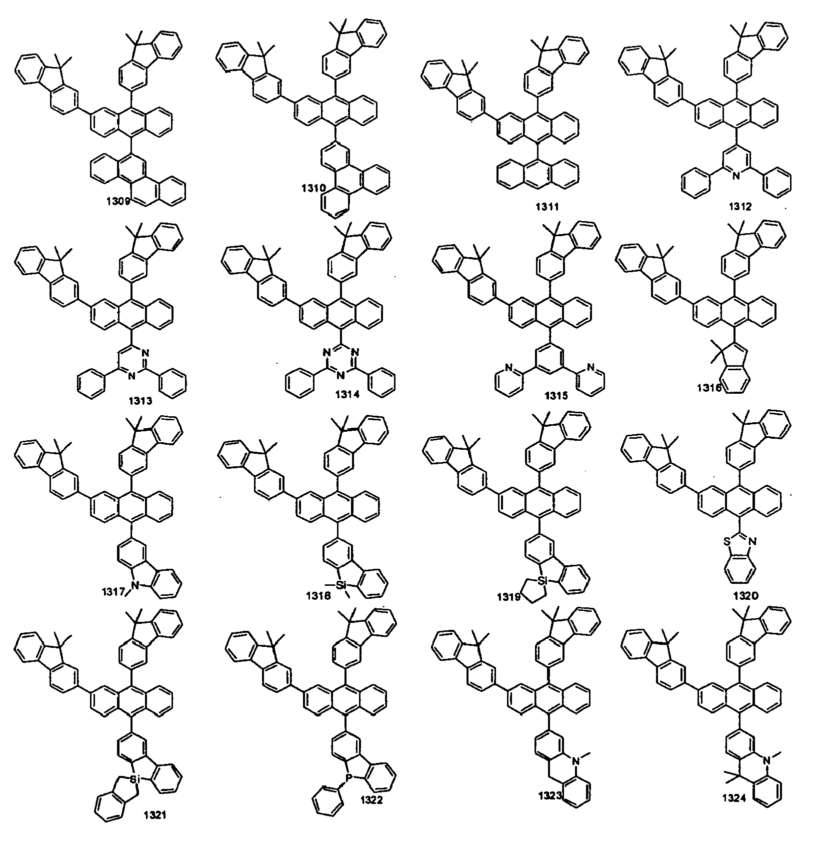

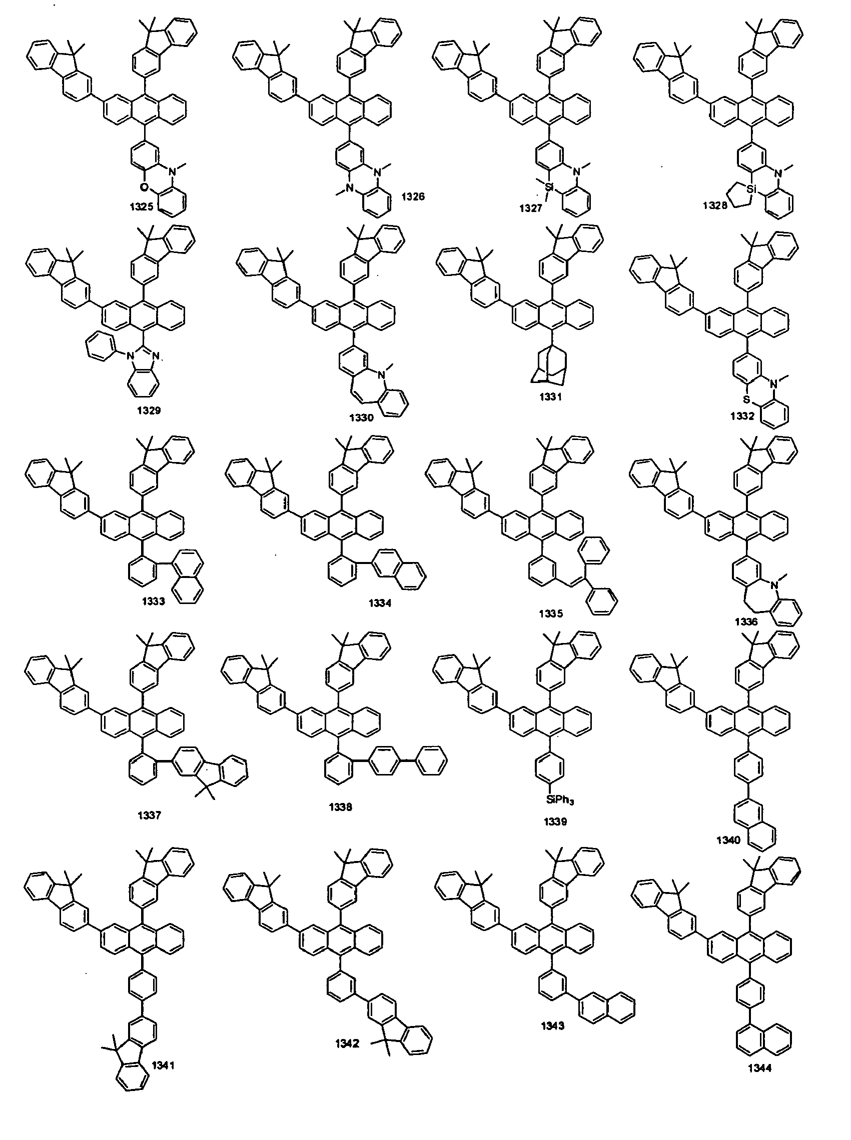

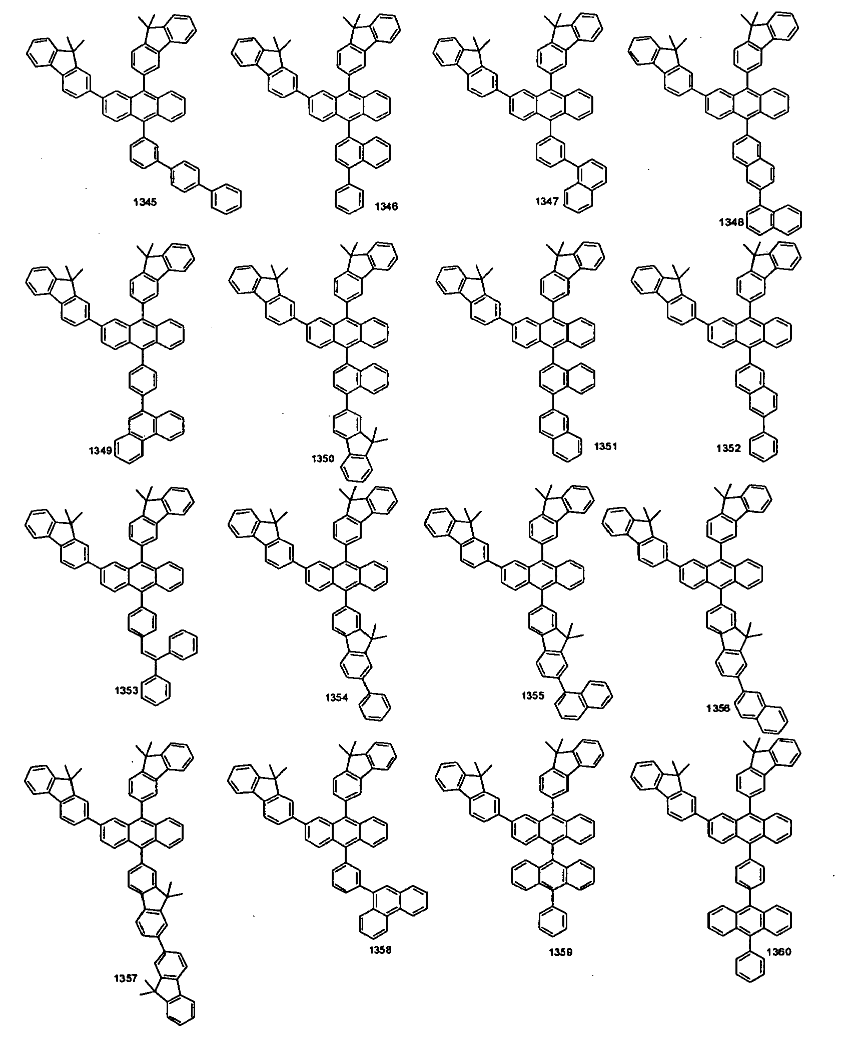

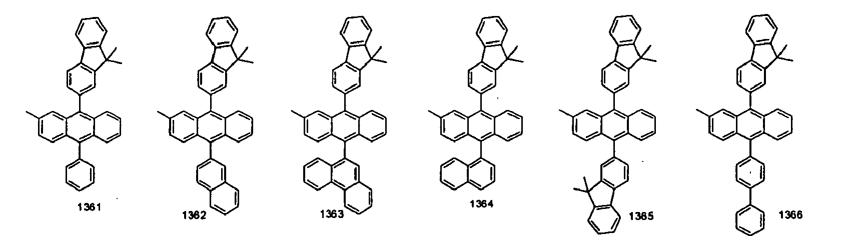

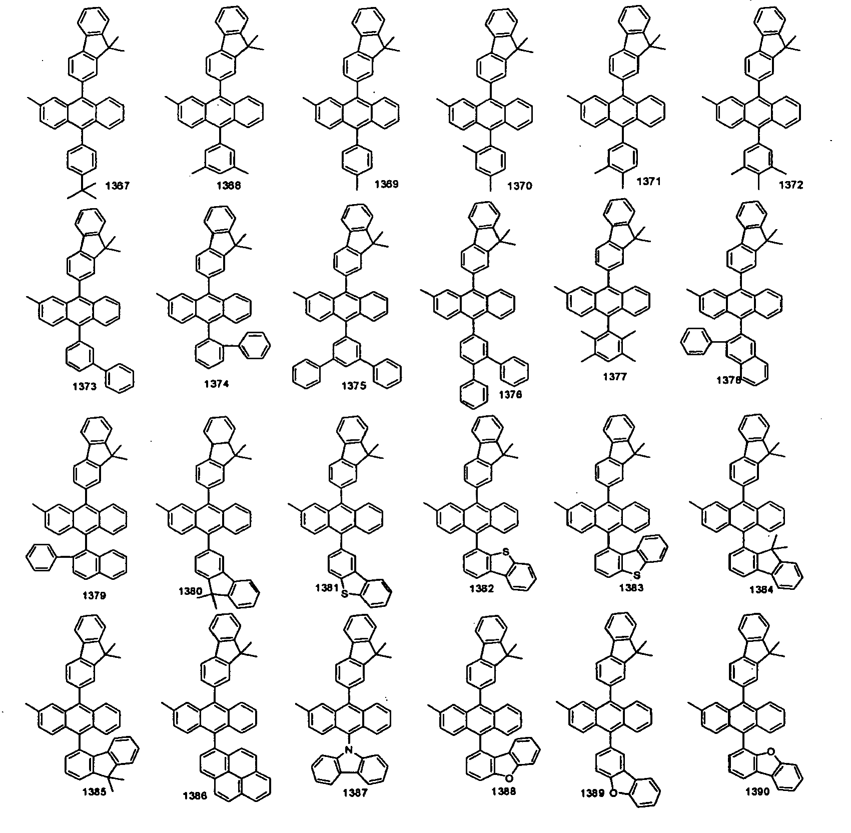

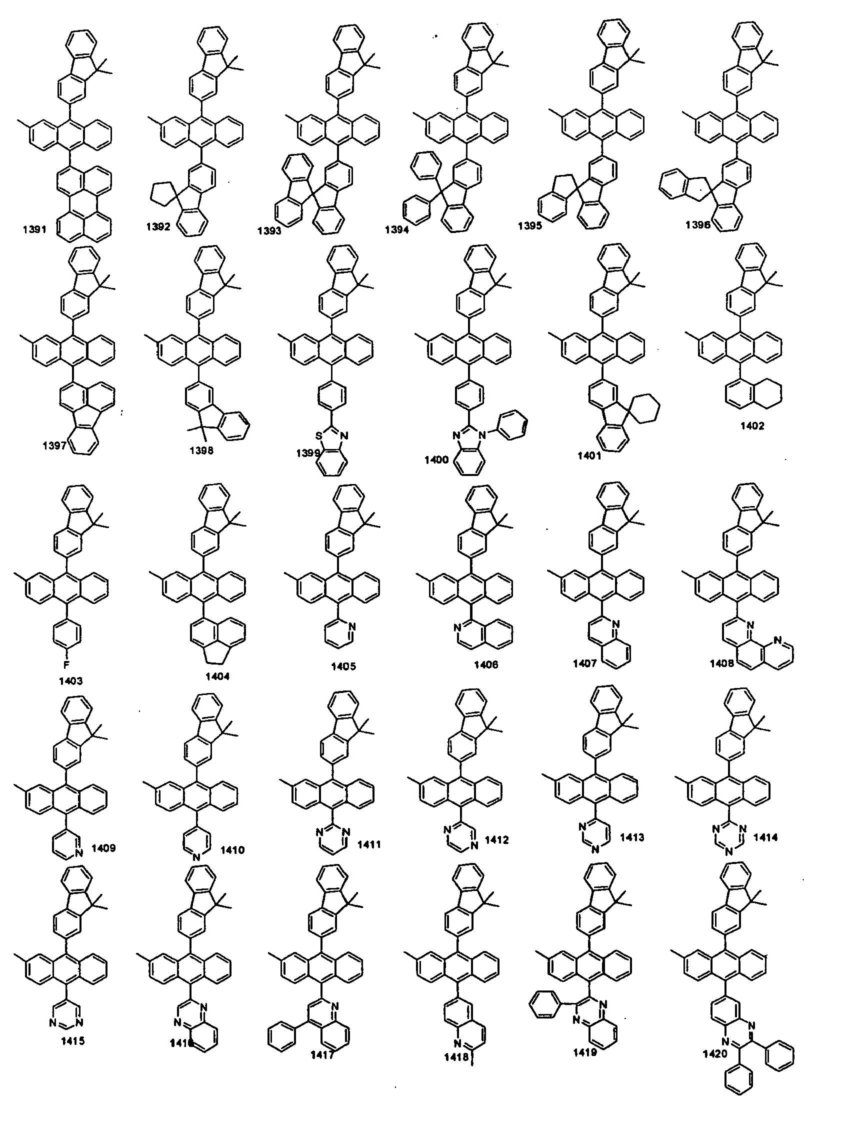

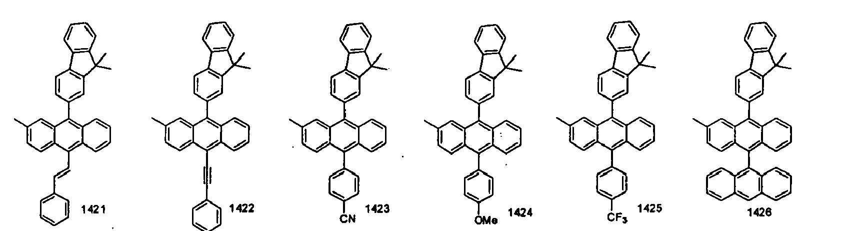

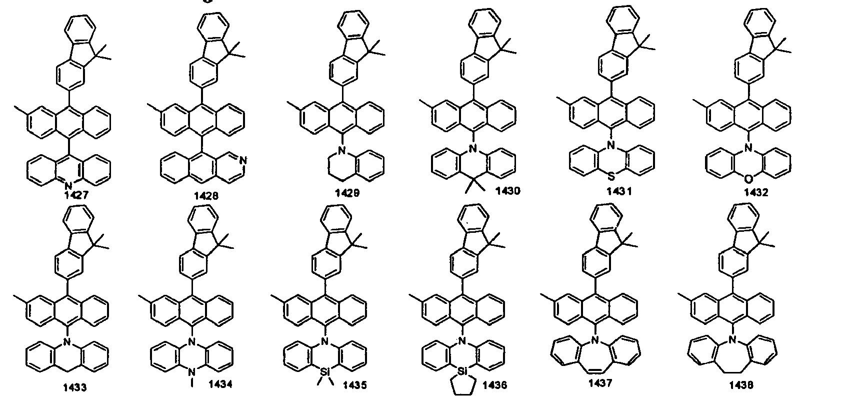

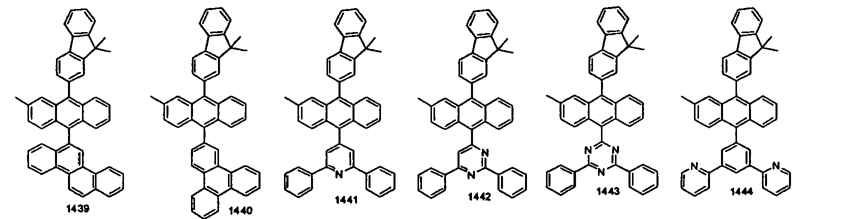

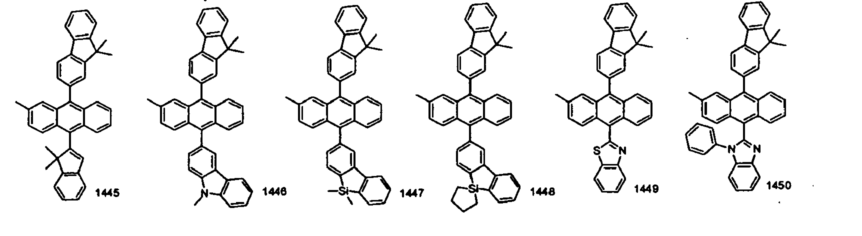

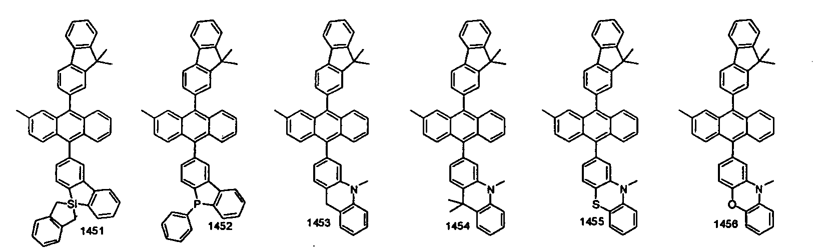

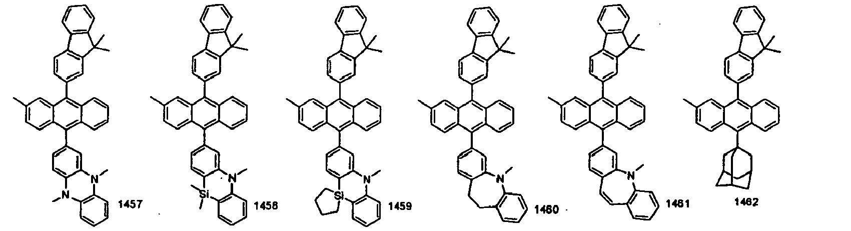

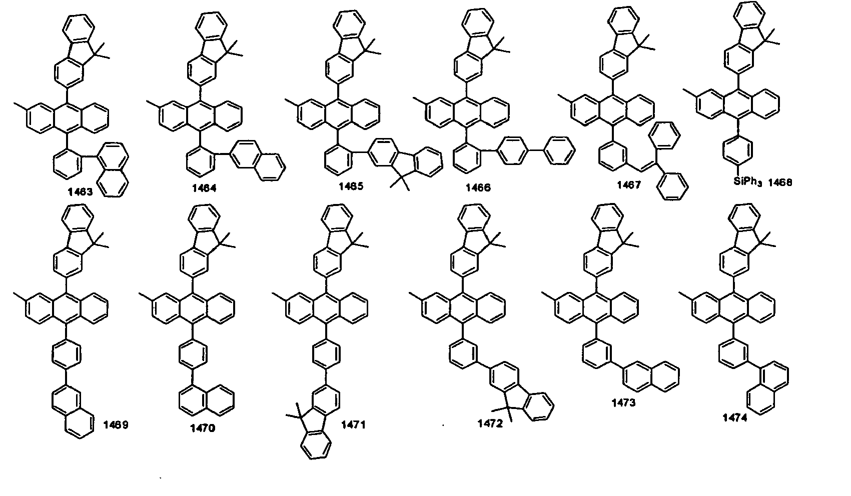

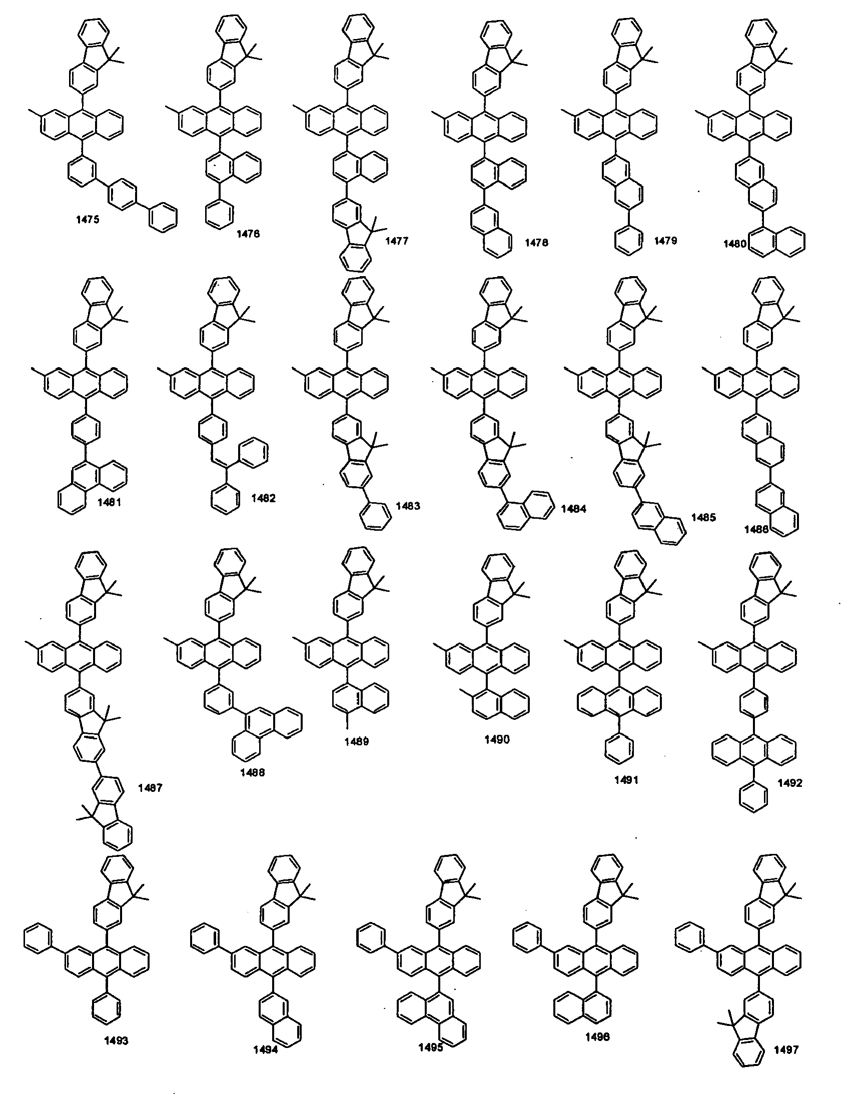

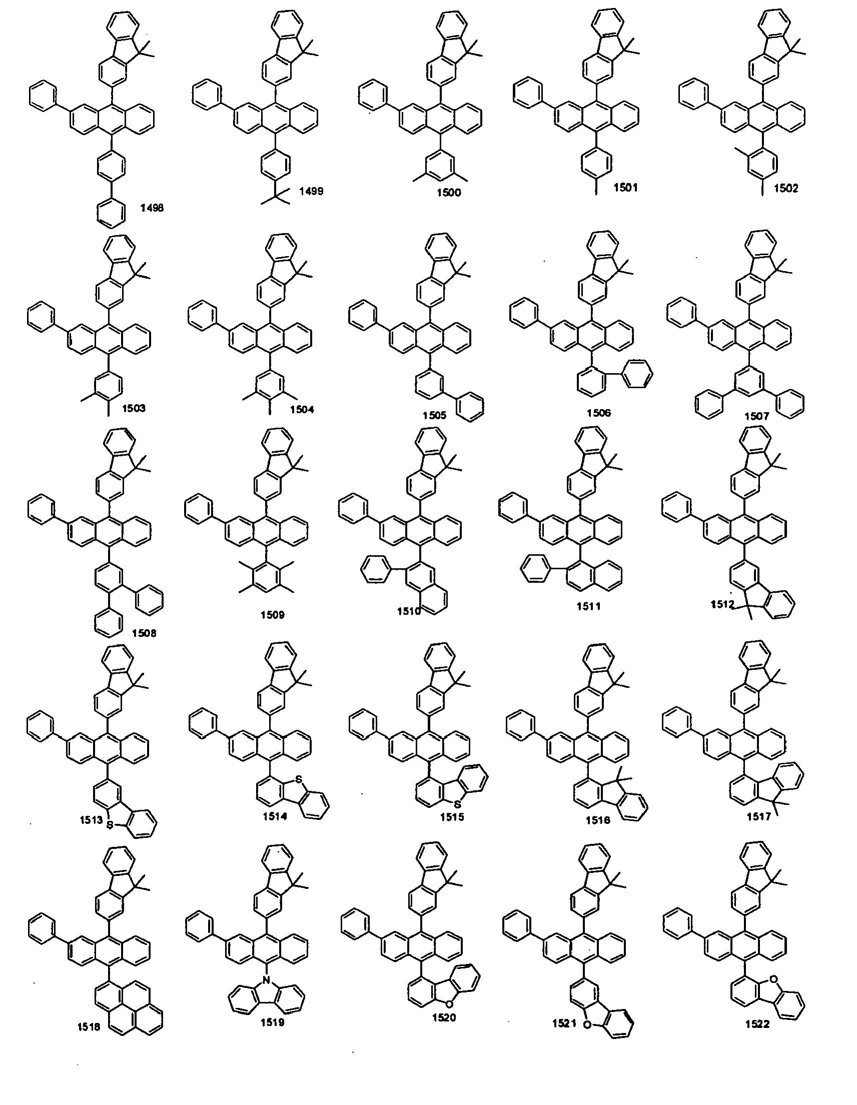

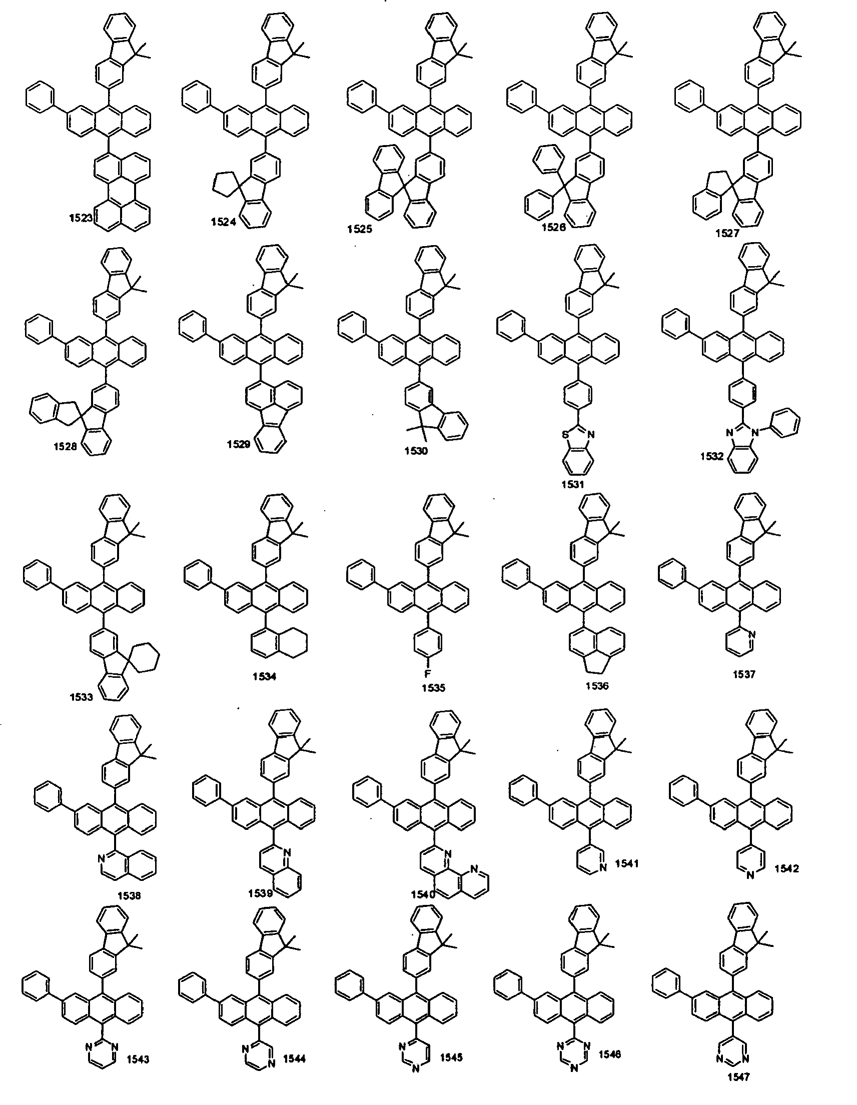

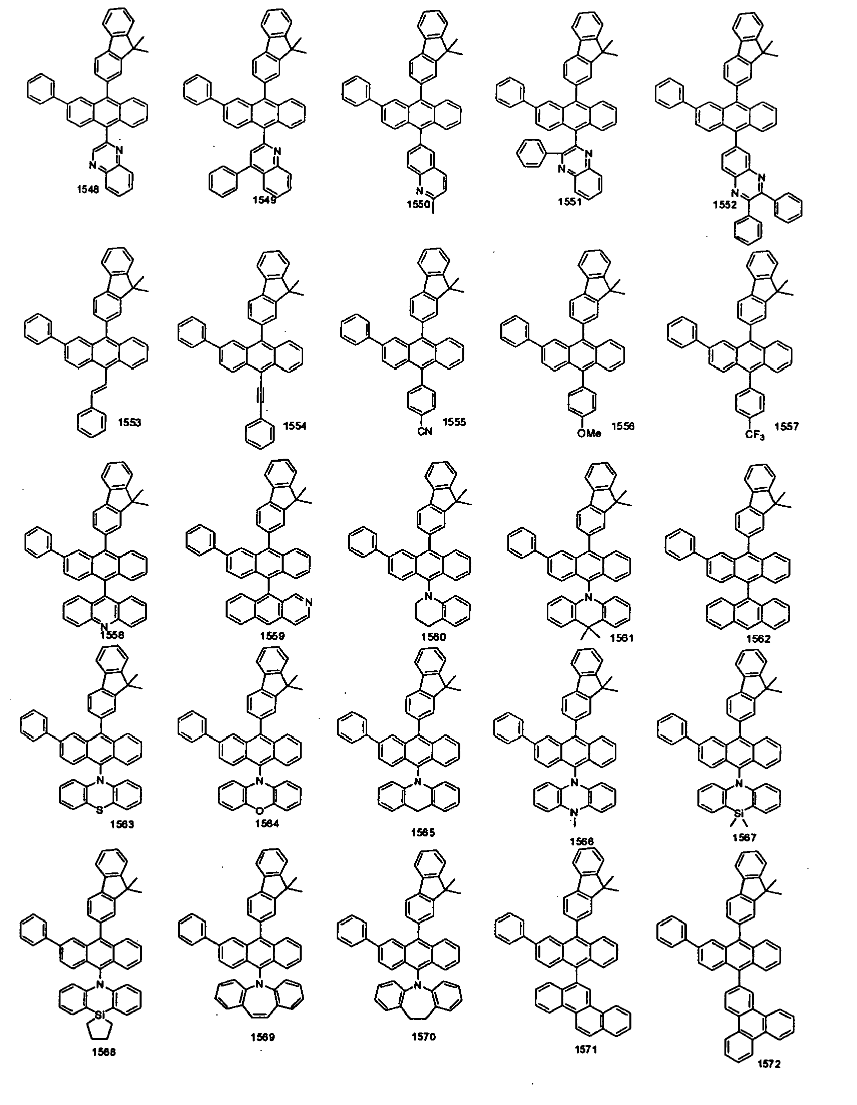

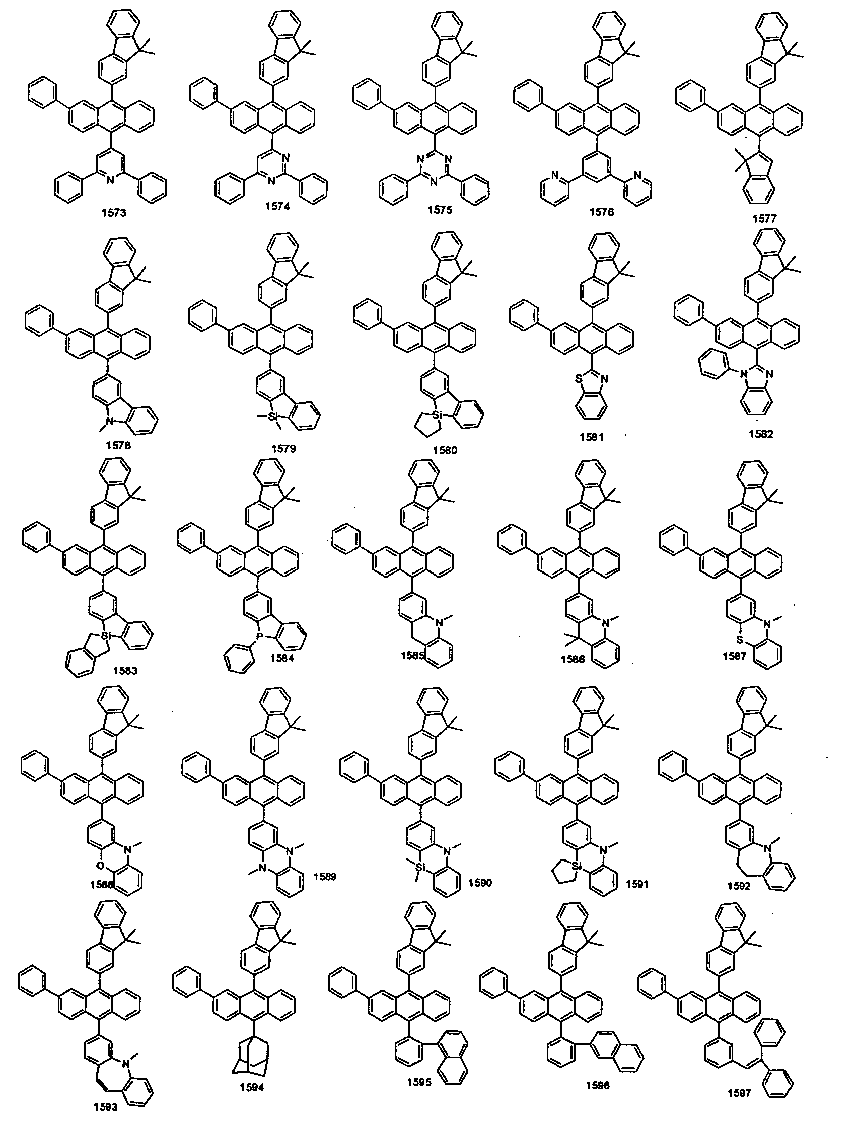

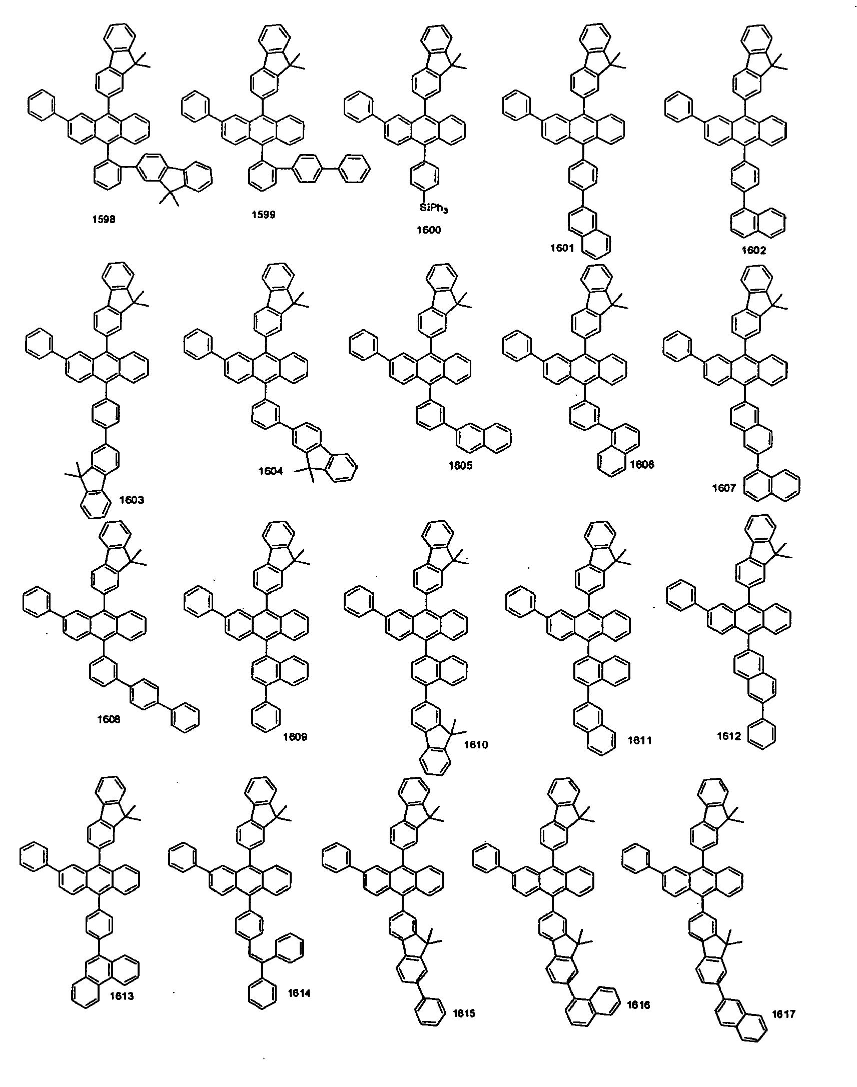

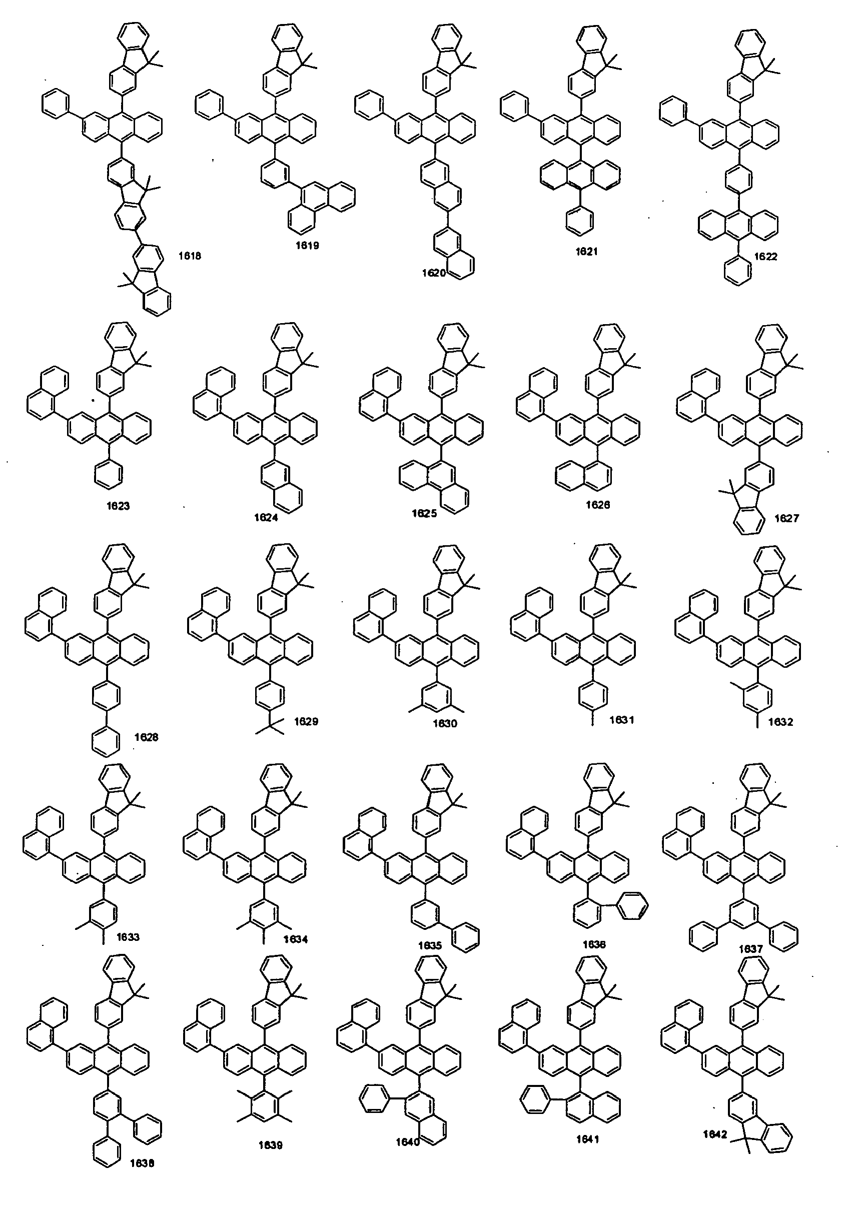

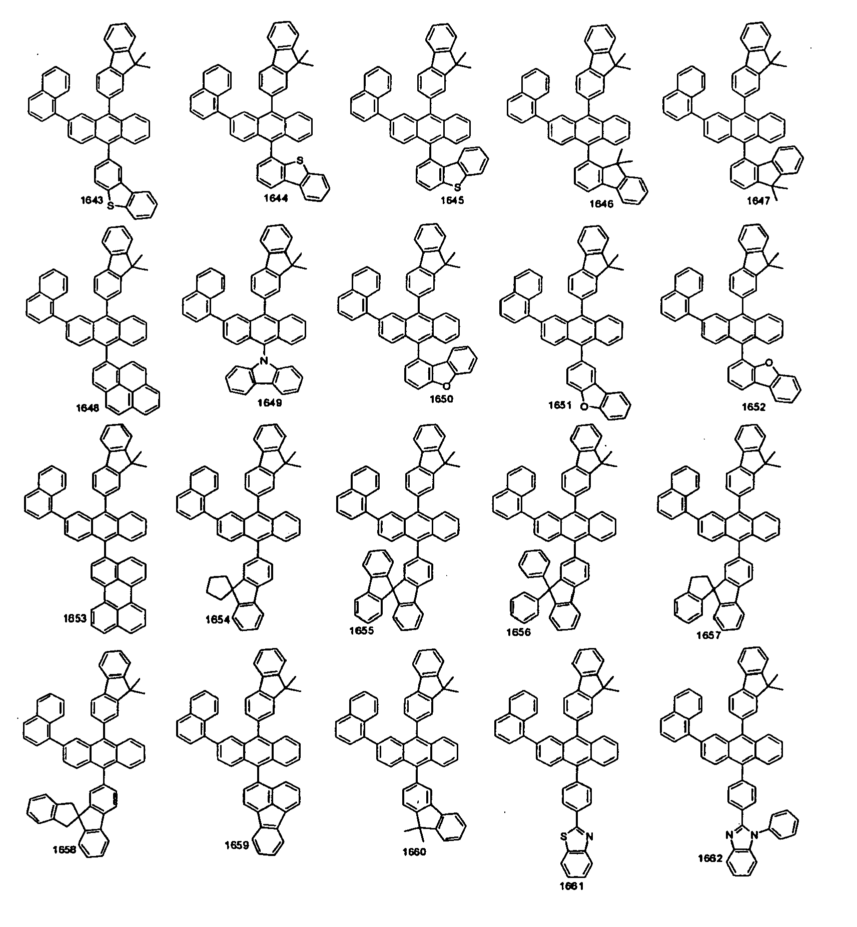

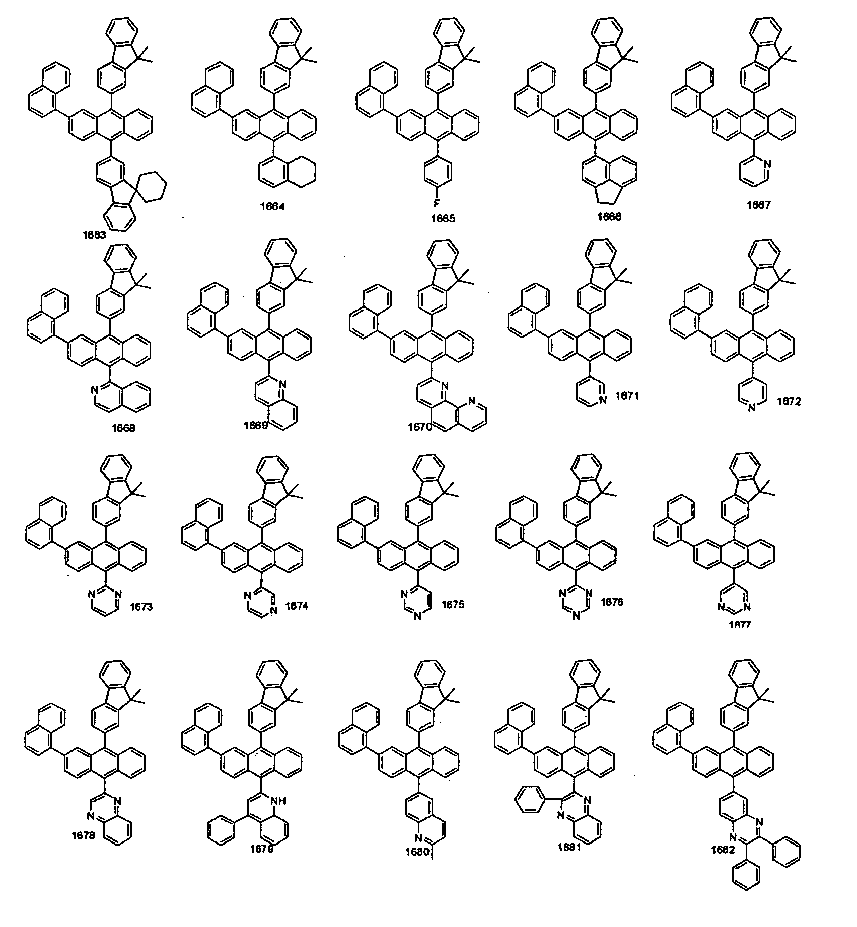

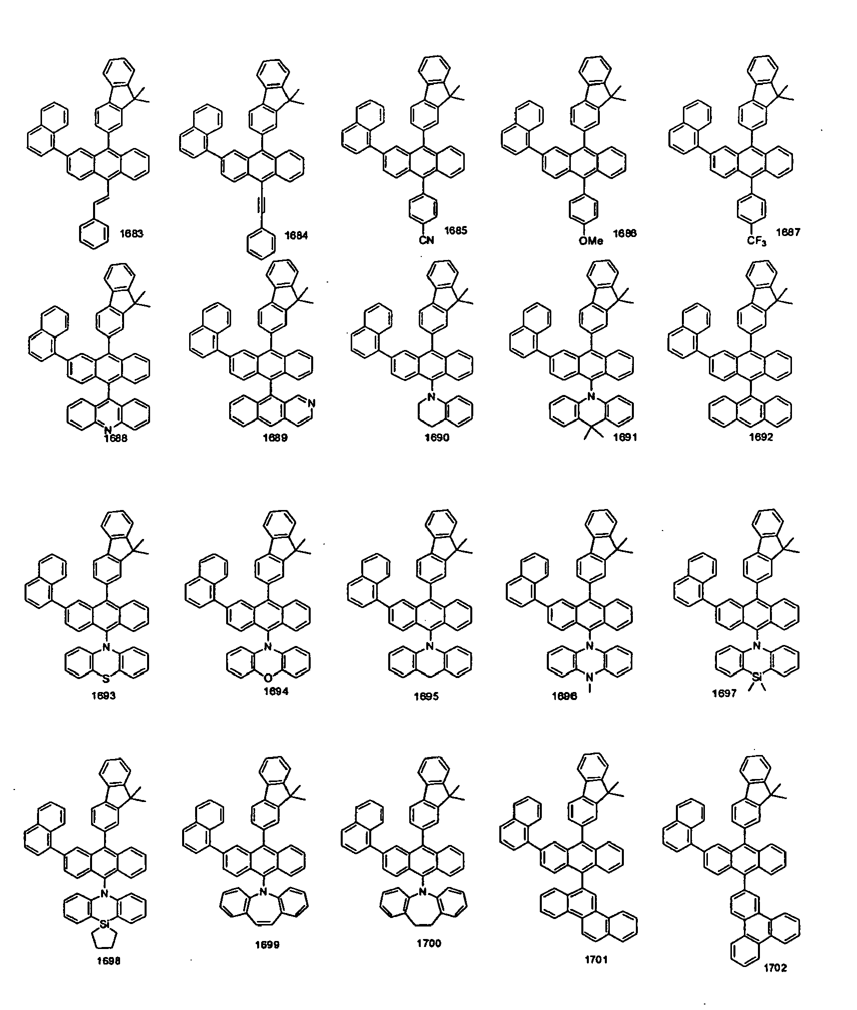

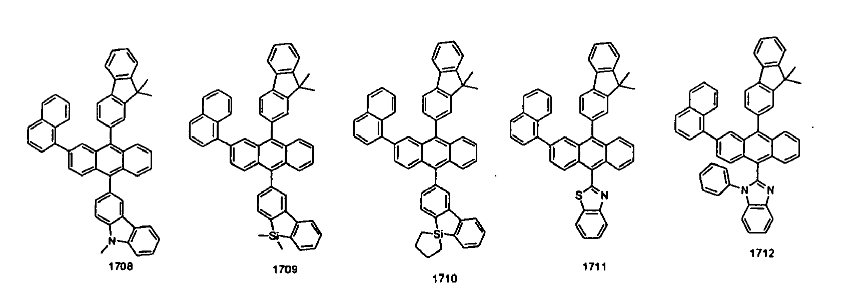

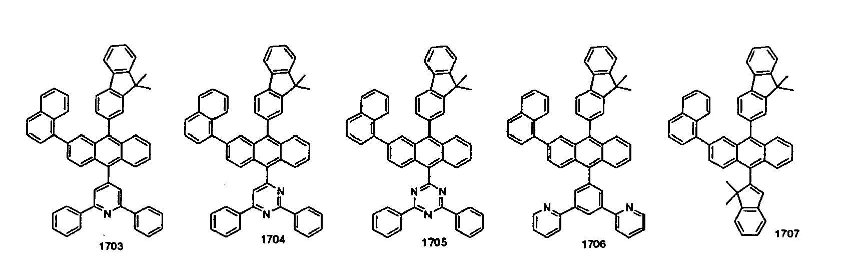

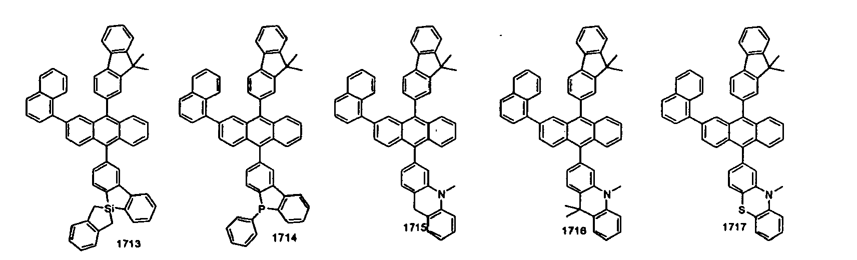

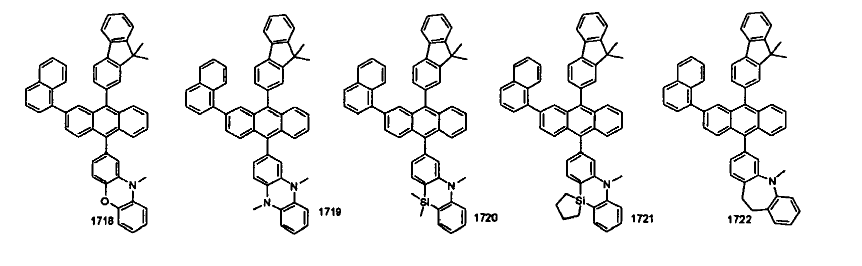

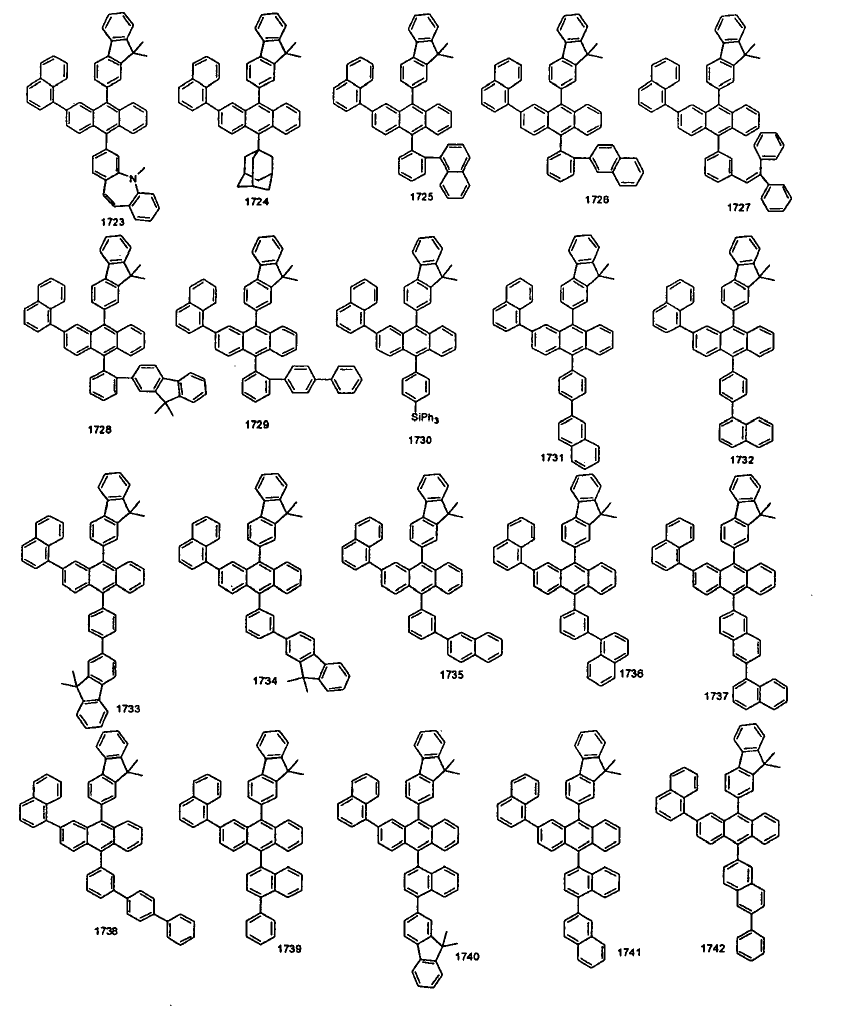

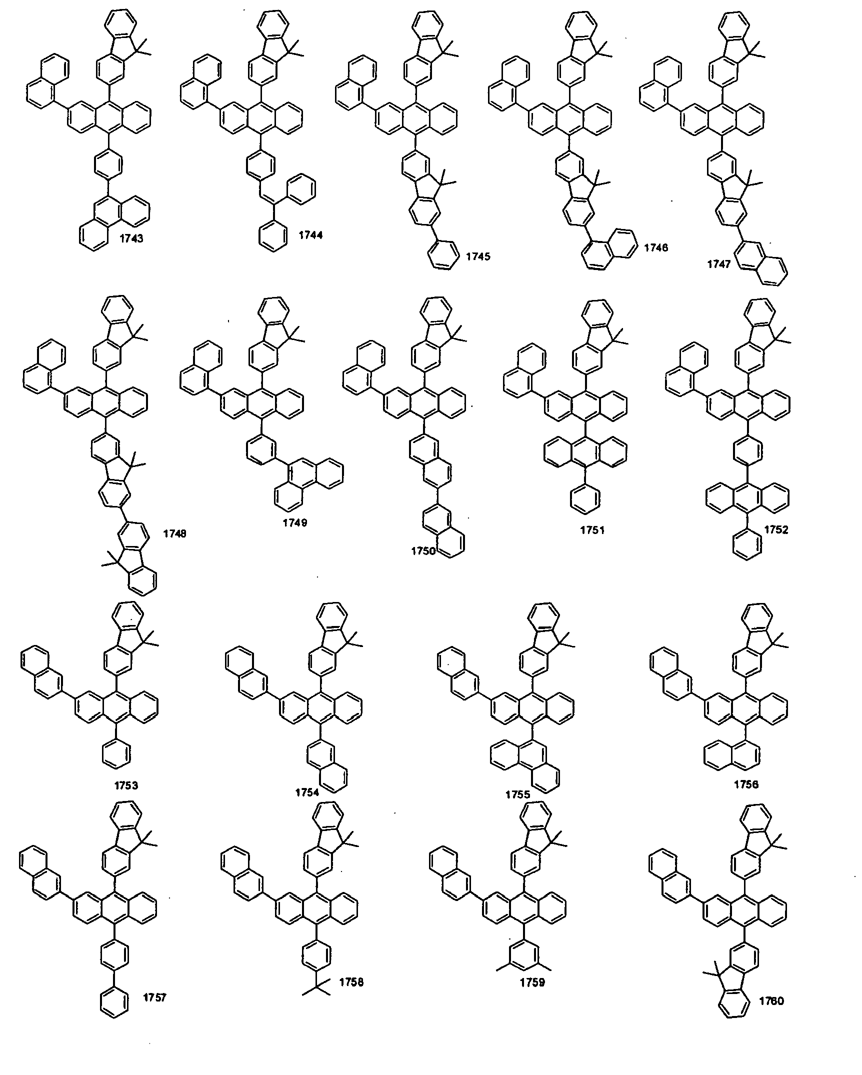

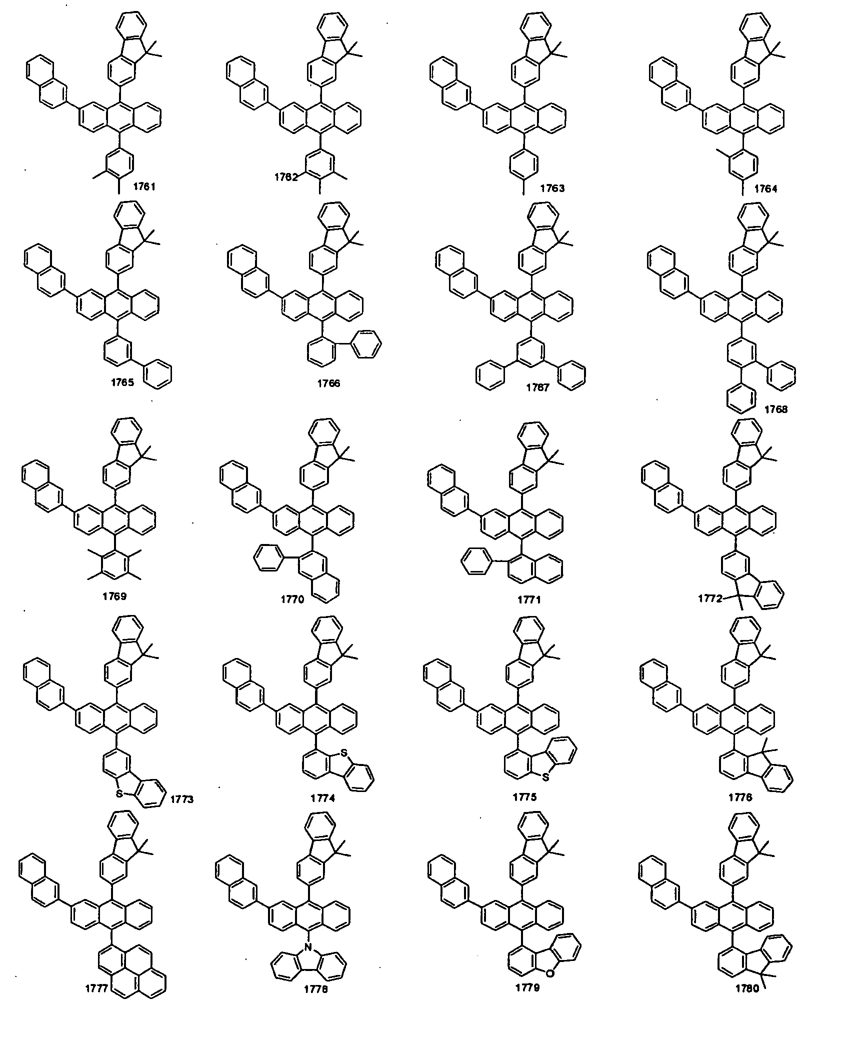

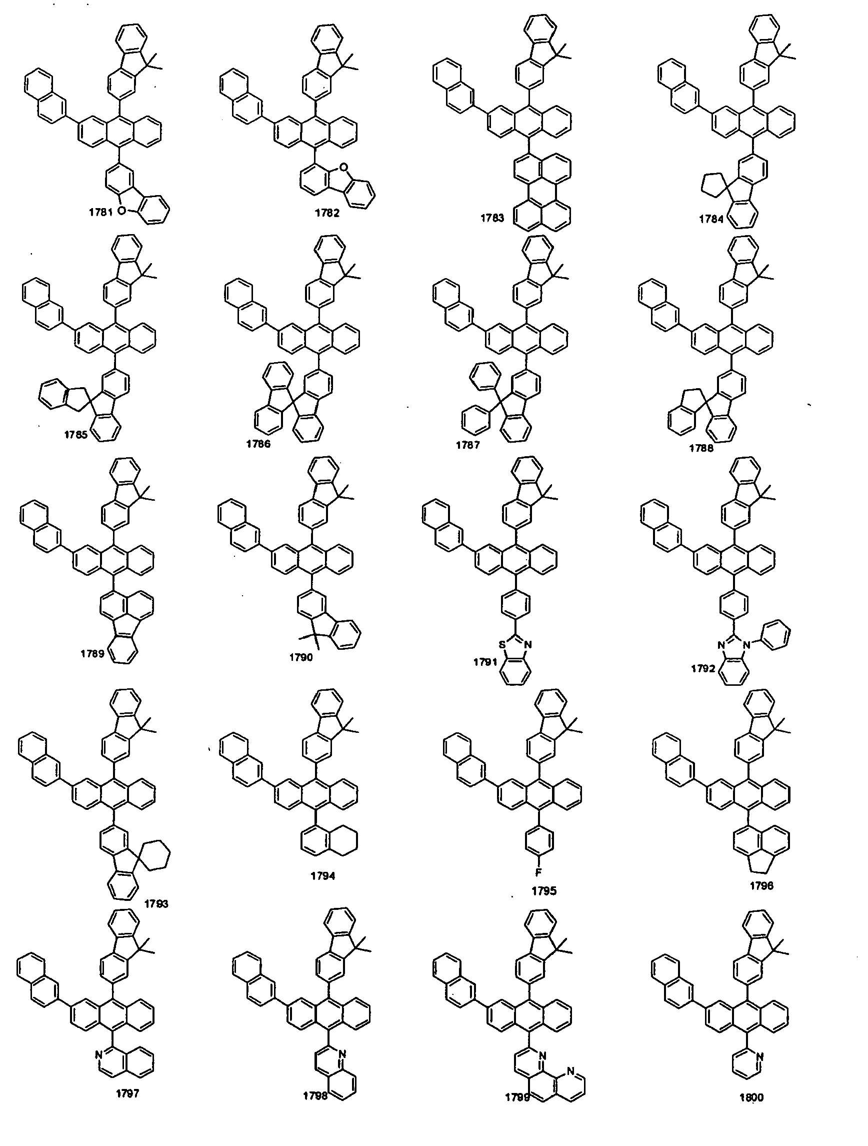

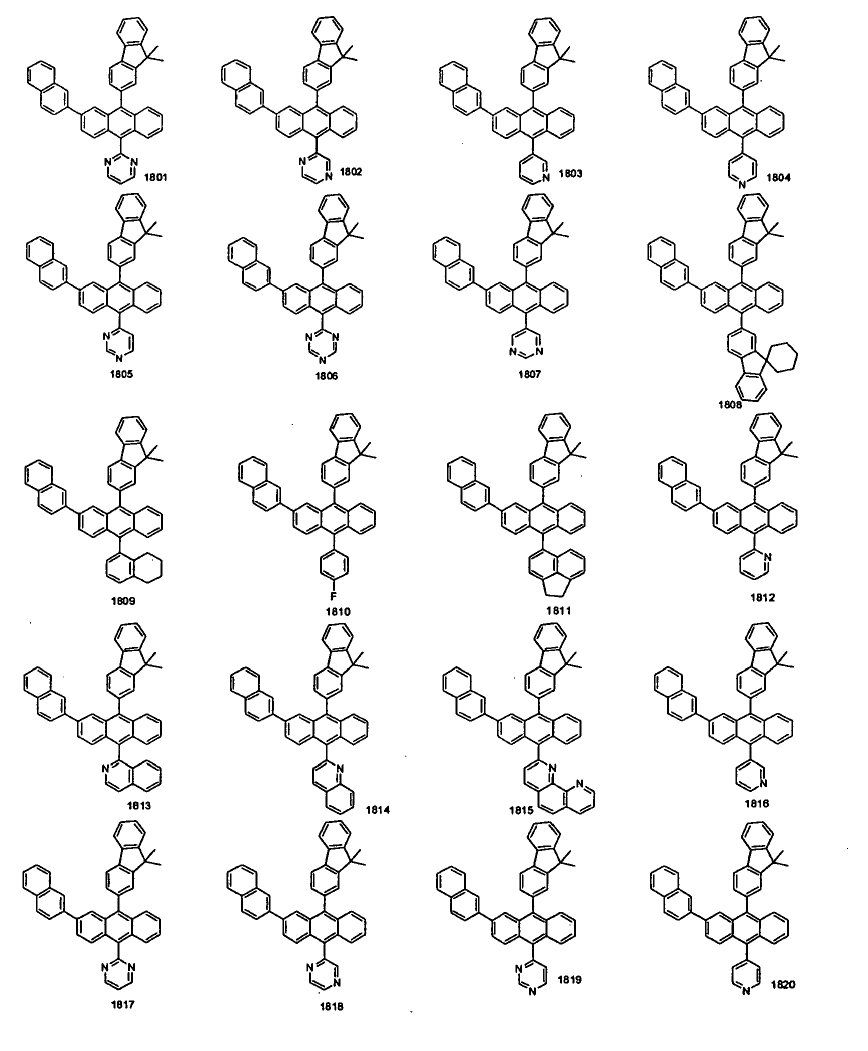

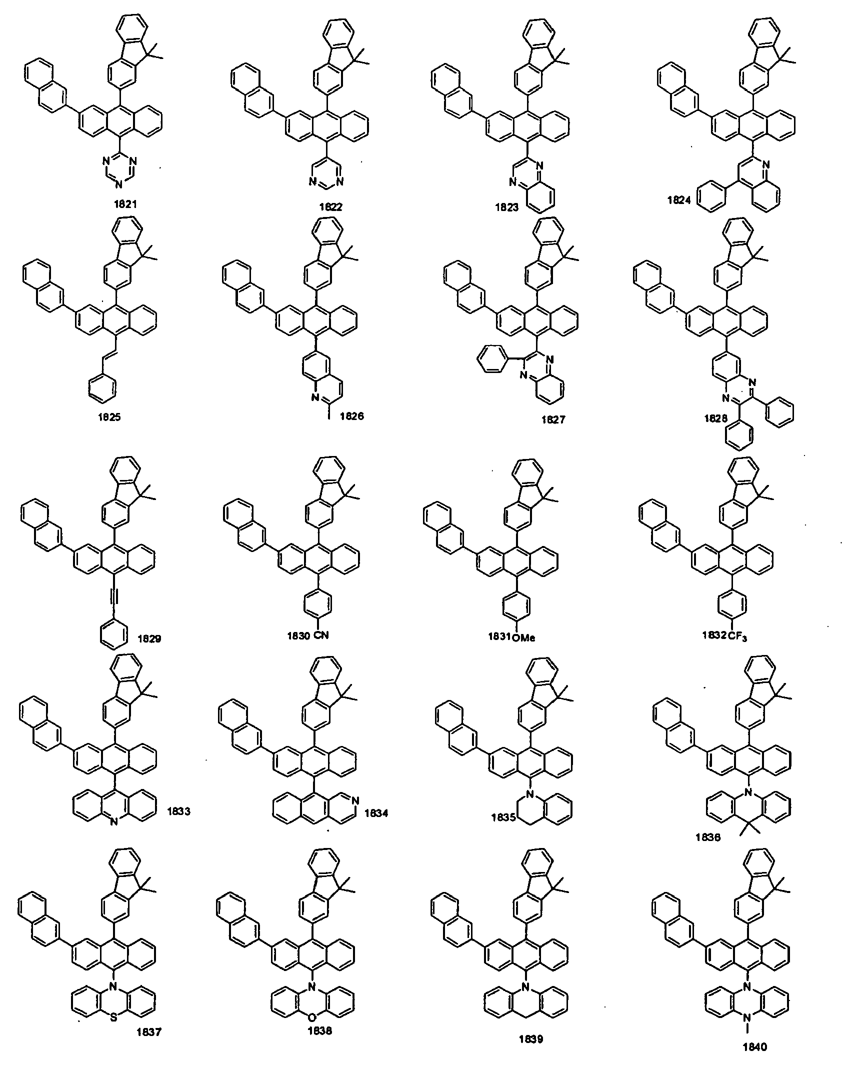

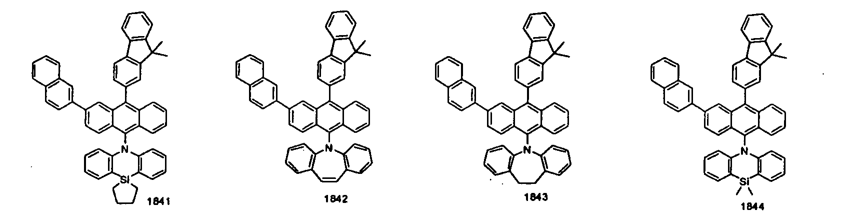

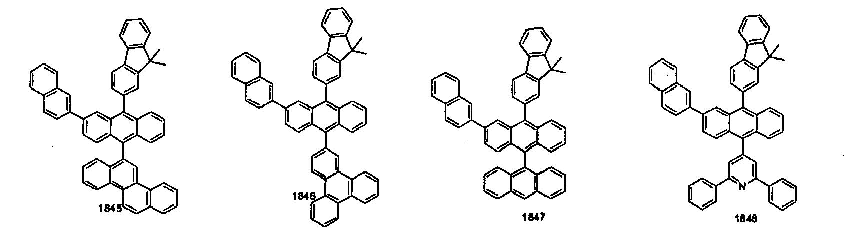

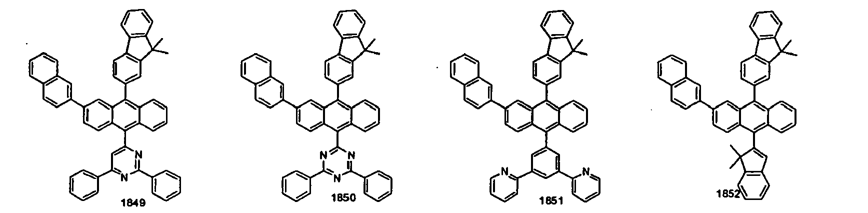

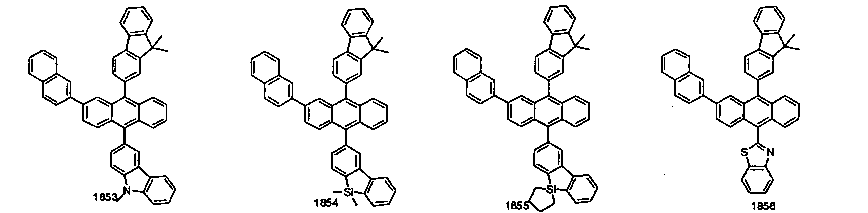

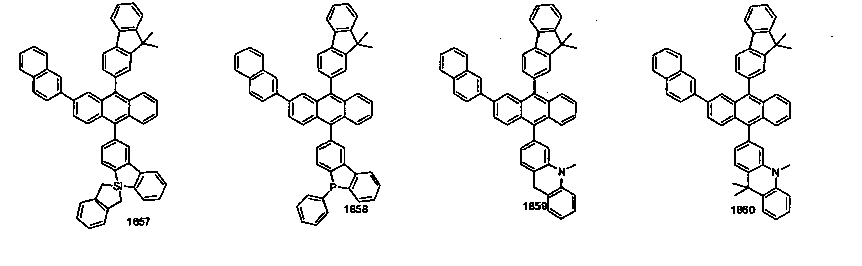

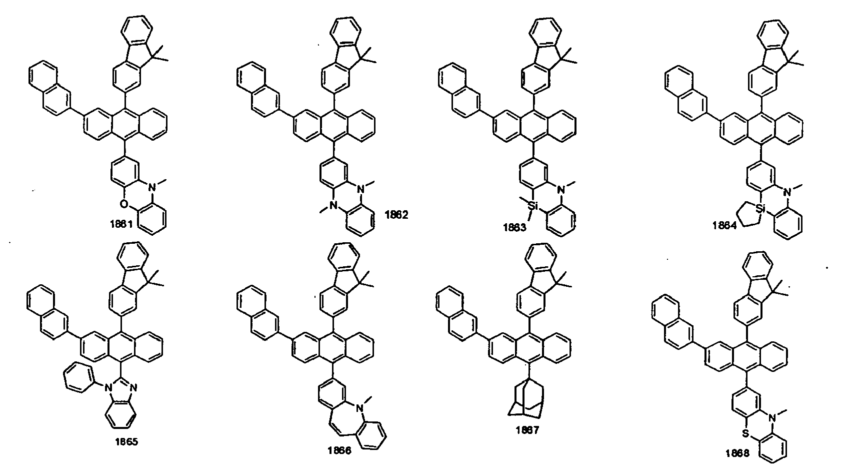

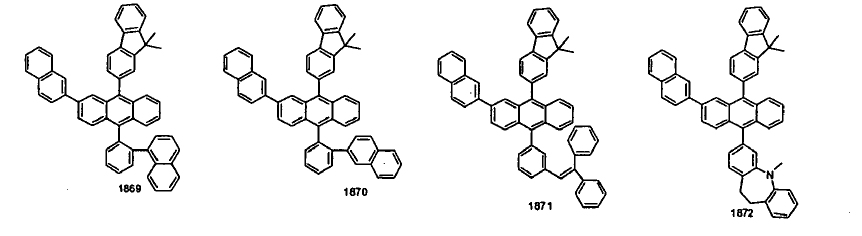

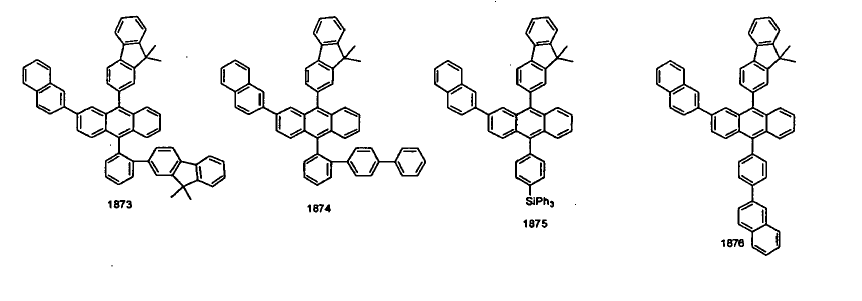

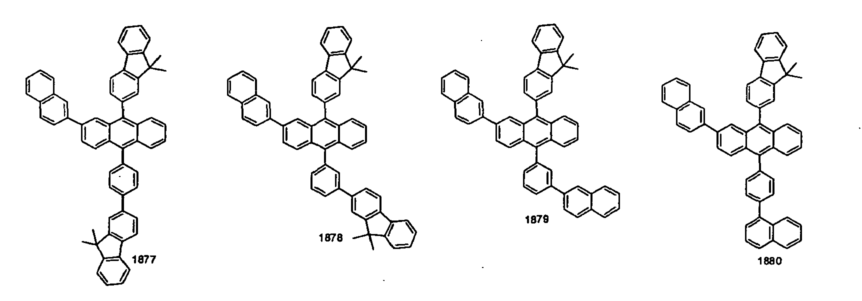

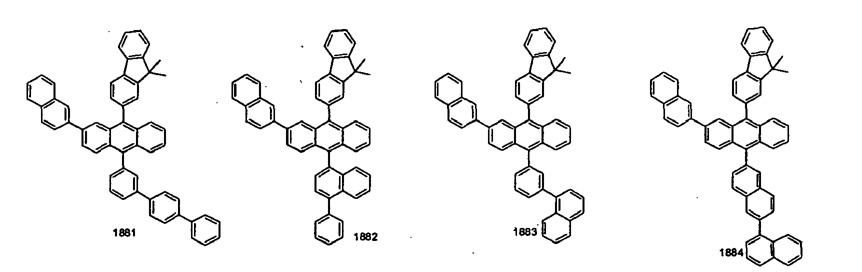

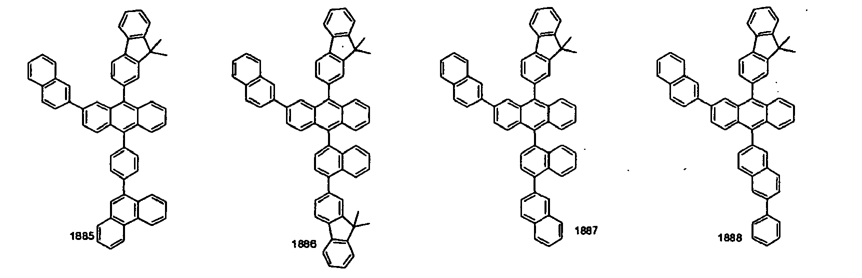

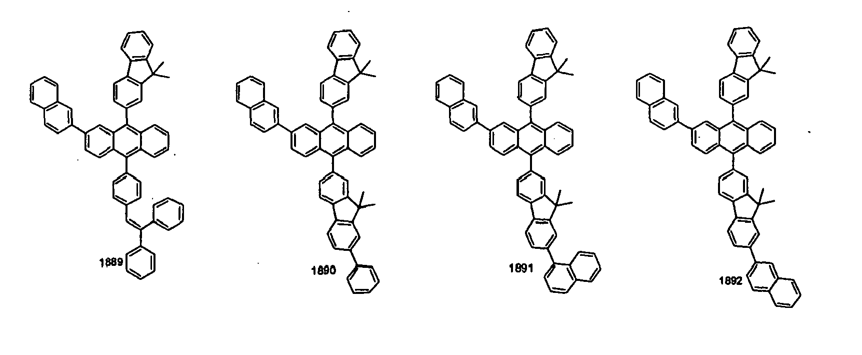

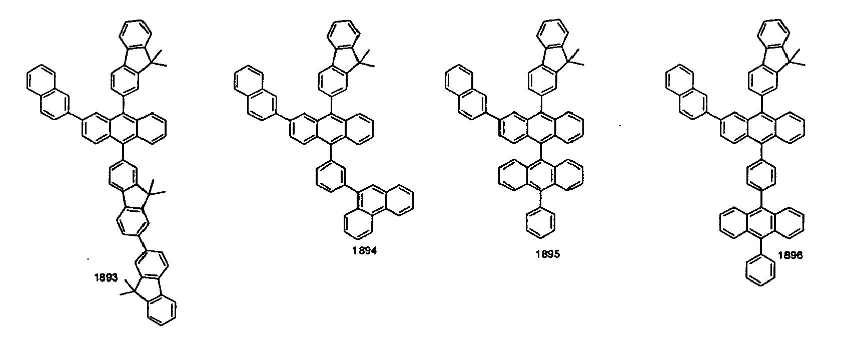

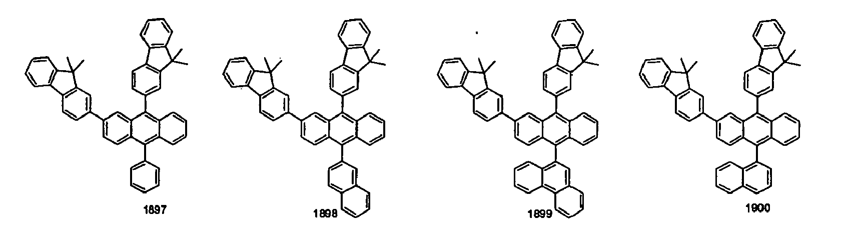

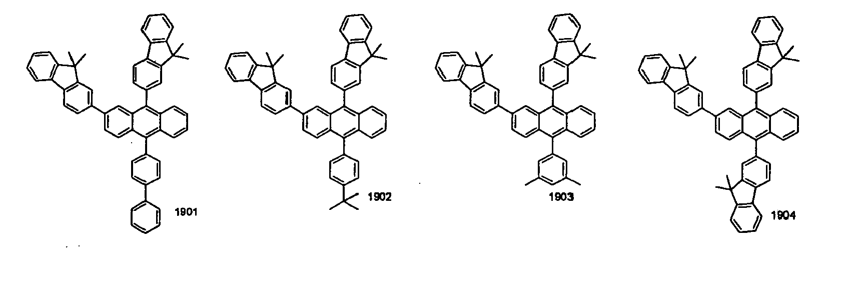

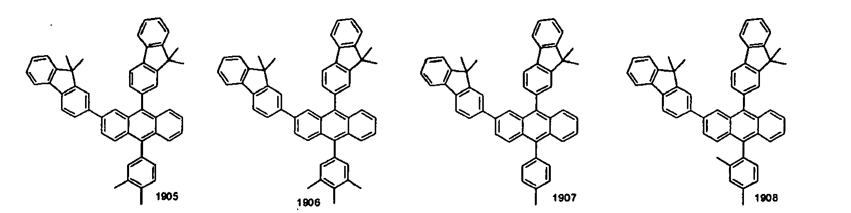

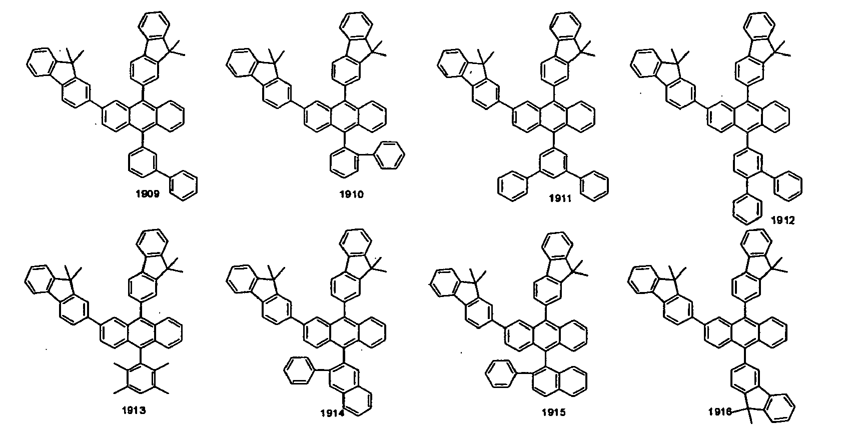

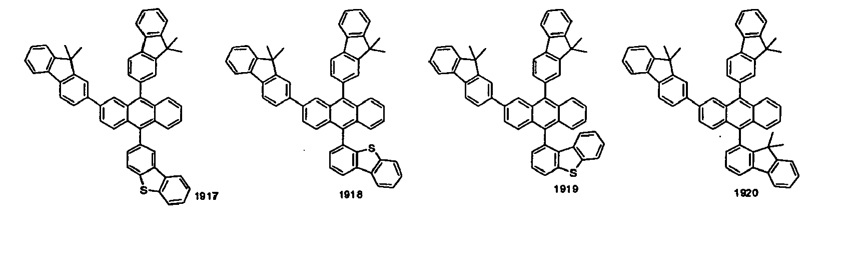

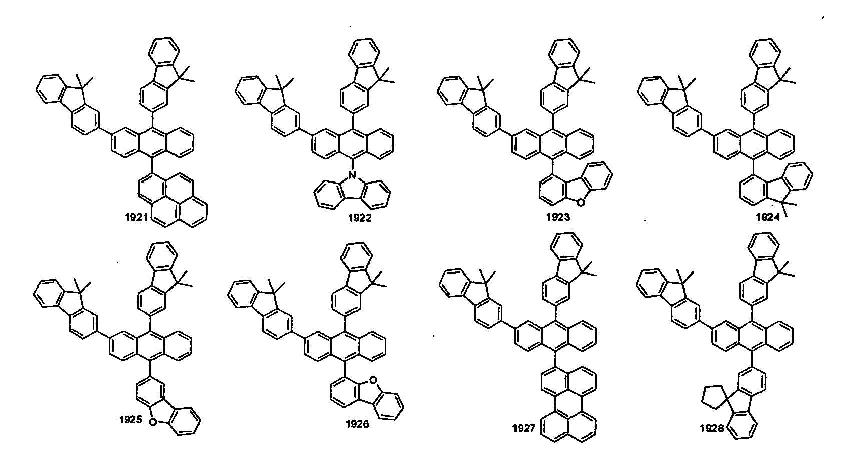

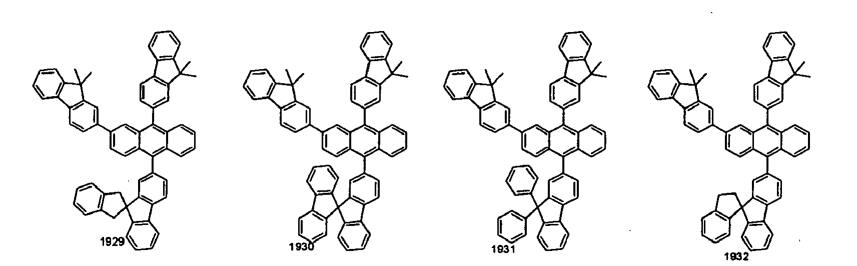

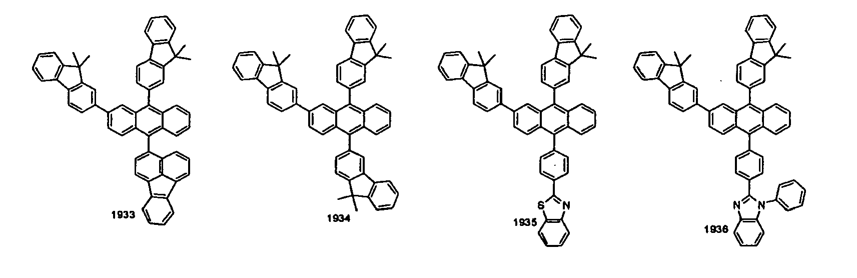

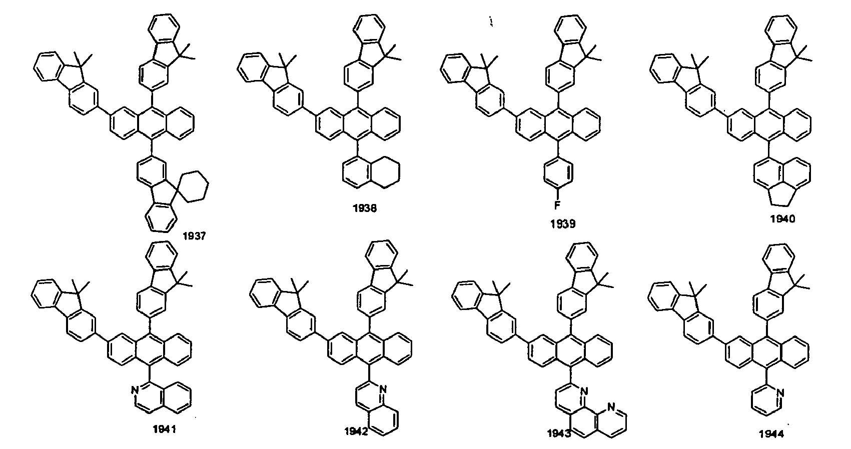

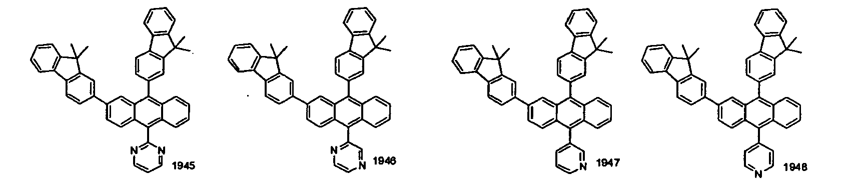

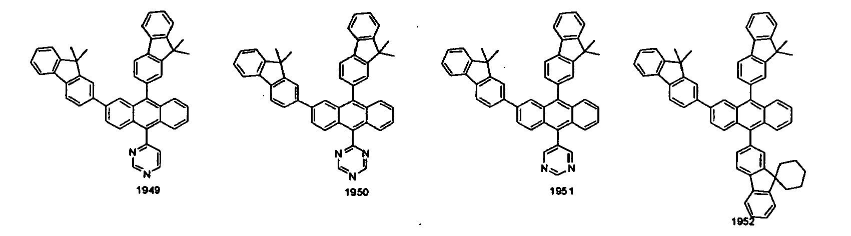

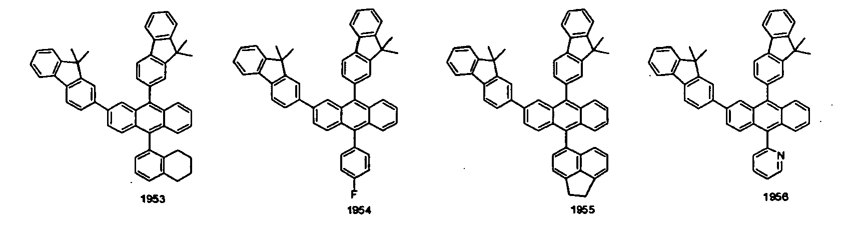

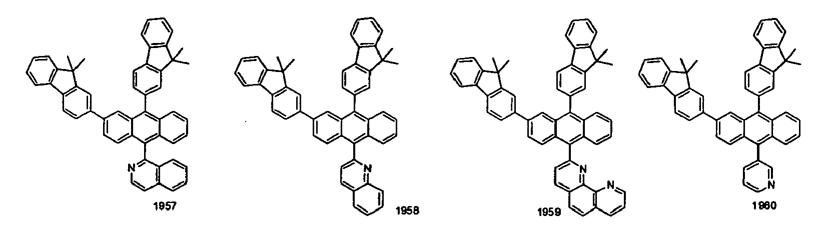

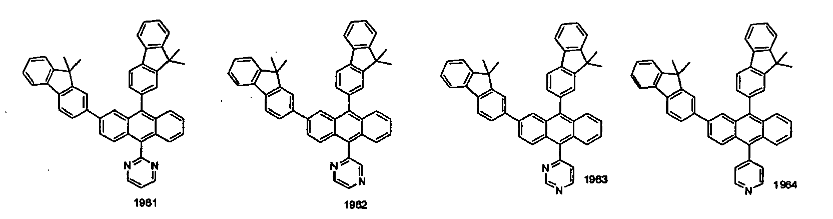

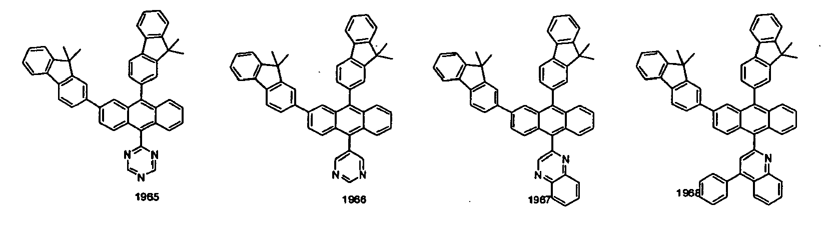

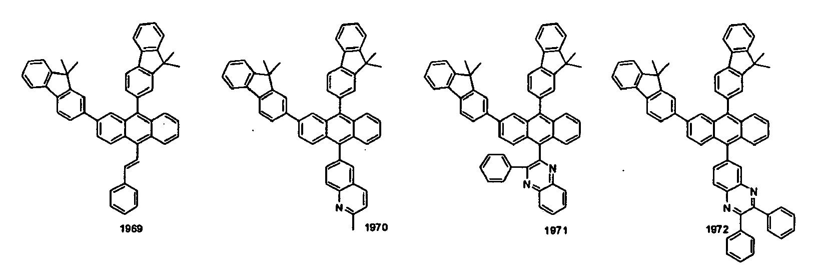

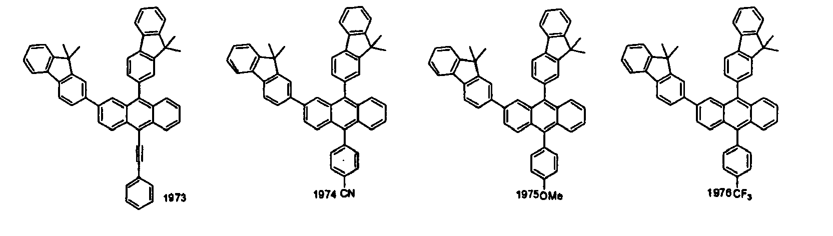

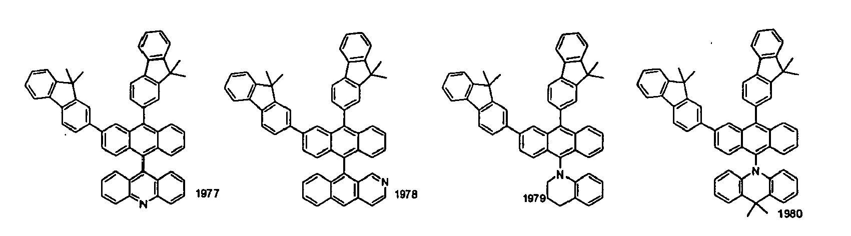

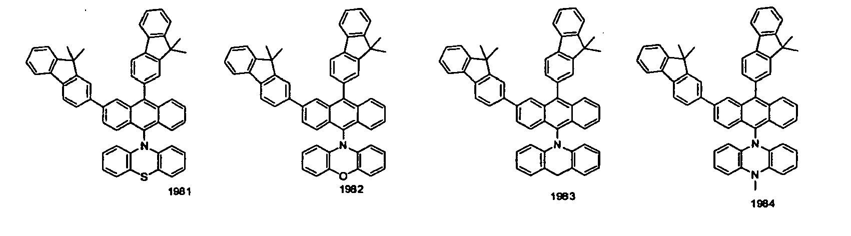

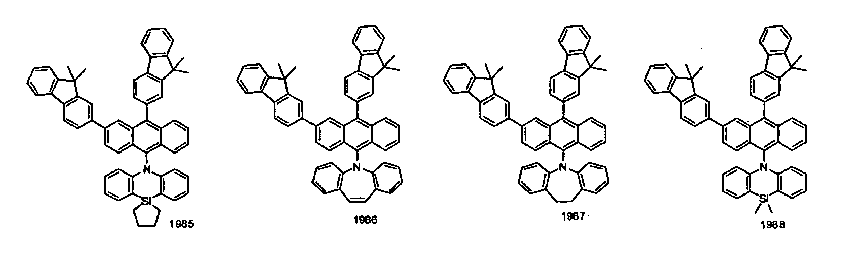

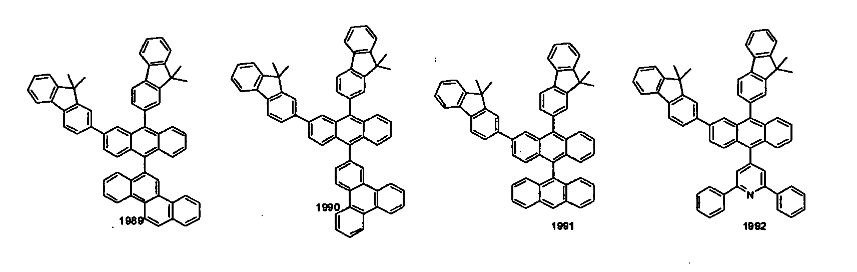

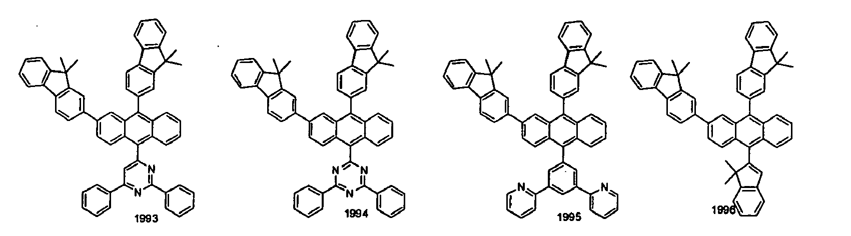

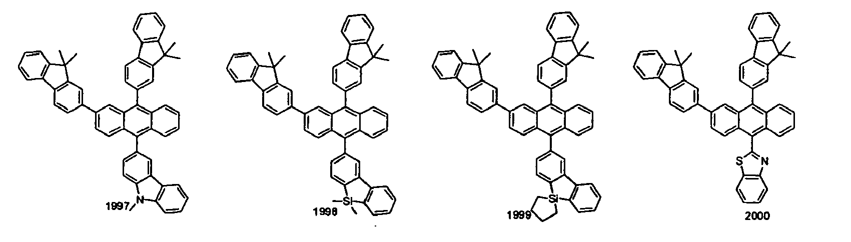

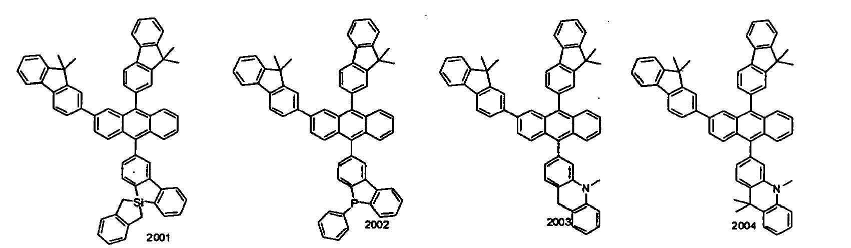

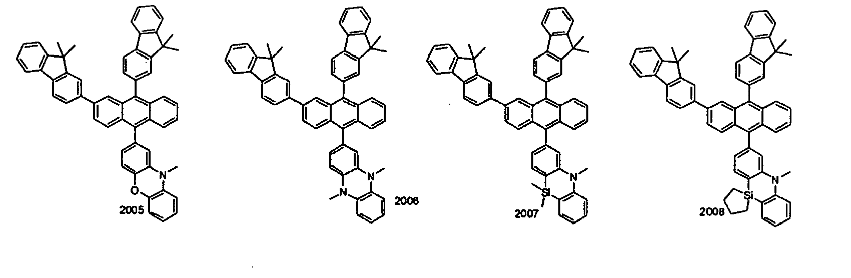

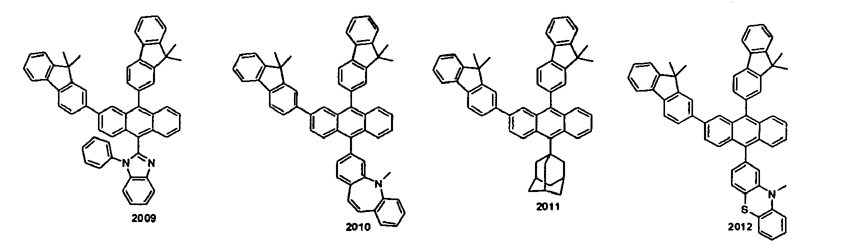

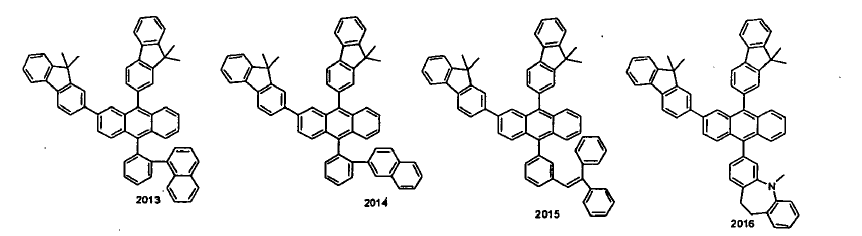

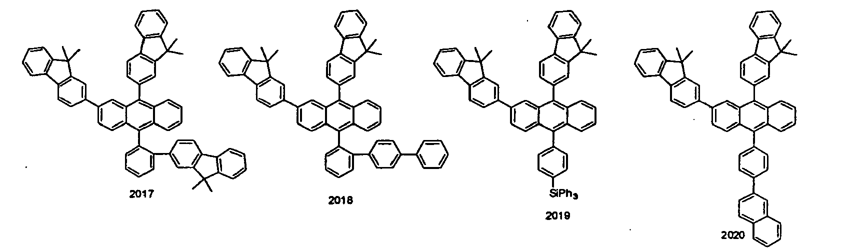

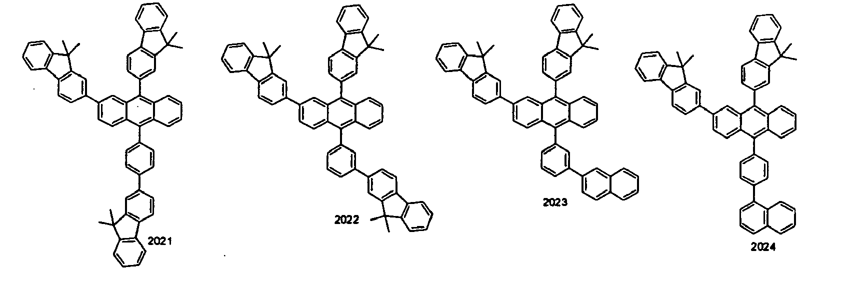

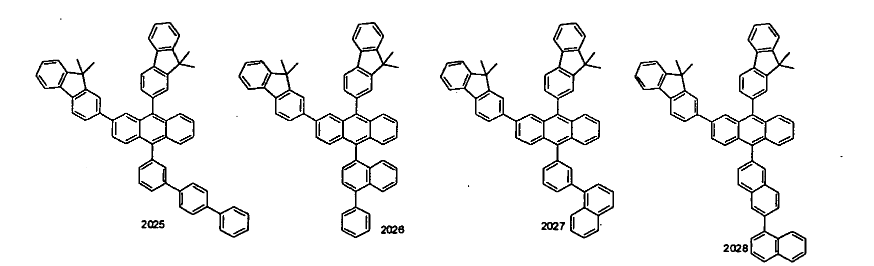

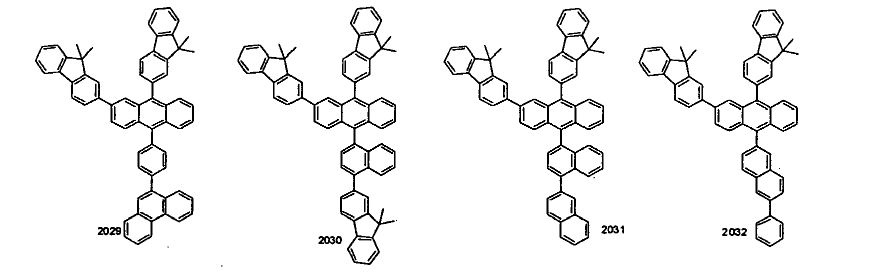

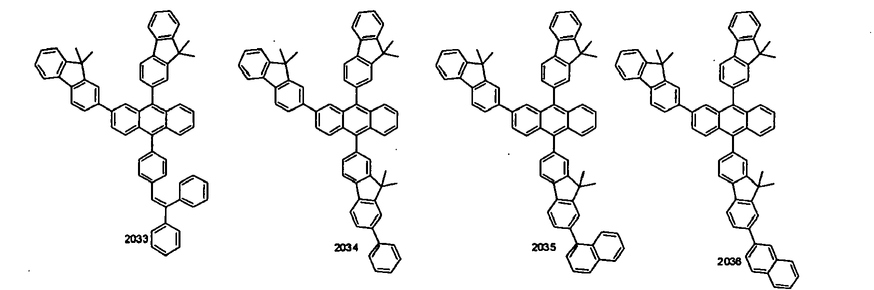

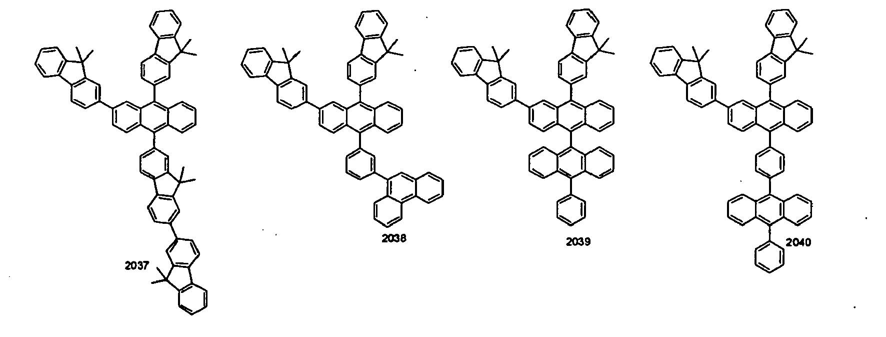

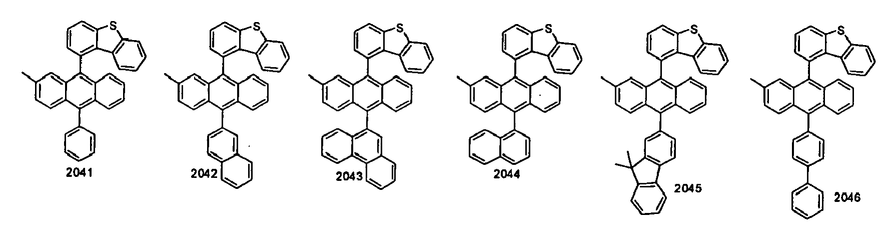

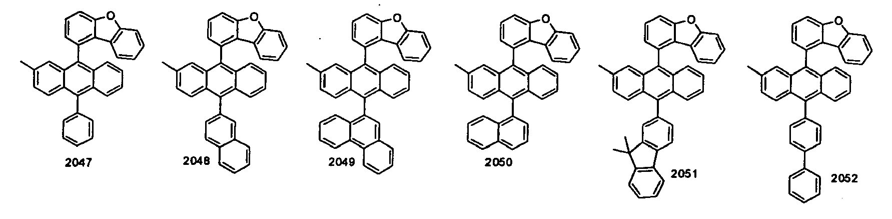

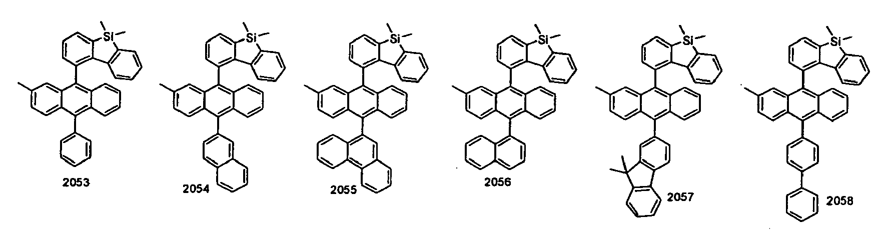

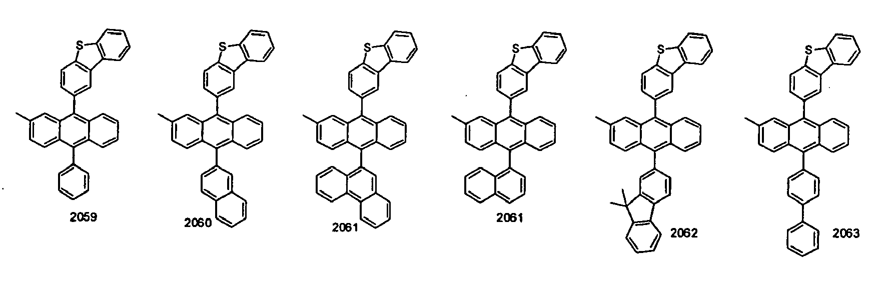

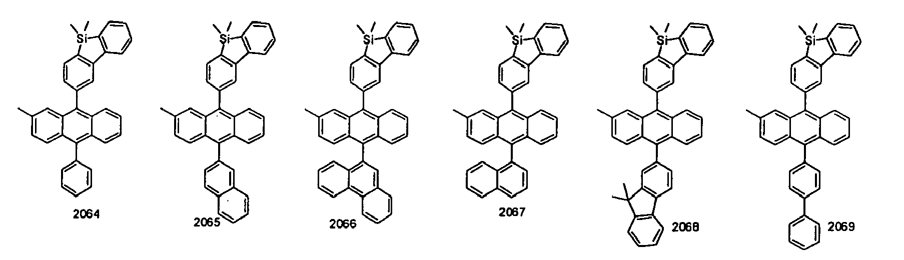

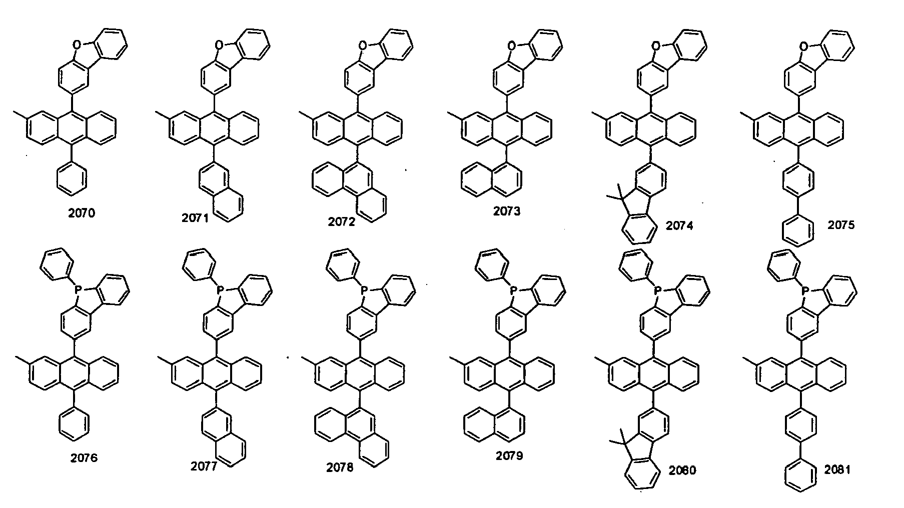

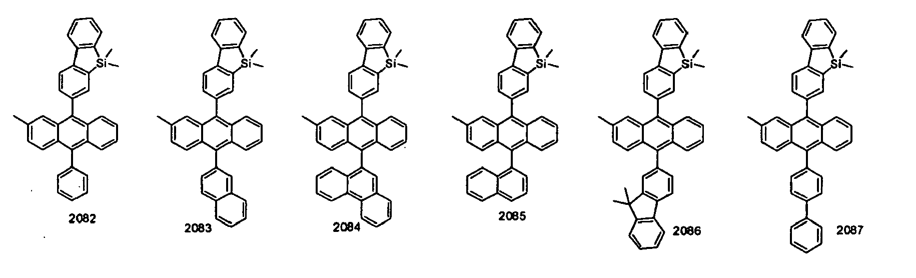

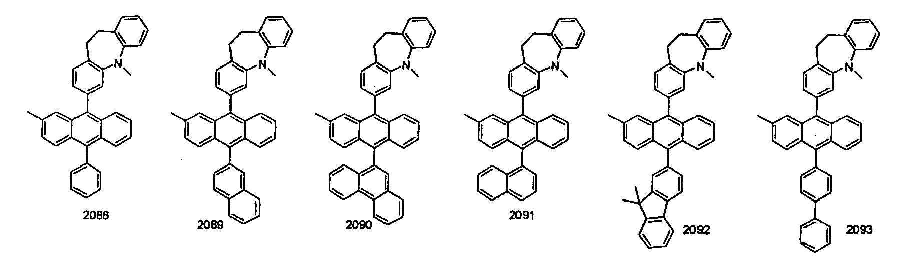

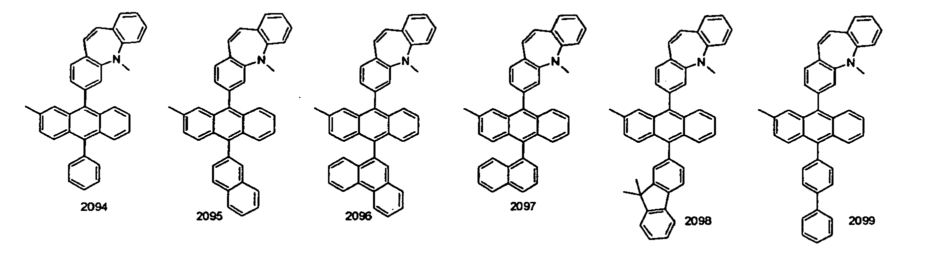

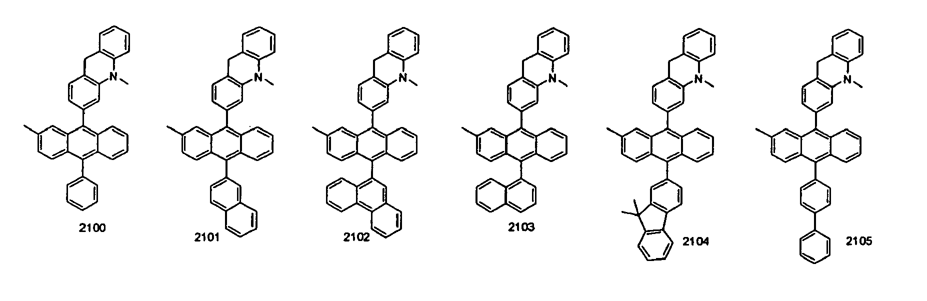

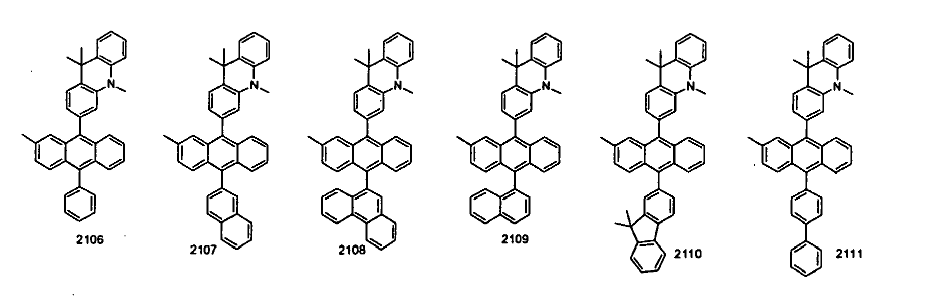

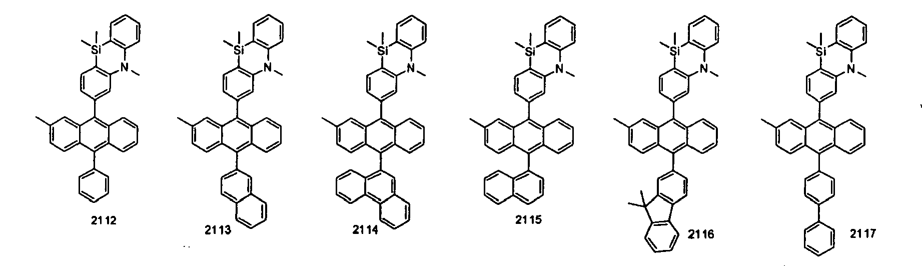

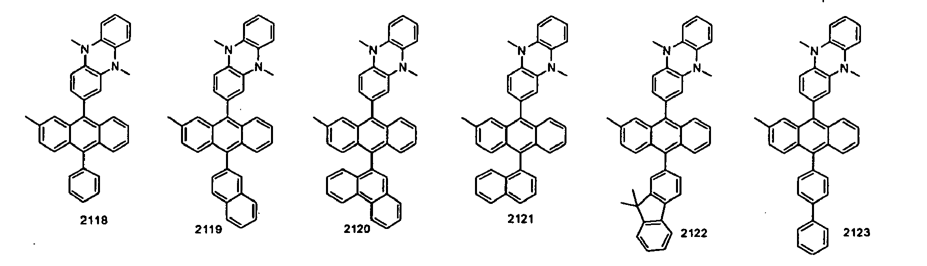

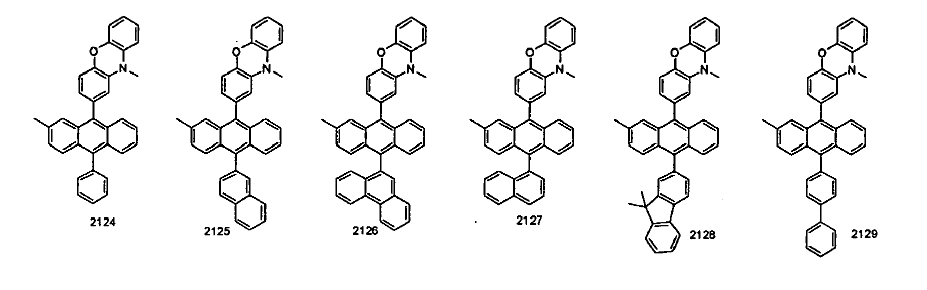

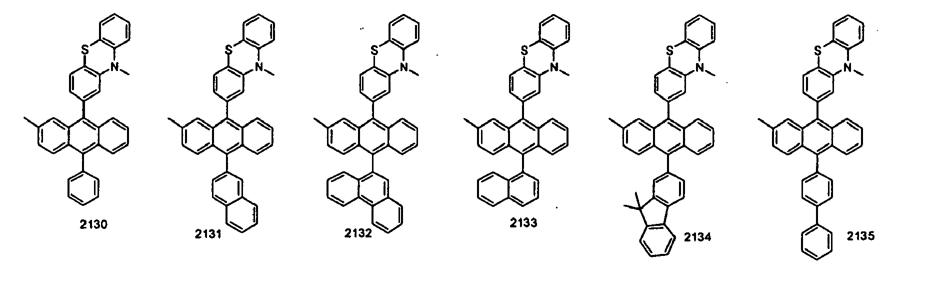

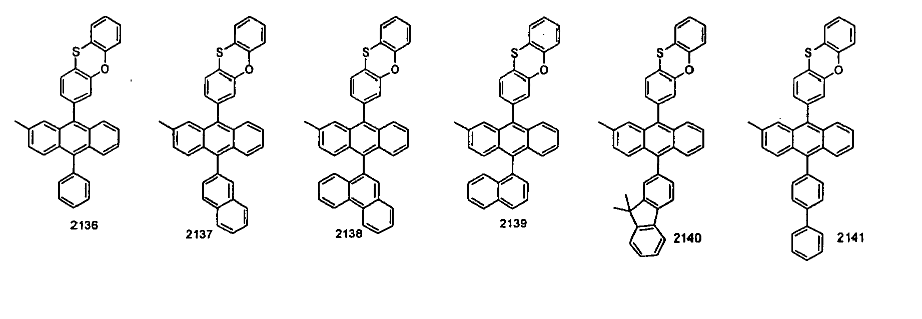

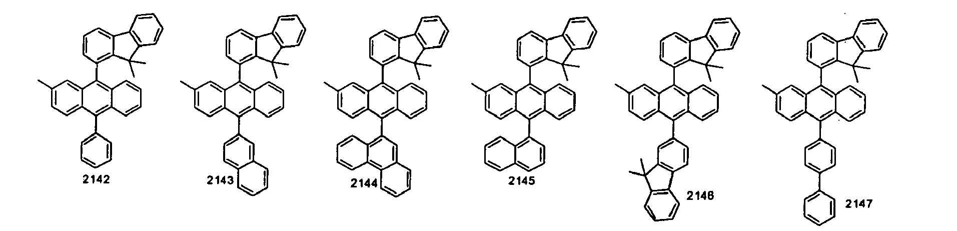

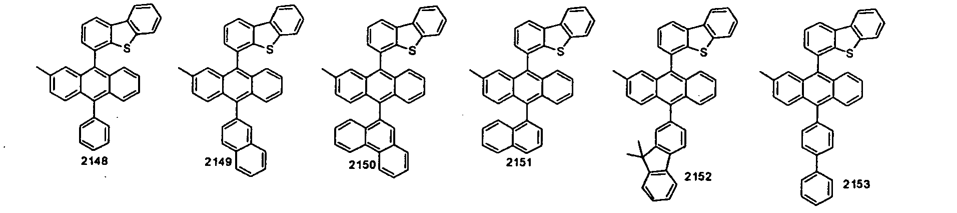

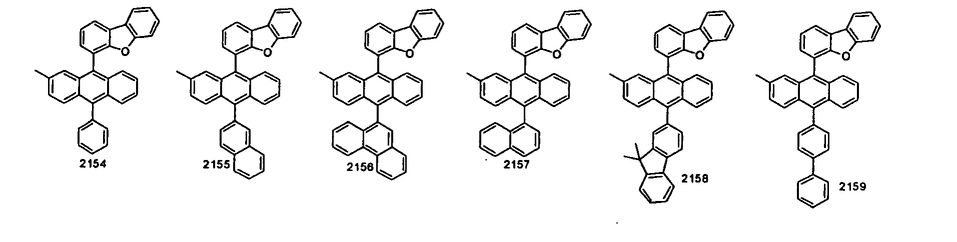

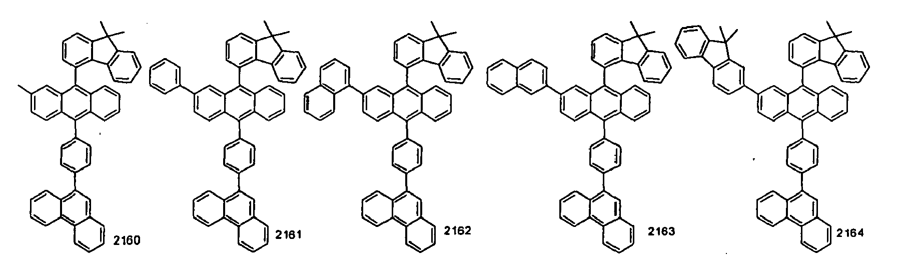

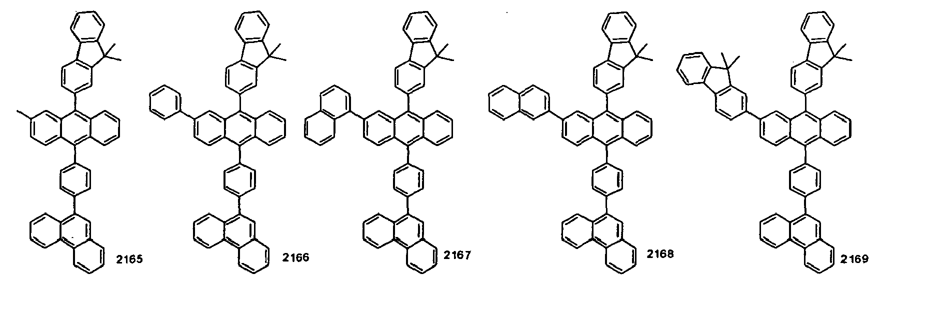

The organic electroluminescent compounds according to the present invention can be prepared as shown by Reaction Scheme (1) : 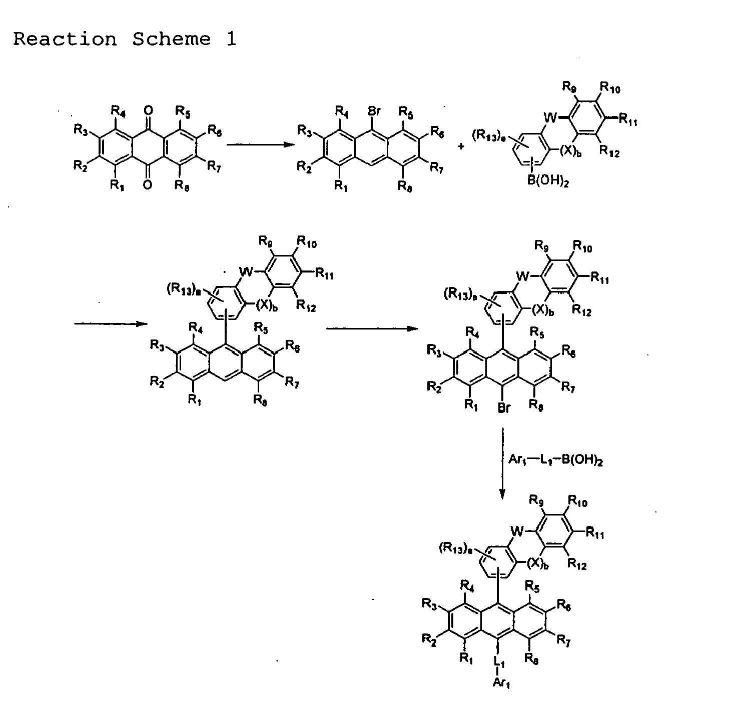 wherein, R₁ through R₁₃, W, X, L₁, Ar₁, a and b are defined as in Chemical Formula (1).

The present invention also provides organic solar cells, which comprises one or more organic electroluminescent compound(s) represented by Chemical Formula (1).

The present invention also provides an organic electroluminescent device which is comprised of a first electrode; a second electrode; and at least one organic layer(s) interposed between the first electrode and the second electrode; wherein the organic layer comprises one or more compound(s) represented by Chemical Formula (1).

The organic electroluminescent device according to the present invention is **characterized in that** the organic layer comprises an electroluminescent layer, which comprises one or more compound(s) represented by Chemical Formula (1) as electroluminescent host, and one or more dopant(s). The dopant applied to the organic electroluminescent device according to the invention is not particularly restricted, but preferably selected from the compounds represented by Chemical Formula (6) or (7).

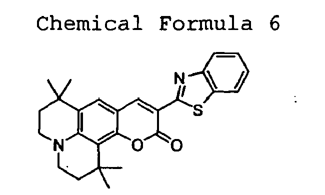

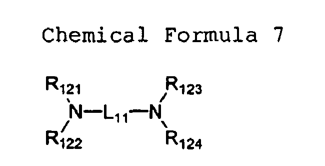

In Chemical Formula (7), L₁₁ represents (C6-C60)arylene with or without one or more substituent(s) selected from a group consisting of halogen, (C1-C60)alkyl, (C6-C60)aryl, (C4-C60)heteroaryl, a 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro and hydroxyl; the alkyl, cycloalkyl, heterocycloalkyl, aryl, heteroaryl, arylsilyl, alkylsilyl, alkylamino and arylamino substituent on the arylene may be further substituted by one or more substituent(s) selected from halogen, (C1-C60)alkyl, (C6-C60)aryl, (C4-C60)heteroaryl, a 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (Cl-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro and hydroxyl;
R₁₂₁ through R₁₂₄ independently represent (C1-C60)alkyl, (C6-C60)aryl, (C4-C60)heteroaryl, (C6-C60)arylamino, (C1-C60)alkylamino, 5- or 6-membered heterocycloalkyl containing one or more heteroatom(s) selected from N, O and S, or (C3-C60)cycloalkyl, or each of R₁₂₁ through R₁₂₄ may be linked to an adjacent substituent via (C3-C60)alkylene or (C3-C60)alkenylene with or without a fused ring to form an alicyclic ring, or a monocyclic or polycyclic aromatic ring; and
the alkyl, aryl, heteroaryl, arylamino, alkylamino, cycloalkyl or heterocycloalkyl of R₁₂₁ through R₁₂₄ may be further substituted by one or more substituent(s) selected from halogen, (C1-C60)alkyl, (C6-C60)aryl, (C4-C60)heteroaryl, a 5- or 6-membered heterocycloalkyl containing one or more heteroatom(s) selected from N, O and S, (C3-C60)cycloalkyl, tri(Cl-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro and hydroxyl.

The electroluminescent layer means the layer where electroluminescence occurs, and it may be a single layer or a multi-layer consisting of two or more layers laminated. When a mixture of host-dopant is used according to the constitution of the present invention, noticeable improvement in luminous efficiency by the electroluminescent host according to the invention could be confirmed. Those results can be achieved by doping concentration of 0.5 to 10% by weight. The host according to the present invention exhibits higher hole and electron conductivity, and excellent stability of the material as compared to other conventional host materials, and provides improved device life as well as luminous efficiency.

Thus, it can be described that use of the compound represented by Chemical Formula (6) or (7) as an electroluminescent dopant significantly supplements electronic drawback of the organic electroluminescent compounds of Chemical Formula (1) according to the present invention.

The dopant compounds represented by Chemical Formula (7) can be exemplified by the following compounds, but are not restricted thereto.

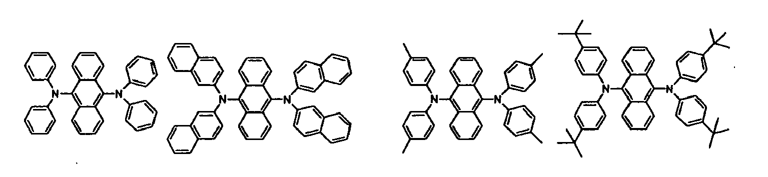

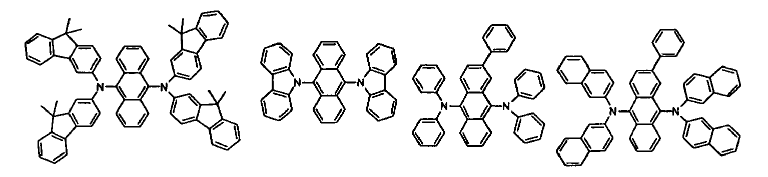

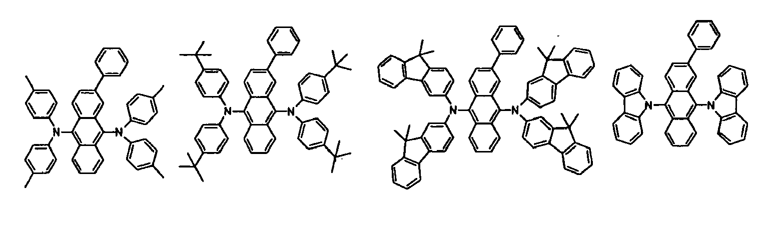

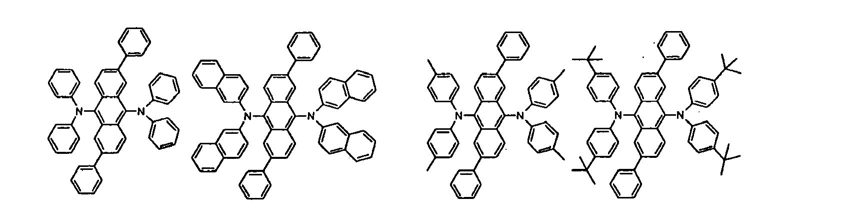

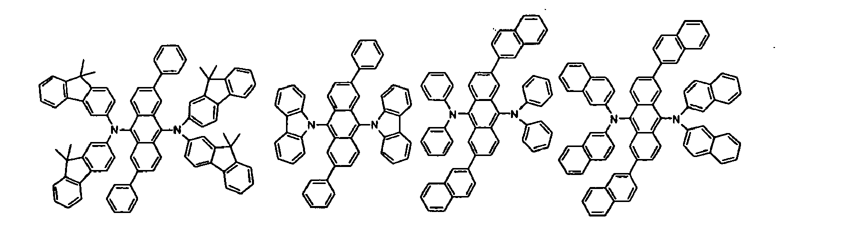

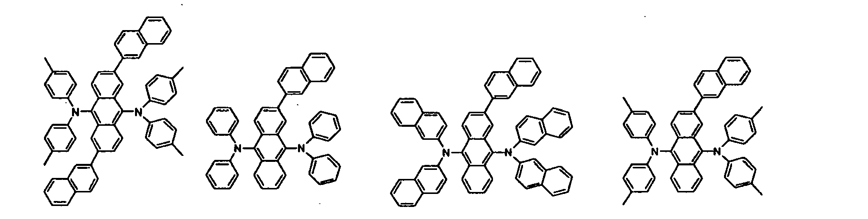

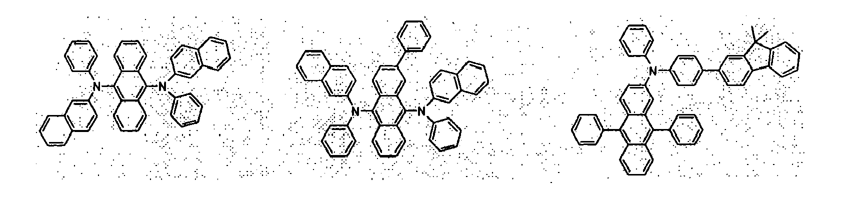

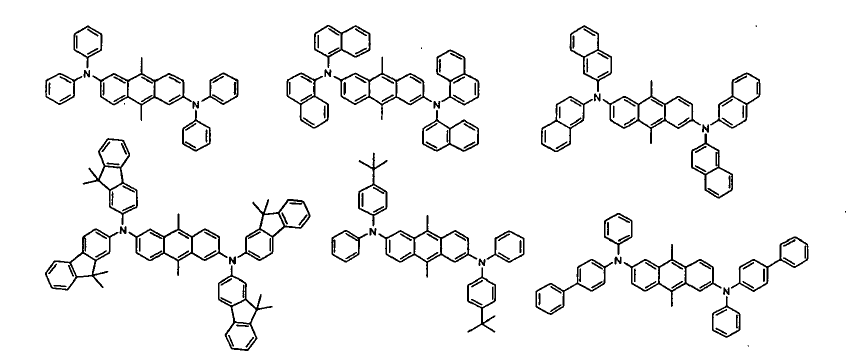

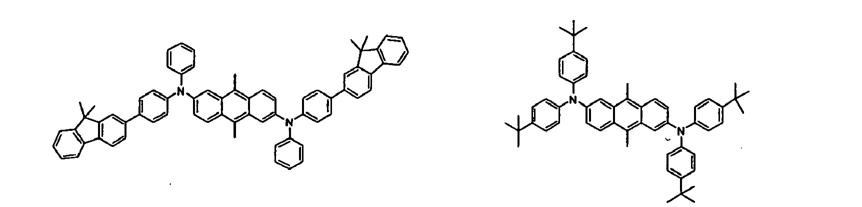

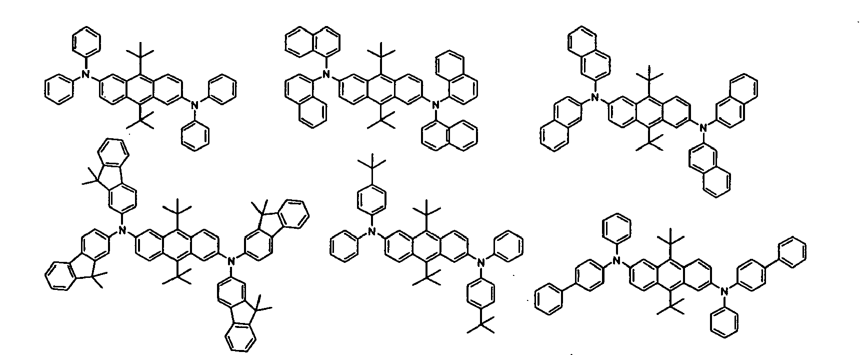

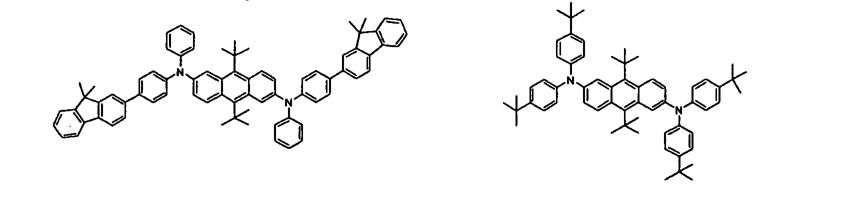

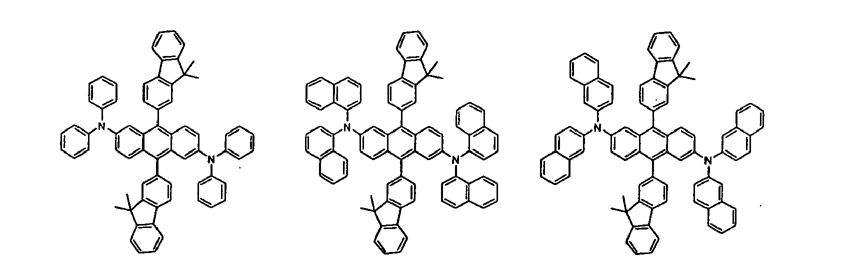

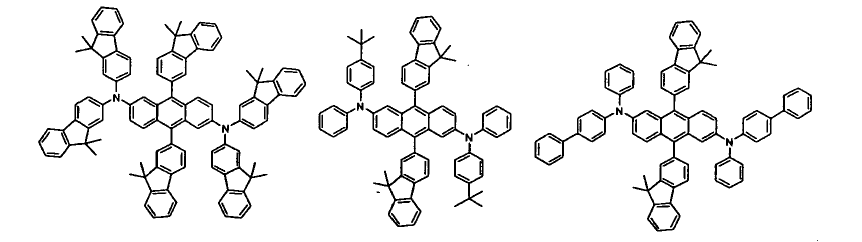

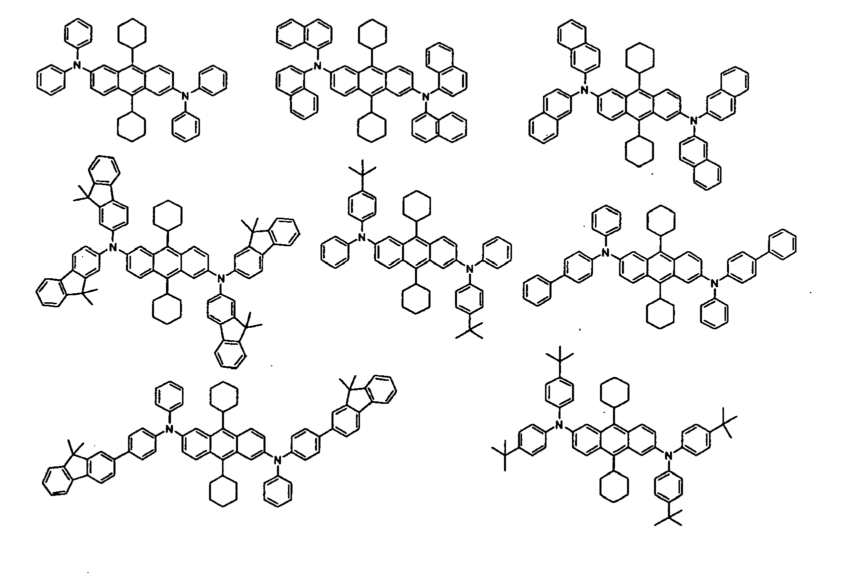

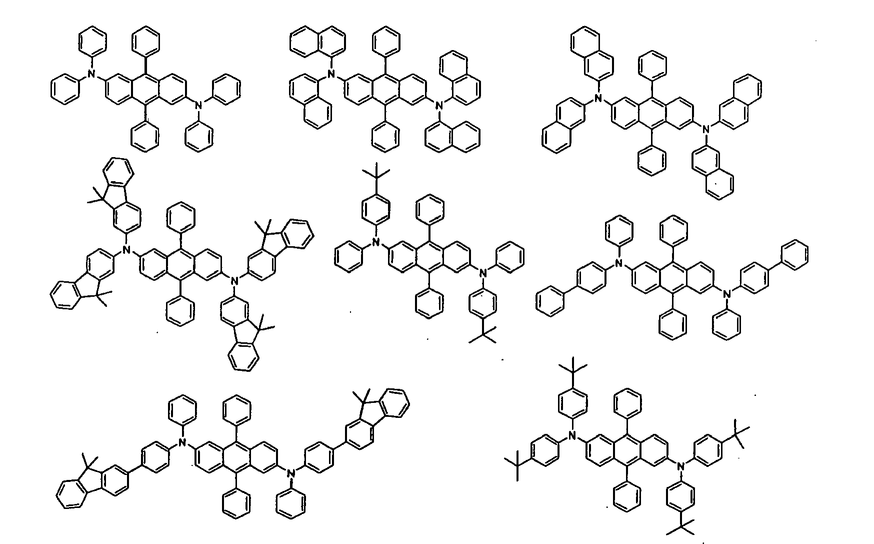

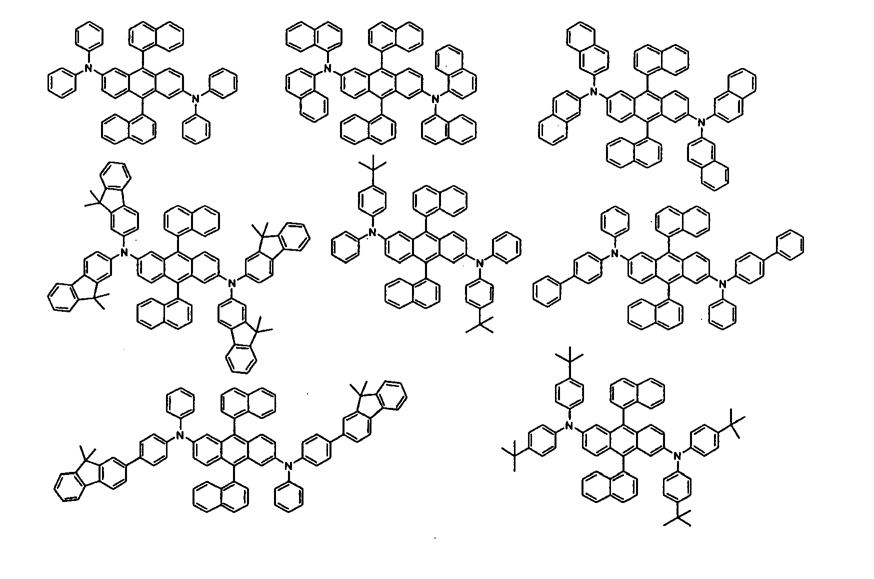

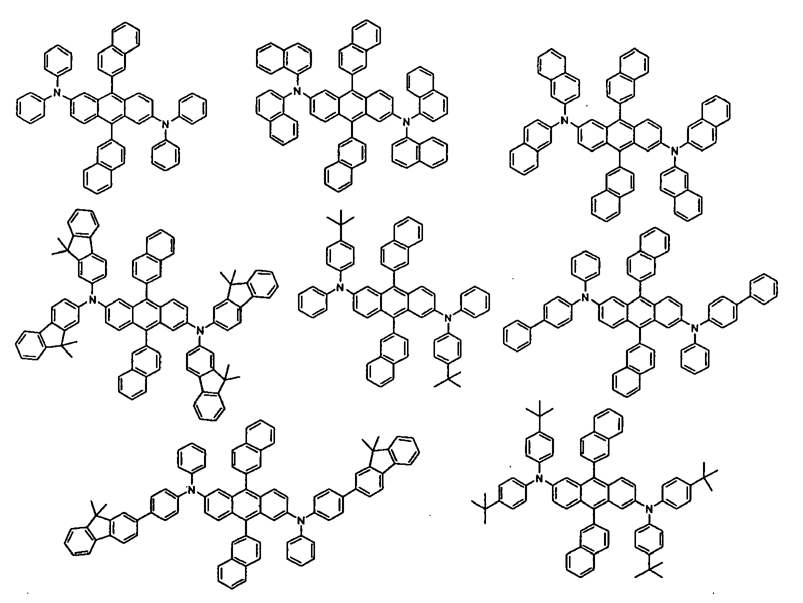

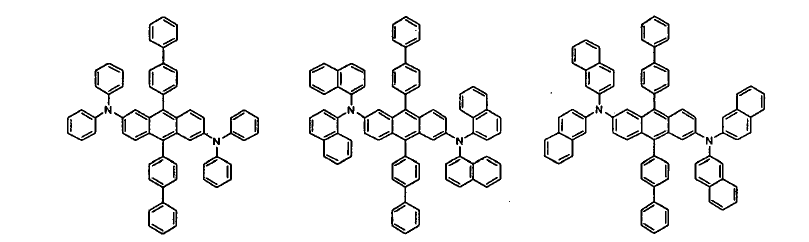

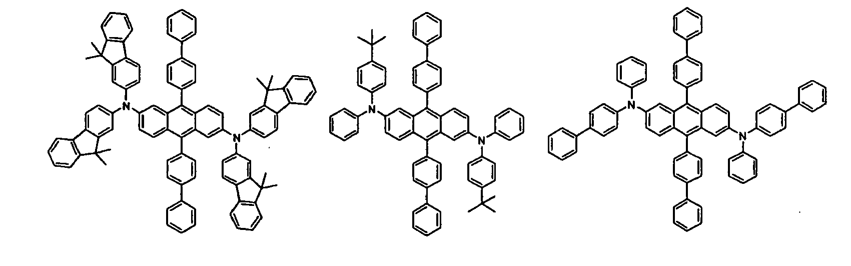

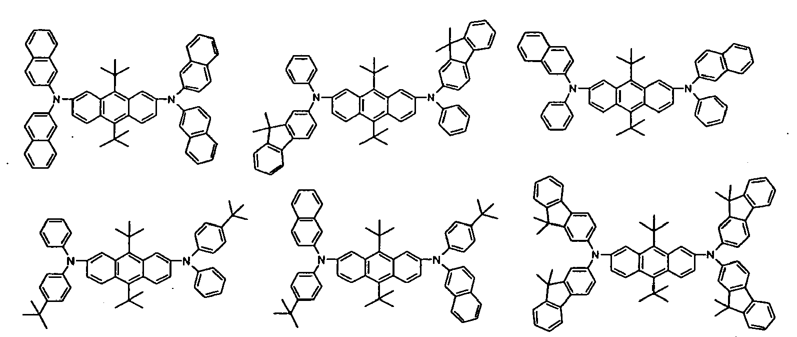

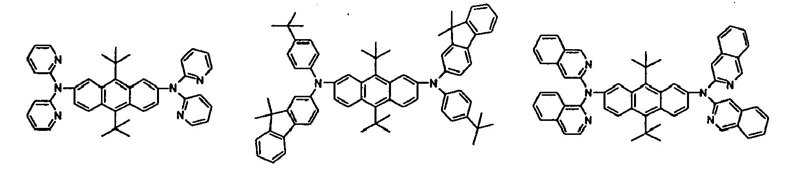

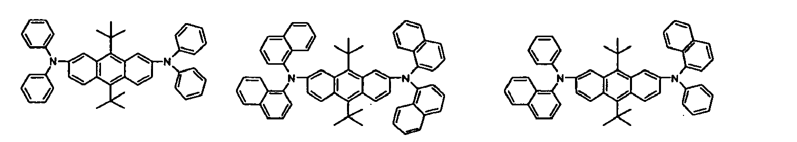

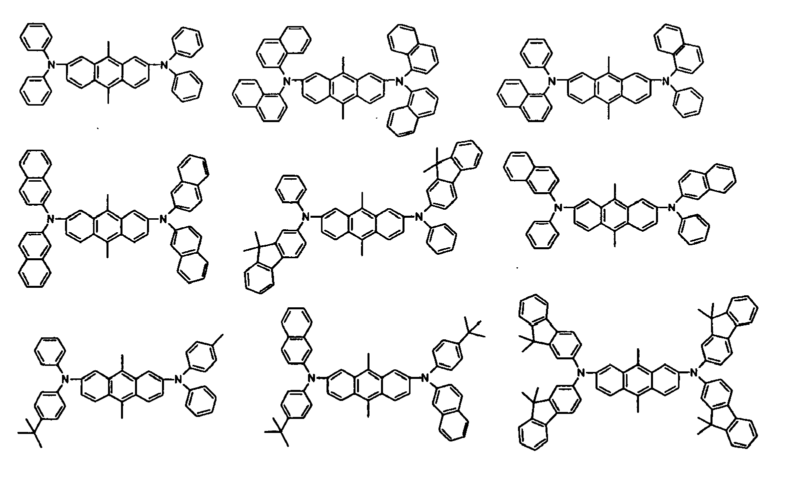

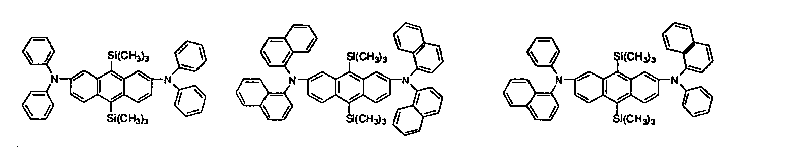

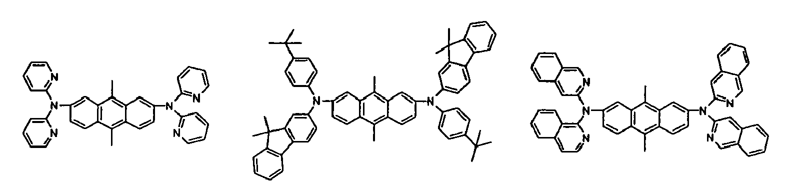

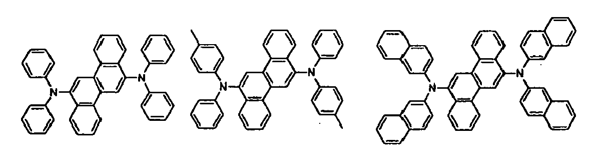

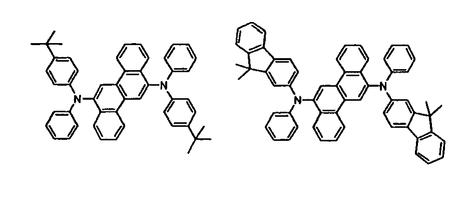

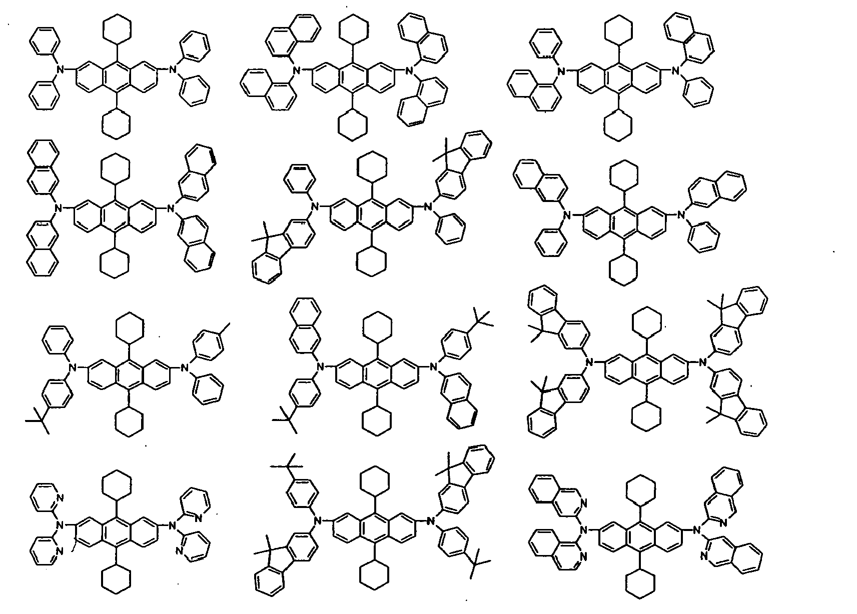

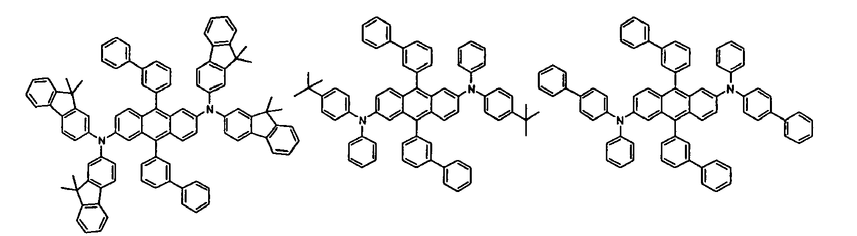

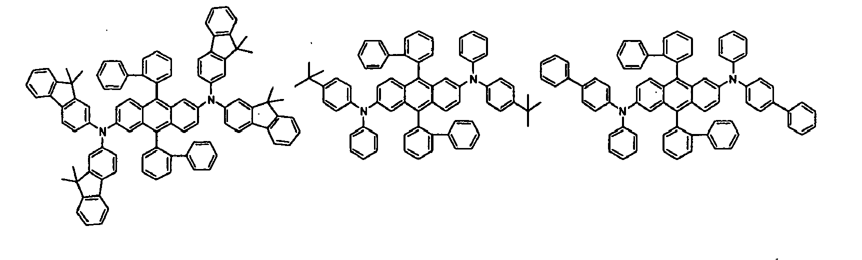

The organic electroluminescent device according to the invention may further comprise one or more compound(s) selected from a group consisting of arylamine compounds and styrylarylamine compounds, as well as the organic electroluminescent compound represented by Chemical Formula (1). Examples of the arylamine or styrylarylamine compounds include the compounds represented by Chemical Formula (8), but they are not restricted thereto: 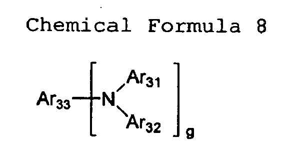 wherein, Ar₃₁ and Ar₃₂ independently represent (C1-C60)alkyl, (C6-C60)aryl, (C4-C60)heteroaryl, (C6-C60)arylamino, (C1-C60)alkylamino, a 5- or 6-membered heterocycloalkyl containing one or more heteroatom(s) selected from N, O and S, or (C3-C60)cycloalkyl, or Ar₃₁ and Ar₃₂ may be linked via (C3-C60)alkylene or (C3-C60)alkenylene with or without a fused ring to form an alicyclic ring, or a monocyclic or polycyclic aromatic ring; the aryl, heteroaryl, arylamino or heterocycloalkyl of Ar₃₁ and Ar₃₂ may be further substituted by one or more substituent(s) selected from halogen, (C1-C60)alkyl, (C2-C60)alkenyl, (C2-C60)alkynyl, (C6-C60)aryl, (C4-C60)heteroaryl, a 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro and hydroxyl;

Ar₃₃ represents (C6-C60)aryl, (C5-C60)heteroaryl or (C6-C60)arylamino; the aryl, heteroaryl or arylamino of Ar₃₃ may be further substituted by one or more substituent(s) selected from halogen, (C1-C60)alkyl, (C6-C60)aryl, (C4-C60)heteroaryl, a 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro and hydroxyl; and
g is an integer from 1 to 4.

The arylamine compounds and styrylarylamine compounds may be more specifically exemplified by the following compounds, but are not restricted thereto.

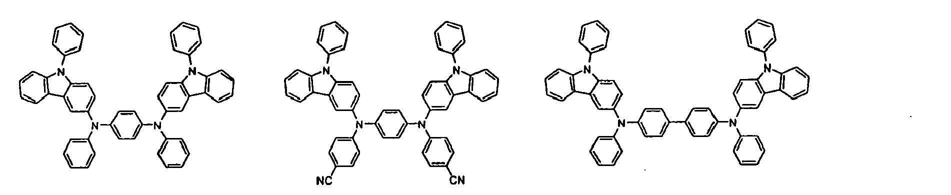

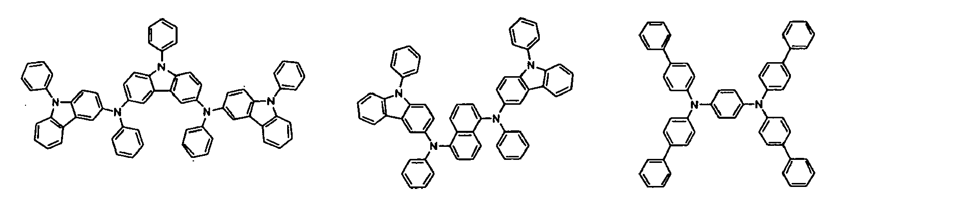

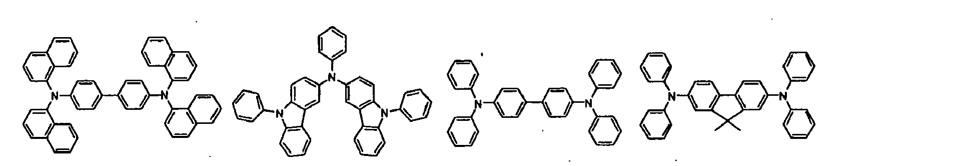

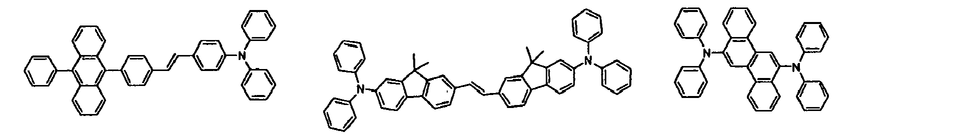

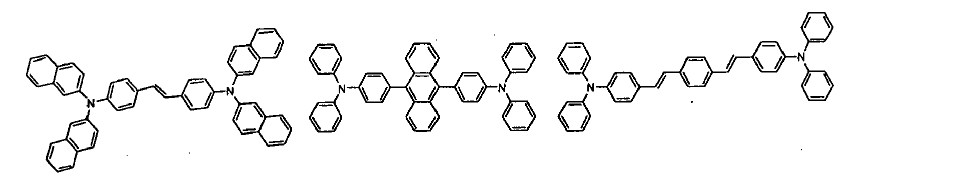

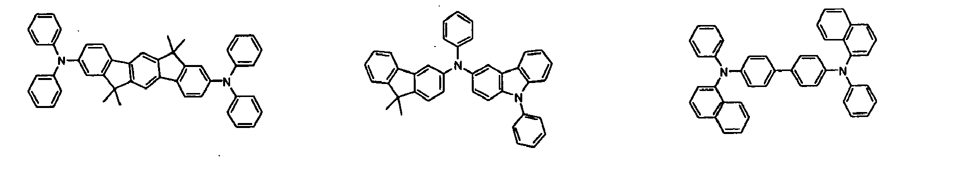

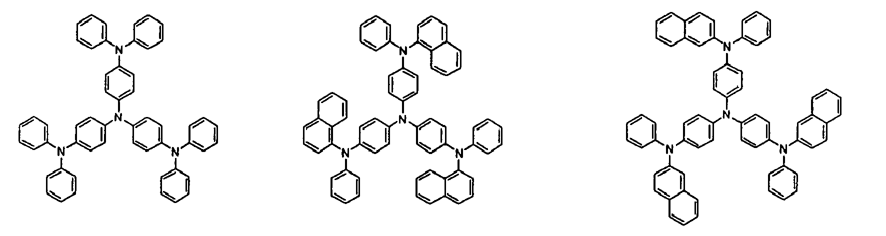

In an organic electroluminescent device according to the present invention, the organic layer may further comprise one or more metal(s) selected from a group consisting of organic metals of Group 1, Group 2, 4^{th} period and 5^{th} period transition metals, lanthanide metals and d-transition elements, as well as the organic electroluminescent compound represented by Chemical Formula (1). The organic layer may comprise a charge generating layer in addition to the electroluminescent layer.

The present invention can realize an organic electroluminescent device having a pixel structure of independent light-emitting mode, which comprises an organic electroluminescent device containing the compound of Chemical Formula (1) as a sub-pixel and one or more sub-pixel(s) comprising one or more metallic compound(s) selected from a group consisting of Ir, Pt, Pd, Rh, Re, Os, Tl, Pb, Bi, In, Sn, Sb, Te, Au and Ag, patterned in parallel at the same time.

Further, the organic electroluminescent device is a white electroluminescent device wherein the organic layer comprises, in addition to the organic electroluminescent compound according to the invention, one or more compound(s) selected from compounds having electroluminescent peak of wavelength of not more than 500 nm, and those having the wavelength of not less than 560 nm, at the same time. Those compounds can be exemplified by the compounds represented by one of Chemical Formulas (9) to (18), but they are not restricted thereto. A white electroluminescent device

M¹L²¹L²²L²³ Chemical Formula 9

In Chemical Formula (9), M¹ is selected from metals from Group 7, 8, 9, 10, 11, 13, 14, 15 and 16 in the Periodic Table of Elements, and ligands L²¹, L²² and L²³ are independently selected from the following structures: 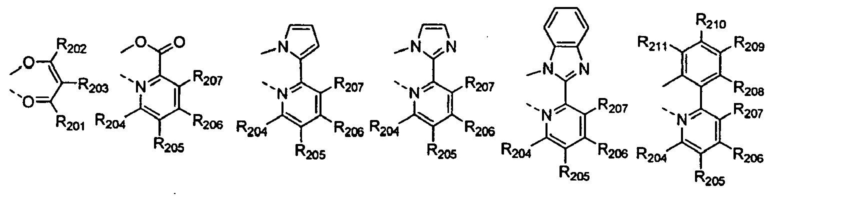 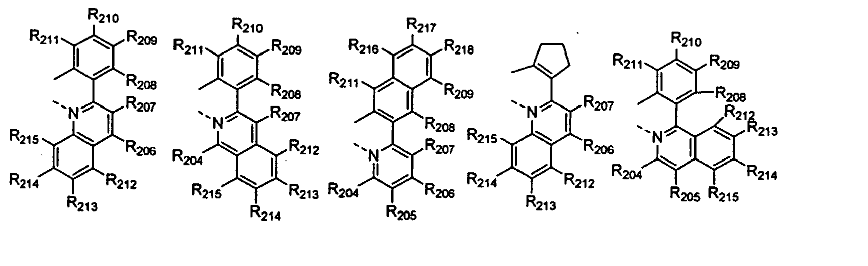 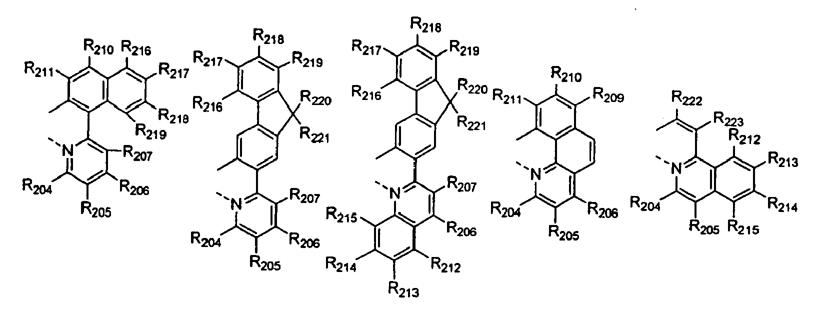 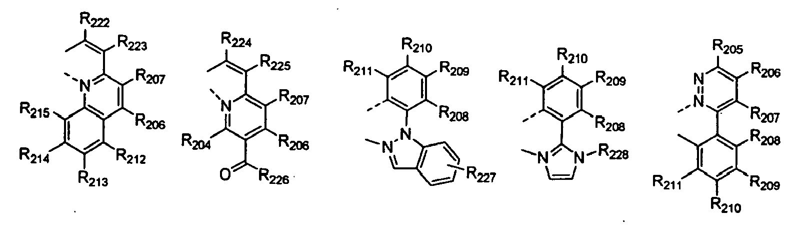 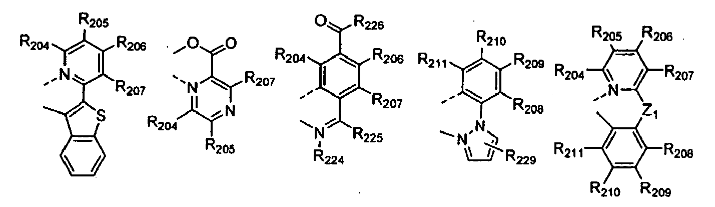
wherein, R₂₀₁ through R₂₀₃ independently represent hydrogen, (C1-C60)alkyl with or without halogen substituent(s), (C6-C60)aryl with or without (C1-C60)alkyl substituent(s), or halogen;
R₂₀₄ through R₂₁₉ independently represent hydrogen, (C1-C60)alkyl, (C1-C30)alkoxy, (C3-C60)cycloalkyl, (C2-C30)alkenyl, (C6-C60)aryl, mono or di(C1-C30)alkylamino, mono or di(C6-30)arylamino, SF₅, tri(C1-C30)alkylsilyl, di(C1-C30)alkyl(C6-C30)arylsilyl, tri(C6-C30)arylsilyl, cyano or halogen, and the alkyl, cycloalkyl, alkenyl or aryl of R₂₀₄ through R₂₁₉ may be further substituted by one or more substituent(s) selected from (C1-C60)alkyl, (C6-C60)aryl and halogen;
R₂₂₀ through R₂₂₃ independently represent hydrogen, (C1-C60)alkyl with or without halogen substituent(s), (C6-C60)aryl with or without (C1-C60)alkyl substituent(s);
R₂₂₄ and R₂₂₅ independently represent hydrogen, linear or branched (C1-C60)alkyl, (C6-C60)aryl or halogen, or R₂₂₄ and R₂₂₅ may be linked via (C3-C12)alkylene or (C3-C12)alkenylene with or without a fused ring to form an alicyclic ring, or a monocyclic or polycyclic aromatic ring; the alkyl or aryl of R₂₂₄ and R₂₂₅, or the alicyclic ring, or the monocyclic or polycyclic aromatic ring formed therefrom via (C3-C12)alkylene or (C3-C12)alkenylene with or without a fused ring may be further substituted by one or more substituent(s) selected from linear or branched (C1-C60)alkyl with or without halogen substituent(s), (C1-C30)alkoxy, halogen, tri(C1-C30)alkylsilyl, tri(C6-C30)arylsilyl and (C6-C60)aryl;
R₂₂₆ represents (C1-C60)alkyl, (C6-C60)aryl, (C5-C60)heteroaryl or halogen;
R₂₂₇ through R₂₂₉ independently represent hydrogen, (C1-C60)alkyl, (C6-C60)aryl or halogen; the alkyl or aryl of R₂₂₆ through R₂₂₉ may be further substituted by halogen or (C1-C60)alkyl;
Z₁ represents 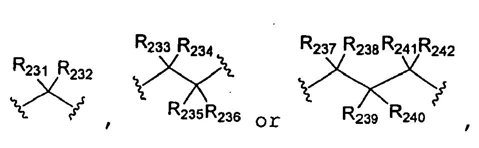 and R₂₃₁ through R₂₄₂ independently represent hydrogen, (C1-C60)alkyl with or without halogen substituent(s), (C1-C30)alkoxy, halogen, (C6-C60)aryl, cyano or (C5-C60)cycloalkyl, or each of R₂₃₁ through R₂₄₂ may be linked to an adjacent substituent via alkylene or alkenylene to form a (C5-C7) spiro-ring or a (C5-C9) fused ring, or each of them may be linked to R₂₀₇ or R₂₀₈ via alkylene or alkenylene to form a (C5-C7) fused ring.

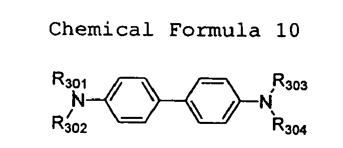

In Chemical Formula (10), R₃₀₁ through R₃₀₄ independently represent (C1-C60)alkyl or (C6-C60)aryl, or each of them may be linked to an adjacent substituent via (C3-C60)alkylene or (C3-C60)alkenylene with or without a fused ring to form an alicyclic ring, or a monocyclic or polycyclic aromatic ring; and the alkyl or aryl of R₃₀₁ through R₃₀₄, or the alicyclic ring, or the monocyclic or polycyclic aromatic ring formed therefrom by linkage via (C3-C60)alkylene or (C3-C60)alkenylene with or without a fused ring may be further substituted by one or more substituent(s) selected from (C1-C60)alkyl with or without halogen substituent(s), (C1-C60)alkoxy, halogen, tri(C1-C60)alkylsilyl, tri(C6-C60)arylsilyl and (C6-C60)aryl.

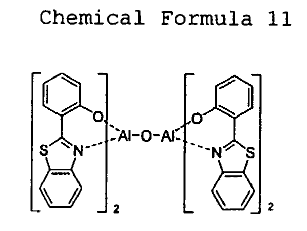

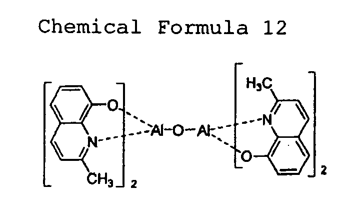

L²⁴L²⁵M²(Q)ₕ Chemical Formula 13

In Chemical Formula (13), the ligands, L²⁴ and L²⁵ are independently selected from the following structures: 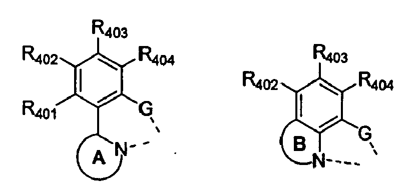
M² is a bivalent or trivalent metal;
h is 0 when M² is a bivalent metal, while h is 1 when M² is a trivalent metal;
Q represents (C6-C60)aryloxy or tri(C6-C60)arylsilyl, and the aryloxy and triarylsilyl of Q may be further substituted by (C1-C60)alkyl or (C6-C60)aryl;
G represents O, S or Se;
ring A represents oxazole, thiazole, imidazole, oxadiazole, thiadiazole, benzoxazole, benzothiazole, benzimidazole, pyridine or quinoline;
ring B represents pyridine or quinoline, and ring B may be further substituted by (C1-C60)alkyl, or phenyl or naphthyl with or without (C1-C60)alkyl substituent(s);
R₄₀₁ through R₄₀₄ independently represent hydrogen, (C1-C60)alkyl, halogen, tri(C1-C60)alkylsilyl, tri(C6-C60)arylsilyl or (C6-C60)aryl, or each of them may be linked to an adjacent substituent via (C3-C60)alkylene or (C3-C60)alkenylene to form a fused ring; the pyridine or quinoline may form a chemical bond with R₄₀₁ to form a fused ring; and
ring A or the aryl group of R₄₀₁ through R₄₀₄ may be further substituted by (C1-C60)alkyl, halogen, (C1-C60)alkyl with halogen substituent(s), phenyl, naphthyl, tri(C1-C60)alkylsilyl, tri(C6-C60)arylsilyl or amino group.

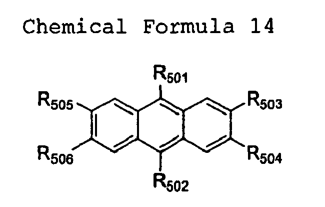

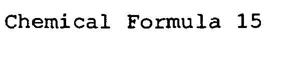

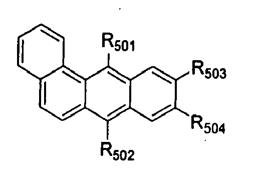

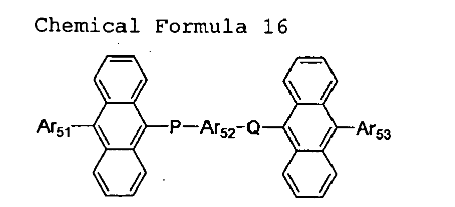

In Chemical Formulas (14) through (16),
R₅₀₁ and R₅₀₂ independently represent (C6-C60)aryl, (C4-C60)heteroaryl, a 5- or 6-membered heterocycloalkyl containing one or more heteroatom(s) selected from N, O and S, or (C3-C60)cycloalkyl, and the aryl or heteroaryl of R₆₁ and R₆₂ may be further substituted by one or more substituent(s) selected from a group consisting of (C1-C60)alkyl, halo(C1-C60)alkyl, (C1-C60)alkoxy, (C3-C60)cycloalkyl, (C6-C60)aryl, (C4-C60)heteroaryl, halogen, cyano, tri(C1-C60)alkylsilyl, di(C1-C60)alkyl(C6-C60)arylsilyl and tri(C6-C60)arylsilyl;
R₅₀₃ through R₅₀₆ independently represent hydrogen, (C1-C60)alkyl, (C1-C60)alkoxy, halogen, (C4-C60)heteroaryl, (C5-C60)cycloalkyl or (C6-C60)aryl, and the heteroayl, cycloalkyl or aryl of R₅₀₃ through R₅₀₆ may be further substituted by one or more substituent(s) selected from a group consisting of (C1-C60)alkyl with or without halogen substituent(s), (C1-C60)alkoxy, (C3-C60)cycloalkyl, halogen, cyano, tri(C1-C60)alkylsilyl, di(C1-C60)alkyl(C6-C60)arylsilyl and tri(C6-C60)arylsilyl;
P and Q independently represent a chemical bond, or (C6-C60)arylene with or without one or more substituent(s) selected from (C1-C60)alkyl, (C1-C60)alkoxy, (C6-C60)aryl, (C4-C60)heteroaryl and halogen;
Ar₅₁ and Ar₅₃ represent (C4-C60)heteroaryl or aryl selected from the following structures: 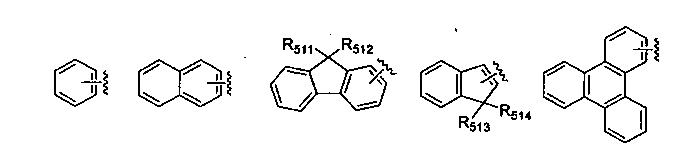 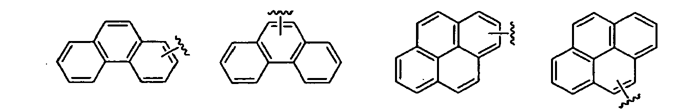 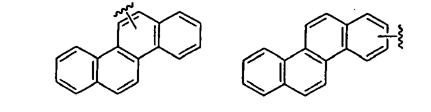
the aryl or heteroaryl of Ar₅₁ and Ar₅₃ may be substituted by one or more substituent(s) selected from (C1-C60)alkyl, (C1-C60)alkoxy, (C6-C60)aryl and (C4-C60)heteroaryl;
Ar₅₂ represents (C6-C60)arylene, (C4-C60)heteroarylene or a compound represented by the following structural formula: 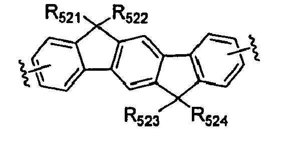
the arylene or heteroarylene of Ar₅₂ may be substituted by one or more substituent(s) selected from (C1-C60)alkyl, (C1-C60)alkoxy, (C6-C60)aryl, (C4-C60)heteroaryl and halogen;
R₅₁₁ through R₅₁₄ independently represent hydrogen, (C1-C60)alkyl or (C6-C60)aryl, or each of them may be linked to an adjacent substituent via (C3-C60)alkylene or (C3-C60)alkenylene with or without a fused ring to form an alicyclic ring, or a monocyclic or polycyclic aromatic ring; and
R₅₂₁ through R₅₂₄ independently represent hydrogen, (C1-C60)alkyl, (C1-C60)alkoxy, (C6-C60)aryl, (C4-C60)heteroaryl or halogen, or each of them may be linked to an adjacent substituent via (C3-C60)alkylene or (C3-C60)alkenylene with or without a fused ring to form an alicyclic ring, or a monocyclic or polycyclic aromatic ring.

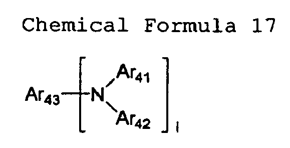

In Chemical Formula (17), Ar₄₁ and Ar₄₂ independently represent (C1-C60)alkyl, (C6-C60)aryl, (C4-C60)heteroaryl, (C6-C60)arylamino, (C1-C60)alkylamino, 5- or 6-membered heterocycloalkyl containing one or more heteroatom(s) selected from N, O and S, or (C3-C60)cycloalkyl, or Ar₄₁ and Ar₄₂ may be linked via (C3-C60)alkylene or (C3-C60)alkenylene with or without a fused ring to form an alicyclic ring, or a monocyclic or polycyclic aromatic ring; the alkyl, aryl, heteroaryl, arylamino, alkylamino, cycloalkyl or heterocycloalkyl of Ar₄₁ and Ar₄₂ may be further substituted by one or more substituent(s) selected from halogen, (C1-C60)alkyl, (C6-C60)aryl, (C4-C60)heteroaryl, a 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro and hydroxyl;
Ar₄₃ represents (C6-C60)arylene, (C4-C60)heteroarylene or arylene represented by one of the following structural formulas: 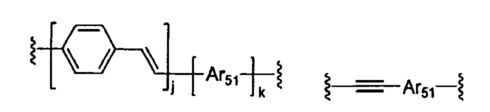 wherein, Ar₅₁ represents (C6-C60)arylene, (C4-C60)heteroarylene;
the arylene or heteroarylene of Ar₄₃ and Ar₅₁ may be further substituted by one or more substituent(s) selected from halogen, (C1-C60)alkyl, (C6-C60)aryl, (C4-C60)heteroaryl, a 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro and hydroxyl;
i is an integer from 1 to 4,
j is an integer from 1 to 4; and
k is an integer of 0 or 1.

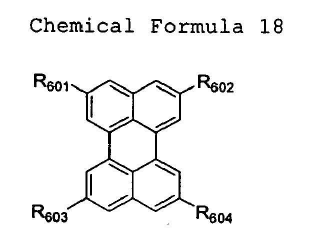

In Chemical Formula (18), R₆₀₁ through R₆₀₄ independently represent hydrogen, halogen, (C1-C60)alkyl, (C6-C60)aryl, (C4-C60)heteroaryl, a 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60) arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or each of R₆₀₁ through R₆₀₄ may be linked to an adjacent substituent via (C3-C60)alkylene or (C3-C60)alkenylene with or without a fused ring to form an alicyclic ring, or a monocyclic or polycyclic aromatic ring; and
the alkyl, alkenyl, alkynyl, cycloalkyl, heterocycloalkyl, aryl, heteroaryl, arylsilyl, alkylsilyl, alkylamino or arylamino of R₆₀₁ through R₆₀₄, or the alicyclic ring, or the monocyclic or polycyclic aromatic ring formed therefrom by linkage to an adjacent substituent via (C3-C60)alkylene or (C3-C60)alkenylene with or without a fused ring may be further substituted by one or more substituent(s) selected from halogen, (C1-C60)alkyl, (C6-C60)aryl, (C4-C60)heteroaryl, a 5-or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro and hydroxyl.

The compounds having electroluminescent peak of wavelength of not more than 500 nm, or those having electroluminescent peak of wavelength of not less than 560 nm, can be exemplified by the following compounds, but they are not restricted thereto.

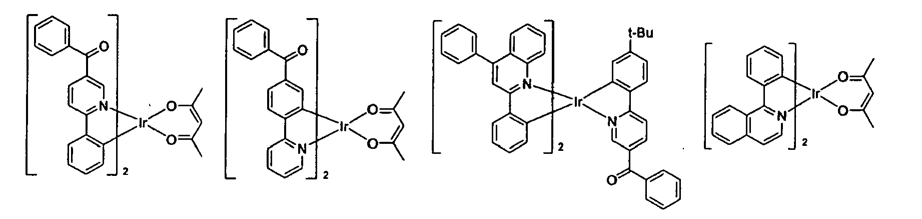

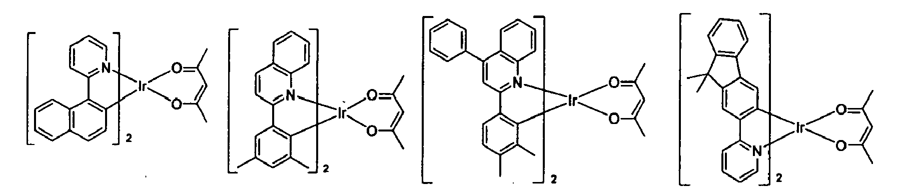

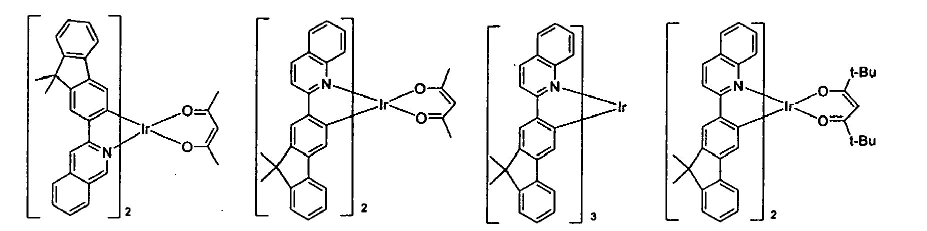

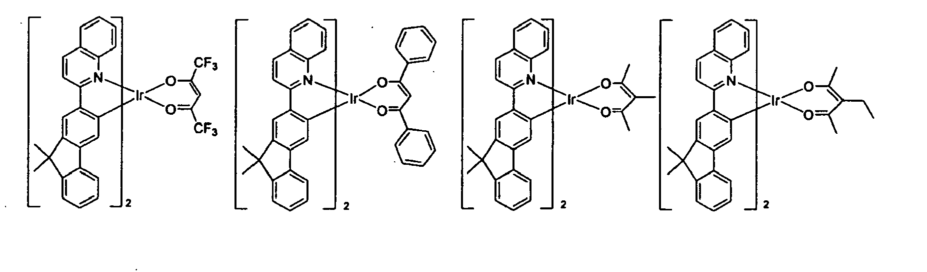

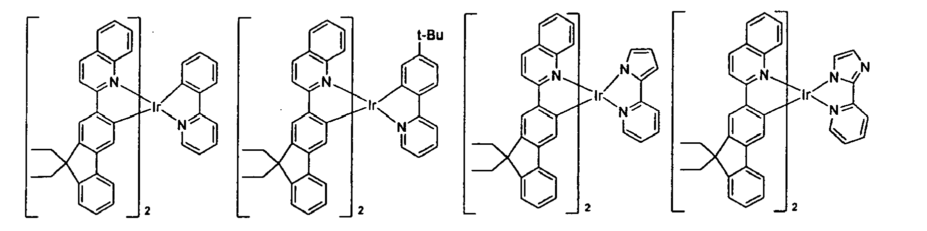

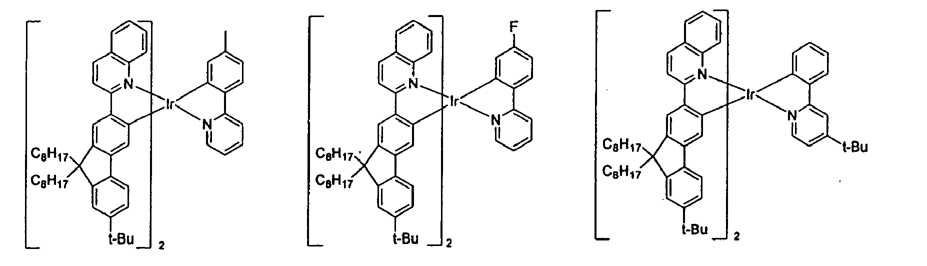

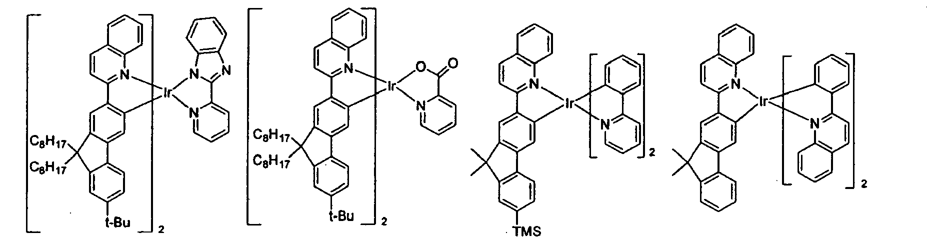

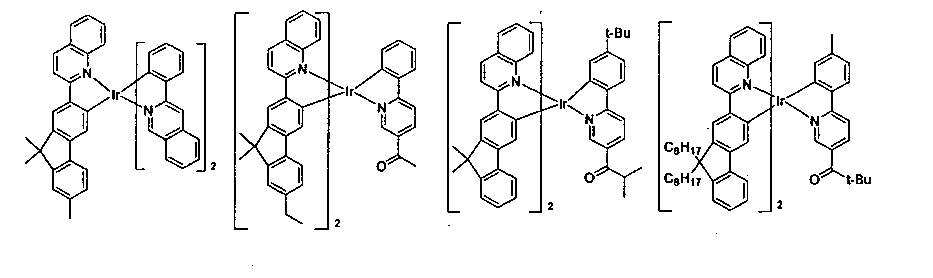

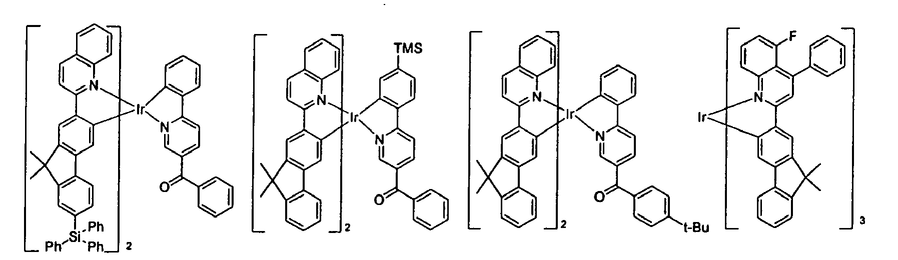

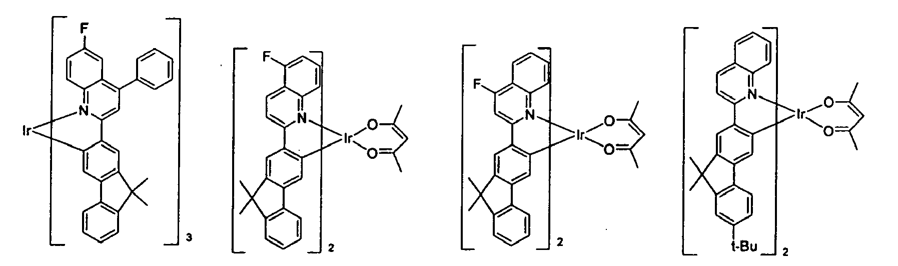

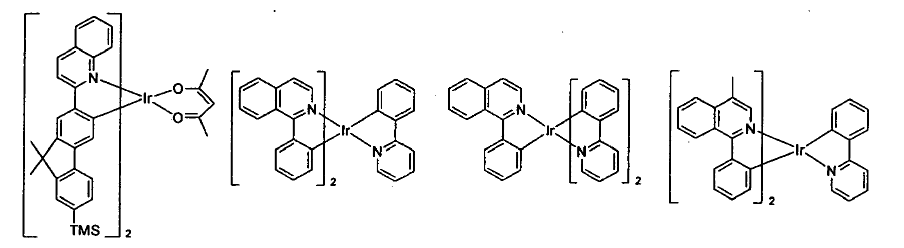

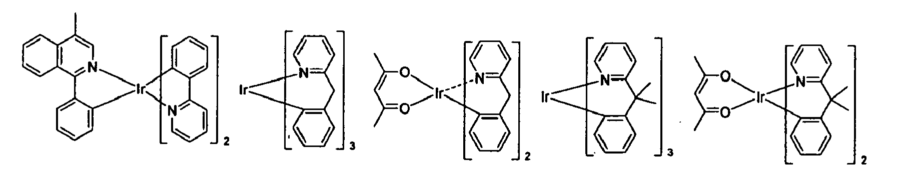

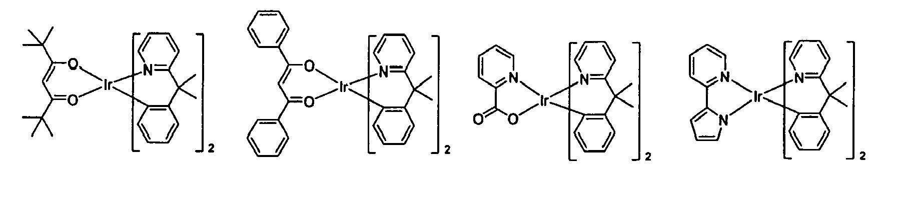

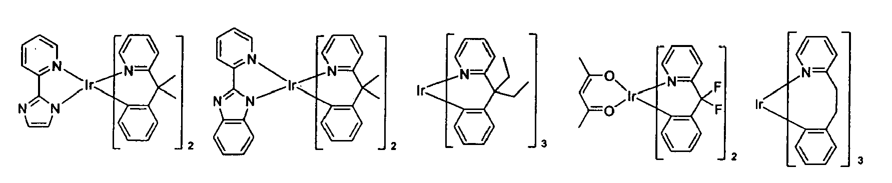

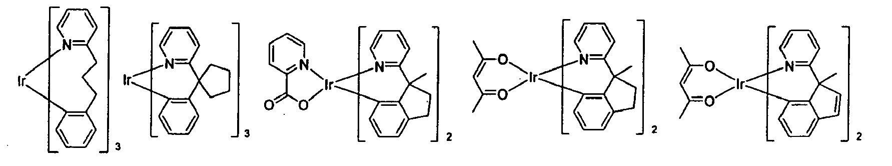

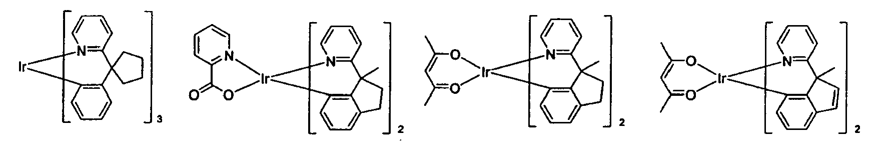

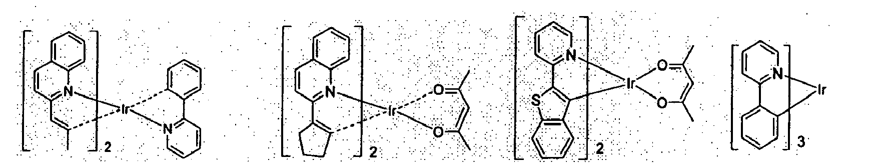

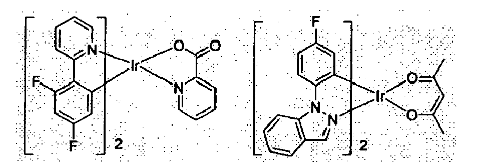

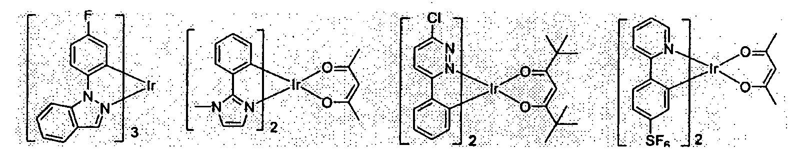

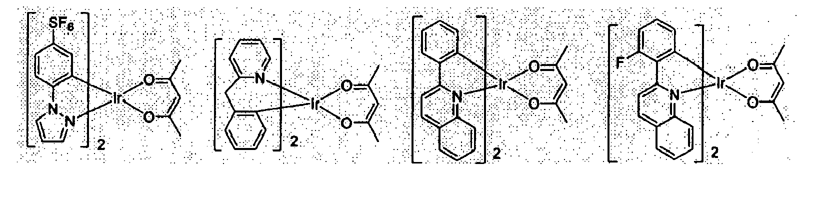

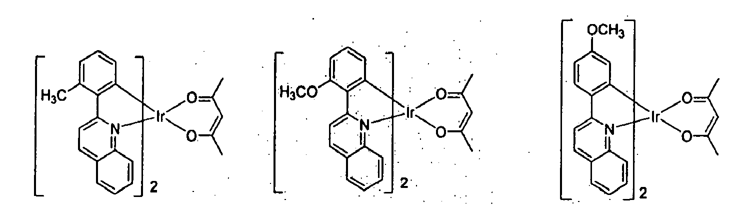

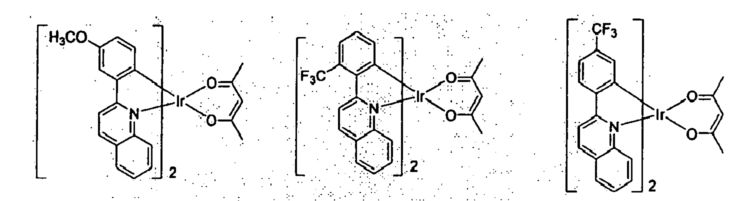

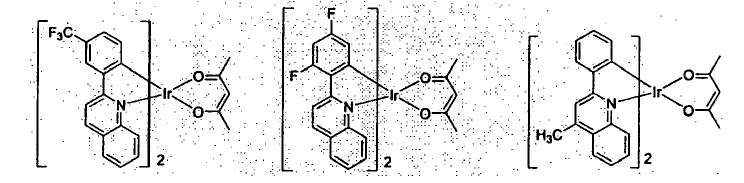

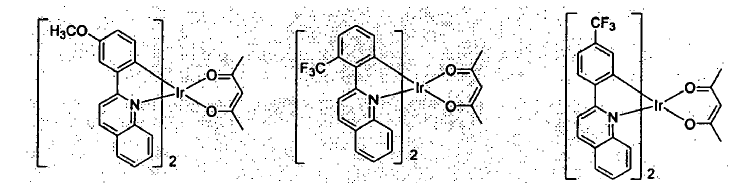

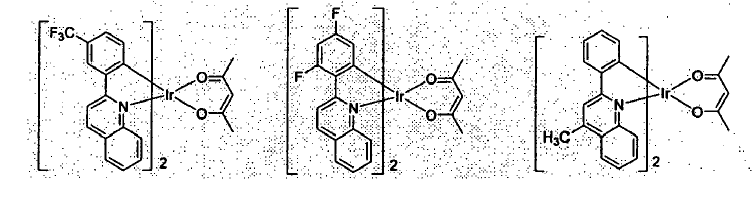

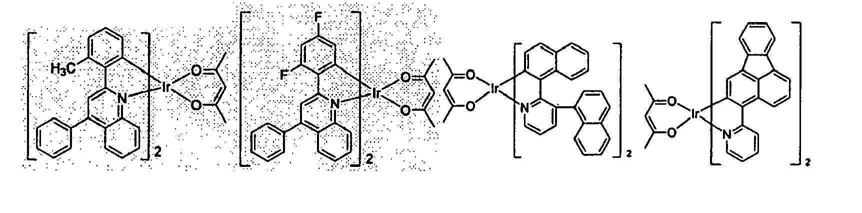

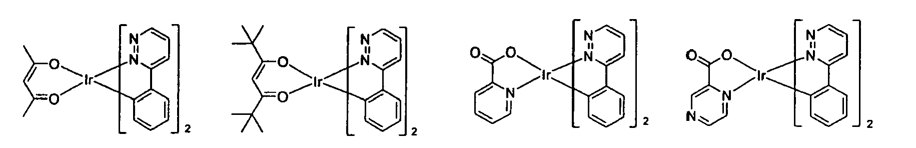

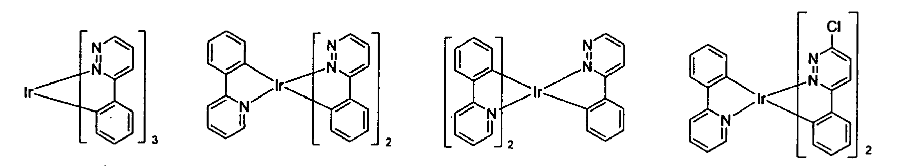

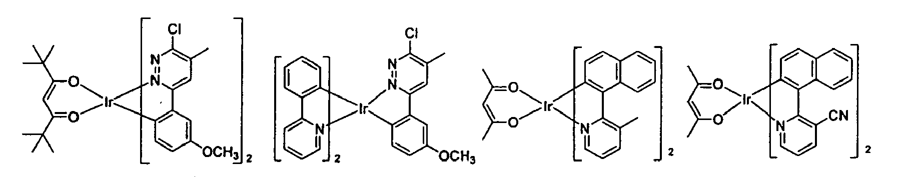

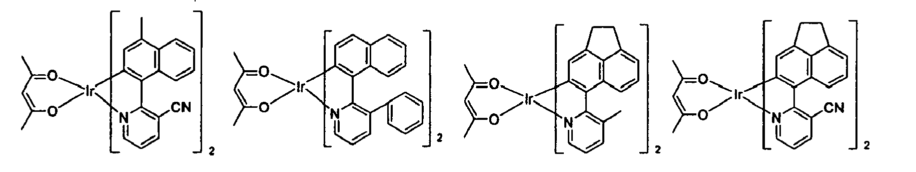

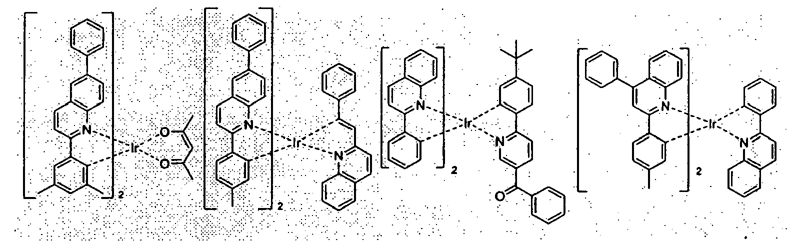

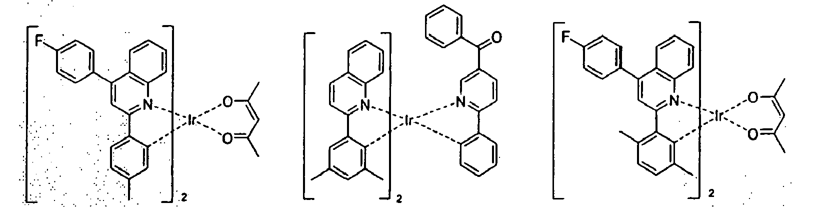

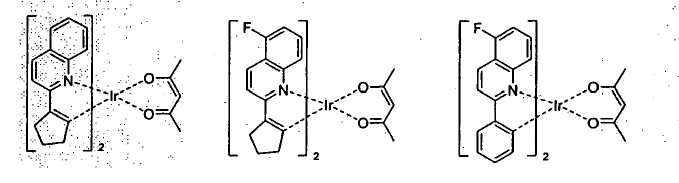

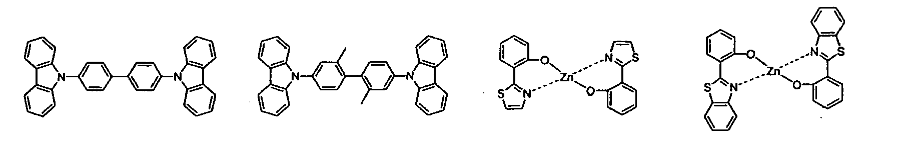

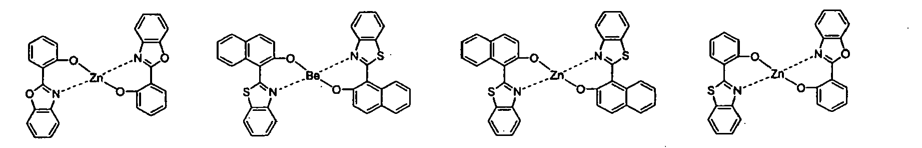

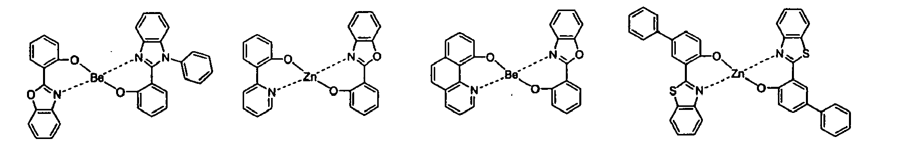

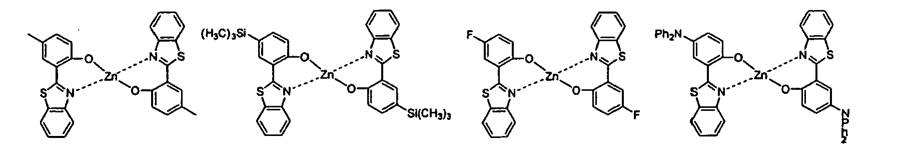

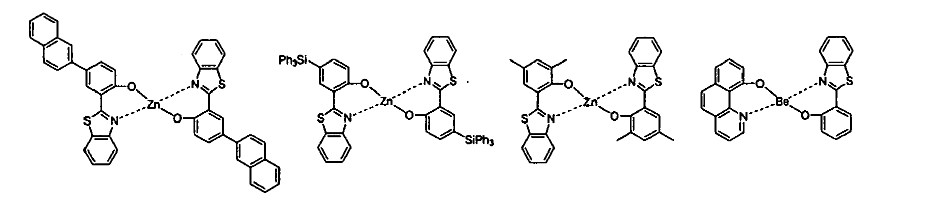

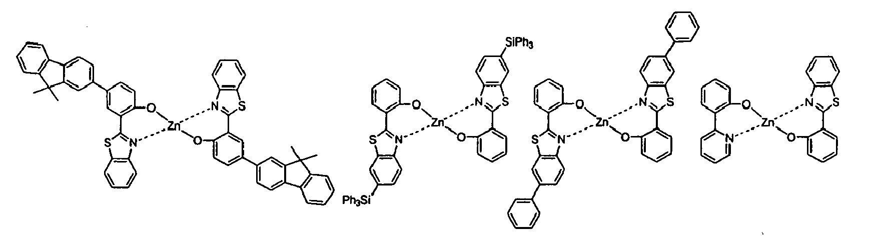

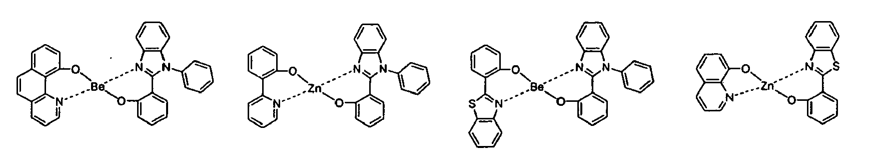

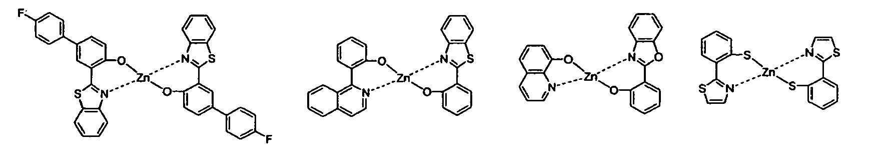

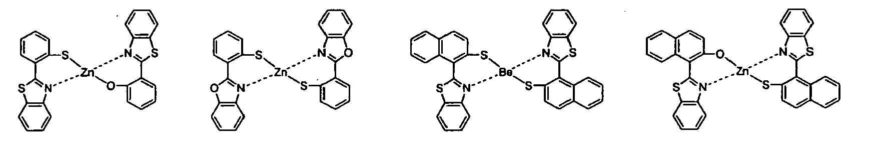

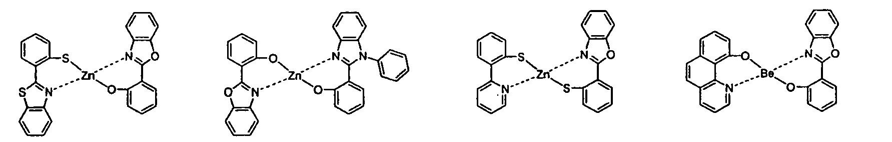

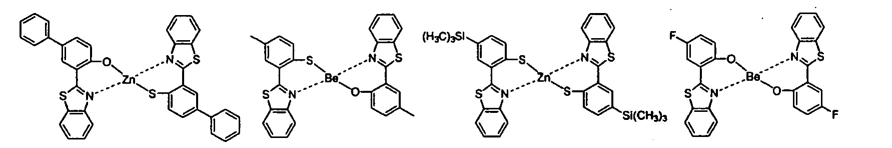

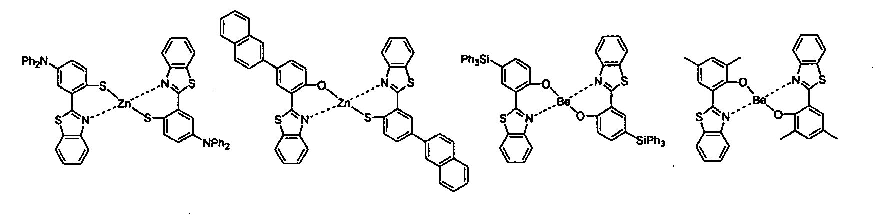

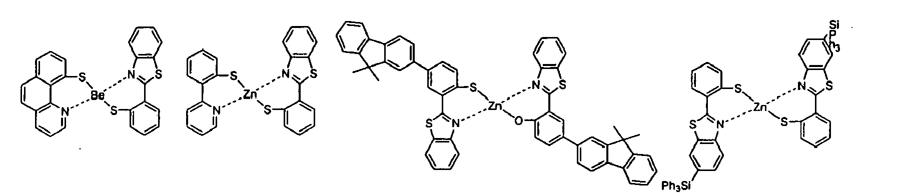

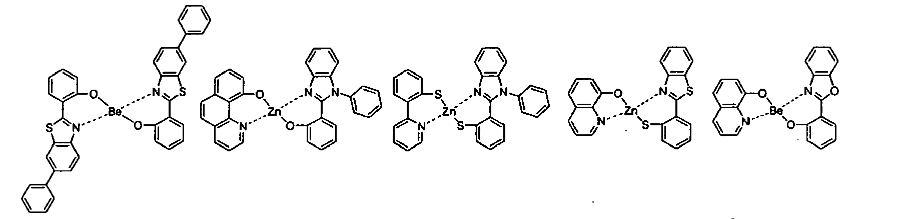

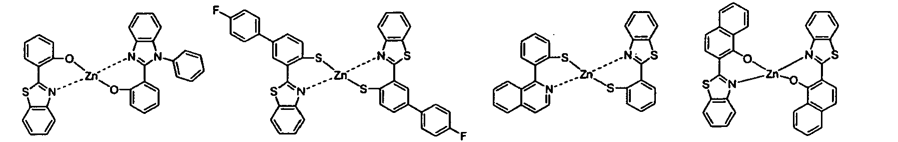

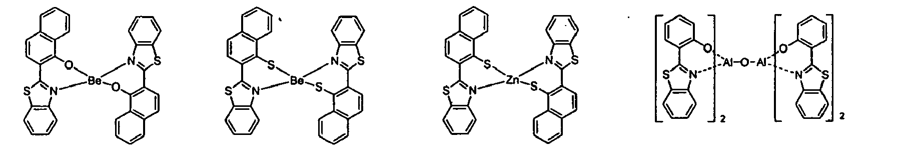

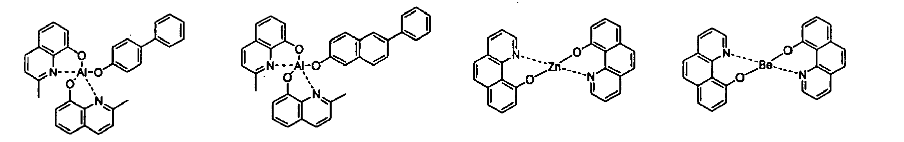

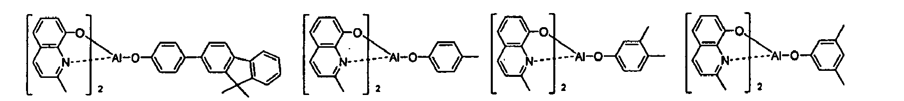

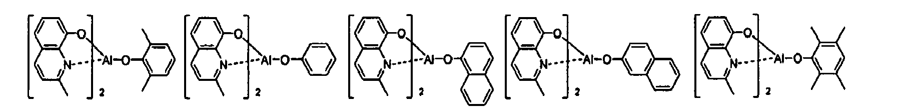

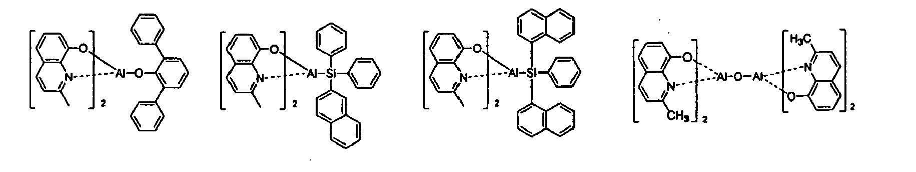

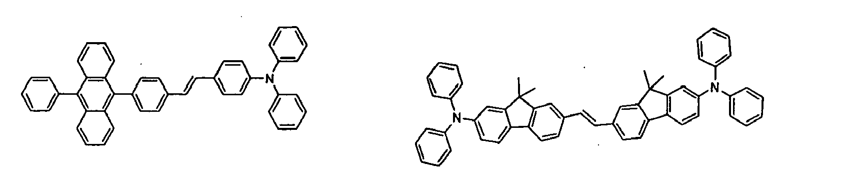

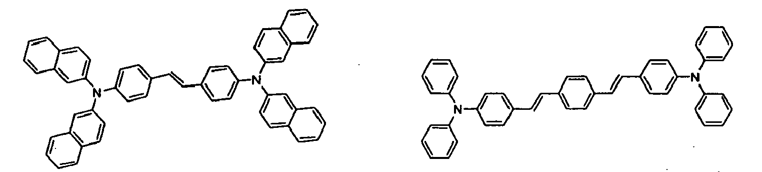

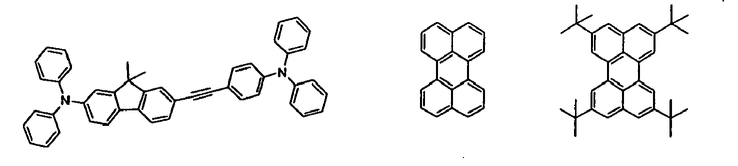

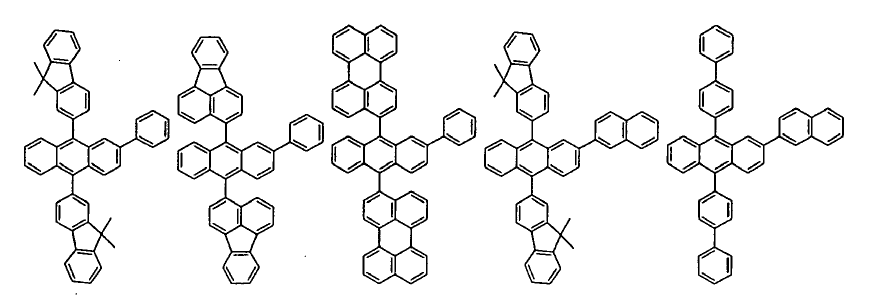

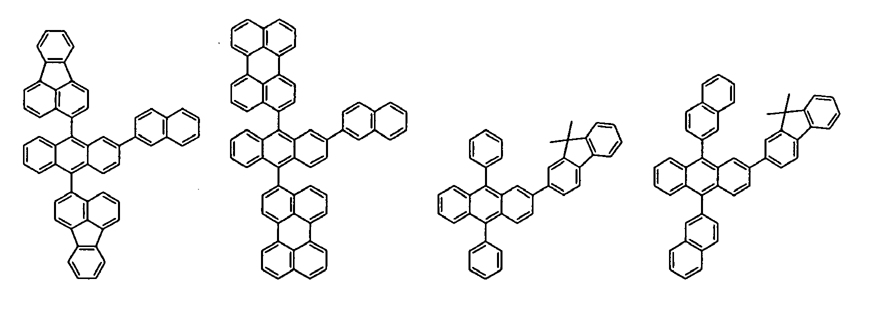

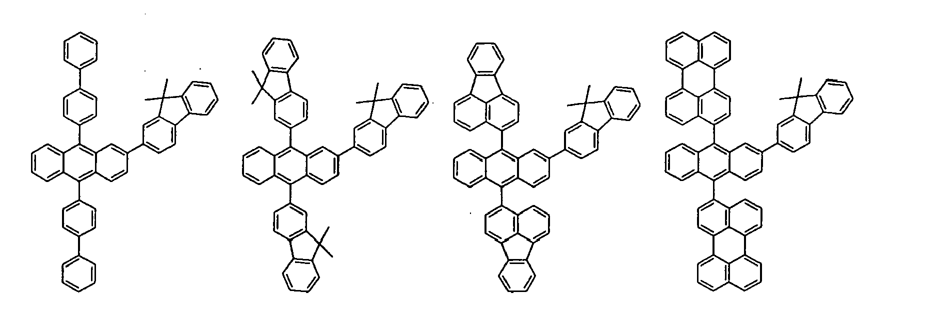

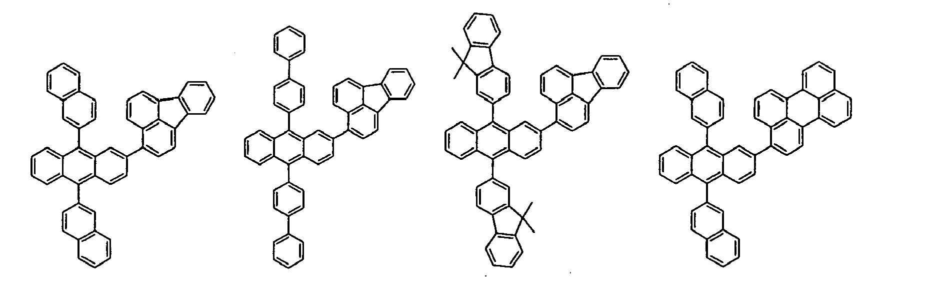

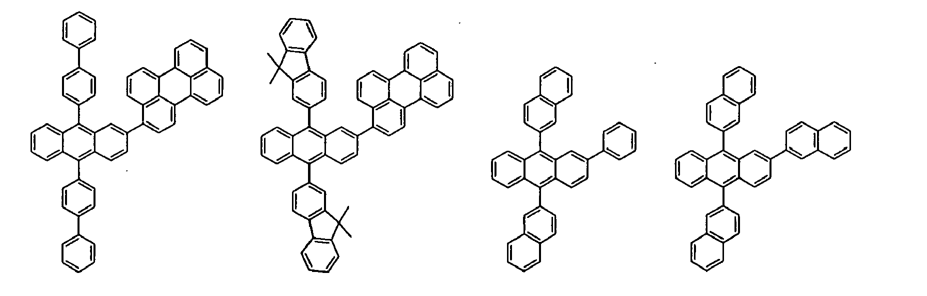

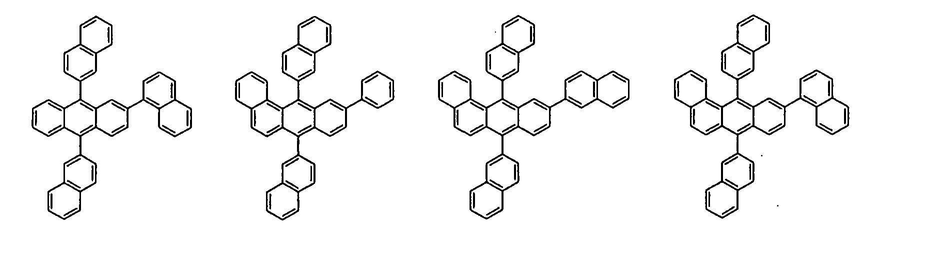

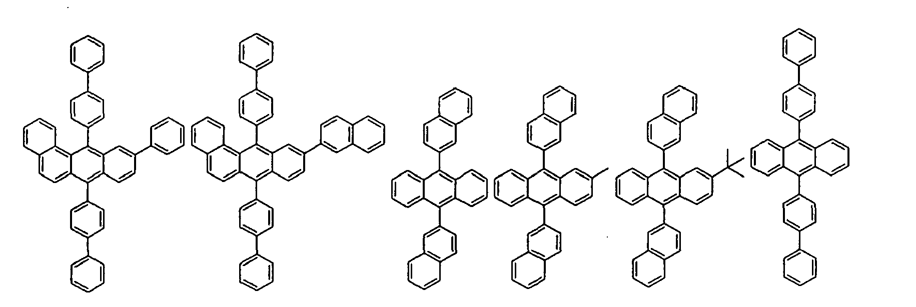

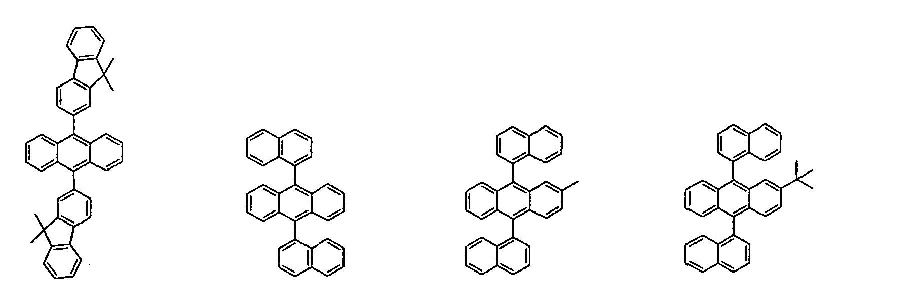

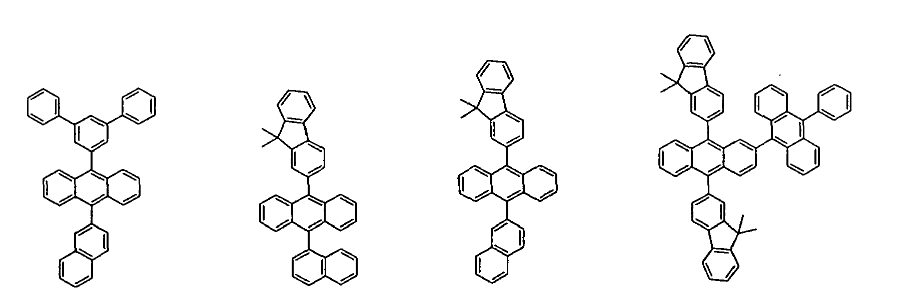

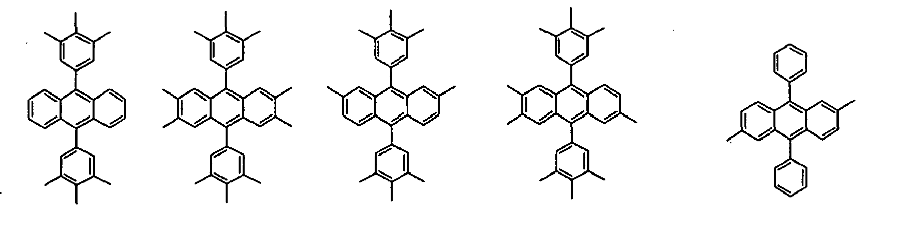

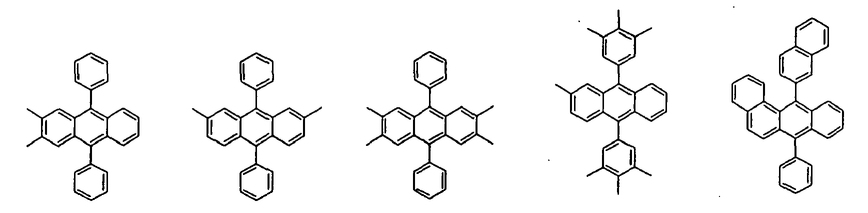

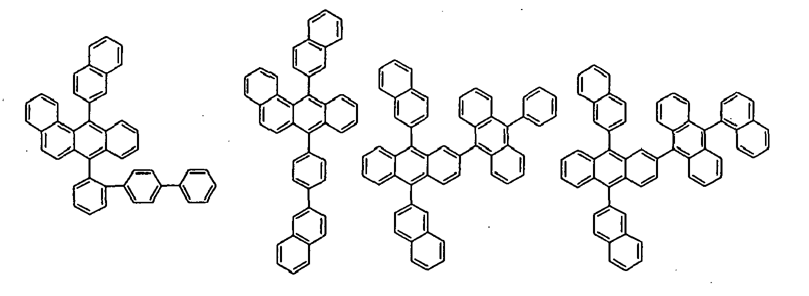

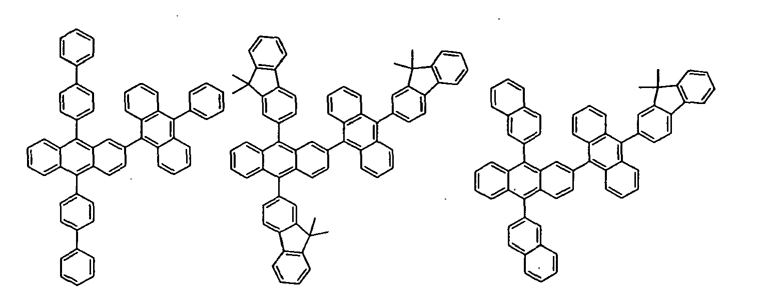

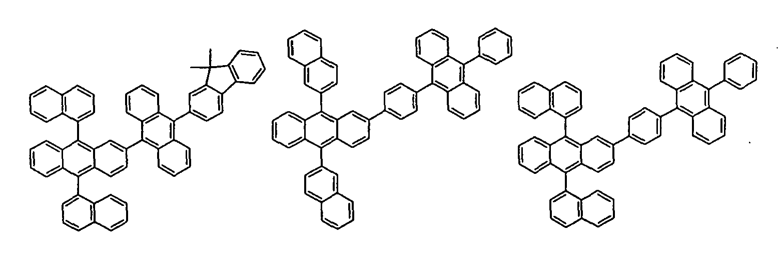

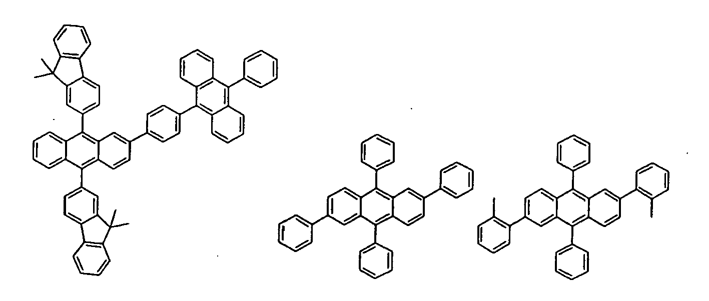

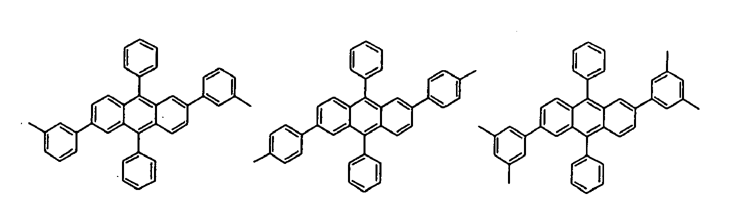

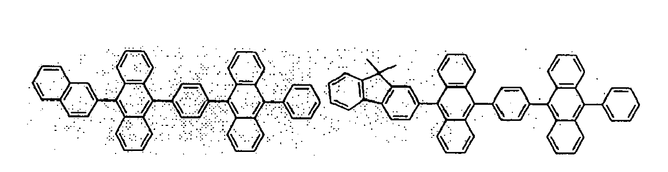

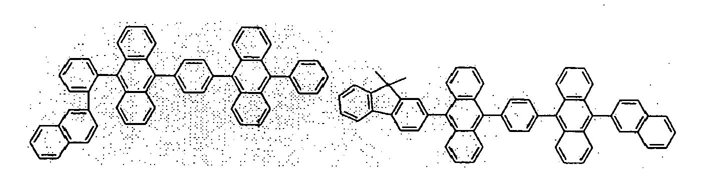

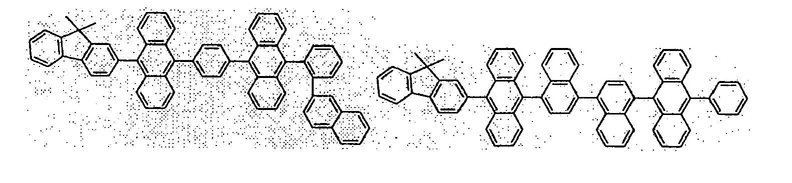

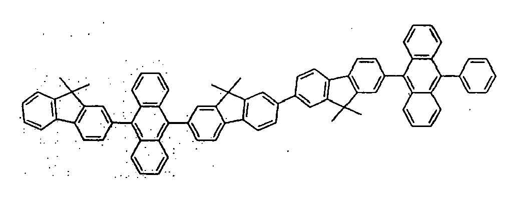

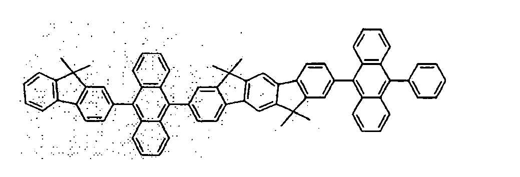

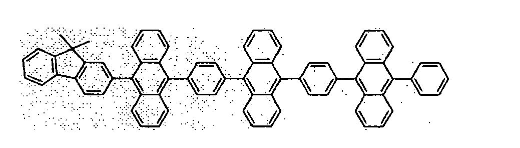

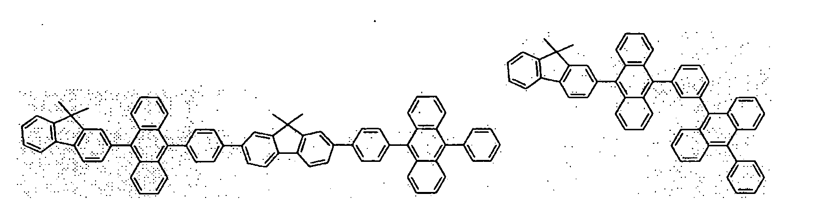

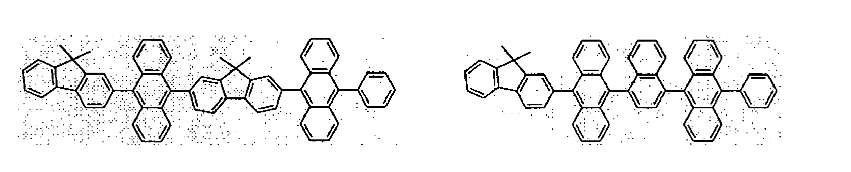

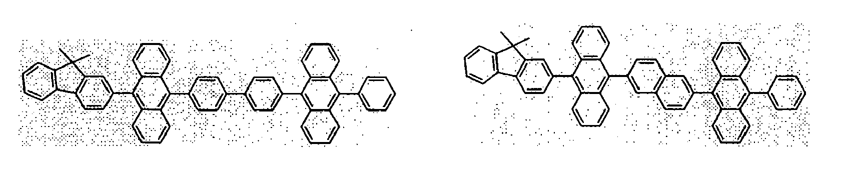

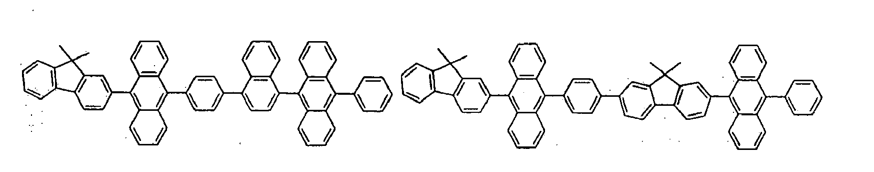

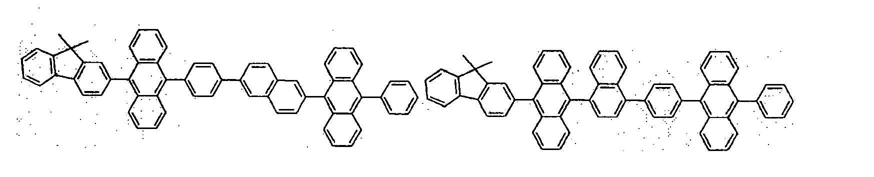

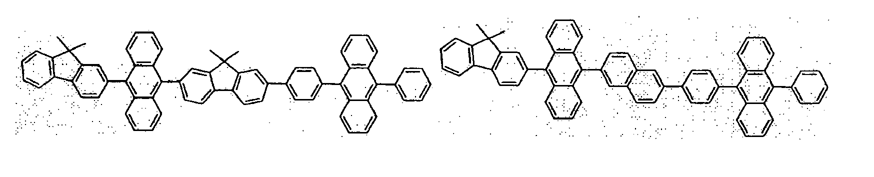

In an organic electroluminescent device according to the present invention, it is preferable to place one or more layer(s) (here-in-below, referred to as the "surface layer") selected from chalcogenide layers, metal halide layers and metal oxide layers, on the inner surface of at least one side of the pair of electrodes. Specifically, it is preferable to arrange a chalcogenide layer of silicon and aluminum metal (including oxides) on the anode surface of the EL medium layer, and a metal halide layer or a metal oxide layer on the cathode surface of the EL medium layer. As the result, stability in operation can be obtained.

Examples of chalcogenides preferably include SiOₓ (1≤X≤2), AlOₓ (1≤X≤1.5), SiON, SiAlON, or the like. Examples of metal halides preferably include LiF, MgF₂, CaF₂, fluorides of rare earth metal, or the like. Examples of metal oxides preferably include Cs₂O, Li₂O, MgO, SrO, BaO, CaO, or the like.

In an organic electroluminescent device according to the present invention, it is also preferable to arrange, on at least one surface of the pair of electrodes thus manufactured, a mixed region of electron transport compound and a reductive dopant, or a mixed region of a hole transport compound with an oxidative dopant. Accordingly, the electron transport compound is reduced to an anion, so that injection and transportation of electrons from the mixed region to an EL medium are facilitated. In addition, since the hole transport compound is oxidized to form a cation, injection and transportation of holes from the mixed region to an EL medium are facilitated. Preferable oxidative dopants include various Lewis acids and acceptor compounds. Preferable reductive dopants include alkali metals, alkali metal compounds, alkaline earth metals, rare-earth metals, and mixtures thereof.

The organic compounds according to the invention, having excellent luminous efficiency and life property of material, can be advantageously employed for manufacturing OLED's having very good operation life.

### Best Mode

The present invention is further described with respect to the representative compounds of the invention, by describing the organic electroluminescent compounds, the processes for preparing the same, and luminescent properties of the device manufactured therefrom in the Examples below, which are provided for illustration of the embodiments only but are not intended to limit the scope of the invention by any means.

### PREPARATION EXAMPLES

### [Preparation Example 1] Preparation of Compound (1493)

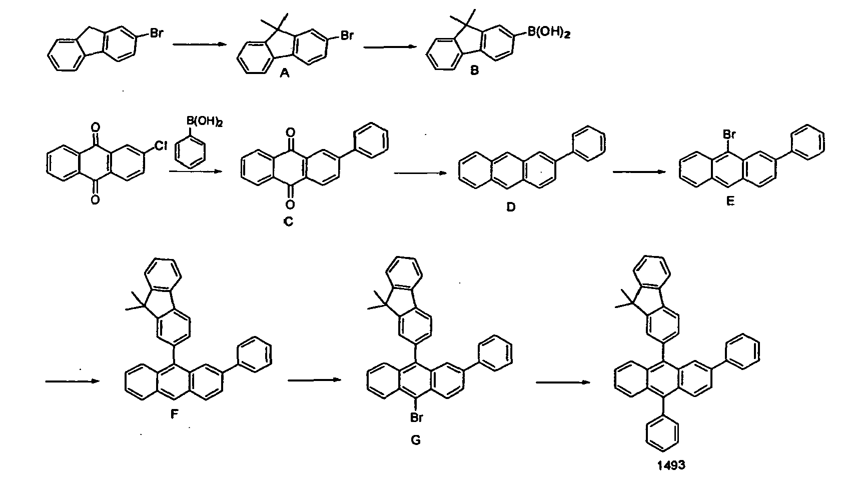

### Preparation of Compound (A)

In dimethyl sulfoxide (400 mL), dissolved were 2-bromofluorene (30.0 g, 122.0 mmol), potassium hydroxide (KOH) (41.2 g, 734.0 mmol), and the solution was cooled to 0°C. After slowly adding water, the mixture was stirred for 30 minutes. While maintaining the temperature at 0°C, methyl iodide (CH₃I) (30.5 g, 489.0 mmol) was slowly poured into the mixture. Then the temperature was raised to room temperature, and the mixture stirred for 12 hours. After adding 10% hydrochloric acid (1 L), the resultant mixture was stirred for 10 minutes. Solid produced was filtered under reduced pressure, and recrystallized from hexane and methanol to obtain Compound (A) (29.5 g, 108.0 mmol).

### Preparation of Compound (B)

Compound (A) (29.5 g, 108.0 mmol) was dissolved in purified tetrahydrofuran (350 mL) under nitrogen atmosphere. After chilling the solution to -78°C, n-butyllithium (n-BuLi, 2.5 M solution in hexane) (56.2 mL, 140.4 mmol) was slowly added dropwise thereto. The mixture was stirred for 1 hour, and trimethylborate (19.6 mL, 172.8 mmol) was added thereto. After slowly raising the temperature to 25°C, the reaction mixture was stirred for one day. The reaction was quenched by adding aqueous 1 M HCl solution (400 mL), and the mixture was extracted with ethyl acetate (300 mL). The extract was dried under reduced pressure, and recrystallized from dichloromethane (20 mL) and hexane (300 mL) to obtain Compound (B) (13.5 g, 56.7 mmol).

### Preparation of Compound (C)

A reaction vessel was charged with 2-chloroanthraquinone (30.0 g, 123.63 mmol), phenylboronic acid (18.09 g, 148.36 mmol) and trans-dichlorobis(triphenylphosphine)palladium (II) (Pd(PPh₃)₂Cl₂) (8.68 g, 12.63 mmol). Toluene solvent (800 mL) was added thereto with stirring, and then ethanol (300 mL). Finally, 2 M sodium carbonate solution (400 mL) was added thereto, and the resultant mixture was heated to 120°C and stirred under reflux. After 3 hours of stirring, the reaction mixture was cooled to room temperature, and water (300 mL) was added thereto. The mixture was extracted with ethyl acetate (300 mL), and the extract was evaporated under reduced pressure to remove the solvent to obtain solid compound. The solid was thoroughly dissolved with tetrahydrofuran solvent (300 mL), and filtered through silica. The solvent was removed via filtration under reduced pressure to obtain Compound (C) (26.8 g, 100.89 mmol).

### Preparation of Compound (D)

A reaction vessel was charged with Compound (C) (85.0 g, 299.07 mmol) and acetic acid (700 mL). While stirring, hydroiodic acid (HI) (700 mL) was added thereto, and then hyperphosphorous acid (H₃PO₂) (600 mL). The resultant mixture was stirred under reflux for 16 hours. Then the reaction mixture was cooled and extracted with dichloromethane, and the extract was purified via column chromatography (dichloromethane/n-hexane = 1/10) to obtain Compound (D) (72.85 g, 286.43 mmol).

### Preparation of Compound (E)

Compound (D) (25.0 g, 98.53 mmol), N-bromosuccinimide (NBS) (19.3 g, 108.38 mmol) and dichloromethane solvent (800 mL) were charged to a reaction vessel, and the mixture was stirred at room temperature for 20 hours. When the reaction was completed, water (800 mL) was added thereto, and the mixture was extracted with dichloromethane (300 mL). The extract was filtered under reduced pressure, and the compound obtained was recrystallized from methanol (500 mL) to obtain the target compound (Compound E) (30.2 g, 90.03 mmol).

### Preparation of Compound (F)

In a reaction vessel, Compound (E) (5.5 g, 14.35 mmol), Compound (B) (4.1 g, 17.22 mmol) and trans-dichlorobis(triphenylphosphine)palladium (II) (Pd(PPh₃)₂Cl₂) (1.0 g, 1.44 mmol) were stirred in toluene solvent (140 mL). Then, ethanol (70 mL) and 2 M sodium carbonate solution (70 mL) were added thereto, and the resultant mixture was stirred under reflux in the presence of nitrogen atmosphere. After 5 hours, the reaction mixture was cooled to room temperature, and methanol (200 mL) was added to thereto. The solid produced was filtered and heated under reflux in methanol (200 mL). Recrystallization gave the target compound (Compound F) (4.0 g, 8.05 mmol).

### Preparation of Compound (G)

In a reaction vessel, Compound (F) (4.0 g, 8.05 mmol) and N-bromosuccinimide (1.72 g, 9.66 mmol) were dissolved in dichloromethane (100 mL), and the solution was stirred at room temperature. After 20 hours, water (200 mL) was added to quench the reaction. The mixture was extracted with dichloromethane (100 mL) and the extract distilled under reduced pressure to remove the solvent. Solid compound thus obtained was heated under reflux in methanol (200 mL). Recrystallization gave Compound (G) (3.6 g, 6.25 mmol).

### Preparation of Compound (1493)

Compound (G) (5.0 g, 8.69 mmol), phenylboronic acid (2.8 g, 11.29 mmol) and trans-dichlorobis(triphenylphosphine)palladium (II) (Pd(PPh₃)₂Cl₂) (0.6 g, 8.7 mmol) were stirred under reflux in 2 M sodium carbonate (Na₂CO₃) solution (15 mL) and toluene solvent (100 mL). After 2 hours, the reaction mixture was extracted with dichloromethane (200 mL), and the extract filtered under reduced pressure. Recrystallization from methanol (300 mL) gave Compound (1493) (4.5 g, 74%).

According to the procedure of Preparation Example 1, organic electroluminescent compounds (Compounds 1 to 2040) were prepared, and the ¹H NMR and MS/FAB data are shown in Table 1.

**Table 1**

| compound | ¹H NMR(CDCl₃, 200 MHz) | MS/FAB | |
|---|---|---|---|
| | | found | calculated |
| 1 | δ = 1.72(6H, s), 2.45(3H, s), 7.25∼7.41(7H, m), 7.51∼7.55(6H, m), 7.63(1H, m), 7.7(1H, m), 7.85∼7.91(4H, m) | 460.61 | 460.21 |
| 3 | δ = 1.72(6H, s), 2.45(3H, s), 7.25∼7.39(6H, m), 7.51∼7.55(2H, m), 7.63(1H, m), 7.7(1H, m), 7.82∼7.93(9H, m), 8.12(2H, m), 8.93(2H, m) | 560.72 | 560.25 |
| 6 | δ = 1.72(6H, s), 2.45(3H, s), 7.25∼7.41(11H, m), 7.51∼7.55(6H, m), 7.63(1H, m), 7.7(1H, m), 7.85∼7.91(4H, m) | 536.70 | 536.25 |
| 15 | δ = 1.72(6H, s), 2.45(3H, s), 7.25∼7.41(8H, m), 7.51∼7.55(10H, m), 7.63∼7.7(5H, m), 7.85∼7.91(4H, m) | 612.80 | 612.28 |
| 25 | δ = 1.72(12H, s), 2.45(3H. s), 7.25∼7.39(9H, m), 7.51∼7.55(4H, m), 7.63(2H, m), 7.7(1H, m), 7.85∼7.91(5H, m) | 576.77 | 576.28 |
| 45 | δ = 1.72(6H, s), 2.45(3H, s), 7(1H, m), 7.25∼7.39(7H, m), 7.51∼7.55(3H, m), 7.63(1H, m), 7.7(1H, m), 7.85∼7.91(4H, m), 8.5(1H, m) | 461.60 | 461.21 |
| 87 | δ = 0.66(6H, s), 1.72(6H, s), 2.45(3H, s), 7.25∼7.39(7H, m), 7.51∼7.63(6H, m), 7.7(1H, m), 7.8∼7.91(7H, m) | 592.84 | 592.26 |
| 113 | δ = 1.72(6H, s), 2.45(3H, s), 7.25∼7.39(6H, m), 7.48∼7.63 (9H, m), 7.7∼7.73(3H, m), 7.85∼7.92(5H, m), 8(2H, m) | 586.76 | 586.27 |
| 125 | δ = 1.72(12H, s), 2.45(3H, s), 7.25∼7.39(6H, m), 7.51∼7.63(8H, m), 7.7∼7.77(4H, m), 7.85∼7.93(7H, m), 8(2H, m) | 702.92 | 702.33 |
| 134 | δ = 1.72(6H, s), 7.28∼7.41(6H, m), 7.51∼7.63(11H, m), 7.73(1H, m), 7.87~8(7H, m), 8.13(1H, m) | 572.74 | 572.25 |
| 137 | δ = 1.72(12H, s), 7.28∼7.41(8H, m), 7.51~7.55(7H, m), 7.61∼7.63 (3H, m), 7.77(1H, m), 7.87~7.97(6H, m), 8.13(1H, m) | 638.84 | 638.30 |
| 145 | δ = 1.72(6H, s), 7.28∼7.41(7H, m), 7.48∼7.63(15H, m), 7.7(1H, m), 7.87∼7.97(4H, m), 8.13(1H, m) | 598.77 | 598.27 |
| 155 | δ = 1.72(6H, s), 7.28∼7.41(6H, m), 7.5-7.63(11H, m), 7.82∼7.98(7H, m), 8.13(1H, m), 8.45(1H, m) | 628.82 | 628.22 |
| 170 | δ = 1.72(12H, s), 7.28∼7.41(8H, m), 7.51~7.55(8H, m), 7.61∼7.63(3H, m), 7.87∼7.97(5H, m), 8.06(1H, m), 8.13(1H, m) | 638.84 | 638.30 |
| 180 | δ = 1.72(6H, s), 7.28∼7,41(7H, m), 7.51~7.63(9H, m), 7.81(1H, m), 7.87~7.97(4H, m), 8.06∼8.13(3H, m), 8.38(1H, m), 8.83(1H, m) | 624.77 | 624.26 |
| 196 | δ = 1.72(6H, s), 3.83(3H, s), 7.05(2H, m), 7.28∼7.41(6H, m), 7.51∼7.55(6H, m), 7.61∼7.68(4H, m), 7.87∼7.97(4H, m), 8.13(1H, m) | 552.70 | 552.25 |
| 212 | δ = 1.72(6H, s), 7.28∼7.41(6H, m), 7.51∼7.55(6H, m), 7.61∼7.63(2H, m), 7.82∼7.97(8H, m), 8.04(1H, m), 8.12∼8.18(4H, m), 8.93(2H, m), 9.15(1H, m) | 672.85 | 672.28 |
| 241 | δ = 1.72(6H, s), 7.25∼7.41(10H, m), 7.51∼7.63(11H, m), 7.73(1H, m), 7.87∼8(7H, m), 8.13(1H, m) | 648.83 | 648.28 |
| 259 | δ = 1.72(6H, s), 7.28∼7.41(6H, m), 7.48-7.63(11H, m), 7.7(1H, m), 7.82∼7.97(9H, m), 8.12∼8.13(3H, m), 8.93(2H, m) | 698.89 | 698.30 |
| 263 | δ = 1.72(6H, s), 7.28~7.41(6H, m), 7.51∼7.55(8H, m), 7.61∼7.63(3H, m), 7.87∼7.97(4H, m), 8.04∼8.13(3H, m), 8.42(1H, m), 8.55(1H, m) | 572.74 | 572.25 |
| 274 | δ = 1.72(6H, s), 2.18(3H, s), 2.34(6H, s), 7.28∼7.39(5H, m), 7.48∼7.55(6H, m), 7.61~7.63(3H, m), 7.87∼7.97(4H, m), 8.04~8.13(3H, m), 8.42(1H, m), 8.55(1H, m) | 614.82 | 614.30 |
| 305 | δ = 1.72(6H, s), 7.28∼7.39(9H, m), 7.51~7.55(4H, m), 7.61∼7.63(3H, m), 7.87∼7.97(4H, m), 8.04∼8.13(3H, m), 8.42(1H, m), 8.55(1H, m) | 590.73 | 590.24 |
| 339 | δ = 1.72(6H, s), 6.63(2H, m), 6.81(2H, m), 6.99∼7.05(4H, m), 7.25∼7.39(7H, m), 7.51∼7.63(7H, m), 7.87∼7.96(4H, m), 8.04~8.08(3H, m), 8.42(1 H, m), 8.55(1 H, m) | 687.78 | 687.29 |
| 366 | δ = 1.72(6H, s), 7.28∼7.39(5H, m), 7.47∼7.63(12H, m), 7.73(1H, m), 7.85(2H, m), 7.87~7.97(10H, m), 8.42(1H, m), 8.55(1H, m) | 698.89 | 698.30 |
| 396 | δ = 1.72(6H, s), 7.28-7.39(5H, m), 7.51∼7.63(10H, m), 7.73(1H, m), 7.87~7.97(10H, m), 8.42(1H, m), 8.55(1H, m) | 622.79 | 622.27 |
| 398 | δ = 1.35(9H, s), 1.72(6H, s), 7.28∼7.39(9H, m), 7.51∼7.63(7H, m), 7.73(1H, m), 7.87∼8(7H, m), 8.13(1H, m) | 628.84 | 628.31 |
| 413 | δ = 1.72(6H, s), 7.28∼7.39(5H, m), 7.5∼7.55(9H, m), 7.73(1H, m,) 7.86∼8(11H, m), 8.13(1H, m), 8.45(1H, m) | 678.88 | 678.24 |
| 442 | δ = 1.72(6H, s), 7.28∼7.39(5H, m), 7.51∼7.63(7H, m), 7.73(1H, m), 7.87∼8(7H, m), 8.13(1H, m), 8.76∼8.79(3H, m) | 574.71 | 574.24 |
| 448 | δ = 1.48(6H, m), 1.72(6H, s), 2.02(4H, m), 7.28~7.39(7H, m), 7.51~7.63(9H, m), 7.73∼7.77(2H, m), 7.87∼8(9H, m), 8.13(1H, m) | 728.96 | 728.34 |
| 468 | δ = 1.72(6H, s), 7.28(1H, m), 7.34(1H, m), 7.38(1H, m), 7.39(2H, m), 7.41∼7.55(17H, m), 7.73(2H, m), 7.87~8(8H, m), 8.13(1H, m), 8.28(1H, m) | 776.96 | 776.32 |
| 492 | δ = 1.69(6H, s), 1.72(6H, s), 6.94(1H, s), 7.22∼7.3(9H, m), 7.51∼7.63(7H, m), 7.73(1H, m), 7.87∼8(7H, m), 8.13(1H, m) | 638.84 | 638.30 |
| 505 | δ = 1.72(6H, s), 7.22(2H, m), 7.28∼7.39(5H, m), 7.45∼7.55(13H, m), 7.73(1H, m), 7.87∼8(7H, m), 8.13(1H,m), 8.56(1H, m) | 688.86 | 688.29 |
| 519 | δ = 1.72(6H, s), 7.28∼7.39(5H, m), 7.48∼7.57(13H, m), 7.7∼7.73(3H, m), 7.87∼8(10H, m), 8.13(1H, m) | 698.89 | 698.30 |
| 538 | δ = 1.72(12H, s), 7.28∼7.39(7H, m), 7.51~7.63(9H, m), 7.73∼7.77(2H, m), 7.87∼8(9H, m), 8.13(1H, m) | 688.90 | 688.31 |
| 547 | δ = 1.72(12H, s), 2.34(3H, s), 7.28∼7.39(11H, m), 7.51~7.55(3H, m), 7.61~7.63(3H, m), 7.77(1H, m), 7.87∼7.97(6H, m), 8.13(1H, m) | 652.86 | 652.31 |
| 571 | δ = 1.72(12H, s), 7.11(4H, m), 7.26∼7.39(15H, m), 7.51~7.55(4H, m), 7.61∼7.63(4H, m), 7.77(2H, m), 7.87~7.97(8H, m), 8.13(1H, m) | 879.14 | 878.39 |
| 578 | δ = 1.72(16H, m), 2.74(4H, m), 6.88(1H, m), 6.98(1H, m), 7.15(1H, m), 7.28∼7.39(7H, m), 7.51∼7.55(3H, m), 7.61∼7.63(3H, m), 7.77(1H, m), 7.87∼7.97(6H, m), 8.13(1H, m) | 692.93 | 692.34 |
| 615 | δ = 1.72(12H, s), 3.83(3H, s), 7.05(2H, m), 7.28∼7.39(7H, m), 7.51∼7.55(3H, m), 7.61 ~7.68(5H, m), 7.77(1H, m), 7.87∼7.97(6H, m), 8.13(1H, m) | 668.86 | 668.31 |
| 642 | δ = 1.72(12H, s), 7.28~7.42(10H, m), 7.48~7.55(4H, m), 7.61~7.63(3H, m), 7.74(1H, m), 7.77(1H, m), 7.78(3H, m), 7.84(1H, m), 7.87~7.97(10H, m) | 820.99 | 820.33 |
| 658 | δ = 1.72(12H, s), 7.25(4H, m), 7.28(2H, m), 7.34∼7.41(15H, m), 7.61~7.63(3H, m), 7.77(1H, m), 7.85~7.97(8H, m), 8.13(1H, m) | 791.03 | 790.36 |
| 679 | δ = 1.72(12H, s), 7.28~7.41(12H, m), 7.51∼7.55(7H, m), 7.61∼7.63(3H, m), 7.77(1H, m), 7.87∼7.97(10H, m), 8.13(1H, m) | 815.05 | 814.36 |
| 692 | δ = 1.72(6H, s), 2.18(3H, s), 2.34(6H, s), 2.45(3H, s), 7.25∼7.28(2H, m), 7.38∼7.39(3H, m), 7.48∼7.55(4H, m), 7.61(1H, m), 7.7(1H, m), 7.85∼7.91(4H, m), 8.06(1H, m) | 502.69 | 502.27 |
| 701 | δ = 1.72(6H, s), 2.45(3H, s), 7.25~7.28(2H, m), 7.38∼7.39(3H, m), 7.5~7.55(4H, m), 7.61(1H, m), 7.7(1H, m), 7.85∼7.91(5H, m), 7.98~8(3H, m), 8.06(1H, m), 8.45(1H, m) | 566.75 | 566.21 |
| 707 | δ = 1.72(6H, s), 2.45(3H, s), 7.25∼7.4(8H, m), 7.5~7.55(3H, m), 7.61~7.63(2H, m), 7.7(1H, m), 7.8(1H, m), 7.87∼7.94(4H, m), 8.06~8.12(2H, m), 8.55(1H, m) | 549.70 | 549.25 |
| 724 | δ = 1.72(6H, s), 2.45(3H, s), 3.52(4H, m), 7.25∼7.31(3H, m), 7.38∼7.46(6H, m), 7.53∼7.55(3H, m), 7.61(1H, m), 7.7(1H, m), 7.85~7.91(4H, m), 8.06(1H, m) | 536.70 | 536.25 |
| 743 | δ = 1.72(6H, s), 2.45(3H, s), 1.25∼7.28(2H, m), 7.38~7.39(3H, m), 7.53∼7.55(2H, m), 7.61(1H, m), 7.7(1H, m), 7.82∼7.91(8H, m), 8.06(1H, m) | 485.62 | 485.21 |
| 750 | δ = 1.72(12H, s), 2.45(3H, s), 6.55(2H, m), 6.73(2H, m), 7.02∼7.05(4H, m), 7.24∼7.28(2H, m), 7.38∼7.39(3H, m), 7.53~7.55(2H, m), 7.61~7.64(2H, m), 7.84∼7.91(4H, m), 8.06(1 H, m) | 591.78 | 591.29 |
| 761 | δ = 1.72(6H, s), 2.45(3H, s), 7.25∼7.28(2H, m), 7.38~7.39(3H, m), 7.47(2H, m), 7.53∼7.55(6H, m), 7.61(1H, m), 7.7(1H, m), 7.85∼7.91(4H, m), 8.06(1H, m), 8.2(2H, m), 8.3(4H, m) | 613.79 | 613.28 |
| 781 | δ = 1.72(6H, s), 2.45(3H, s), 3.2(3H, s), 6.6(1H, m), 6.77(1H, m), 6.86(1H, m), 6.96∼6.99(3H, m), 7.08(1H, m), 7.25~7.39(7H, m), 7.53~7.55(2H, m), 7.61(1H, m), 7.7(1H, m), 7.85∼7.91(4H, m), 8.06(1 H, m) | 589.77 | 589.28 |
| 798 | δ = 1.72(6H, s), 2.45(3H, s), 7.25∼7.28(2H, m), 7.38∼7.39(3H, m), 7.53∼7.61(8H, m), 7.7∼7.73(2H, m), 7.85~7.92(5H, m), 8∼8.06(5H, m), 8.55(2H, m) | 636.82 | 636.28 |
| 809 | δ = 1.72(6H, s), 2.45(3H, s), 2.62(3H, s), 7.06(1H, m), 7.25∼7.28(2H, m), 7.38∼7.39(3H, m), 7.52∼7.55(4H, 7.61(1H, m), 7.7(1H, m), 7.82∼7.91(5H, m), 8.06(1H, m), 8.18(1H, m), 8.52(1H, m) | 524.69 | 524.25 |
| 821 | δ = 1.72(6H, s), 2.34(3H, s), 7.28∼7.41(9H, m), 7.51∼7.55(6H, m), 7.61(2H, m), 7.87∼7.97(4H, m), 8.06(1H, m), 8.13(1H, m) | 536.70 | 536.25 |
| 835 | δ = 1.72(6H, s), 7.28(1H, m), 7.38∼7.41(4H, m), 7.5~7.61(11H, m), 7.82∼7.98(7H, m), 8.06(1H, m), 8.13(1H, m), 8.45(1H, m) | 628.82 | 628.22 |
| 859 | δ = 1.72(6H, s), 7.28(1H, m), 7.35∼7.41(5H, m), 7.51~7.61(9H, m), 7.78(1H, m), 7.87∼7.98(5H, m), 8.06∼8.13(4H, m) | 573.72 | 573.25 |
| 874 | δ = 1.72(6H, s), 7.28(1H, m), 7.38∼7.41(7H, m), 7.51∼7.61(10H, m), 7.87∼7.97(4H, m), 8.06(1H, m), 8.13(1H, m) | 546.70 | 546.23 |
| 880 | δ = 1.72(6H, s), 1.96(2H, m), 2.76(2H, m), 3.06(2H, m), 6.55(1H, m), 6.72(1H, m), 7.05∼7.07(2H, m), 7.28(1H, m), 7.38∼7.41(4H, m), 7.51∼7.61(8H, m), 7.87∼7.96(4H, m), 8.06∼8.07(2H, m) | 577.76 | 577.28 |
| 891 | δ = 1.72(6H, s), 7.28(1H, m), 7.38∼7.41(4H, m), 7.51~7.55(6H, m), 7.61(2H, m), 7.82∼7.91(8H, m), 7.93(1H, s), 7.97(1H, m), 8.06~8.13(4H, m), 8.93(3H, m) | 672.85 | 672.28 |
| 920 | δ = 1.72(6H, s), 7.28(1H, m), 7.37(6H, m), 7.38∼7.46(21H, m), 7.61(2H, m), 7.87∼7.97(6H, m), 8.06(1H, m), 8.13(1H, m) | 781.07 | 780.32 |
| 930 | δ = 1.72(12H, s), 7.28(2H, m), 7.38∼7.41(5H, m), 7.51∼7.55(9H, m), 7.61∼7.63(3H, m), 7.77(1H, m), 7.87∼8.06(9H, m), 8.13(1H, m), 8.55(2H, m) | 764.99 | 764.34 |
| 941 | δ = 1.72(6H, s), 7.28(1H, m), 7.38∼7.41(9H, m), 7.51∼7.55(10H, m), 7.61(2H, m), 7.87∼7.97(8H, m), 8.06(1H, m), 8.13(1H, m) | 698.89 | 698.30 |
| 955 | δ = 1.72(6H, s), 7.28(1H, m), 7.38∼7.41(4H, m). 7.48∼7.61(14H, m), 7.7(1H, m), 7.87∼7.97(4H, m), 8.04∼8.13(4H, m), 8.42(1H, m), 8.55(1H, m) | 648.83 | 648.28 |
| 994 | δ = 1.72(6H, s), 7.28(1 H, m), 7.38∼7.39(3H, m), 7.53∼7.55(4H, m), 7.61(3H, m), 7.87∼7.97(4H, m), 8.04∼8.13(4H, m), 8.42(1H, m), 8.55(1H, m), 8.76∼8.79(3H, m) | 574.71 | 574.24 |
| 1003 | δ = 1.72(6H, s), 6.95(2H, m), 7.28∼7.3(2H, m), 7.38∼7.39(3H, m), 7.45(2H, m), 7.53∼7.55(4H, m), 7.61 (3H, m), 7.72(2H, m), 7.87∼7.97(4H, m), 8.04∼8.13(4H, m), 8.42(1H, m), 8.55(1H, m) | 598.77 | 598.27 |
| 1026 | δ = 1.72(6H, s), 7(2H, m), 7.26∼7.28(3H, m), 7.38∼7.39(3H, m), 7.51∼7.55(6H, m), 7.61(3H, m), 7.87∼7.97(4H, m), 8.04∼8.13(4H, m), 8.23(2H, m), 8.42(1H, m), 8.5∼8.55(3H, m), 8.68(1H, m) | 726.90 | 726.30 |
| 1049 | δ = 1.72(6H, s), 7.25∼7.28(5H, m), 7.38∼7.47(14H, m), 7.61(3H, m), 7.85∼7.97(6H, m), 8.04∼8.13(4H, m), 8.42(1H, m), 8.55(1H, m) | 724.93 | 724.31 |
| 1055 | δ = 1.72(6H, s), 7.28(1H, m), 7.38∼7.39(3H, m), 7.48∼7.61(13H, m), 7.7∼7.73(2H, m), 7.87(1H, m), 7.91∼8(10H, m), 8.42(1H, m), 8.55(1H, m) | 698.89 | 698.30 |
| 1065 | δ = 1.72(12H, s), 7.28(1H, m), 7.38∼7.41(4H, m), 7.51∼7.55(8H, m), 7.61∼7.63(5H, m), 7.77(2H, m), 7.87∼7.97(6H, m), 8.04∼8.13(4H, m), 8.42(1 H, m), 8.55(1 H, m) | 764.99 | 764.34 |
| 1087 | δ = 1.72(6H, s), 7.28(1H, m), 7.38∼7.41(5H, m), 7.51∼7.55(18H, m), 7.73(1H, m), 7.87∼8(7H, m), 8.06(1H, m), 8.13(1H, m) | 724.93 | 724.31 |
| 1110 | δ = 1.72(12H, s), 7.28(2H, m), 7.38∼7.39(4H, m), 7.53∼7.61(10H, m), 7.73(1H, m), 7.87∼8(8H, m), 8.06(2H, m), 8.13(1H, m) | 688.90 | 688.31 |
| 1131 | δ = 1.72(6H, s), 3.52(4H, m), 7.28∼7.31(2H, m), 7.38∼7.46(6H, m), 7.53∼7.61(8H, m), 7.73(1H, m), 7.87∼8(7H, m), 8.06(1H, m), 8.13(1H, m) | 648.83 | 648.28 |
| 1139 | δ = 1.72(6H, s), 7.28(1H, m), 7.38∼7.39(3H, m), 7.53∼7.61(7H, m), 7.73(1H, m), 7.87∼8(7H, m), 8.06∼8.13(3H, m), 8.86(1H, m), 9.27(1 H, m) | 574.71 | 574.24 |
| 1146 | δ = 1.72(6H, s), 2.53(3H, s), 7.12(1H, m), 7.28(1H, m), 7.38∼7.39(3H, m), 7.53∼7.61(7H, m), 7.73(1H, m), 7.87∼8.06(10H, m), 8.13(1H, m), 8.2(1 H, m), 8.32(1H, m) | 637.81 | 637.28 |
| 1170 | δ = 1.72(6H, s), 7.28(1H, m), 7.38∼7.41(5H, m), 7.51∼7.61(11H, m), 7.73(1H, m), 7.87∼8(7H, m), 8.06(1H, m), 8.13(1H, m), 8.28(4H, m) | 727.89 | 727.30 |
| 1191 | δ = 1.72(6H, s), 6.92(1H, s), 7.28∼7.3(3H, m), 7.37∼7.39(4H, m), 7.45∼7.53(16H, m), 7.68∼7.73(2H, m), 7.87∼8(7H, m), 8.06∼8.07(2H, m), 8.13(1H, m) | 750.96 | 750.33 |
| 1214 | δ = 1.72(6H, s), 7.28(1H, m), 7.38∼7.39(3H, m), 7.48∼7.61(10H, m), 7.7∼7.73(2H, m), 7.82∼7.91(12H, m), 8.06∼8.13(4H, m), 8.93(2H, m) | 748.95 | 748.31 |
| 1220 | δ = 1.72(12H, s), 7.28(2H, m), 7.38∼7.39(4H, m), 7.53∼7.55(5H, m), 7.61∼7.63(4H, m), 7.77(1H, m), 7.87∼7.97(6H, m), 8.04∼8.13(4H, m), 8.42(1H, m), 8.55(1 H, m) | 688.90 | 688.31 |
| 1242 | δ = 1.72(12H, s), 7.25∼7.4(9H, m), 7.5∼7.63(8H, m), 7.77(1H, m), 7.87∼8(7H, m), 8.06∼8.12(3H, m), 8.55(1H, m) | 727.93 | 727.32 |
| 1258 | δ = 1.72(12H, m), 2.74(8H, m), 6.88(1H, m), 6.98(1H, m), 7.15(1H, m), 7.28(2H, m), 7.38∼7.39(4H, m), 7.53∼7.55(3H, m), 7.61∼7.63(3H, m), 7.77(1H, m), 7.87∼7.97(6H, m), 8.06(1H, m), 8.13(1H, m) | 692.93 | 692.34 |
| 1275 | δ = 1.72(12H, s), 3.52(4H, m), 7.28∼7.31(3H, m), 7.38∼7.46(7H, m), 7.53∼7.55(4H, m), 7.61∼7.63(3H, m), 7.77(1H, m), 7.87∼7.97(6H, m), 8.06(1H, m), 8.13(1H, m) | 714.93 | 714.33 |
| 1287 | δ = 1.72(12H, s), 7.28(2H, m), 7.38∼7.39(4H, m), 7.53∼7.55(3H, m), 7.61∼7.67(5H, m), 7.77∼7.8(3H, m), 7.87∼7.97(6H, m), 8.06(1H, m), 8.13(1H, m), 8.7(1H, s) | 690.87 | 690.30 |
| 1293 | δ = 1.72(12H, s), 7.28(2H, m), 7.38∼7.41(7H, m), 7.53∼7.63(8H, m), 7.77(1H, m), 7.87∼7.97(6H, m), 8.06(1H, m), 8.13(1H, m) | 662.86 | 662.30 |
| 1309 | δ = 1.72(12H, s), 7.28(2H, m), 7.38∼7.39(4H, m), 7.53∼7.55(3H, m), 7.61∼7.63(3H, m), 7.77∼7.93(12H, 7.97(1H, m), 8.06∼8.13(4H, m), 8.93(3H, m) | m), 789.01 | 788.34 |
| 1317 | δ = 1.72(12H, s), 3.82(3H, s), 7.28∼7.29(3H, m), 7.38∼7.39(5H, m), 7.5∼7.55(4H, m), 7.61∼7.63(3H, m), 7.77(3H, m), 7.87∼7.97(6H, m), 8.06∼8.13(3H, m), 8.27(1H, m) | 741.96 | 741.34 |
| 1342 | δ = 1.72(18H, s), 7.28(3H, m), 7.38∼7.39(5H, m), 7.48∼7.63(11H, m), 7.7(1H, m), 7.77(2H, m), 7.87∼7.97(8H, m), 8.06(1H, m), 8.13(1H, m) | 831.09 | 830.39 |
| 1361 | δ = 1.72(6H, s), 2.45(3H, s), 7.25∼7.28(2H, m), 7.38∼7.41(4H, m), 7.51∼7.55(5H, m), 7.63(1H, m), 7.7(1H, m), 7.77(1H, m), 7.85∼7.93(5H, m) | 460.61 | 460.22 |
| 1365 | δ = 1.72(12H, s), 2.45(3H, s), 7.25∼7.28(3H, m), 7.38∼7.39(4H, m), 7.55(2H, m), 7.63(2H, m), 7.7(1H, m), 7.77(2H, m), 7.85∼7.93(7H, m) | 576.77 | 576.28 |
| 1381 | δ = 1.72(6H, s), 2.45(3H, s), 7.25∼7.28(2H, m), 7.38∼7.39(3H, m), 7.5∼7.55(3H, m), 7.63(1H, m), 7.7(1H, m), 7.77(1H, m), 7.85∼8(9H, m), 8.45(1 H, m) | 566.75 | 566.21 |
| 1405 | δ = 1.72(6H, s), 2.45(3H, s), 7(1H, m), 7.25∼7.28(3H, m), 7.38∼7.39(3H, m), 7.51∼7.55(2H, m), 7.63(1H, m), 77(1H, m), 7.77(1H, m), 7.85∼7.93(5H, m), 8.5(1H, m) | 461.60 | 461.21 |
| 1424 | δ = 1.72(6H, s), 2.45(3H, s), 3.83(3H. s), 7.05(2H, m), 7.25∼7.28(2H, m), 7.38∼7.39(3H, m), 7.55(1H, m), 7.63∼7.7(4H, m), 7.77(1H, m), 7.85∼7.93(5H, m) | 490.63 | 490.23 |
| 1441 | δ = 1.72(6H, s), 2.45(3H, s), 7.25∼7.28(2H, m), 7.38∼7.39(3H, m), 7.47(2H, m), 7.54∼7.55(5H, m), 7.63(1H, m), 7.7(1H, m), 7.77(1H, m), 7.85∼7.93(5H, m), 8.2(2H, m), 8.3(4H, m) | 613.79 | 613.28 |
| 1456 | δ = 1.72(6H, s), 2.45(3H, s), 3.2(3H, s), 6.56(1H, m), 6.73(1H, m), 6.82(1H, m), 6.92∼6.98(3H, m), 7.07(1H, m), 7.25∼7.28(2H, m), 7.38∼7.39(3H, m), 7.55(1H, m), 7.63(1H, m), 7.7(1H, m), 7.77(1H, m), 7.85∼7.93(5H, m) | 579.73 | 579.26 |
| 1472 | δ = 1.72(12H, s), 2.45(3H, s), 7.25∼7.28(3H, m), 7.38∼7.39(4H, m), 7.48(2H, m), 7.55∼7.63(5H, m), 7.7(2H, m), 7.77(2H, m), 7.85∼7.93(7H, m) | 652.86 | 652.31 |
| 1478 | δ = 1.72(6H, s), 2.45(3H, s), 7.25∼7.28(2H, m), 7.38∼7.39(3H, m), 7.55∼7.63(7H, m), 7.7∼7.77(3H, m), 7.85∼7.93(6H, m), 8∼8.01(4H, m), 8.55(2H, m) | 652.86 | 652.31 |
| 1489 | δ = 1.72(6H, s), 2.45(3H, s), 2.62(3H, s), 7.06(1H, m), 7.25∼7.28(2H, m), 7.38∼7.39(3H, m), 7.52∼7.55(3H, m), 7.63(1H, m), 7.7(1H, m), 7.77∼7.93(7H, m), 8.18(1H, m), 8.52(1H, m) | 524.69 | 524.25 |
| 1501 | δ = 1.72(6H, s), 2.34(3H, s), 7.28∼7.41(9H, m), 7.51∼7.55(5H, m), 7.61∼7.63(2H, m), 7.77(1H, m), 7.87∼7.97(5H, m), 8.13(1H, m) | 536.70 | 536.25 |
| 1507 | δ = 1.72(6H, s), 7.28(1H, m), 7.38∼7.41(6H, m), 7.51∼7.55(13H, m), 7.61∼7.66(5H, m), 7.77(1H, m), 7.87∼7.97(5H, m), 8.13(1H, m) | 674.87 | 674.30 |
| 1519 | δ = 1.72(6H, s), 7.25∼7.41(8H, m), 7.5∼7.63(9H, m), 7.77(1H, m), 7.87∼8(6H, m), 8.1∼8.12(2H, m), 8.55(1H, m) | 611.77 | 611.26 |
| 1526 | δ = 1.72(6H, s), 7.11(4H, m), 7.26∼7.41(13H, m), 7.51∼7.55(6H, m), 7.61∼7.63(3H, m), 7.77(2H, m), 7.87∼7.97(7H, m), 8.13(1H, m) | 762.98 | 762.33 |
| 1537 | δ = 1.72(6H, s), 7(1H, m), 7.26∼7.28(2H, m), 7.38∼7.41(4H, m), 7.51∼7.55(6H, m), 7.61∼7.63(2H, m), 7.77(1H, m), 7.87∼7.97(5H, m), 8.13(1H, m), 8.5(1H, m) | 523.66 | 523.23 |
| 1547 | δ = 1.72(6H, s), 7.28(1H, m), 7.38∼7.41(4H, m), 7.51∼7.55(5H, m), 7.61∼7.63(2H, m), 7.77(1H, m), 7.87∼7.97(5H, m), 8.13(1H, m), 9(2H, m), 9.22(1 H, m) | 524.65 | 524.23 |
| 1569 | δ = 1.72(6H, s), 6.63(2H, m), 6.81(2H, m), 6.99∼7.05(4H, m), 7.25∼7.28(3H, m), 7.38∼7.41(4H, m), 7.51∼7.63(7H, m), 7.77(1H, m), 7.87∼7.96(5H, m), 8.07(1H, m) | 637.81 | 637.28 |
| 1595 | δ = 1.72(6H, s), 7.28(1H, m), 7.38∼7.55(13H, m), 7.61∼7.63(3H, m), 7.77(1H, m), 7.85∼7.97(7H, m), 8.04∼8.13(3H, m), 8.42(1 H, m), 8.55(1 H, m) | 684.83 | 684.28 |
| 1601 | δ = 1.72(6H, s), 7.25∼7.28(5H, m), 7.38∼7.41(4H, m), 7.51∼7.63(10H, m), 7.73∼7.77(2H, m), 7.87∼8(8H, m), 8.13(1H, m) | 648.83 | 648.28 |
| 1613 | δ = 1.72(6H, s), 7.25∼7.28(5H, m), 7.38∼7.41(4H, m), 7.51∼7.55(5H, m), 7.61∼7.63(2H, m), 7.77∼7.97(11H, m), 8.12∼8.13(3H, m), 8.93(2H, m) | 698.89 | 698.30 |
| 1630 | δ = 1.72(6H, s), 2.34(6H, s), 7.28∼7.31(2H, m), 7.38∼7.39(3H, m), 7.55∼7.63(8H, m). 7.77(1H, m), 7.87∼7.97(5H, m), 8.04∼8.13(3H, m), 8.42(1H, m), 8.55(1H, m) | 600.79 | 600.28 |
| 1642 | δ = 1.72(12H, s), 7.28(2H, m), 7.38∼7.39(4H, m), 7.53∼7.55(5H, m), 7.61∼7.63(4H, m), 7.77(1H, m), 7.87∼7.97(6H, m), 8.04∼8.13(4H, m), 8.42(1H, m), 8.55(1H, m) | 688.90 | 688.31 |
| 1643 | δ = 1.72(6H, s), 7.28(1H, m), 7.38∼7.39(3H, m), 7.5∼7.55(5H, m), 7.61∼7.63(3H, m), 7.77(1H, m), 7.86(1H, m), 7.87(1H, m), 7.91∼7.98(10H, m), 8.42∼8.45(2H, m), 8.55(1H, m) | 678.88 | 678.24 |
| 1664 | δ = 1.72(10H, m). 2.74(4H, m), 6.88(1H, m), 6.98(1H, m), 7.15(1H, m), 7.28(1H, m), 7.38∼7.39(3H, m), 7.55(3H, m), 7.61∼7.63(3H, m), 7.77(1H, m), 7.87∼7.97(5H, m), 8.04∼8.13(3H, m), 8.42(1H, m), 8.55(1H, m) | 626.83 | 626.30 |
| 1670 | δ = 1.72(6H, s), 7.28(1H, m), 7.35∼7.39(4H, m), 7.55∼7.63(7H, m), 7.77∼7.81(2H, m), 7.87∼7.97(5H, m). 8.04∼8.13(5H, m), 8.38∼8.42(2H, m), 8.55(1H, m), 8.83(1H, m) | 674.83 | 674.27 |
| 1709 | δ = 0.66(6H, s), 1.72(6H, s), 7.28∼7.39(5H, m), 7.52∼7.63(9H, m), 7.77∼7.87(9H, m), 8.04∼8.13(3H, m), 8.42(1H, m), 8.55(1H, m) | 704.97 | 704.29 |
| 1731 | δ = 1.72(6H, s), 7.25∼7.28(5H, m), 7.38∼7.39(3H, m), 7.55∼7.63(9H, m), 7.73∼7.77(2H, m), 7.87(1H, m), 7.91∼7.97(10H, m), 8.42(1H, m), 8.55(1H, m) | 698.89 | 698.30 |
| 1739 | δ = 1.72(6H, s), 7.28(1H, m), 7.38∼7.41(4H, m), 7.51∼7.55(7H, m), 7.61∼7.63(3H, m), 7.77∼7.79(3H, m), 7.87∼7.97(10H, m), 8.42(1H, m), 8.55(3H, m) | 698.89 | 698.30 |
| 1747 | δ = 1.72(12H, s), 7.28(1H, m), 7.38∼7.39(3H, m), 7.55∼7.63(11H, m), 7.73∼7.77(4H, m), 7.87(1H, m), 7.91∼7.97(12H, m), 8.42(1H, m), 8.55(1H, m) | 815.05 | 814.36 |
| 1755 | δ = 1.72(6H, s), 7.28(1H, m), 7.38∼7.39(3H, m), 7.55∼7.63(6H, m), 7.73(1H, m), 7.77(1H, m), 7.82∼7.91(13H, m), 8.12∼8.13(3H, m), 8.93(2H, m) | 672.85 | 672.28 |
| 1756 | δ = 1.72(6H, s), 7.28(1H, m), 7.38∼7.39(3H, m), 7.55∼7.63(9H, m), 7.73∼7.77(2H, m), 7.87(1H, m), 7.91∼7.97(10H, m), 8.42(1H, m), 8.55(1H, m) | 622.79 | 622.27 |
| 1779 | δ = 1.72(6H, s), 7.28∼7.44(7H, m), 7.55∼7.66(8H, m), 7.73∼7.77(3H, m), 7.87∼8(9H, m), 8.13(1H, m) | 662.82 | 662.26 |
| 1795 | δ = 1.72(6H, s), 7.28∼7.3(3H, m), 7.38∼7.39(5H, m), 7.55∼7.63(6H, m), 7.73∼7.77(2H, m), 7.87∼8(8H, m), 8.13(1H, m) | 590.73 | 590.24 |
| 1800 | δ = 1.72(6H, s), 7(1H, m), 7.26∼7.28(2H, m), 7.38∼7.39(3H, m), 7.51∼7.63(7H, m), 7.73∼7.77(2H, m), 7.87∼8(8H, m), 8.13(1H, m), 8.5(1H, m) | 573.72 | 573.25 |
| 1814 | δ = 1.72(6H, s), 7.28(1H, m), 7.35∼7.39(4H, m), 7.55∼7.63(7H, m), 7.73∼7.78(3H, m), 7.87∼8(9H, m), 8.06∼8.13(3H, m) | 623.78 | 623.26 |
| 1830 | δ = 1.72(6H, s), 7.28(1H, m), 7.38∼7.39(3H, m), 7.55-7.63(6H, m), 7.73(1H, m), 7.77(1H, m), 7.82∼7.91(12H, m), 8.13(1H, m) | 597.75 | 597.25 |
| 1846 | δ = 1.72(6H, s), 7.28(1H, m), 7.38∼7.39(3H, m), 7.55∼7.63(6H, m), 7.73(1H, m), 7.77(1H, m), 7.82(2H, m), 7.87∼7.92(11H, m), 722.91 8.12~8.18(4H, m), 8.93(2H, m), 9.15(1H, m) | 722.91 | 722.30 |
| 1874 | δ = 1.72(6H, s), 7.25∼7.28(5H, m), 7.38(1H, m), 7.39(2H, m), 7.41(1H, m), 7.47~7.55(12H, m), 7.73~7.77(2H, m), 724.93 7.85∼8(10H, m), 8.13(1H, m) | 724.93 | 724.31 |
| 1875 | δ = 1.72(6H, s), 7.28(1H, m). 7.37∼7.39(9H, m), 7.46∼7.63(17H, m), 7.73∼7.77(2H, m), 7.87∼8(10H, m), 831.12 8.13(1H, m) | 831.12 | 830.34 |
| 1882 | δ = 1.72(6H, s), 7.28(1H, m), 7.38∼7.41 (4H, m), 7.51∼7.63(10H, m), 7.73∼7.79(4H, m), 7.87∼8.01(1 OH, m), 8.13(1H, m), 8.55(2H, m) | 698.89 | 698.30 |
| 1892 | δ = 1.72(12H, s), 7.28(1H, m). 7.38∼7.39(3H, m), 7.55∼7.63(11H, m), 7.73∼7.77(5H, m), 7.87∼8(13H, m), 815.05 8.13(1H, m) | 815.05 | 814.36 |
| 1905 | δ = 1.72(12H, s), 2.34(6H, s), 7.14∼7.17(2H, m), 7.28(2H, m). 7.38∼7.39(4H, m), 7.55(2H, m), 7.61∼7.67(4H, m), 7.77(2H, m), 666.89 7.87∼7.97(7H, m), 8.13(1H, m) | 666.89 | 666.33 |
| 1912 | δ = 1.72(12H, s), 7.21(1H, m), 7.28(2H, m), 7.38∼7.41(6H, m), 7.51∼7.55(6H, m), 7.61∼7.63(3H, m), 7.76∼7.79(7H, m), 791.03 7.87∼7.97(8H, m), 8.13(1H, m) | 791.03 | 790.36 |
| 1919 | δ = 1.72(12H, s), 7.28(2H, m), 7.38∼7.39(4H, m), 7.5∼7.63(8H, 744.98 744.29 m), 7.77∼7.91(12H, m), 8.13(1H, m), 8.45(1H, m) | 744.98 | 744.29 |
| 1931 | δ = 1.72(12H, s), 7.11(4H, m), 7.26∼7.39(14H, m), 7.55(3H, m), 7.61∼7.63(4H, m), 7.77(3H, m), 7.87∼7.97(9H, m), 8,13(1H, m) | 879.14 | 878.39 |
| 1968 | δ = 1.72(12H, s). 7.28(2H, m). 7.38∼7.41(5H, m), 7.51∼7.63(8H, m), 7.64(1H, s), 7.77∼7.79(5H, m), 7.87∼7.98(8H, m), 8.06(1H, m), 8.13(1H, m) | 765.98 | 765.34 |
| 1977 | δ = 1.72(12H, s), 7.28(2H, m), 7.38∼7.39(4H, m), 7.55~7.63(7H, m), 7.77∼7.78(4H, m), 7.87∼7.98(9H, m), 8.06(2H, m), 8.13(1 H, m) | 739.94 | 739.32 |
| 1998 | δ = 0.66(6H, s), 1.72(12H, s), 7.28∼7.39(7H, 7.52∼7.63(8H, m), 7.77∼7.87(12H, m), 8.13(1H, m) | 771.07 | 770.34 |
| 2007 | δ = 0.66(6H, s), 1.72(12H, s), 3.2(3H, s), 6.69∼6.7(2H, m), 6.96(1H, m), 7.21∼7.39(10H, m), 7.55(2H, m), 7.61∼7.63(3H, m), 7.77(2H, m), 7.87∼7.97(7H, m). 8.13(1H, m) | 800.11 | 799.36 |
| 2009 | δ = 1.72(12H, s), 7.22(2H, m), 7.28(2H, m), 7.38∼7.39(4H, m). 7.45∼7.63(11H, m), 7.77(2H, m), 7.87∼7.97(7H, m), 8.13(1H, m), 8.56(1H, m) | 754.96 | 754.33 |
| 2013 | δ = 1.72(12H, s), 7.28(2H, m), 7.38∼7.39(4H, m), 7.47(2H, m), 7.55(4H, m), 7.61∼7.63(4H, m), 7.77(2H, m), 7.85∼7.97(9H, m), 8.04∼8.13(3H, m), 8.42(1H, m), 8.55(1H, m) | 764.99 | 764.34 |
| 2018 | δ = 1.72(12H, s), 7.25∼7.28(6H, m), 7.38∼7.55(13H, m), 7.61∼7.63(3H, m), 7.77(2H, m), 7.85∼7.97(9H, m), 8.13(1H, m) | 791.03 | 791.36 |
| 2020 | δ = 1.72(12H, s), 7.25∼7.28(6H, m), 7.38∼7.39(4H, m), 7.55∼7.63(8H, m), 7.73∼7.77(3H, m), 7.87∼8(10H, m), 8.13(1H, m) | 764.99 | 764.34 |
| 2023 | δ = 1.72(12H, s), 7.28(2H, m), 7.38∼7.39(4H, m), 7.48(2H, m), 7.55∼7.63(9H, m), 7.7∼7.77(4H, m), 7.87∼8(10H, m), 8.13(1H, m) | 764.99 | 764.34 |
| 2028 | δ = 1.72(12H, s), 7.28(2H, m), 7.38∼7.39(4H, m), 7.55∼7.63(10H, m), 7.73∼7.77(4H, m), 7.87∼7.97(9H, m), 8.04∼8.13(3H, m), 8.42(1H, m), 8.55(1H, m) | 815.05 | 814.36 |
| 2030 | δ = 1.72(18H, s), 7.28(3H, m), 7.38∼7.39(5H, m), 7.55(5H, m), 7.61∼7.63(4H, m), 7.77(3H, m), 7.87∼8.01(11H, m), 8.13(1H, m), 8.55(2H, m) | 881.15 | 880.41 |
| 2038 | δ = 1.72(12H, s), 7.28(2H, m), 7.38∼7.39(4H, m), 7.48(2H, m), 7.55∼7.63(6H, m), 7.7(1H, m), 7.77∼7.97(14H, 8.12∼8.13(3H, m), 8.93(2H, m) | 815.05 | 814.36 |
| 2040 | δ = 1.72(12H, s). 7.25∼7.28(6H, m), 7.38∼7.41(9H, m), 7.51∼7.55(6H, m), 7.61∼7.63(3H, m), 7.77(2H, m), 7.87∼7.97(11H, m), 8.13(1H, m) | 891.15 | 890.39 |

[Example 1] Manufacture of an OLED employing organic electroluminescent compound according to the invention

An OLED device was manufactured by using an electroluminescent material according to the invention.

First, a transparent electrode ITO thin film (15 Ω/□) (2) prepared from glass for OLED (produced by Samsung-Corning) (1) was subjected to ultrasonic washing with trichloroethylene, acetone, ethanol and distilled water, sequentially, and stored in isopropanol before use.

Then, an ITO substrate was equipped in a substrate folder of a vacuum vapor-deposit device, and 4,4',4"-tris(N,N-(2-naphthyl)-phenylamino)triphenylamine (2-TNATA) was placed in a cell of the vacuum vapor-deposit device, which was then ventilated up to 10⁻⁶ torr of vacuum in the chamber. Electric current was applied to the cell to evaporate 2-TNATA, thereby providing vapor-deposit of a hole injecting layer (3) having 60 nm of thickness on the ITO substrate.

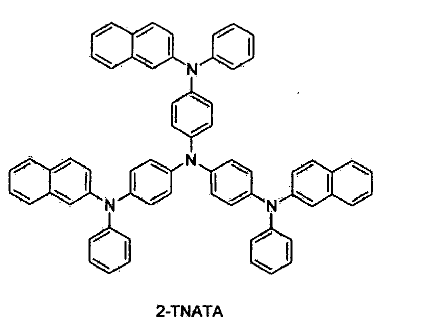

Then, to another cell of the vacuum vapor-deposit device, charged was N,N'-bis(a-naphthyl)-N,N'-diphenyl-4,4'-diamine (NPB), and electric current was applied to the cell to evaporate NPB, thereby providing vapor-deposit of a hole transport layer (4) of 20 nm of thickness on the hole injecting layer.

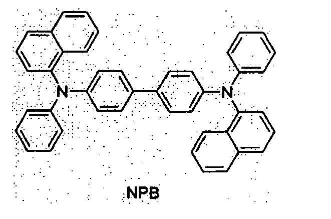

After forming the hole injecting layer and the hole transport layer, an electroluminescent layer was formed according to the following procedure. To one cell of a vacuum vapor-deposit device, charged was a compound according to the present invention (e.g. Compound 3) as host material, while Compound (E) was charged to another cell as dopant. The two materials were evaporated at different rates to carry out doping at 2 to 5 mol% on the basis of the host, to vapor-deposit an electroluminescent layer (5) having 30 nm of thickness on the hole transport layer.

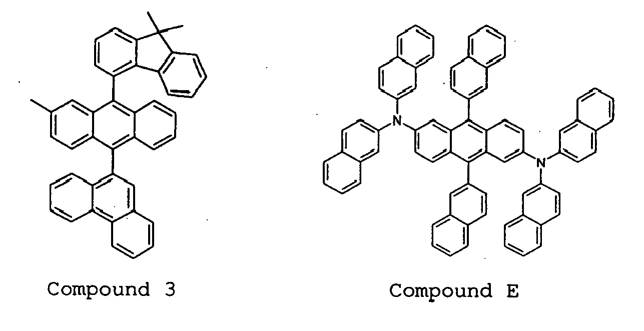

Then, tris(8-hydroxyquinoline)aluminum (III) (Alq) (of which the structure is shown below) was vapor-deposited as an electron transport layer (6) in a thickness of 20 nm, and then lithium quinolate (Liq) was vapor-deposited as an electron injecting layer (7) in a thickness of 1 to 2 nm. Thereafter, an Al cathode (8) was vapor-deposited in a thickness of 150 nm by using another vacuum vapor-deposit device to manufacture an OLED.

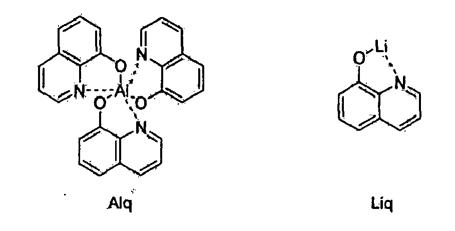

Each compound was employed as electroluminescent material for an OLED after purifying via vacuum sublimation at 10⁻⁶ torr.

### [Comparative Example 1] Manufacture of an OLED by using conventional electroluminescent material

After forming a hole injecting layer and a hole transport layer according to the same procedure described in Example 1, tris(8-hydroxyquinoline)-aluminum (III) (Alq) was charged to another cell of said vacuum vapor-deposit device as electroluminescent host material, and Coumarin 545T (C545T) (of which the structure is shown below) was charged to still another cell. Then the two materials were evaporated at different rates to carry out doping, and thus providing an electroluminescent layer having 30 nm of thickness vapor-deposited on the hole transport layer. The doping concentration was preferably from 1 to 3% by weight on the basis of Alq.

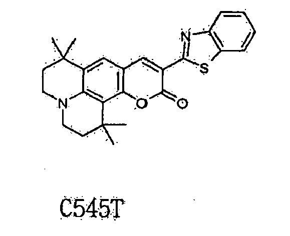

Then, an electron transport layer and electron injecting layer were vapor-deposited according to the same procedure as in Example 1, and an Al cathode was vapor-deposited thereon with a thickness of 150 nm by using another vacuum vapor-deposit device to manufacture an OLED.

### [Example 2] Electroluminescent properties of OLED's manufactured

The luminous efficiencies of the OLED's comprising the organic electroluminescent compound according to the present invention (Examples 1) or conventional EL compound (Comparative Example 1) were measured at 5,000 cd/m², respectively, and the results are shown in Table 2.

Since the electroluminescent properties in high luminance region are very important, particularly in case of green electroluminescent materials, the data at high luminance (about 20,000 cd/m²) are attached in order to reflect the properties.

**Table 2**

| No. | Host | Dopant | Doping concentration (mol%) | Luminous efficiency (cd/A) | Color |
|---|---|---|---|---|---|
| | | | | @5000cd/m* | |
| 1 | 3 | Compound E | 3 | 18.5 | Green |
| 2 | 173 | Compound E | 3 | 18.9 | Green |
| 3 | 285 | Compound E | 3 | 20.3 | Green |
| 4 | 456 | Compound E | 3 | 18.2 | Green |
| 5 | 567 | Compound E | 3 | 19.5 | Green |
| 6 | 765 | Compound E | 3 | 19.7 | Green |
| 7 | 892 | Compound E | 3 | 18.5 | Green |
| 8 | 1058 | Compound E | 3 | 19.6 | Green |
| 9 | 1087 | Compound E | 3 | 20.7 | Green |
| 10 | 1250 | Compound E | 3 | 21.8 | Green |
| 11 | 1381 | Compound E | 3 | 20.1 | Green |
| 12 | 1518 | Compound E | 3 | 18.3 | Green |
| 13 | 1710 | Compound E | 3 | 18.6 | Green |
| 14 | 1767 | Compound E | 3 | 19.2 | Green |
| 15 | 1899 | Compound E | 3 | 18.9 | Green |
| Comp.1 | Alq | Compound C545T | 1 | 10.3 | Green |

As can be seen from Table 2, when the material according to the invention was applied to a green electroluminescent device, the device wherein Compound (E) was doped at 3.0% to Compound (1250) (an organic electroluminescent compound according to the invention) showed more than twice of luminous efficiency as compared to the device employing conventional Alq:C545T (Comparative Example 2).

Accordingly, the organic electroluminescent compounds according to the present invention can be used as green electroluminescent material of high efficiency. Moreover, the device, to which the host material according to the invention was applied, showed noticeable improvement in view of color purity. The improvement in both color purity and luminous efficiency proves that the materials of the present invention have excellent properties.

## Claims

1. An organic electroluminescent compound represented by Chemical Formula (1): 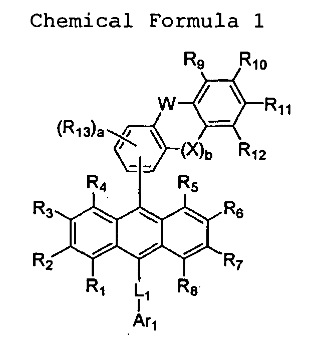
wherein, R₁ through R₈ independently represent hydrogen, halogen, (C1-C60)alkyl, (C6-C60)aryl, (C3-C60)heteroaryl, 5-or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or each of R₁ through R₈ may be linked to an adjacent substituent via (C3-C60)alkylene or (C3-C60)alkenylene to form an alicyclic ring, or a monocyclic or polycyclic aromatic ring; provided that R₁ through R₈ cannot be hydrogen all at the same time;
R₉ through R₁₂ independently represent hydrogen, halogen, (C1-C60)alkyl, (C6-C60)aryl, (C3-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or each of R₉ through R₁₂ may be linked to an adjacent substituent via (C3-C60)alkylene or (C3-C60)alkenylene to form an alicyclic ring, or a monocyclic or polycyclic aromatic ring;
R₁₃ represents halogen, (C1-C60)alkyl, (C6-C60)aryl, (C3-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl;
W and X independently represent a chemical bond, - C(R₁₄) (R₁₅)-, -N(R₁₆)-, -S-, -O-, -Si(R₁₇)(R₁₈)-, -P(R₁₉)-,-C(=O)-, -B(R₂₀)-, -In(R₂₁)-, -Se-, -Ge(R₂₂)(R₂₃)-, -Sn(R₂₄)(R₂₅)-or -Ga(R₂₆)-;
wherein R₁₄ through R₂₆ independently represent hydrogen, halogen, (C1-C60)alkyl, (C6-C60)aryl, (C3-C60)heteroaryl, 5-or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or R₁₄ and R₁₅, R₁₇ and R₁₈, R₂₂ and R₂₃, or R₂₄ and R₂₅ may be linked via (C3-C60)alkylene or (C3-C60)alkenylene with or without a fused ring to form an alicyclic ring, or a monocyclic or polycyclic aromatic ring;
L₁ represents a chemical bond, (C6-C60)arylene or (C3-C60)heteroarylene, and the arylene or heteroarylene of L₁ may be further substituted by one or more substituent(s) selected from (C1-C60)alkyl, halogen, cyano, (C1-C60)alkoxy, (C3-C60)cycloalkyl, (C6-C60)aryl, (C3-C60)heteroaryl, adamantyl, (C7-C60)bicycloalkyl, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro, hydroxyl, tri(C1-C30)alkylsilyl, di(C1-C30)alkyl(C6-C30)arylsilyl and tri(C6-C30)arylsilyl;
Ar₁ represents (C1-C60)alkyl, (C6-C60)aryl, (C3-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or a substituent selected from the following structures: 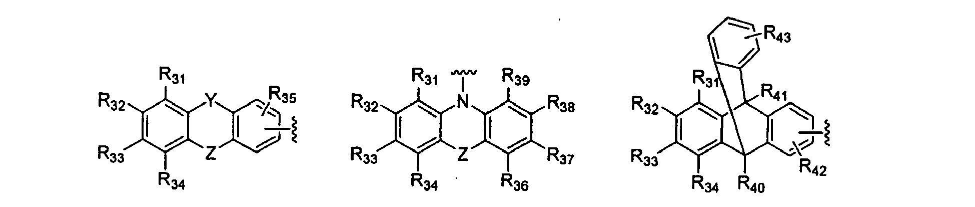
wherein, R₃₁ through R₄₃ independently represent hydrogen, halogen, (C1-C60)alkyl, (C6-C60)aryl, (C3-C60)heteroaryl, 5-or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or each of R₃₁ through R₄₃ may be linked to an adjacent substituent via (C3-C60)alkylene or (C3-C60)alkenylene with or without a fused ring to form an alicyclic ring, or a monocyclic or polycyclic aromatic ring;
Y and Z independently represent a chemical bond, - (CR₅₁R₅₂)_{c}-, -N(R₅₃)-, -S-, -O-, -Si(R₅₄)(R₅₅)-, -P(R₅₆)-, -C(=O)-, -B(R₅₇)-, -In(R₅₈)-, -Se-, -Ge(R₅₉)(R₆₀)-, -Sn(R₆₁)(R₆₂)-, Ga(R6₃)- or -(R₆₄)C=C(R₆₅)-;
wherein R₅₁ through R₆₅ independently represent hydrogen, halogen, (C1-C60)alkyl, (C6-C60)aryl, (C3-C60)heteroaryl, 5-or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or R₅₁ and R₅₂, R₅₄ and R₅₅, R₅₉ and R₆₀, R₆₁ and R₆₂ or R₆₄and R₆₅ may be linked via (C3-C60)alkylene r (C3-C60)alkenylene with or without a fused ring to form an alicyclic ring, or a monocyclic or polycyclic aromatic ring;
the alkyl, aryl, heteroaryl, heterocycloalkyl, cycloalkyl, trialkylsilyl, dialkylarylsilyl, triarylsilyl, adamantyl, bicycloalkyl, alkenyl, alkynyl, alkylamino or arylamino of Ar₁, R₁ through R₂₆, R₃₁ through R₄₃ and R₅₁ through R₆₃ may be further substituted by halogen, (C1-C60)alkyl, (C6-C60)aryl, (C3-C60)heteroaryl, 5- or 6-membered heterocyloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl;
a is an integer from 0 to 3; and
b and c independently represent an integer from 1 to 4.

2. The organic electroluminescent compound according to claim 1, which is selected from the compounds represented by one of Chemical Formulas (2) to (5): 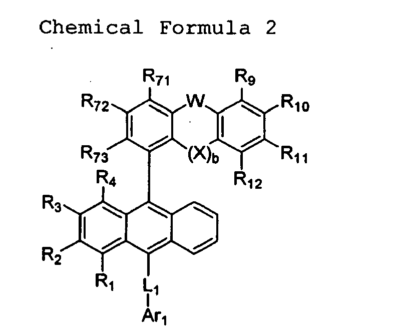 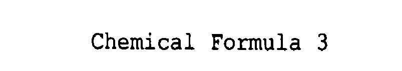 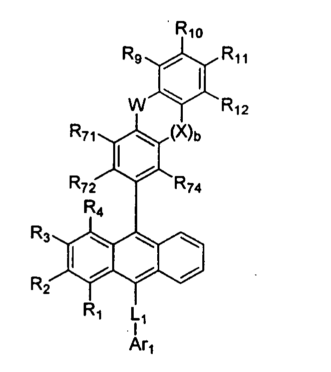 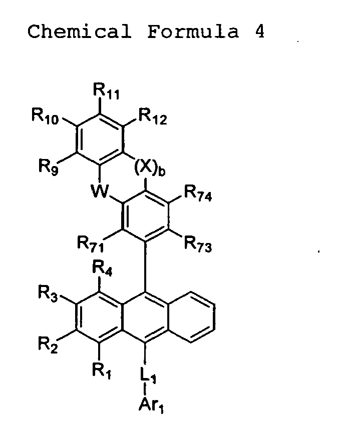 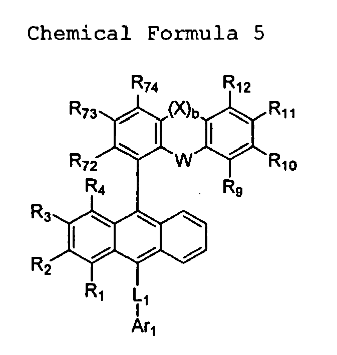
wherein, L₁, Ar₁, R₉ through R₁₂, X, W and b are defined as in Claim 1;
R₁ through R₄ independently represent hydrogen, halogen, (C1-C60)alkyl, (C6-C60)aryl, (C3-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, provided that R₁ through R₄ cannot be hydrogen all at the same time;
R₇₁ through R₇₄ independently represent hydrogen, halogen, (C1-C60)alkyl, (C6-C60)aryl, (C3-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl; and
the alkyl, aryl, heteroaryl, heterocycloalkyl, cycloalkyl, trialkylsilyl, dialkylarylsilyl, triarylsilyl, adamantyl, bicycloalkyl, alkenyl, alkynyl, alkylamino or arylamino of R₁ through R₄, and R₇₁ through R₇₄ may be further substituted by halogen, (C1-C60)alkyl, (C6-C60)aryl, (C3-C60)heteroaryl, 5-or 6-membered heterocyloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl.

3. The organic electroluminescent compound according to claim 2, wherein 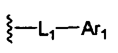 is independently selected from the following structures: 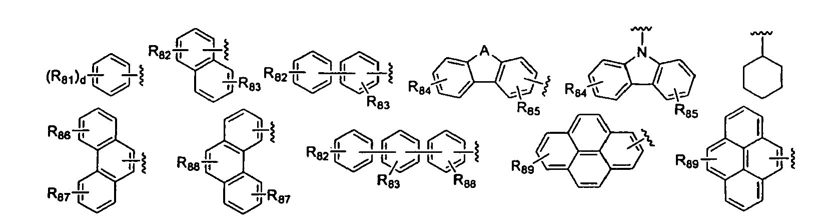 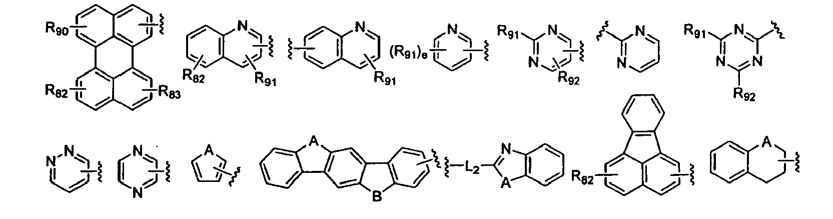 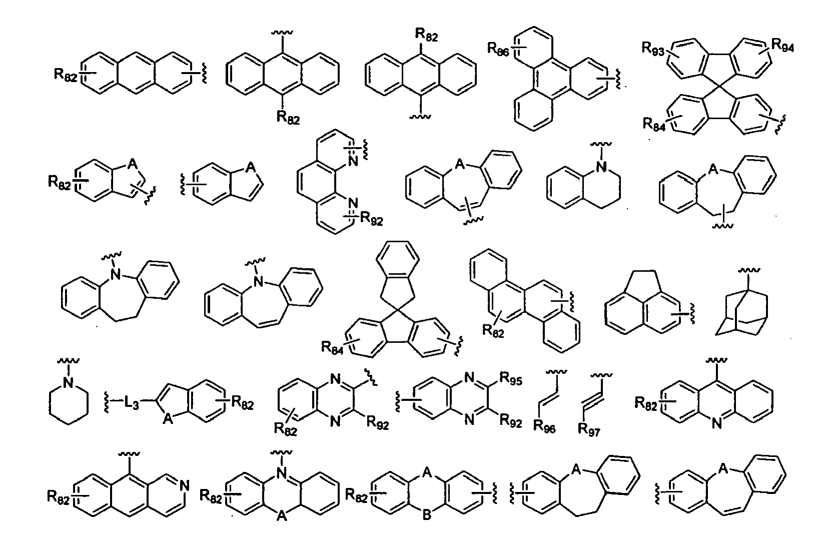
wherein, R₈₁ through R₉₇ independently represent hydrogen, halogen, (C1-C60)alkyl, (C6-C60)aryl, (C3-C60)heteroaryl, 5-or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl; the alkyl, aryl, heteroaryl, heterocycloalkyl, cycloalkyl, trialkylsilyl, dialkylarylsilyl, triarylsilyl, adamantyl, bicycloalkyl, alkenyl, alkynyl, alkylamino or arylamino of R₈₁ through R₉₇ may be further substituted by halogen, (C1-C60)alkyl, (C6-C60)aryl, (C3-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl;
L₂ and L₃ independently represent a chemical bond, (C6-C60)arylene or (C3-C60)heteroarylene; the arylene or heteroarylene of L₂ and L₃ may be further substituted by one or more substituent(s) selected from (C1-C60)alkyl, halogen, cyano, (C1-C60)alkoxy, (C3-C60)cycloalkyl, (C6-C60)aryl, (C3-C60)heteroaryl, adamantyl, (C7-C60)bicycloalkyl, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro, hydroxyl, tri(C1-C30)alkylsilyl, di(C1-C30)alkyl(C6-C30)arylsilyl and tri(C6-C30)arylsilyl;
A and B independently represent a chemical bond, - C(R₁₀₁)(R₁₀₂)-, -N(R₁₀₃)-, -S-, -O-, -Si(R₁₀₄)(R₁₀₅)-, -P(R₁₀₆)-, - C(=O)-, -B(R₁₀₇)-, -In(R₁₀₈)-, -Se-, -Ge(R₁₀₉)(R₁₁₀)-, - Sn(R₁₁₁)(R₁₁₂)- or -Ga(R₁₁₃)-;
wherein R₁₀₁ through R₁₁₃ independently represent hydrogen, halogen, (C1-C60)alkyl, (C6-C60)aryl, (C3-C60)heteroaryl, 5-or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or R₁₀₁ and R₁₀₂, R₁₀₄ and R₁₀₅, R₁₀₉ and R₁₁₀, or R₁₁₁ and R₁₁₂ may be linked via (C3-C60)alkylene or (C3-C60)alkenylene with or without a fused ring to form an alicyclic ring, or a monocyclic or polycyclic aromatic ring;
d is an integer from 1 to 5; and
e is an integer from 1 to 4.

4. An organic electroluminescent device which is comprised of a first electrode; a second electrode; and at least one organic layer(s) interposed between the first electrode and the second electrode; wherein the organic layer comprises an electroluminescent layer comprising an organic electroluminescent compound represented by Chemical Formula (1) : 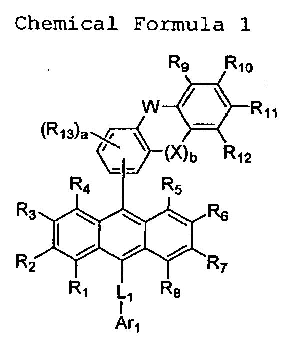
wherein, R₁ through R₈ independently represent hydrogen, halogen, (C1-C60)alkyl, (C6-C60)aryl, (C3-C60)heteroaryl, 5-or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or each of R₁ through R₈ may be linked to an adjacent substituent via (C3-C60)alkylene or (C3-C60)alkenylene to form an alicyclic ring, or a monocyclic or polycyclic aromatic ring; provided that R₁ through R₈ cannot be hydrogen all at the same time;
R₉ through R₁₂ independently represent hydrogen, halogen, (C1-C60)alkyl, (C6-C60)aryl, (C3-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or each of R₉ through R₁₂ may be linked to an adjacent substituent via (C3-C60)alkylene or (C3-C60)alkenylene to form an alicyclic ring, or a monocyclic or polycyclic aromatic ring;
R₁₃ represents halogen, (C1-C60)alkyl, (C6-C60)aryl, (C3-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl;
W and X independently represent a chemical bond, - C(R₁₄)(R₁₅)-, -N(R₁₆)-, -S-, -O-, -Si(R₁₇)(R₁₈)-, -P(R₁₉)-, - C(=O)-, -B(R₂₀)-, -In(R₂₁)-, -Se-, -Ge(R₂₂)(R₂₃)-, -Sn(R₂₄)(R₂₅)-or -Ga(R₂₆)-;
wherein R₁₄ through R₂₆ independently represent hydrogen, halogen, (C1-C60)alkyl, (C6-C60)aryl, (C3-C60)heteroaryl, 5-or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or R₁₄ and R₁₅, R₁₇ and R₁₈, R₂₂ and R₂₃, or R₂₄ and R₂₅ may be linked via (C3-C60)alkylene or (C3-C60)alkenylene with or without a fused ring to form an alicyclic ring, or a monocyclic or polycyclic aromatic ring;
L₁ represents a chemical bond, (C6-C60)arylene or (C3-C60)heteroarylene, and the arylene or heteroarylene of L₁ may be further substituted by one or more substituent(s) selected from (C1-C60)alkyl, halogen, cyano, (C1-C60)alkoxy, (C3-C60)cycloalkyl, (C6-C60)aryl, (C3-C60)heteroaryl, adamantyl, (C7-C60)bicycloalkyl, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro, hydroxyl, tri(C1-C30)alkylsilyl, di(C1-C30)alkyl(C6-C30)arylsilyl and tri(C6-C30)arylsilyl;
Ar₁ represents (C1-C60)alkyl, (C6-C60)aryl, (C3-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or a substituent selected from the following structures: 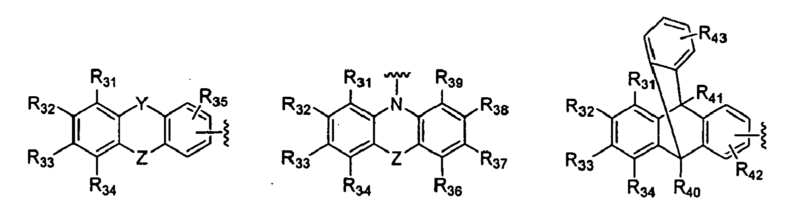
wherein, R₃₁ through R₄₃ independently represent hydrogen, halogen, (C1-C60)alkyl, (C6-C60)aryl, (C3-C60)heteroaryl, 5-or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or each of R₃₁ through R₄₃ may be linked to an adjacent substituent via (C3-C60)alkylene or (C3-C60)alkenylene with or without a fused ring to form an alicyclic ring, or a monocyclic or polycyclic aromatic ring;
Y and Z independently represent a chemical bond, - (CR₅₁R₅₂)_{c}-, -N(R₅₃)-, -S-, -O-, -Si(R₅₄)(R₅₅)-, -P(R₅₆)-, -C(=O)-, -B(R₅₇)-, -In(R₅₈)-, -Se-, -Ge(R₅₉)(R₆₀)-, -Sn(R₆₁)(R₆₂)-, - Ga(R₆₃)- or -(R₆₄)C=C(R₆₅)-;
wherein R₅₁ through R₆₅ independently represent hydrogen, halogen, (C1-C60)alkyl, (C6-C60)aryl, (C3-C60)heteroaryl, 5-or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or R₅₁ and R₅₂, R₅₄ and R₅₅, R₅₉ and R₆₀, R₆₁ and R₆₂ or R₆₄and R₆₅ may be linked via (C3-C60)alkylene r (C3-C60)alkenylene with or without a fused ring to form an alicyclic ring, or a monocyclic or polycyclic aromatic ring;
the alkyl, aryl, heteroaryl, heterocycloalkyl, cycloalkyl, trialkylsilyl, dialkylarylsilyl, triarylsilyl, adamantyl, bicycloalkyl, alkenyl, alkynyl, alkylamino or arylamino of Ar₁, R₁ through R₂₆, R₃₁ through R₄₃ and R₅₁ through R₆₃ may be further substituted by halogen, (C1-C60)alkyl, (C6-C60)aryl, (C3-C60)heteroaryl, 5- or 6-membered heterocyloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl;
a is an integer from 0 to 3; and
b and c independently represent an integer from 1 to 4 and one or more dopant(s) selected from the compounds represented by Chemical Formula (6) or (7): 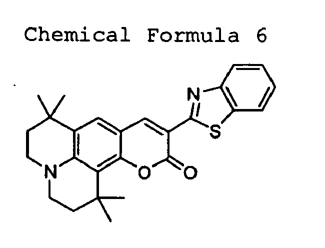 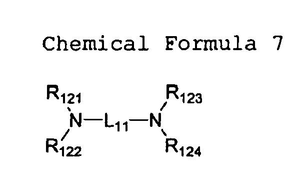
wherein, L₁₁ represents (C6-C60)arylene with or without one or more substituent(s) selected from a group consisting of halogen, (C1-C60)alkyl, (C6-C60)aryl, (C4-C60)heteroaryl, a 5-or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro and hydroxyl; the alkyl, cycloalkyl, heterocycloalkyl, aryl, heteroaryl, arylsilyl, alkylsilyl, alkylamino and arylamino substituent on the arylene may be further substituted by one or more substituent(s) selected from halogen, (C1-C60)alkyl, (C6-C60)aryl, (C4-C60)heteroaryl, a 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro and hydroxyl;
R₁₂₁ through R₁₂₄ independently represent (C1-C60)alkyl, (C6-C60)aryl, (C4-C60)heteroaryl, (C6-C60)arylamino, (C1-C60)alkylamino, 5- or 6-membered heterocycloalkyl containing one or more heteroatom(s) selected from N, O and S, or (C3-C60)cycloalkyl, or each of R₁₂₁ through R₁₂₄ may be linked to an adjacent substituent via (C3-C60)alkylene or (C3-C60)alkenylene with or without a fused ring to form an alicyclic ring, or a monocyclic or polycyclic aromatic ring; and
the alkyl, aryl, heteroaryl, arylamino, alkylamino, cycloalkyl or heterocycloalkyl of R₁₂₁ through R₁₂₄ may be further substituted by one or more substituent(s) selected from halogen, (C1-C60)alkyl, (C6-C60)aryl, (C4-C60)heteroaryl, a 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro and hydroxyl.

5. The organic electroluminescent device according to claim 4, wherein the organic layer comprises one or more compound(s) selected from a group consisting of arylamine compounds and styrylarylamine compounds.

6. The organic electroluminescent device according to claim 4, wherein the organic layer comprises one or more metal(s) selected from a group consisting of organic metals of Group 1, Group 2, 4^{th} period and 5^{th} period transition metals, lanthanide metals and d-transition elements.

7. The organic electroluminescent device according to claim 4, wherein the organic layer comprises a charge generating layer as well as an electroluminescent layer.

8. A white electroluminescent device which comprises an organic electroluminescent compound represented by Chemical Formula (1): 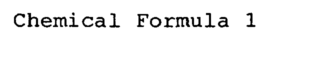 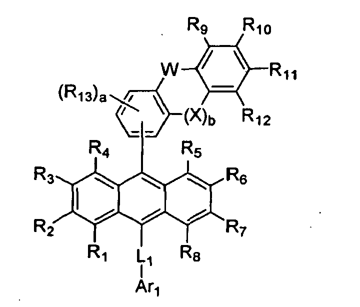
wherein, R₁ through R₈ independently represent hydrogen, halogen, (C1-C60)alkyl, (C6-C60)aryl, (C3-C60)heteroaryl, 5-or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or each of R₁ through R₈ may be linked to an adjacent substituent via (C3-C60)alkylene or (C3-C60)alkenylene to form an alicyclic ring, or a monocyclic or polycyclic aromatic ring; provided that R₁ through R₈ cannot be hydrogen all at the same time;
R₉ through R₁₂ independently represent hydrogen, halogen, (C1-C60)alkyl, (C6-C60)aryl, (C3-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or each of R₉ through R₁₂ may be linked to an adjacent substituent via (C3-C60)alkylene or (C3-C60)alkenylene to form an alicyclic ring, or a monocyclic or polycyclic aromatic ring;
R₁₃ represents halogen, (C1-C60)alkyl, (C6-C60)aryl, (C3-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl;
W and X independently represent a chemical bond, - C(R₁₄)(R₁₅)-, -N(R₁₆)-, -S-, -O-, -Si(R₁₇)(R₁₈)-, -P(R₁₉)-,-C(=O)-, -B(R₂₀)-, -In(R₂₁)-, -Se-, -Ge(R₂₂)(R₂₃)-, -Sn(R₂₄)(R_{2S})-or -Ga(R₂₆)-;
wherein R₁₉ through R₂₆ independently represent hydrogen, halogen, (C1-C60)alkyl, (C6-C60)aryl, (C3-C60)heteroaryl, 5-or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or R₁₄ and R₁₅, R₁₇ and R₁₈, R₂₂ and R₂₃, or R₂₄ and R₂₅ may be linked via (C3-C60) alkylene or (C3-C60)alkenylene with or without a fused ring to form an alicyclic ring, or a monocyclic or polycyclic aromatic ring;
L₁ represents a chemical bond, (C6-C60)arylene or (C3-C60)heteroarylene, and the arylene or heteroarylene of L₁ may be further substituted by one or more substituent(s) selected from (C1-C60)alkyl, halogen, cyano, (C1-C60)alkoxy, (C3-C60)cycloalkyl, (C6-C60)aryl, (C3-C60)heteroaryl, adamantyl, (C7-C60)bicycloalkyl, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro, hydroxyl, tri(C1-C30)alkylsilyl, di(C1-C30)alkyl(C6-C30)arylsilyl and tri(C6-C30)arylsilyl;
Ar₁ represents (C1-C60)alkyl, (C6-C60)aryl, (C3-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or a substituent selected from the following structures: 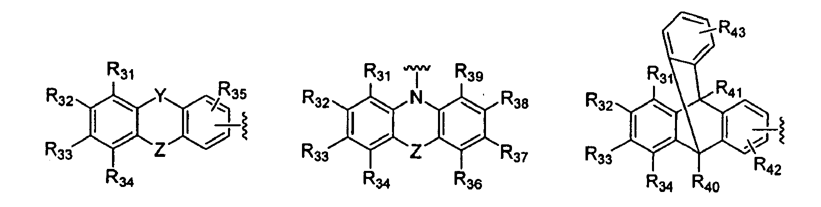
wherein, R₃₁ through R₄₃ independently represent hydrogen, halogen, (C1-C60)alkyl, (C6-C60)aryl, (C3-C60)heteroaryl, 5-or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or each of R₃₁ through R₄₃ may be linked to an adjacent substituent via (C3-C60)alkylene or (C3-C60)alkenylene with or without a fused ring to form an alicyclic ring, or a monocyclic or polycyclic aromatic ring;
Y and Z independently represent a chemical bond, - (CR₅₁R₅₂)_{c}-, -N(R₅₃)-, -S-, -O-, -Si(R₅₄)(R₅₅)-, -P(R₅₆)-, -C(=O)-, ₋B(R₅₇)-, -In(R₅₈)-, -Se-, -Ge(R₅₉)(R₆₀)-, ₋Sn(R₆₁)(R₆₂)-, - Ga(R₆₃)- or -(R₆₄)C=C(R₆₅)-;
wherein R₅₁ through R₆₅ independently represent hydrogen, halogen, (C1-C60)alkyl, (C6-C60)aryl, (C3-C60)heteroaryl, 5-or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or R₅₁, and R₅₂, R₅₄ and R₅₅, R₅₉ and R₆₀, R₆₁ and R₆₂ or R₆₄and R₆₅ may be linked via (C3-C60)alkylene r (C3-C60)alkenylene with or without a fused ring to form an alicyclic ring, or a monocyclic or polycyclic aromatic ring;
the alkyl, aryl, heteroaryl, heterocycloalkyl, cycloalkyl, trialkylsilyl, dialkylarylsilyl, triarylsilyl, adamantyl, bicycloalkyl, alkenyl, alkynyl, alkylamino or arylamino of Ar₁, R₁ through R₂₆, R₃₁ through R₄₃ and R₅₁, through R₆₃ may be further substituted by halogen, (C1-C60)alkyl, (C6-C60)aryl, (C3-C60)heteroaryl, 5- or 6-membered heterocyloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl;
a is an integer from 0 to 3; and
b and c independently represent an integer from 1 to 4.

9. The organic solar cell which comprises an organic electroluminescent compound represented by Chemical Formula (1) : 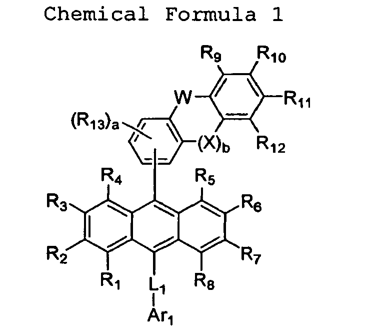
wherein, R₁ through R₈ independently represent hydrogen, halogen, (C1-C60)alkyl, (C6-C60)aryl, (C3-C60)heteroaryl, 5-or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or each of R₁ through R₈ may be linked to an adjacent substituent via (C3-C60)alkylene or (C3-C60)alkenylene to form an alicyclic ring, or a monocyclic or polycyclic aromatic ring; provided that R₁ through R₈ cannot be hydrogen all at the same time;
R₉ through R₁₂ independently represent hydrogen, halogen, (C1-C60)alkyl, (C6-C60)aryl, (C3-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or each of R₉ through R₁₂ may be linked to an adjacent substituent via (C3-C60)alkylene or (C3-C60)alkenylene to form an alicyclic ring, or a monocyclic or polycyclic aromatic ring;
R₁₃ represents halogen, (C1-C60)alkyl, (C6-C60)aryl, (C3-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl;
W and X independently represent a chemical bond, - C(R₁₄)(R₁₅)-, -N(R₁₆)-, -S-, -O-, -Si(R₁₇)(R₁₈)-, -P(R₁₉)-, - C(=O)-, -B(R₂₀)-, -In(R₂₁)-, -Se-, -Ge(R₂₂)(R₂₃)-, -Sn(R₂₄)(R₂₅)-or -Ga(R₂₆)-;
wherein R₁₄ through R₂₆ independently represent hydrogen, halogen, (C1-C60)alkyl, (C6-C60)aryl, (C3-C60)heteroaryl, 5-or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or R₁₄ and R₁₅, R₁₇ and R₁₈, R₂₂ and R₂₃, or R₂₄ and R₂₅ may be linked via (C3-C60)alkylene or (C3-C60)alkenylene with or without a fused ring to form an alicyclic ring, or a monocyclic or polycyclic aromatic ring;
L₁ represents a chemical bond, (C6-C60)arylene or (C3-C60)heteroarylene, and the arylene or heteroarylene of L₁ may be further substituted by one or more substituent(s) selected from (C1-C60)alkyl, halogen, cyano, (C1-C60)alkoxy, (C3-C60)cycloalkyl, (C6-C60)aryl, (C3-C60)heteroaryl, adamantyl, (C7-C60)bicycloalkyl, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro, hydroxyl, tri(C1-C30)alkylsilyl, di(C1-C30)alkyl(C6-C30)arylsilyl and tri(C6-C30)arylsilyl;
Ar₁ represents (C1-C60)alkyl, (C6-C60)aryl, (C3-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or a substituent selected from the following structures: 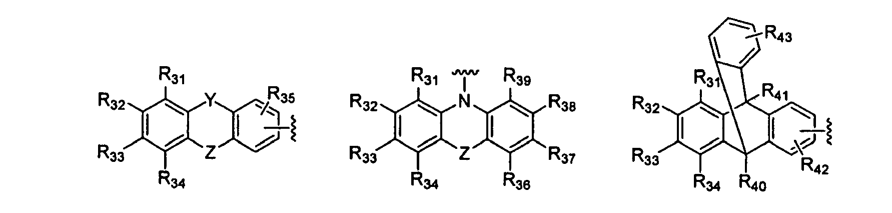
wherein, R₃₁ through R₄₃ independently represent hydrogen, halogen, (C1-C60)alkyl, (C6-C60)aryl, (C3-C60)heteroaryl, 5-or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or each of R₃₁ through R₄₃ may be linked to an adjacent substituent via (C3-C60)alkylene or (C3-C60)alkenylene with or without a fused ring to form an alicyclic ring, or a monocyclic or polycyclic aromatic ring;
Y and Z independently represent a chemical bond, - (CR₅₁R₅₂)c-, -N(R₅₃)-, -S-, -O-, -Si(R₅₄)(R₅₅)-, -P(R₅₆)-, -C(=O)-, -B(R₅₇)-, -In(R₅₈)-, -Se-, -Ge(R₅₉)(R₆₀)-, -Sn(R₆₁)(R₆₂)-, - Ga(R₆₃)- or -(R₆₄)C=C(R₆₅)-;
wherein R₅₁ through R₆₅ independently represent hydrogen, halogen, (C1-C60)alkyl, (C6-C60)aryl, (C3-C60)heteroaryl, 5-or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or R₅₁ and R₅₂, R₅₄ and R₅₅, R₅₉ and R₆₀, R₆₁ and R₆₂ or R₆₄and R₆₅ may be linked via (C3-C60) alkylene r (C3-C60)alkenylene with or without a fused ring to form an alicyclic ring, or a monocyclic or polycyclic aromatic ring;
the alkyl, aryl, heteroaryl, heterocycloalkyl, cycloalkyl, trialkylsilyl, dialkylarylsilyl, triarylsilyl, adamantyl, bicycloalkyl, alkenyl, alkynyl, alkylamino or arylamino of Ar₁, R₁ through R₂₆, R₃₁ through R₄₃ and R₅₁ through R₆₃ may be further substituted by halogen, (C1-C60)alkyl, (C6-C60)aryl, (C3-C60)heteroaryl, 5- or 6-membered heterocyloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl;
a is an integer from 0 to 3; and
b and c independently represent an integer from 1 to 4.
